# EUROPEAN PATENT APPLICATION

(11) **EP 2 058 309 A1**
(43) Date of publication of application: **13.05.2009**
(21) Application number: 07791901.7
(22) Date of filing: 03.08.2007
(51) Int. Cl.: C07D 471/04, A61K 31/437, A61K 31/444, A61K 31/496, A61K 31/5025, A61K 31/506, A61K 31/5377, A61K 31/541, A61P 3/02, A61P 3/10, A61P 5/00, A61P 7/02, A61P 9/00, A61P 9/10, A61P 17/00, A61P 19/00, A61P 19/10, A61P 25/00, A61P 25/28, A61P 25/30, A61P 29/00

(54) **FUSED HETEROCYCLIC COMPOUND**

(30) Priority: 04.08.2006 JP 2006213960
(71) Applicant: Takeda Pharmaceutical Company Limited, Osaka- shi, Osaka 541-0045 (JP)
(72) Inventor: UCHIKAWA, Osamu, Osaka-shi Osaka 532-8686 (JP); SAKAI, Nozomu, Osaka-shi Osaka 532-8686 (JP); TERAO, Yoshito, Osaka-shi Osaka 532-8686 (JP); SUZUKI, Hideo, Osaka-shi Osaka 532-8686 (JP)
(74) Representative: Jones, Nicholas Andrew
(86) International application number: PCT/JP2007/065227
(87) International publication number: WO 2008/016131

(57) **Abstract**

The present invention provides a compound represented by the formula (I): 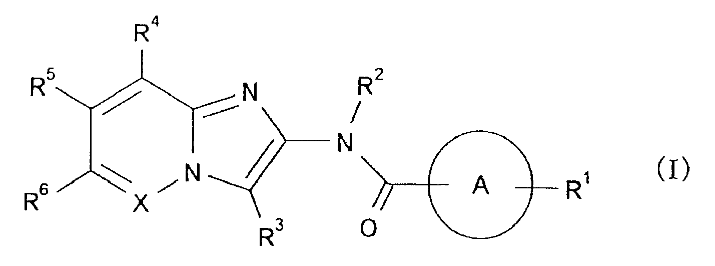 wherein
ring A is a ring which is optionally further substituted;
R¹ is a hydrogen atom or a substituent;
R² is a hydrogen atom or a substituent;
R³ is a hydrogen atom or a substituent;
R⁴ is a hydrogen atom or a substituent;
R⁵ is a hydrogen atom or a substituent;
R⁶ is a hydrogen atom or a substituent;
X is =N- or =C(Z)- (Z is a hydrogen atom or a substituent);
when X is =C(Z)-, Z and R⁶ are optionally bonded to each other to form, together with the carbon atom bonded thereto, an optionally substituted ring,
provided that when X is =CH-, then R⁶ is not optionally substituted 2-piperidinyl, excluding N-imidazo[1,2-a]pyridin-2-yl-4-methyl-benzamide, N-imidazo[1,2-a]pyridin-2-yl-benzamide and N-(7-methylimidazo[1,2-a]pyridin-2-yl)-benzamide, or a salt thereof, and a pharmaceutical agent containing same. The compound of the present invention has an ASK1 inhibitory action, and is useful as a pharmaceutical agent such as an agent for the prophylaxis or treatment of diabetes, inflammatory diseases and the like, and the like.

[Sequence Listing]

SEQUENCE LISTING

<110>  Takeda Pharmaceutical Company Limited

<120>  FUSED HETEROCYCLIC COMPOUND

<130>  091093

<150>  JP 2006-213960
<151>  2006-08-04

<160>  2

<170>  PatentIn version 3.3

<210>  1
<211>  65
<212>  DNA
<213>  Artificial

<220>
<223>  PCR primer

<400>  1
aaaagtcgac atggactaca aggacgacga tgacaaggtg aacaccatta ccgaagagaa     60

gggga                                                                 65


<210>  2
<211>  39
<212>  DNA
<213>  Artificial

<220>
<223>  PCR primer

<400>  2
aaagcggccg ctcaagtctg tttgtttcga aagtcaatg                            39

## Description

### Technical Field

The present invention relates to an imidazopyridine compound and an imidazopyridazine compound having an apoptosis signal regulating kinase 1 (hereinafter sometimes to be abbreviated as "ASK1") inhibitory action, and are useful as therapeutic agents for diabetes, inflammatory disease and the like.

### Background of the Invention

Mitogen-activated protein (MAP) kinase cascade is a signal transduction mechanism wherein MAP kinase kinase kinase (MAPKKK) activated by physicochemical stress or inflammatory cytokines such as tumor necrosis factor-α (TNF-α), interleukin-1 (IL-1) and the like sequentially activates MAP kinase kinase (MAPKK) and MAP kinase (MAPK). Responsive to such stimulations, the cells show phenotypes of survival, growth, differentiation, death (apoptosis) and the like. c-Jun N-terminal kinase (JNK) and p38 MAP kinase (p38) are known MAPKs that are responsible for a part of the signal transduction pathway that induces apoptosis (see, for example, Science, 270: 1326 (1995)).

JNK and p38 are each activated by MKK4/MKK7 and MKK3/MKK6 which are MAPKKs. These MAPKKs are activated by MAPKKK called apoptosis signal regulating kinase 1 (ASK1) (see, for example, Science, 275: 90-94 (1997)). MAPKKK has been reported to include many besides ASK1. ASK1 characteristically has an ability to induce apoptosis in cells through signal transduction via activation of JNK and/or p38. In recent years, activation of ASK1 is suggested to be also involved in the differentiation of keratinocytes and cell differentiation such as neurite elongation and the like of PC12 cells. Thus, it has been clarified that ASK1 plays a key role not only in apoptosis but also control of the fate of cells.

As mentioned above, since ASK1 is an important molecule that controls the subsequent fate of cells, it is considered that various factors are involved its activation/inactivation to place it under complicated control. For example, protein phosphatase 5 (PP5) is considered to directly bounds to ASK1 under H₂O₂ stimulation, and sets activated ASK1 back in an inactivated state by dephosphorylation of threonine (see, for example, EMBO Journal, 20: 6028-6036 (2001)). In addition, it has also been reported that thioredoxin, which is a redox regulator, is constitutively bound to the N-terminal domain of ASK1 and acts as an ASK1 activation inhibitor when an oxidative stress is absent; when an oxidative stress is applied, it is released from ASK1 to cause activation of ASK1 (see, for example, EMBO Journal, 17: 2596-2606 (1998)); 14-3-3 protein inhibits activation of ASK1 by binding to the C-terminal domain thereof (see, for example, Proceedings of National Academy of Sciences, USA, 96: 8511-8515 (1999)); and the like.

On the other hand, it has been suggested that ASK1 is closely related to the induction of apoptosis by endoplasmic reticulum stress and the production of cytokine chemokine, since a treatment of ASK1 knockout (KO) mouse cell with an endoplasmic reticulum stress inducing agent results in significant suppression of apoptosis as compared to wild-type mouse cell and stimulation of KO mouse cell with LPS results in significant suppression of cytokine chemokine production as compared to wild-type mouse cell, and therefore, ASK1 inhibitory substances such as the above-mentioned thioredoxin, 14-3-3 protein and the like, ASK1 dominant-negative mutant, ASK1 antisense oligonucleotide and the like are effective for the prophylaxis or treatment of endoplasmic reticulum stress associated diseases such as neurodegenerative diseases (e.g., polyglutamine disease etc.) and the like, as well as immune diseases (see, for example, WO02/38179 and WO03/00826).

It has been reported that, in a pathology model study using ASK1 knockout mouse, a diabetes model prepared by administration of streptozocin to ASK1 knockout mouse shows suppressed progression of neuropathy as compared to wild-type mouse treated in the same manner, ASK1 knockout mouse on a high-fat diet shows a significantly suppressed increase in the blood glucose level as compared to wild-type mouse, diabetic nephropathy model (db/db) mouse shows an increased ASK1 expression level in the kidney as compared to normal mouse, chronic obliterative pulmonary disease (COPD) model prepared by exposing ASK1 knockout mouse to cigarette smoke shows suppressed inflammatory changes in the lung as compared to wild-type mouse treated in the same manner, and exposure of wild-type mouse to cigarette smoke increases the expression level of activated ASK1 in the lung (see, for example, WO2005/103288). These results demonstrate that ASK1 is deeply involved in the onset and progression of diabetes and complications thereof as well as COPD, and inhibition of the expression or activity of ASK1 is effective for the prophylaxis or treatment of diabetes and complications thereof as well as inflammatory diseases such as COPD.

On the other hand, as imidazopyridine compounds, non-patent reference 1 describes compounds represented by the following formulas (A), (B) and (C):

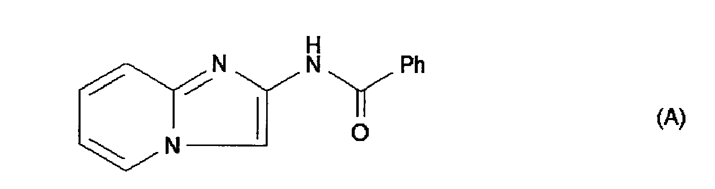

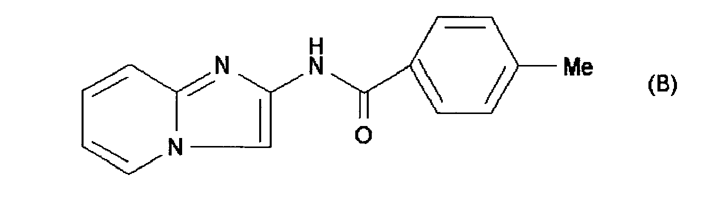

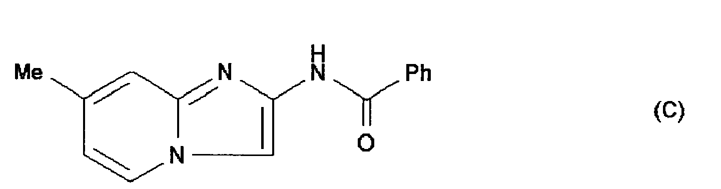

wherein Me is methyl and Ph is phenyl;
patent reference 1 describes a compound represented by the following formula:

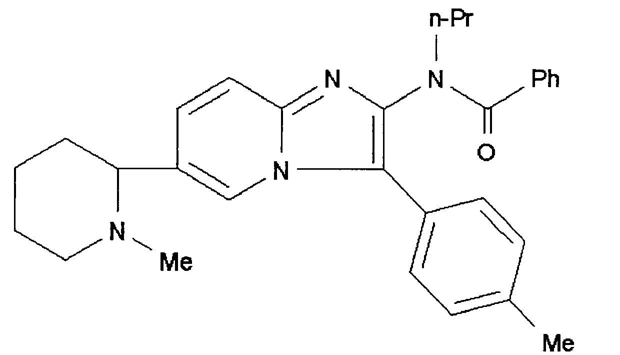

wherein Me is methyl, nPr is n-propyl and Ph is phenyl.
In addition, patent reference 2 describes that a compound represented by the following formula:

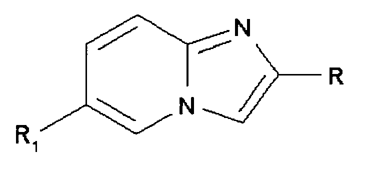

wherein R is 5- or 6-membered monocyclic aromatic heterocycle and the like, or the formula:

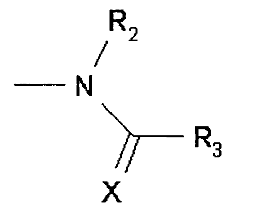

wherein X is O or S; R₂ is a hydrogen atom or lower alkyl; R₃ is a hydrogen atom, C₁₋₈ alkyl, cycloalkyl, aryl, aralkyl, heteroaryl, amino monosubstituted by lower alkyl, amino disubstituted by lower alkyl, cycloalkylamino, lower alkoxy, lower alkylthio, aryloxy, arylthio, heteroaryloxy or heteroarylthio; and
R₁ is a hydrogen atom, lower alkyl, lower alkoxy, lower alkylthio, phenyl, naphthyl, phenoxy, naphthyloxy, nitro or amino, or the formula:

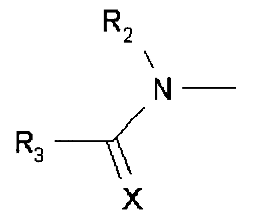

wherein X, R₂ and R₃ are as defined above, is useful as an anthelmintic or fungicide.
However, the above-mentioned references do not disclose that the imidazopyridine derivatives have an ASK1 inhibitory action.
patent reference 1: WO2004/103991
patent reference 2: US Patent No. 3701780
non-patent reference 1: Dopovidi Natsional'noi Akademii Nauk Ukraini (2005), (4), 128-133

### Disclosure of the Invention

### Problems to be Solved by the Invention

The present invention aims to provide a compound having an ASK1 inhibitory action, and useful as a pharmaceutical agent, for example, an agent for the prophylaxis or treatment of diabetes, inflammatory diseases and the like, and the like.

### Means of Solving the Problems

The present inventors have conducted various intensive studies and found that a compound represented by the following formula (I) unexpectedly has a superior ASK1 inhibitory action and superior properties as a pharmaceutical product such as stability and the like, and can be a safe and useful pharmaceutical agent. Based on these findings, they have completed the present invention.

Accordingly, the present invention relates to:
[1] a compound represented by the formula (I):

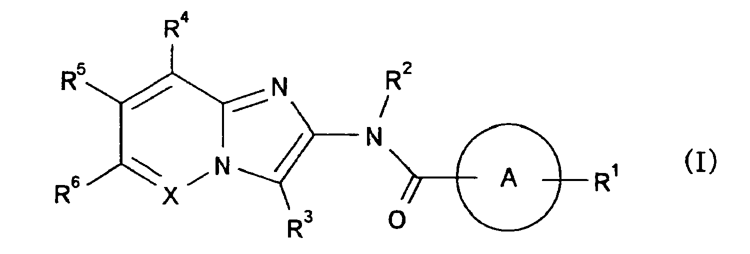

wherein
ring A is a ring which is optionally further substituted;
R¹ is a hydrogen atom or a substituent;
R² is a hydrogen atom or a substituent;
R³ is a hydrogen atom or a substituent;
R⁴ is a hydrogen atom or a substituent;
R⁵ is a hydrogen atom or a substituent;
R⁶ is a hydrogen atom or a substituent;
X is =N- or =C(Z)- (Z is a hydrogen atom or a substituent); when X is =C(Z)-, Z and R⁶ are optionally bonded to each other to form, together with the carbon atom bonded thereto, an optionally substituted ring,
provided that when X is =CH-, then R⁶ is not optionally substituted 2-piperidinyl, excluding N-imidazo[1,2-a]pyridin-2-yl-4-methyl-benzamide, N-imidazo[1,2-a]pyridin-2-yl-benzamide and N-(7-methylimidazo[1,2-a]pyridin-2-yl)-benzamide, or a salt thereof (hereinafter sometimes to be abbreviated as "compound (I)");
[2] the compound of the above-mentioned [1], wherein X is =C(Z)- (Z is a hydrogen atom or a substituent);
[3] the compound of the above-mentioned [1], wherein X is =N-, and ring A is an aromatic hydrocarbon optionally further having substituent(s) or a 5-membered heterocycle optionally having substituent(s);
[4] the compound of the above-mentioned [2] or [3], wherein R¹ is
   (1) a hydrogen atom,
   (2) C₁₋₆ alkyl optionally substituted by 1 to 3 substituents selected from
      (a) an aromatic heterocyclic group,
      (b) amino optionally mono- or di-substituted by substituent(s) selected from
         (i) C₁₋₆ alkyl,
         (ii) C₁₋₆ alkyl-carbonyl optionally substituted by 1 to 3 substituents selected from hydroxy and an aromatic heterocyclic group,
         (iii) C₆₋₁₄ aryl-carbonyl,
         (iv) C₁₋₆ alkyl-sulfonyl, and
         (v) aromatic heterocyclyl-carbonyl,
      (c) cyano,
      (d) carboxyl,
      (e) C₁₋₆ alkoxy-carbonyl,
      (f) N-hydroxycarbamoyl,
      (g) carbamoyl optionally mono- or di-substituted by C₁₋₆ alkyl optionally substituted by 1 to 3 substituents selected from (i) cyano, (ii) hydroxy, (iii) an aromatic heterocyclic group, and (iv) a non-aromatic heterocyclic group optionally having 1 to 3 C₁₋₆ alkyl,
      (h) a halogen atom,
      (i) di-C₁₋₆ alkylphosphoryl,
      (j) hydroxy, and
      (k) a non-aromatic heterocyclic group;
   (3) C₃₋₁₀ cycloalkyl optionally substituted by 1 to 3 substituents selected from
      (a) C₁₋₆ alkoxy-carbonyl, and
      (b) carboxyl;
   (4) an aromatic heterocyclic group;
   (5) hydroxy optionally substituted by C₁₋₆ alkyl;
   (6) amino optionally mono- or di-substituted by substituent(s) selected from
      (a) C₁₋₆ alkyl, and
      (b) C₁₋₆ alkyl-sulfonyl;
   (7) cyano;
   (8) carboxyl;
   (9) C₁₋₆ alkoxy-carbonyl;
   (10) C₁₋₆ alkyl-sulfonyl;
   (11) sulfamoyl optionally mono- or di-substituted by substituent(s) selected from
      (a) C₁₋₆ alkyl, and
      (b) C₁₋₆ alkyl-carbonyl; or
   (12) a halogen atom;
[5] the compound of the above-mentioned [2] or [3], wherein R² is a hydrogen atom;
[6] the compound of the above-mentioned [2] or [3], wherein R³ is a hydrogen atom, C₁₋₆ alkyl or a halogen atom;
[7] the compound of the above-mentioned [2] or [3], wherein R⁴ is a hydrogen atom;
[8] the compound of the above-mentioned [2] or [3], wherein R⁵ is a hydrogen atom;
[9] the compound of the above-mentioned [2] or [3], wherein R⁶ is
   (1) a hydrogen atom;
   (2) C₁₋₆ alkyl optionally substituted by 1 to 3 substituents selected from
      (a) hydroxy,
      (b) amino optionally mono- or di-substituted by C₁₋₆ alkyl, and
      (c) a halogen atom;
   (3) C₆₋₁₄ aryl optionally substituted by 1 to 3 substituents selected from
      (a) C₁₋₆ alkyl optionally substituted by 1 to 3 halogen atoms,
      (b) C₁₋₆ alkoxy optionally substituted by 1 to 3 halogen atoms,
      (c) C₁₋₆ alkoxy-carbonyl,
      (d) amino optionally mono- or di-substituted by substituent(s) selected from
         (i) C₁₋₆ alkyl, and
         (ii) C₁₋₆ alkyl-carbonyl,
      (e) C₁₋₆ alkyl-sulfonyl,
      (f) cyano, and
      (g) C₁₋₃ alkylenedioxy;
   (4) an aromatic heterocyclic group optionally substituted by 1 to 3 substituents selected from
      (a) C₁₋₆ alkyl optionally substituted by 1 to 3 substituents selected from
         (i) hydroxy optionally substituted by a non-aromatic heterocyclic group, and
         (ii) cyano,
      (b) C₆₋₁₄ aryl,
      (c) an aromatic heterocyclic group,
      (d) C₁₋₆ alkoxy, and
      (e) a halogen atom;
   (5) a non-aromatic heterocyclic group optionally substituted by 1 to 3 substituents selected from
      (a) C₁₋₆ alkyl optionally substituted by 1 to 3 substituents selected from
         (i) C₆₋₁₄ aryl,
         (ii) an aromatic heterocyclic group, and
         (iii) hydroxy optionally substituted by C₁₋₆ alkyl,
      (b) C₆₋₁₄ aryl optionally substituted by 1 to 3 substituents selected from C₁₋₆ alkoxy and a halogen atom,
      (c) an aromatic heterocyclic group,
      (d) hydroxy,
      (e) amino optionally mono- or di-substituted by C₁₋₆ alkyl-carbonyl,
      (f) carboxyl,
      (g) carbamoyl optionally mono- or di-substituted by C₇₋₁₃ aralkyl,
      (h) C₁₋₆ alkyl-sulfonyl, and
      (i) C₆₋₁₄ aryl-sulfonyl;
   (6) hydroxy optionally substituted by a substituent selected from
      (a) C₆₋₁₄ aryl optionally substituted by 1 to 3 substituents selected from
         (i) C₁₋₆ alkyl optionally substituted by 1 to 3 substituents selected from
            (A) a halogen atom,
            (B) amino optionally mono- or di-substituted by C₁₋₆ alkyl, and
            (C) an aromatic heterocyclic group optionally substituted by 1 to 3 C₁₋₆ alkyl optionally substituted by 1 to 3 hydroxy,
         (ii) an aromatic heterocyclic group optionally substituted by 1 to 3 C₁₋₆ alkyl,
         (iii) a non-aromatic heterocyclic group,
         (iv) C₁₋₆ alkoxy optionally substituted by 1 to 3 substituents selected from (A) a halogen atom and (B) carboxyl,
         (v) amino optionally mono- or di-substituted by substituent(s) selected from
            (A) C₁₋₆ alkyl,
            (B) C₁₋₆ alkyl-carbonyl,
            (C) C₃₋₁₀ cycloalkyl-carbonyl,
            (D) C₆₋₁₄ aryl-carbonyl optionally substituted by 1 to 3 substituents selected from (1') C₁₋₆ alkyl optionally substituted by 1 to 3 halogen atoms, (2') a halogen atom, and, (3') C₃₋₁₀ cycloalkyl optionally substituted by 1 to 3 cyano, and
            (E) aromatic heterocyclyl-carbonyl optionally substituted by 1 to 3 C₁₋₆ alkyl,
         (vi) carboxyl, and
         (vii) non-aromatic heterocyclyl-carbonyl optionally substituted by 1 to 3 substituents selected from C₁₋₆ alkyl and oxo,
      (b) an aromatic heterocyclic group optionally substituted by 1 to 3 substituents selected from
         (i) C₁₋₆ alkyl,
         (ii) C₁₋₆ alkyl-carbonyl, and
         (iii) carboxyl, and
      (c) a non-aromatic heterocyclic group optionally substituted by 1 to 3 substituents selected from C₁₋₆ alkyl and oxo;
   (7) mercapto optionally substituted by
      (a) an aromatic heterocyclic group optionally substituted by 1 to 3 substituents selected from
         (i) C₁₋₆ alkyl optionally substituted by 1 to 3 halogen atoms,
         (ii) C₆₋₁₄ aryl, and
         (iii) an aromatic heterocyclic group;
   (8) amino optionally mono- or di-substituted by substituent(s) selected from
      (a) C₁₋₆ alkyl optionally substituted by 1 to 3 substituents selected from
         (i) an aromatic heterocyclic group,
         (ii) hydroxy,
         (iii) C₁₋₆ alkoxy, and
         (iv) amino optionally mono- or di-substituted by C₁₋₆ alkyl,
      (b) C₃₋₁₀ cycloalkyl,
      (c) C₇₋₁₃ aralkyl, and
      (d) a non-aromatic heterocyclic group optionally substituted by 1 to 3 C₁₋₆ alkyl;
   (9) cyano;
   (10) C₁₋₆ alkyl-carbonyl optionally substituted by 1 to 3 hydroxy;
   (11) a halogen atom; or
   (12) hydroxy substituted by C₆₋₁₄ aryl fused with C₃₋₁₀ cycloalkane optionally substituted by oxo;
[10] the compound of the above-mentioned [2], wherein Z is a hydrogen atom, or C₁₋₆ alkyl optionally substituted by 1 to 3 hydroxy, or Z and R⁶ are bonded to each other to form, together with the carbon atom bonded thereto, an aromatic hydrocarbon or a heterocycle, each optionally substituted by 1 to 3 C₁₋₆ alkyl;
[11] the following compound or a salt thereof:
   4-tert-butyl-N-(1,2-dihydrofuro[2,3-e]imidazo[1,2-a]pyridin-7-yl)benzamide,
   4-tert-butyl-N-[6-(1H-imidazol-1-yl)imidazo[1,2-a]pyridin-2-yl]benzamide,
   6-tert-butyl-N-[6-(1H-imidazol-1-yl)imidazo[1,2-a]pyridin-2-yl]nicotinamide,
   4-(2-hydroxy-1,1-dimethylethyl)-N-[6-(1H-imidazol-1-yl)imidazo[1,2-a]pyridin-2-yl]benzamide,
   4-tert-butyl-N-[6-(1H-pyrazol-4-yl)imidazo[1,2-a]pyridin-2-yl]benzamide,
   4-(1-cyano-1-methylethyl)-N-[6-(1H-imidazol-1-yl)imidazo[1,2-a]pyridin-2-yl]benzamide,
   4-tert-butyl-N-[6-(1H-imidazol-1-yl)imidazo[1,2-b]pyridazin-2-yl]benzamide;
[12] a prodrug of the compound of the above-mentioned [1];
[13] a pharmaceutical agent comprising the compound of the above-mentioned [1], or a prodrug thereof;
[14] the pharmaceutical agent of the above-mentioned [13], which is an ASK1 inhibitor;
[15] the pharmaceutical agent of the above-mentioned [13], which is an agent for the prophylaxis or treatment of diabetes;
[16] the pharmaceutical agent of the above-mentioned [13], which is an agent for the prophylaxis or treatment of an inflammatory disease;
[17] a method of preventing or treating diabetes and/or an inflammatory disease, comprising administering an effective amount of the compound of the above-mentioned [1] or a prodrug thereof to a mammal;
[18] use of the compound of the above-mentioned [1] or a prodrug thereof for the production of an agent for the prophylaxis or treatment of diabetes and/or an inflammatory disease; and the like.

### Effect of the Invention

The compound of the present invention has a superior ASK1 inhibitory activity, and is useful as a pharmaceutical agent, for example, an agent for the prophylaxis or treatment of diabetes, an inflammatory disease and the like, and the like.

### [Detailed Description of the Invention]

In the present specification, unless otherwise specified, the "halogen atom" means a fluorine atom, a chlorine atom, a bromine atom or an iodine atom.
In the present specification, unless otherwise specified, the "C₁₋₃ alkylenedioxy" means methylenedioxy, ethylenedioxy and the like.
In the present specification, unless otherwise specified, the "C₁₋₆ alkyl" means methyl, ethyl, propyl, isopropyl, butyl, isobutyl, sec-butyl, tert-butyl, pentyl, isopentyl, neopentyl, tert-pentyl, 1-ethylpropyl, hexyl, isohexyl, 1,1-dimethylbutyl, 2,2-dimethylbutyl, 3,3-dimethylbutyl, 2-ethylbutyl and the like.
In the present specification, unless otherwise specified, the "C₁₋₆ alkoxy" means methoxy, ethoxy, propoxy, isopropoxy, butoxy, isobutoxy, sec-butoxy, tert-butoxy and the like.
In the present specification, unless otherwise specified, the "C₁₋₆ alkoxy-carbonyl" means methoxycarbonyl, ethoxycarbonyl, propoxycarbonyl, tert-butoxycarbonyl and the like.
In the present specification, unless otherwise specified, the "C₁₋₆ alkyl-carbonyl" means acetyl, propanoyl, butanoyl, isobutanoyl, pentanoyl, isopentanoyl, hexanoyl and the like.

The definition of each symbol used in the formula (I) is described in detail in the following.
R¹, R², R³, R⁴, R⁵, R⁶ and Z are the same or different and each is a hydrogen atom or a substituent.
Examples of the "substituent" for R¹, R², R³, R⁴, R⁵, R⁶ or Z include "optionally substituted hydrocarbon group", "optionally substituted heterocyclic group", "optionally substituted hydroxy", "optionally substituted mercapto", "optionally substituted amino", "cyano". "nitro", "acyl". "halogen atom" and the like.

Examples of the "hydrocarbon group" of the aforementioned "optionally substituted hydrocarbon group" include C₁₋₁₀ alkyl, C₂₋₁₀ alkenyl, C₂₋₁₀ alkynyl, C₃₋₁₀ cycloalkyl, C₃₋₁₀ cycloalkenyl, C₄₋₁₀ cycloalkadienyl, C₆₋₁₄ aryl, C₇₋₁₃ aralkyl, C₈₋₁₃ arylalkenyl, C₃₋₁₀ cycloalkyl-C₁₋₆ alkyl and the like.

Here, examples of the C₁₋₁₀ alkyl include methyl, ethyl, propyl, isopropyl, butyl, isobutyl, sec-butyl, tert-butyl, pentyl, isopentyl, neopentyl, tert-pentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.
Examples of the C₂₋₁₀ alkenyl include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.
Examples of the C₂₋₁₀ alkynyl include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.

Examples of the C₃₋₁₀ cycloalkyl include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like.
Examples of the C₃₋₁₀ cycloalkenyl include 2-cyclopenten-1-yl, 3-cyclopenten-1-yl, 2-cyclohexen-1-yl, 3-cyclohexen-1-yl and the like.
Examples of the C₄₋₁₀ cycloalkadienyl include 2,4-cyclopentadien-1-yl, 2,4-cyclohexadien-1-yl, 2,5-cyclohexadien-1-yl and the like.
The above-mentioned C₃₋₁₀ cycloalkyl, C₃₋₁₀ cycloalkenyl and C₄₋₁₀ cycloalkadienyl each may be fused with a benzene ring, and examples of such fused ring group include indanyl, dihydronaphthyl, tetrahydronaphthyl, fluorenyl and the like.

Examples of the C₆₋₁₄ aryl include phenyl, naphthyl, anthryl, phenanthryl, acenaphthyl, biphenylyl and the like.
Examples of the C₇₋₁₃ aralkyl include benzyl, phenethyl, naphthylmethyl, biphenylylmethyl and the like.
Examples of the C₈₋₁₃ arylalkenyl include styryl and the like.
Examples of the C₃₋₁₀ cycloalkyl-C₁₋₆ alkyl include cyclohexylmethyl and the like.

The C₁₋₁₀ alkyl, C₂₋₁₀ alkenyl and C₂₋₁₀ alkynyl exemplified as the aforementioned "hydrocarbon group" optionally have 1 to 3 substituents at substitutable position(s).
Examples of such substituent include
(1) C₃₋₁₀ cycloalkyl (e.g., cyclopropyl, cyclohexyl) optionally substituted by 1 to 3 substituents selected from
   (a) C₁₋₆ alkyl optionally substituted by 1 to 3 halogen atoms,
   (b) hydroxy,
   (c) C₁₋₆ alkoxy, and
   (d) a halogen atom;
(2) C₆₋₁₄ aryl (e.g., phenyl, naphthyl) optionally substituted by 1 to 3 substituents selected from
   (a) C₁₋₆ alkyl optionally substituted by 1 to 3 halogen atoms,
   (b) hydroxy,
   (c) C₁₋₆ alkoxy, and
   (d) a halogen atom;
(3) an aromatic heterocyclic group (e.g., thienyl, furyl, pyridyl, oxazolyl, thiazolyl, tetrazolyl, oxadiazolyl, pyrazinyl, quinolyl, indolyl, pyrimidinyl, imidazolyl, pyrazolyl, thiadiazolyl, isoxazolyl) optionally substituted by 1 to 3 substituents selected from
   (a) C₁₋₆ alkyl optionally substituted by 1 to 3 substituents selected from a halogen atom and hydroxy,
   (b) hydroxy,
   (c) C₁₋₆ alkoxy, and
   (d) a halogen atom;
(4) a non-aromatic heterocyclic group (e.g., tetrahydrofuryl, morpholinyl, thiomorpholinyl, piperidinyl, pyrrolidinyl, piperazinyl, dioxolyl, dioxolanyl, 1,3-dihydro-2-benzofuranyl, thiazolidinyl, oxooxadiazolyl) optionally substituted by 1 to 3 substituents selected from
   (a) C₁₋₆ alkyl optionally substituted by 1 to 3 halogen atoms,
   (b) hydroxy,
   (c) C₁₋₆ alkoxy, and
   (d) a halogen atom;
(5) amino optionally mono- or di-substituted by substituent(s) selected from
   (a) C₁₋₆ alkyl optionally substituted by 1 to 3 substituents selected from
      (i) C₁₋₆ alkoxy,
      (ii) C₆₋₁₄ aryloxy (e.g., phenoxy),
      (iii) carboxyl,
      (iv) C₁₋₆ alkoxy-carbonyl, and
      (v) hydroxy,
   (b) C₇₋₁₃ aralkyloxy (e.g., benzyloxy),
   (c) C₁₋₆ alkyl-carbonyl optionally substituted by 1 to 3 substituents selected from
      (i) hydroxy, and
      (ii) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl),
   (d) C₃₋₁₀ cycloalkyl-carbonyl (e.g., cyclopropylcarbonyl, cyclopentylcarbonyl, cyclohexylcarbonyl),
   (e) C₁₋₆ alkoxy-carbonyl,
   (f) C₆₋₁₄ aryl-carbonyl (e.g., benzoyl) optionally substituted by 1 to 3 substituents selected from
      (i) C₁₋₆ alkyl optionally substituted by 1 to 3 halogen atoms (e.g., trifluoromethyl), and
      (ii) a halogen atom,
   (g) C₇₋₁₃ aralkyl-carbonyl (e.g., benzylcarbonyl, phenethylcarbonyl),
   (h) aromatic heterocyclyl-carbonyl (e.g., thienylcarbonyl, furylcarbonyl, pyridylcarbonyl, oxazolylcarbonyl, thiazolylcarbonyl, tetrazolylcarbonyl, oxadiazolylcarbonyl, pyrazinylcarbonyl, quinolylcarbonyl, indolylcarbonyl, imidazolylcarbonyl, pyrazolylcarbonyl, thiadiazolylcarbonyl, isoxazolylcarbonyl) optionally substituted by 1 to 3 C₁₋₆ alkyl,
   (i) aromatic heterocyclyl-carbamoyl (e.g., thienylcarbamoyl, furylcarbamoyl, pyridylcarbamoyl, oxazolylcarbamoyl, thiazolylcarbamoyl, tetrazolylcarbamoyl, oxadiazolylcarbamoyl, pyrazinylcarbamoyl, quinolylcarbamoyl, indolylcarbamoyl, imidazolylcarbamoyl, pyrazolylcarbamoyl, thiadiazolylcarbamoyl, isoxazolylcarbamoyl),
   (j) C₁₋₆ alkyl-carbamoyl (e.g., methylcarbamoyl, ethylcarbamoyl),
   (k) C₆₋₁₄ aryl-carbamoyl (e.g., phenylcarbamoyl, 1-naphthylcarbamoyl, 2-naphthylcarbamoyl),
   (l) C₇₋₁₃ aralkyl-carbamoyl (e.g., benzylcarbamoyl),
   (m) C₁₋₆ alkyl-sulfonyl (e.g., methylsulfonyl, ethylsulfonyl, isopropylsulfonyl),
   (n) C₆₋₁₄ aryl-sulfonyl (e.g., benzenesulfonyl, toluenesulfonyl, 1-naphthalenesulfonyl, 2-naphthalenesulfonyl), and
   (o) C₇₋₁₃ aralkyl-sulfonyl (e.g., benzylsulfonyl);
(6) amidino;
(7) C₁₋₆ alkyl-carbonyl optionally substituted by 1 to 3 halogen atoms;
(8) C₁₋₆ alkoxy-carbonyl optionally substituted by 1 to 3 halogen atoms;
(9) C₁₋₆ alkylsulfonyl (e.g., methylsulfonyl) optionally substituted by 1 to 3 halogen atoms;
(10) carbamoyl optionally mono- or di-substituted by substituent(s) selected from
   (a) C₁₋₆ alkyl optionally substituted by 1 to 3 substituents selected from
      (i) a halogen atom,
      (ii) cyano,
      (iii) hydroxy, and
      (iv) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl),
   (b) C₆₋₁₄ aryl (e.g., phenyl),
   (c) C₇₋₁₃ aralkyl (e.g., benzyl), and
   (d) aromatic heterocyclyl-C₁₋₆ alkyl (e.g., furfuryl);
(11) thiocarbamoyl optionally substituted by C₁₋₆ alkyl optionally mono- or di-substituted by 1 to 3 halogen atoms;
(12) sulfamoyl optionally substituted by C₁₋₆ alkyl optionally mono- or di-substituted by 1 to 3 halogen atoms;
(13) carboxyl;
(14) hydroxy;
(15) C₁₋₆ alkoxy optionally substituted by 1 to 3 substituents selected from
   (a) a halogen atom,
   (b) carboxyl,
   (c) C₁₋₆ alkoxy, and
   (d) C₁₋₆ alkoxy-carbonyl;
(16) C₂₋₆ alkenyloxy (e.g., ethenyloxy) optionally substituted by 1 to 3 halogen atoms;
(17) C₃₋₁₀ cycloalkyloxy (e.g., cyclohexyloxy);
(18) C₇₋₁₃ aralkyloxy (e.g., benzyloxy);
(19) C₆₋₁₄ aryloxy (e.g., phenyloxy, naphthyloxy);
(20) C₁₋₆ alkyl-carbonyloxy (e.g., acetyloxy, tert-butylcarbonyloxy);
(21) mercapto;
(22) C₁₋₆ alkylthio (e.g., methylthio, ethylthio) optionally substituted by 1 to 3 halogen atoms;
(23) C₇₋₁₃ aralkylthio (e.g., benzylthio);
(24) C₆₋₁₄ arylthio (e.g., phenylthio, naphthylthio);
(25) sulfo;
(26) cyano;
(27) azido;
(28) nitro;
(29) nitroso;
(30) a halogen atom;
(31) C₁₋₆ alkylsulfinyl (e.g., methylsulfinyl);
(32) C₃₋₁₀ cycloalkyl-C₁₋₆ alkyloxy (e.g., cyclopropylmethyloxy);
(33) C₁₋₃ alkylenedioxy;
(34) N-hydroxycarbamoyl;
(35) mono or di-C₁₋₆ alkylphosphoryl (e.g., diethylphosphoryl);
(36) C₆₋₁₄ arylsulfonyl (e.g., phenylsulfonyl);
(37) non-aromatic heterocyclyl-carbonyl (e.g., tetrahydrofurylcarbonyl, morpholinylcarbonyl, thiomorpholinylcarbonyl, piperidinylcarbonyl, pyrrolidinylcarbonyl, piperazinylcarbonyl, dioxolylcarbonyl, dioxolanylcarbonyl, 1,3-dihydro-2-benzofuranylcarbonyl, thiazolidinylcarbonyl) optionally substituted by 1 to 3 substituents selected from
   (a) C₁₋₆ alkyl optionally substituted by 1 to 3 halogen atoms,
   (b) hydroxy,
   (c) C₁₋₆ alkoxy, and
   (d) a halogen atom;
      and the like. When the number of substituents is two or more, the substituents may be the same or different.

The C₃₋₁₀ cycloalkyl, C₃₋₁₀ cycloalkenyl, C₄₋₁₀ cycloalkadienyl, C₆₋₁₄ aryl, C₇₋₁₃ aralkyl, C₈₋₁₃ arylalkenyl and C₃₋₁₀ cycloalkyl-C₁₋₆ alkyl exemplified as the aforementioned "hydrocarbon group" optionally have 1 to 3 substituents at substitutable position(s).
Examples of such substituent include
(1) the aforementioned groups exemplified as the substituents that C₁₋₁₀ alkyl and the like may have;
(2) C₁₋₆ alkyl optionally substituted by 1 to 3 substituents selected from
   (a) a halogen atom,
   (b) carboxyl,
   (c) hydroxy optionally substituted by a non-aromatic heterocyclic group (e.g., piperidino, tetrahydropyranyl),
   (d) amino optionally mono- or di-substituted by C₁₋₆ alkyl,
   (e) C₆₋₁₄ aryl (e.g., phenyl),
   (f) C₁₋₆ alkoxy-carbonyl,
   (g) C₁₋₆ alkyl-carbonyloxy (e.g., acetyloxy, tert-butylcarbonyloxy),
   (h) carbamoyl,
   (i) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl) optionally substituted 1 to 3 C₁₋₆ alkyl optionally substituted by 1 to 3 hydroxy (e.g., hydroxymethyl), and
   (j) a non-aromatic heterocyclic group (e.g., piperidino, tetrahydropyranyl);
(3) C₂₋₆ alkenyl (e.g., ethenyl, 1-propenyl) optionally substituted by 1 to 3 substituents selected from
   (a) a halogen atom,
   (b) carboxyl,
   (c) C₁₋₆ alkoxy-carbonyl, and
   (d) carbamoyl;
(4) C₇₋₁₃ aralkyl (e.g., benzyl) optionally substituted by 1 to 3 substituents selected from
   (a) C₁₋₆ alkyl optionally substituted by 1 to 3 halogen atoms,
   (b) hydroxy,
   (c) C₁₋₆ alkoxy, and
   (d) a halogen atom;
      and the like. When the number of substituents is two or more, the substituents may be the same or different.

Examples of the "heterocyclic group" of the aforementioned "optionally substituted heterocyclic group" include an aromatic heterocyclic group and a non-aromatic heterocyclic group.
Here, examples of the aromatic heterocyclic group include a 4- to 7-membered (preferably 5- or 6-membered) monocyclic aromatic heterocyclic group containing, as a ring constituting atom besides carbon atom, 1 to 4 hetero atoms selected from an oxygen atom, a sulfur atom and a nitrogen atom, and a fused aromatic heterocyclic group. Examples of the fused aromatic heterocyclic group include a group wherein such 4- to 7-membered monocyclic aromatic heterocyclic group is fused with one or two selected from 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene), a benzene ring and the like, and the like.
Preferable examples of the aromatic heterocyclic group include
monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-5-yl, 1,2,4-triazin-3-yl, 1,2,4-triazin-6-yl) and the like;
fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl, 4-benzofuranyl, 5-benzofuranyl, 6-benzofuranyl, 7-benzofuranyl), benzothienyl (e.g., 2-benzothienyl, 3-benzothienyl), benzoxazolyl (e.g., 2-benzoxazolyl), benzisoxazolyl (e.g., 7-benzisooxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like;
and the like.

Examples of the non-aromatic heterocyclic group include a 4- to 7-membered (preferably 5- or 6-membered) a monocyclic non-aromatic heterocyclic group, containing, as a ring constituting atom besides carbon atom, 1 to 4 hetero atoms selected from an oxygen atom, a sulfur atom and a nitrogen atom, and a fused non-aromatic heterocyclic group. Examples of the fused non-aromatic heterocyclic group include a group wherein such 4- to 7-membered monocyclic non-aromatic heterocyclic group is fused with one or two selected from a 5-or 6-membered aromatic or non-aromatic heterocycle containing 1 or 2 nitrogen atoms (e.g., pyrrole, imidazole, pyrazole, pyrazine, pyridine, pyrimidine), a 5-membered aromatic or non-aromatic heterocycle containing one sulfur atom (e.g., thiophene), a benzene ring and the like, and the like.
Preferable examples of the non-aromatic heterocyclic group include
monocyclic non-aromatic heterocyclic groups such as pyrrolidinyl (e.g., 1-pyrrolidinyl, 2-pyrrolidinyl), piperidinyl (e.g., piperidino, 2-piperidinyl, 3-piperidinyl, 4-piperidinyl), homopiperidinyl (e.g., homopiperidino, 2-homopiperidinyl, 3-homopiperidinyl, 4-homopiperidinyl), tetrahydropyridyl (e.g., 1,2,3,6-tetrahydropyridin-1-yl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimine-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, pyranyl (e.g., 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidetetrahydrothiopyranyl (e.g., 1-oxidetetrahydrothiopyran-4-yl), 1,1-dioxidetetrahydrothiopyranyl (e.g., 1,1-dioxidetetrahydrothiopyran-4-yl), tetrahydrofuryl (e.g., tetrahydrofuran-3-yl, tetrahydrofuran-2-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl), oxodihydrooxadiazolyl (e.g., 5-oxo-4,5-dihydro-1,2,4-oxadiazol-3-yl) and the like;
fused non-aromatic heterocyclic groups such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydro-1-benzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydro-1-benzofuran-3-yl), chromenyl (e.g., 4H-chromen-2-yl, 2H-chromen-3-yl, 2H-chromene-7-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl, 1,2,3,4-tetrahydroisoquinolin-2-yl), dihydrophthalazinyl (e.g., 1,4-dihydrophthalazin-4-yl), tetrahydrobenzoazepinyl (e.g., 2,3,4,5-tetrahydro-1H-benzo[c]azepin-1-yl) and the like;
and the like.

The "heterocyclic group" of the aforementioned "optionally substituted heterocyclic group" optionally has 1 to 3 substituents at substitutable position(s). Examples of such substituent include those exemplified as the substituents that C₃₋₁₀ cycloalkyl exemplified as the "hydrocarbon group" of the aforementioned "optionally substituted hydrocarbon group" may have, and the like. When the number of substituents is two or more, the substituents may be the same or different.

Examples of the aforementioned "optionally substituted hydroxy" include hydroxy optionally substituted by a substituent selected from C₁₋₁₀ alkyl, C₂₋₁₀ alkenyl, C₃₋₁₀ cycloalkyl, C₃₋₁₀ cycloalkenyl, C₆₋₁₄ aryl, C₇₋₁₃ aralkyl, C₈₋₁₃ arylalkenyl, C₁₋₆ alkyl-carbonyl, heterocyclic group etc., each being optionally substituted.

Moreover, examples of the aforementioned "optionally substituted hydroxy" include hydroxy substituted by C₆₋₁₄ aryl fused with C₃₋₁₀ cycloalkane optionally substituted by oxo (e.g., 5-oxotetrahydrobenzoannulen-2-yl).
Here, examples of the "C₁₋₁₀ alkyl, C₂₋₁₀ alkenyl, C₃₋₁₀ cycloalkyl, C₃₋₁₀ cycloalkenyl, C₆₋₁₄ aryl, C₇₋₁₃ aralkyl and C₈₋₁₃ arylalkenyl, each being optionally substituted", include those exemplified as the aforementioned "optionally substituted hydrocarbon group".

Examples of the "optionally substituted heterocyclic group" as the substituent of the aforementioned "optionally substituted hydroxy" include those exemplified as the "optionally substituted heterocyclic group" as the "substituent" for R¹ and the like.

Examples of the aforementioned "optionally substituted mercapto" include mercapto optionally substituted by substituent (s) selected from C₁₋₁₀ alkyl, C₂₋₁₀ alkenyl, C₃₋₁₀ cycloalkyl, C₃₋₁₀ cycloalkenyl, C₆₋₁₄ aryl, C₇₋₁₃ aralkyl, C₈₋₁₃ arylalkenyl, C₁₋₆ alkyl-carbonyl, heterocyclic group etc., each being optionally substituted.
Here, Examples of the "C₁₋₁₀ alkyl, C₂₋₁₀ alkenyl, C₃₋₁₀ cycloalkyl, C₃₋₁₀ cycloalkenyl, C₆₋₁₄ aryl, C₇₋₁₃ aralkyl and C₈₋₁₃ arylalkenyl, each being optionally substituted" include those exemplified as the aforementioned "optionally substituted hydrocarbon group".

Examples of the "optionally substituted heterocyclic group" as the substituent of the aforementioned "optionally substituted mercapto" include those exemplified as the "optionally substituted heterocyclic group" as the "substituent" for R¹ and the like.

Examples of the aforementioned "optionally substituted amino" include amino optionally substituted by one or two substituents selected from C₁₋₁₀ alkyl, C₂₋₁₀ alkenyl, C₃₋₁₀ cycloalkyl, C₃₋₁₀ cycloalkenyl, C₆₋₁₄ aryl, C₇₋₁₃ aralkyl, C₈₋₁₃ arylalkenyl and heterocyclic group, each being optionally substituted; acyl etc.
Here, Examples of the "C₁₋₁₀ alkyl, C₂₋₁₀ alkenyl, C₃₋₁₀ cycloalkyl, C₃₋₁₀ cycloalkenyl, C₆₋₁₄ aryl, C₇₋₁₃ aralkyl, C₈₋₁₃ arylalkenyl, each being optionally substituted" include those exemplified as the aforementioned "optionally substituted hydrocarbon group".
Examples of the "optionally substituted heterocyclic group" as the substituent of the aforementioned "optionally substituted amino" include those exemplified as the "optionally substituted heterocyclic group" as the "substituent" for R¹ and the like. Of these, an optionally substituted 5- or 6-membered non-aromatic heterocyclic group (e.g., pyrrolidine, piperidine) is preferable.

Examples of the "acyl" exemplified as the substituent of the "optionally substituted amino" and the "substituent" for R¹, R², R³, R⁴, R⁵, R⁶ or Z include groups represented by the formulas: -COR^{a}, -CO-OR^{a}, -SO₂R^{a}, -SOR^{a}, -CO-NR^{a}, R^{b}', -SO₂-NR^{a}'R^{b}', -SO₂-NR^{a}' (COR^{b}') or -CS-NR^{a}' R^{b}' wherein R^{a} is a hydrogen atom, an optionally substituted hydrocarbon group or an optionally substituted heterocyclic group, R^{a}' is a hydrogen atom, hydroxy, an optionally substituted hydrocarbon group or an optionally substituted heterocyclic group, R^{b}' is a hydrogen atom, an optionally substituted hydrocarbon group or an optionally substituted heterocyclic group, or R^{a}' and R^{b}' form, together with the adjacent nitrogen atom, an optionally substituted nitrogen-containing heterocycle, and the like.
Examples of the "optionally substituted hydrocarbon group" and "optionally substituted heterocyclic group" for R^{a}, R^{a}' or R^{b}' include those similar to the "optionally substituted hydrocarbon group" and "optionally substituted heterocyclic group" exemplified as the "substituent" for R¹ and the like.

Examples of the "nitrogen-containing heterocycle" of the "optionally substituted nitrogen-containing heterocycle" formed by R^{a}' and R^{b}' together with the adjacent nitrogen atom include a 5- to 7-membered nitrogen-containing heterocycle containing, as a ring constituting atom besides carbon atom, at least one nitrogen atom, and optionally further containing 1 or 2 hetero atoms selected from an oxygen atom, a sulfur atom and a nitrogen atom. Preferable examples of the nitrogen-containing heterocycle include pyrrolidine, imidazolidine, pyrazolidine, piperidine, piperazine, morpholine, thiomorpholine, oxopiperazine and the like.
The nitrogen-containing heterocycle optionally has 1 to 3 (preferably 1 or 2) substituents at substitutable position(s). Examples of such substituent include those exemplified as the substituent that the C₃₋₁₀ cycloalkyl group and the like exemplified as the "hydrocarbon group" of the aforementioned "optionally substituted hydrocarbon group" may have. When the number of substituents is two or more, the substituents may be the same or different.

Preferable examples of the "acyl" include
(1) formyl;
(2) carboxyl;
(3) C₁₋₆ alkyl-carbonyl optionally substituted by 1 to 3 substituents selected from hydroxy and an aromatic heterocyclic group;
(4) C₁₋₆ alkoxy-carbonyl optionally substituted by 1 to 3 substituents selected from
   (a) carboxyl,
   (b) carbamoyl,
   (c) thiocarbamoyl,
   (d) C₁₋₆ alkoxy-carbonyl, and
   (e) C₁₋₆ alkyl-carbonyloxy;
(5) C₃₋₁₀ cycloalkyl-carbonyl (e.g., cyclopropylcarbonyl, cyclopentylcarbonyl, cyclohexylcarbonyl);
(6) C₆₋₁₄ aryl-carbonyl (e.g., benzoyl, 1-naphthoyl, 2-naphthoyl) optionally substituted by 1 to 3 substituents selected from
   (a) a halogen atom,
   (b) cyano,
   (c) C₁₋₆ alkyl optionally substituted by 1 to 3 halogen atoms,
   (d) C₁₋₆ alkoxy,
   (e) carboxyl,
   (f) C₁₋₆ alkoxy-carbonyl, and
   (g) carbamoyl;
(7) C₆₋₁₄ aryloxy-carbonyl (e.g., phenyloxycarbonyl, naphthyloxycarbonyl) optionally substituted by 1 to 3 substituents selected from
   (a) carboxyl,
   (b) C₁₋₆ alkoxy-carbonyl, and
   (c) carbamoyl;
(8) C₇₋₁₃ aralkyloxy-carbonyl optionally substituted by 1 to 3 substituents selected from
   (a) carboxyl,
   (b) carbamoyl,
   (c) thiocarbamoyl,
   (d) C₁₋₆ alkoxy-carbonyl,
   (e) a halogen atom,
   (f) cyano,
   (g) nitro,
   (h) C₁₋₆ alkoxy,
   (i) C₁₋₆ alkyl-sulfonyl, and
   (j) C₁₋₆ alkyl
   (e.g., benzyloxycarbonyl, phenethyloxycarbonyl, carboxybenzyloxycarbonyl, methoxycarbonylbenzyloxycarbonyl, biphenylylmethoxycarbonyl);
(9) carbamoyl optionally mono- or di-substituted by C₁₋₆ alkyl optionally substituted by 1 to 3 substituents selected from cyano, hydroxy, aromatic heterocyclic group (e.g., thienyl, furyl, pyridyl, oxazolyl, thiazolyl, tetrazolyl, oxadiazolyl, pyrazinyl, quinolyl, indolyl, pyrimidinyl, imidazolyl, pyrazolyl, thiadiazolyl, isoxazolyl), a halogen atom, and C₁₋₆ alkoxy
   (e.g., methylcarbamoyl, ethylcarbamoyl, dimethylcarbamoyl, diethylcarbamoyl, ethylmethylcarbamoyl, propylcarbamoyl, isopropylcarbamoyl, butylcarbamoyl, isobutylcarbamoyl, trifluoroethylcarbamoyl, N-methoxyethyl-N-methylcarbamoyl);
(10) C₁₋₆ alkylsulfonyl optionally substituted by 1 to 3 substituents selected from
   (a) carboxyl,
   (b) carbamoyl, and
   (c) C₁₋₆ alkoxy-carbonyl
   (e.g., methylsulfonyl, carboxymethylsulfonyl);
(11) C₁₋₆ alkylsulfinyl (e. g., methylsulfinyl);
(12) thiocarbamoyl;
(13) C₇₋₁₃ aralkyl-carbonyl (e.g., benzylcarbonyl, phenethylcarbonyl);
(14) aromatic heterocyclyl-carbonyl (e.g., furylcarbonyl, thienylcarbonyl, thiazolylcarbonyl, pyrazolylcarbonyl, pyridylcarbonyl, pyrazinylcarbonyl, benzofuranylcarbonyl, benzothienylcarbonyl, quinoxalinylcarbonyl, thiadiazolylcarbonyl, isoxazolylcarbonyl) optionally substituted by 1 to 3 substituents selected from
   (a) C₁₋₆ alkyl,
   (b) C₆₋₁₄ aryl,
   (c) C₇₋₁₃ aralkyl,
   (d) C₁₋₆ alkoxy,
   (e) carboxyl,
   (f) C₁₋₆ alkoxy-carbonyl, and
   (g) carbamoyl;
(15) sulfamoyl optionally mono- or di-substituted by substituent(s) selected from
   (a) C₁₋₆ alkyl optionally substituted by 1 to 3 substituents selected from a halogen atom and C₁₋₆ alkoxy, and
   (b) C₁₋₆ alkyl-carbonyl
      (e.g., methylsulfamoyl, ethylsulfamoyl, dimethylsulfamoyl, diethylsulfamoyl, ethylmethylsulfamoyl, propylsulfamoyl, isopropylsulfamoyl, butylsulfamoyl, isobutylsulfamoyl, trifluoroethylsulfamoyl, N-methoxyethyl-N-methylsulfamoyl, acetylsulfamoyl);
(16) N-hydroxycarbamoyl;
(17) non-aromatic heterocyclyl-carbonyl (e.g., tetrahydrofurylcarbonyl, morpholinylcarbonyl, thiomorpholinylcarbonyl, piperidinylcarbonyl, pyrrolidinylcarbonyl, piperazinylcarbonyl, dioxolylcarbonyl, dioxolanylcarbonyl, 1,3-dihydro-2-benzofuranylcarbonyl, thiazolidinylcarbonyl) optionally substituted by 1 to 3 substituents selected from
   (a) C₁₋₆ alkyl optionally substituted by 1 to 3 halogen atoms,
   (b) hydroxy,
   (c) C₁₋₆ alkoxy, and
   (d) a halogen atom;
      and the like.

R¹ is preferably a hydrogen atom, optionally substituted C₁₋₁₀ alkyl (preferably, optionally substituted C₁₋₆ alkyl), optionally substituted C₃₋₁₀ cycloalkyl, an optionally substituted aromatic heterocyclic group, optionally substituted hydroxy, optionally substituted amino, cyano, carboxyl, optionally substituted C₁₋₆ alkoxy-carbonyl, optionally substituted C₁₋₆ alkyl-sulfonyl, optionally substituted sulfamoyl, a halogen atom and the like.
R¹ is more preferably
(1) a hydrogen atom,
(2) C₁₋₆ alkyl (e.g., methyl, propyl, isopropyl, tert-butyl, 2-methylbutan-2-yl) optionally substituted by 1 to 3 substituents selected from
   (a) an aromatic heterocyclic group (e.g., tetrazolyl, oxadiazolyl),
   (b) amino optionally mono- or di-substituted by substituent(s) selected from
      (i) C₁₋₆ alkyl (e.g., propyl),
      (ii) C₁₋₆ alkyl-carbonyl (e.g., acetyl, propionyl) optionally substituted by 1 to 3 substituents selected from hydroxy and an aromatic heterocyclic group (e.g., imidazolyl),
      (iii) C₆₋₁₄ aryl-carbonyl (e.g., benzoyl),
      (iv) C₁₋₆ alkyl-sulfonyl (e.g., methylsulfonyl), and
      (v) aromatic heterocyclyl-carbonyl (e.g., imidazolylcarbonyl),
   (c) cyano,
   (d) carboxyl,
   (e) C₁₋₆ alkoxy-carbonyl (e.g., methoxycarbonyl, ethoxycarbonyl),
   (f) N-hydroxycarbamoyl,
   (g) carbamoyl optionally mono- or di-substituted by C₁₋₆ alkyl (e.g., methyl, ethyl, propyl) optionally substituted by 1 to 3 substituents selected from (i) cyano, (ii) hydroxy, and (iii) an aromatic heterocyclic group (e.g., tetrazolyl) and (iv) a non-aromatic heterocyclic group (e.g., dioxolyl) optionally having 1 to 3 C₁₋₆ alkyl,
   (h) a halogen atom (e.g., fluorine atom),
   (i) di-C₁₋₆ alkylphosphoryl (e.g., diethylphosphoryl),
   (j) hydroxy,
   (k) a non-aromatic heterocyclic group (e.g., oxoxadiazolinyl);
(3) C₃₋₁₀ cycloalkyl (e.g., cyclopropyl, cyclobutyl, cyclopentyl, cyclohexyl) optionally substituted by 1 to 3 substituents selected from
   (a) C₁₋₆ alkoxy-carbonyl (e.g., methoxycarbonyl), and
   (b) carboxyl;
(4) an aromatic heterocyclic group (e.g., pyridyl, furyl, imidazolyl, pyrazolyl);
(5) hydroxy optionally substituted by C₁₋₆ alkyl (methyl);
(6) amino optionally mono- or di-substituted by substituent(s) selected from
   (a) C₁₋₆ alkyl (e.g., methyl, ethyl), and
   (b) C₁₋₆ alkyl-sulfonyl (e.g., methylsulfonyl);
(7) cyano;
(8) carboxyl;
(9) C₁₋₆ alkoxy-carbonyl (e.g., methoxycarbonyl);
(10) C₁₋₆ alkyl-sulfonyl (e.g., methylsulfonyl);
(11) sulfamoyl optionally mono- or di-substituted by substituent(s) selected from
   (a) C₁₋₆ alkyl (e.g., methyl), and
   (b) C₁₋₆ alkyl-carbonyl (e.g., acetyl);
(12) a halogen atom (e.g., chlorine atom);
   and the like.

R² is preferably a hydrogen atom.
R³ is preferably a hydrogen atom, optionally substituted C₁₋₁₀ alkyl (preferably, optionally substituted C₁₋₆ alkyl) or a halogen atom, more preferably, a hydrogen atom, C₁₋₆ alkyl (e.g., methyl) or a halogen atom (e.g., bromine atom).
R⁴ is preferably a hydrogen atom.
R⁵ is preferably a hydrogen atom.

R⁶ is preferably a hydrogen atom, optionally substituted C₁₋₁₀ alkyl (preferably, optionally substituted C₁₋₆ alkyl), optionally substituted C₆₋₁₄ aryl, an optionally substituted aromatic heterocyclic group, an optionally substituted non-aromatic heterocyclic group, optionally substituted hydroxy, optionally substituted mercapto, optionally substituted amino, cyano, optionally substituted C₁₋₆ alkyl-carbonyl, halogen atom and the like.
R⁶ is more preferably
(1) a hydrogen atom;
(2) C₁₋₆ alkyl (e.g., methyl, ethyl) optionally substituted by 1 to 3 substituents selected from
   (a) hydroxy,
   (b) amino optionally mono- or di-substituted by C₁₋₆ alkyl (e.g., methyl), and
   (c) a halogen atom (e.g., fluorine atom);
(3) C₆₋₁₄ aryl (e.g., phenyl) optionally substituted by 1 to 3 substituents selected from
   (a) C₁₋₆ alkyl (e.g., methyl) optionally substituted by 1 to 3 halogen atoms (e.g., fluorine atom)
      (specific example, trifluoromethyl),
   (b) C₁₋₆ alkoxy (e.g., methoxy) optionally substituted by 1 to 3 halogen atoms (e.g., fluorine atom)
      (specific example, methoxy, trifluoromethoxy),
   (c) C₁₋₆ alkoxy-carbonyl (e.g., methoxycarbonyl, ethoxycarbonyl),
   (d) amino optionally mono- or di-substituted by substituent(s) selected from
      (i) C₁₋₆ alkyl (e.g., methyl), and
      (ii) C₁₋₆ alkyl-carbonyl (e.g., acetyl),
   (e) C₁₋₆ alkyl-sulfonyl (e.g., methylsulfonyl),
   (f) cyano, and
   (g) C₁₋₃ alkylenedioxy (e.g., methylenedioxy);
(4) an aromatic heterocyclic group (e.g., thienyl, furyl, pyrrolyl, imidazolyl, pyrazolyl, oxazolyl, triazolyl, pyridyl, pyridazinyl, indolyl) optionally substituted by 1 to 3 substituents selected from
   (a) C₁₋₆ alkyl (e.g., methyl, ethyl, isopropyl, tert-butyl) optionally substituted by 1 to 3 substituents selected from
      (i) hydroxy optionally substituted by a non-aromatic heterocyclic group (e.g., tetrahydropyranyl), and
      (ii) cyano,
   (b) C₆₋₁₄ aryl (e.g., phenyl),
   (c) an aromatic heterocyclic group (e.g., pyridyl),
   (d) C₁₋₆ alkoxy (e.g., methoxy), and
   (e) a halogen atom (e.g., fluorine atom, chlorine atom, bromine atom);
(5) a non-aromatic heterocyclic group (e.g., pyrrolidinyl, piperidinyl, homopiperidinyl, tetrahydropyridyl, piperazinyl, morpholinyl, thiomorpholinyl, tetrahydroisoquinolinyl, dihydroisoindolyl, tetrahydrobenzoazepinyl) optionally substituted by 1 to 3 substituents selected from
   (a) C₁₋₆ alkyl (e.g., methyl, ethyl) optionally substituted by 1 to 3 substituents selected from
      (i) C₆₋₁₄ aryl (e.g., phenyl),
      (ii) an aromatic heterocyclic group (e.g., pyridyl), and
      (iii) hydroxy optionally substituted by C₁₋₆ alkyl (e.g., methyl),
   (b) C₆₋₁₄ aryl (e.g., phenyl) optionally substituted by 1 to 3 substituents selected from C₁₋₆ alkoxy (e.g., methoxy) and a halogen atom (e.g., fluorine atom),
   (c) an aromatic heterocyclic group (e.g., pyridyl, pyrimidinyl),
   (d) hydroxy,
   (e) amino optionally mono- or di-substituted by C₁₋₆ alkyl-carbonyl (e.g., acetyl),
   (f) carboxyl,
   (g) carbamoyl optionally mono- or di-substituted by C₇₋₁₃ aralkyl (e.g., benzyl),
   (h) C₁₋₆ alkyl-sulfonyl (e.g., ethylsulfonyl), and
   (i) C₆₋₁₄ aryl-sulfonyl (e.g., benzenesulfonyl);
(6) hydroxy optionally substituted by a substituent selected from
   (a) C₆₋₁₄ aryl (e.g., phenyl) optionally substituted by 1 to 3 substituents selected from
      (i) C₁₋₆ alkyl (e.g., ethyl, propyl, butyl) optionally substituted by 1 to 3 substituents selected from
         (A) a halogen atom (e.g., fluorine atom),
         (B) amino optionally mono- or di-substituted by C₁₋₆ alkyl (e.g., methyl), and
         (C) aromatic heterocyclic group (e.g., imidazolyl) optionally substituted by 1 to 3 C₁₋₆ alkyl (e.g., methyl) optionally substituted by 1 to 3 hydroxy (specific example, hydroxymethyl),
      (ii) aromatic heterocyclic group (e.g., imidazolyl, pyrazolyl) optionally substituted by 1 to 3 C₁₋₆ alkyl (e.g., methyl),
      (iii) a non-aromatic heterocyclic group (e.g., morpholinyl),
      (iv) C₁₋₆ alkoxy optionally substituted by 1 to 3 substituents selected from (A) a halogen atom (e.g., fluorine atom) and (B) carboxyl (e.g., methoxy),
      (v) amino optionally mono- or di-substituted by substituent(s) selected from
         (A) C₁₋₆ alkyl (e.g., methyl, ethyl),
         (B) C₁₋₆ alkyl-carbonyl (e.g., acetyl, tert-butylcarbonyl),
         (C) C₃₋₁₀ cycloalkyl-carbonyl (e.g., cyclopropylcarbonyl, cyclohexylcarbonyl),
         (D) C₆₋₁₄ aryl-carbonyl (e.g., benzoyl) optionally substituted by 1 to 3 substituents selected from (1') C₁₋₆ alkyl (e.g., methyl) optionally substituted by 1 to 3 halogen atoms (e.g., fluorine atom) (specific example, trifluoromethyl), (2') a halogen atom (e.g., fluorine atom, chlorine atom), and (3') C₃₋₁₀ cycloalkyl optionally substituted by 1 to 3 cyano (specific example, 1-cyanocyclopropyl), and
         (E) aromatic heterocyclyl-carbonyl (e.g., thienylcarbonyl, furylcarbonyl, pyridylcarbonyl, pyrazolylcarbonyl, thiadiazolylcarbonyl, isoxazolylcarbonyl) optionally substituted by 1 to 3 C₁₋₆ alkyl (e.g., methyl),
      (vi) carboxyl, and
      (vii) non-aromatic heterocyclyl-carbonyl (e.g., pyrrolidinylcarbonyl) optionally substituted by 1 to 3 substituents selected from C₁₋₆ alkyl (e.g., methyl) and oxo,
   (b) an aromatic heterocyclic group (e.g., pyridyl, benzofuranyl) optionally substituted by 1 to 3 substituents selected from
      (i) C₁₋₆ alkyl (e.g., methyl),
      (ii) C₁₋₆ alkyl-carbonyl (e.g., acetyl), and
      (iii) carboxyl, and
   (c) a non-aromatic heterocyclic group (e.g., pyrrolidinyl, piperidinyl, chromenyl) optionally substituted by 1 to 3 substituents selected from C₁₋₆ alkyl (e.g., methyl) and oxo;
(7) mercapto optionally substituted by
   (a) an aromatic heterocyclic group (e.g., pyridyl, imidazolyl, thiazolyl, thiadiazolyl, triazolyl) optionally substituted by 1 to 3 substituents selected from
      (i) C₁₋₆ alkyl (e.g., methyl) optionally substituted by 1 to 3 halogen atoms (e.g., fluorine atom)
         (specific example, methyl, trifluoromethyl),
      (ii) C₆₋₁₄ aryl (e.g., phenyl), and
      (iii) an aromatic heterocyclic group (e.g., pyridyl);
(8) amino optionally mono- or di-substituted by substituent(s) selected from
   (a) C₁₋₆ alkyl (e.g., methyl, ethyl, propyl) optionally substituted by 1 to 3 substituents selected from
      (i) an aromatic heterocyclic group (e.g., pyridyl, imidazolyl),
      (ii) hydroxy,
      (iii) C₁₋₆ alkoxy (e.g., methoxy), and
      (iv) amino optionally mono- or di-substituted by C₁₋₆ alkyl (e.g., methyl),
   (b) C₃₋₁₀ cycloalkyl (e.g., cyclopropyl, cyclopentyl, cyclohexyl),
   (c) C₇₋₁₃ aralkyl (e.g., benzyl), and
   (d) a non-aromatic heterocyclic group (e.g.,
   pyrrolidinyl, piperidinyl) optionally substituted by 1 to 3 C₁₋₆ alkyl (e.g., methyl);
(9) cyano;
(10) C₁₋₆ alkyl-carbonyl (e.g., acetyl) optionally substituted by 1 to 3 hydroxy;
(11) a halogen atom (e.g., fluorine atom, chlorine atom, bromine atom, iodine atom);
(12) hydroxy substituted by a C₆₋₁₄ aryl group (e.g., phenyl) fused with C₃₋₁₀ cycloalkane (e.g., cycloheptane) optionally substituted by oxo (specific example, 5-oxotetrahydrobenzoannulen-2-yl);
   and the like.

Z is preferably a hydrogen atom or optionally substituted C₁₋₁₀ alkyl (preferably, optionally substituted C₁₋₆ alkyl), more preferably a hydrogen atom, or a C₁₋₆ alkyl group (e.g., methyl) optionally substituted by 1 to 3 hydroxy.

Examples of the "ring" of the "optionally further substituted ring for ring A" include "alicyclic hydrocarbon", "aromatic hydrocarbon", "heterocycle" and the like.
Examples of the "alicyclic hydrocarbon" include saturated or unsaturated monocyclic or fused polycyclic alicyclic hydrocarbon such as C₃₋₁₀ cycloalkane, C₃₋₁₀ cycloalkene and C₄₋₁₀ cycloalkanediene, and a bicyclic fused ring thereof with benzene and the like.
Examples of the C₃₋₁₀ cycloalkane include cyclopropane, cyclobutane, cyclopentane, cyclohexane, cycloheptane, cyclooctane, bicyclo[2.2.1]heptane, bicyclo[2.2.2]octane, bicyclo[3.2.1]octane, bicyclo[3.2.2]nonane, bicyclo[3.3.1]nonane, bicyclo[4.2.1]nonane, bicyclo[4.3.1]decane, adamantane and the like.
Examples of the C₃₋₁₀ cycloalkene include cyclopentene, cyclohexene, cycloheptene, cyclooctene and the like.
Examples of the C₄₋₁₀ cycloalkadiene include 2,4-cyclopentadiene, 2,4-cyclohexadiene and the like.
As the "aromatic hydrocarbon", monocyclic or fused polycyclic aromatic hydrocarbon is used. Preferred are C₆₋₁₄ aromatic hydrocarbon and the like. Specifically, for example, benzene, naphthalene, anthracene, phenanthrene, acenaphthylene, phenalene, biphenyl and the like is used, and benzene, naphthalene and the like are preferably used.

Examples of the "heterocycle" include aromatic heterocycle and non-aromatic heterocycle.
Here, examples of the aromatic heterocycle include a 4-to 7-membered (preferably 5- or 6-membered) monocyclic aromatic heterocycle containing, as a ring constituting atom besides carbon atom, 1 to 4 hetero atoms selected from an oxygen atom, a sulfur atom and a nitrogen atom, and a fused aromatic heterocycle. Examples of the fused aromatic heterocycle include a ring wherein such 4- to 7-membered monocyclic aromatic heterocycle is fused with one or two selected from a 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene), a benzene ring etc., and the like.
Preferable examples of the aromatic heterocycle include monocyclic aromatic heterocycles such as furan, thiophene, pyridine, pyrimidine, pyridazine, pyrazine, pyrrole, imidazole, pyrazole, thiazole, isothiazole, oxazole, isoxazole, oxadiazole, thiadiazole, triazole, tetrazole, triazine and the like;
fused non-aromatic heterocycles such as quinoline, isoquinoline, quinazoline, quinoxaline, benzofuran, benzothiophene, benzoxazole, benzisoxazole, benzothiazole, benzimidazole, benzotriazole, indole, indazole, pyrrolopyrazine (e.g., 1H-pyrrolo[2,3-b]pyrazine, 1H-pyrrolo[2,3-b]pyrazine), imidazopyridine (e.g., 1H-imidazo[4,5-b]pyridine, 1H-imidazo[4,5-c]pyridine, 2H-imidazo[1,2-a]pyridine), imidazopyrazine (e.g., 1H-imidazo[4,5-b]pyrazine), pyrazolopyridine (e.g., 1H-pyrazolo[4,3-c]pyridine), pyrazolothienyl (e.g., 2H-pyrazolo[3,4-b]thiophen-2-yl), pyrazolotriazine (e.g., pyrazolo[5,1-c][1,2,4]triazine) and the like.

Examples of the non-aromatic heterocycle include a 4- to 7-membered (preferably 5- or 6-membered) monocyclic non-aromatic heterocycle containing, as a ring constituting atom besides carbon atom, 1 to 4 hetero atoms selected from an oxygen atom, a sulfur atom and a nitrogen atom, and a fused non-aromatic heterocycle. Examples of the fused non-aromatic heterocycle include a ring wherein such 4- to 7-membered monocyclic non-aromatic heterocycle is fused with one or two selected from a 5- or 6-membered aromatic or non-aromatic heterocycle containing 1 or 2 nitrogen atoms (e.g., pyrrole, imidazole, pyrazole, pyrazine, pyridine, pyrimidine), a 5-membered aromatic or non-aromatic heterocycle containing one sulfur atom (e.g., thiophene), a benzene ring etc., and the like.
Preferable examples of the non-aromatic heterocycle include monocyclic non-aromatic heterocycles such as pyrrolidine, piperidine, homopiperidine, tetrahydropyridine, morpholine, thiomorpholine, piperazine, hexamethylenimine, oxazolidine, thiazolidine, imidazolidine, oxazoline, thiazoline, imidazoline, dioxole, dioxolane, dihydrooxadiazole, 2-thioxo-1,3-oxazolidine, pyran, tetrahydropyran, thiopyran, tetrahydrothiopyran, 1-oxidetetrahydrothiopyran, 1,1-dioxidetetrahydrothiopyran, tetrahydrofuran, pyrazolidine, pyrazoline, tetrahydropyrimidine, dihydrotriazole, tetrahydrotriazole and the like; fused non-aromatic heterocycles such as dihydroindole, dihydroisoindole, dihydrobenzofuran, dihydrobenzodioxin, dihydrobenzodioxepine, tetrahydrobenzofuran, chromene, dihydroquinoline, tetrahydroquinoline, dihydroisoquinoline, tetrahydroisoquinoline, dihydrophthalazine, tetrahydrobenzoazepine and the like;
and the like.

The "ring" of the aforementioned "optionally further substituted ring" may have, besides a group represented by the formula (I)

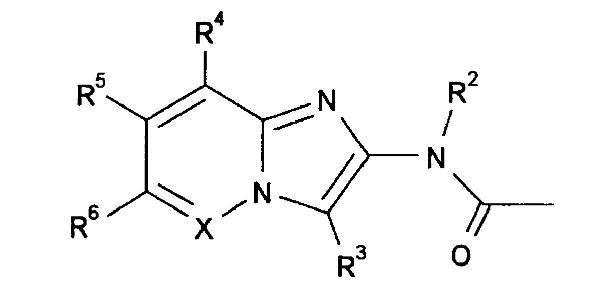

and R¹ group, 1 to 3 substituents at substitutable position(s). Examples of such substituent include those exemplified as the substituent that the C₃₋₁₀ cycloalkyl group and the like exemplified as the "hydrocarbon group" of the aforementioned "optionally substituted hydrocarbon group" may have. When the number of substituents is two or more, the substituents may be the same or different.

Ring A is preferably optionally substituted aromatic hydrocarbon (more preferably, benzene), or optionally substituted 5-membered aromatic heterocycle (more preferably, furan, thiophene, isoxazole).

As the "ring" of the "optionally substituted ring" formed by Z and R⁶ together with the adjacent nitrogen atom bonded thereto, those exemplified as the "ring" of the "optionally substituted ring" for the aforementioned ring A, which have a vinylene structure in the ring, can be mentioned.
Examples of such ring include those exemplified as the "alicyclic hydrocarbon" or "aromatic hydrocarbon" (e.g., benzene);
monocyclic aromatic heterocycles such as furan, thiophene, pyridine, pyrimidine, pyridazine, pyrazine, pyrrole, imidazole, pyrazole, thiazole, isothiazole, oxazole, isoxazole, 1,2,3-oxadiazole, 1,2,5-oxadiazole, 1,2,3-thiadiazole, 1,2,5-thiadiazole, 1,2,3-triazole and the like;
fused aromatic heterocycle such as quinoline, isoquinoline, quinazoline, quinoxaline, benzofuran, benzothiophene, benzoxazole, benzisoxazole, benzothiazole, benzimidazole, benzotriazole, indole, indazole, pyrrolopyrazine (e.g., 1H-pyrrolo[2,3-b]pyrazine, 1H-pyrrolo[2,3-b]pyrazine), imidazopyridine (e.g., 1H-imidazo[4,5-b]pyridine, 1H-imidazo[4,5-c]pyridine, 2H-imidazo[1,2-a]pyridine), imidazopyrazine (e.g., 1H-imidazo[4,5-b]pyrazine), pyrazolopyridine (e.g., 1H-pyrazolo[4,3-c]pyridine), pyrazolothienyl (e.g., 2H-pyrazolo[3,4-b]thiophen-2-yl), pyrazolotriazine (e.g., pyrazolo[5,1-c][1,2,4]triazine) and the like;
monocyclic non-aromatic heterocycles such as tetrahydropyridine, oxazoline, thiazoline, imidazoline, dioxole, dioxolane, dihydroxadiazole, pyran, thiopyran, pyrazolidine, pyrazoline, tetrahydropyrimidine, dihydrofuran (e.g., 2,3-dihydrofuran) and the like;
fused non-aromatic heterocycle such as dihydroindole, dihydroisoindole, dihydrobenzofuran, dihydrobenzodioxine, dihydrobenzodioxepine, tetrahydrobenzofuran, chromene, dihydroquinoline, tetrahydroquinoline, dihydroisoquinoline, tetrahydroisoquinoline, dihydrophthalazine, tetrahydrobenzoazepine and the like;
and the like.
The "ring" of the aforementioned "optionally substituted ring" is preferably benzene, dihydrofuran or imidazole.

The "ring" of the aforementioned "optionally substituted ring" optionally has 1 to 3 substituents at substitutable position(s). Examples of such substituent include those exemplified as the substituents that C₃₋₁₀ cycloalkyl and the like exemplified as the "hydrocarbon group" of the aforementioned "optionally substituted hydrocarbon group" may have. Of those, C₁₋₆ alkyl is preferable. When the number of substituents is two or more, the substituents may be the same or different.

The "optionally substituted ring" formed by Z and R⁶ together with the adjacent nitrogen atom bonded thereto is preferably an aromatic hydrocarbon (preferably, benzene) or a heterocycle (preferably, dihydrofuran, imidazole), each being optionally substituted by 1 to 3 C₁₋₆ alkyl.

The compounds shown in the following (i) - (iv) are excluded from the scope of compound (I).
(i) a compound wherein X is =CH-, and R⁶ is optionally substituted 2-piperidinyl,
(ii) N-imidazo[1,2-a]pyridin-2-yl-4-methyl-benzamide,
(iii) N-imidazo[1,2-a]pyridin-2-yl-benzamide, and
(iv) N-(7-methylimidazo[1,2-a]pyridin-2-yl)-benzamide.

Preferable examples of compound (I) are as follows. (imidazopyridine derivative)
(I-1)
A compound wherein ring A is aromatic hydrocarbon (preferably, benzene) or aromatic heterocycle (preferably, furan, thiophene, isoxazole, pyridine);

R¹ is
(1) a hydrogen atom,
(2) C₁₋₆ alkyl (e.g., methyl, propyl, isopropyl, tert-butyl, 2-methylbutan-2-yl) optionally substituted by 1 to 3 substituents selected from
   (a) an aromatic heterocyclic group (e.g., tetrazolyl, oxadiazolyl),
   (b) amino optionally mono- or di-substituted by substituent(s) selected from
      (i) C₁₋₆ alkyl (e.g., propyl),
      (ii) C₁₋₆ alkyl-carbonyl (e.g., acetyl, propionyl) optionally substituted by 1 to 3 substituents selected from hydroxy and aromatic heterocyclic group (e.g., imidazolyl),
      (iii) C₆₋₁₄ aryl-carbonyl (e.g., benzoyl),
      (iv) C₁₋₆ alkyl-sulfonyl (e.g., methylsulfonyl), and
      (v) aromatic heterocyclyl-carbonyl (e.g., imidazolylcarbonyl),
   (c) cyano,
   (d) carboxyl,
   (e) C₁₋₆ alkoxy-carbonyl (e.g., methoxycarbonyl, ethoxycarbonyl),
   (f) N-hydroxycarbamoyl,
   (g) carbamoyl optionally mono- or di-substituted by C₁₋₆ alkyl (e.g., methyl, ethyl, propyl) optionally substituted by 1 to 3 substituents selected from (i) cyano, (ii) hydroxy, (iii) an aromatic heterocyclic group (e.g., tetrazolyl), and (iv) a non-aromatic heterocyclic group (e.g., dioxolyl) optionally having 1 to 3 C₁₋₆ alkyl (e.g., methyl, propyl, isopropyl, tert-butyl, 2-methylbutan-2-yl),
   (h) a halogen atom (e.g., fluorine atom),
   (i) di-C₁₋₆ alkylphosphoryl (e.g., diethylphosphoryl),
   (j) hydroxy, and
   (k) a non-aromatic heterocyclic group (e.g., oxoxadiazolyl);
(3) C₃₋₁₀ cycloalkyl (e.g., cyclopropyl, cyclobutyl, cyclopentyl, cyclohexyl) optionally substituted by 1 to 3 substituents selected from
   (a) C₁₋₆ alkoxy-carbonyl (e.g., methoxycarbonyl), and
   (b) carboxyl;
(4) an aromatic heterocyclic group (e.g., pyridyl, furyl, imidazolyl, pyrazolyl);
(5) hydroxy optionally substituted by C₁₋₆ alkyl (e.g., methyl);
(6) amino optionally mono- or di-substituted by substituent(s) selected from
   (a) C₁₋₆ alkyl (e.g., methyl, ethyl), and
   (b) C₁₋₆ alkyl-sulfonyl (e.g., methylsulfonyl);
(7) cyano;
(8) carboxyl;
(9) C₁₋₆ alkoxy-carbonyl (e.g., methoxycarbonyl);
(10) C₁₋₆ alkyl-sulfonyl (e.g., methylsulfonyl);
(11) sulfamoyl optionally mono- or di-substituted by substituent(s) selected from
   (a) C₁₋₆ alkyl (e.g., methyl), and
   (b) C₁₋₆ alkyl-carbonyl (e.g., acetyl); or
(12) a halogen atom (e.g., chlorine atom);

R² is a hydrogen atom,
R³ is a hydrogen atom, C₁₋₆ alkyl (e.g., methyl) or a halogen atom (e.g., bromine atom),
R⁴ is a hydrogen atom,
R⁵ is a hydrogen atom,

R⁶ is
(1) a hydrogen atom;
(2) C₁₋₆ alkyl (e.g., methyl, ethyl) optionally substituted by 1 to 3 substituents selected from
   (a) hydroxy,
   (b) amino optionally mono- or di-substituted by C₁₋₆ alkyl (e.g., methyl), and
   (c) a halogen atom (e.g., fluorine atom);
(3) C₆₋₁₄ aryl (e.g., phenyl) optionally substituted by 1 to 3 substituents selected from
   (a) C₁₋₆ alkyl (e.g., methyl) optionally substituted by 1 to 3 halogen atoms (e.g., fluorine atom)
      (specific example, trifluoromethyl),
   (b) C₁₋₆ alkoxy (e.g., methoxy) optionally substituted by 1 to 3 halogen atoms (e.g., fluorine atom)
      (specific example, methoxy, trifluoromethoxy),
   (c) C₁₋₆ alkoxy-carbonyl (e.g., methoxycarbonyl, ethoxycarbonyl),
   (d) amino optionally mono- or di-substituted by substituent(s) selected from
      (i) C₁₋₆ alkyl (e.g., methyl), and
      (ii) C₁₋₆ alkyl-carbonyl (e.g., acetyl),
   (e) C₁₋₆ alkyl-sulfonyl (e.g., methylsulfonyl),
   (f) cyano, and
   (g) C₁₋₃ alkylenedioxy (e.g., methylenedioxy);
(4) an aromatic heterocyclic group (e.g., thienyl, furyl, pyrrolyl, imidazolyl, pyrazolyl, oxazolyl, triazolyl, pyridyl, pyridazinyl, indolyl) optionally substituted by 1 to 3 substituents selected from
   (a) C₁₋₆ alkyl (e.g., methyl, ethyl, isopropyl, tert-butyl) optionally substituted by 1 to 3 substituents selected from
      (i) hydroxy optionally substituted by a non-aromatic heterocyclic group (e.g., tetrahydropyranyl), and
      (ii) cyano,
   (b) C₆₋₁₄ aryl (e.g., phenyl),
   (c) an aromatic heterocyclic group (e.g., pyridyl),
   (d) C₁₋₆ alkoxy (e.g., methoxy), and
   (e) a halogen atom (e.g., fluorine atom, chlorine atom, bromine atom);
(5) a non-aromatic heterocyclic group (e.g., pyrrolidinyl, piperidinyl, homopiperidinyl, tetrahydropyridyl, piperazinyl, morpholinyl, thiomorpholinyl, tetrahydroisoquinolinyl, dihydroisoindolyl, tetrahydrobenzoazepinyl) optionally substituted by 1 to 3 substituents selected from
   (a) C₁₋₆ alkyl (e.g., methyl, ethyl) optionally substituted by 1 to 3 substituents selected from
      (i) C₆₋₁₄ aryl (e.g., phenyl),
      (ii) an aromatic heterocyclic group (e.g., pyridyl), and
      (iii) hydroxy optionally substituted by C₁₋₆ alkyl (e.g., methyl),
   (b) C₆₋₁₄ aryl (e.g., phenyl) optionally substituted by 1 to 3 substituents selected from C₁₋₆ alkoxy (e.g., methoxy), and a halogen atom (e.g., fluorine atom),
   (c) an aromatic heterocyclic group (e.g., pyridyl, pyrimidinyl),
   (d) hydroxy,
   (e) amino optionally mono- or di-substituted by C₁₋₆ alkyl-carbonyl (e.g., acetyl),
   (f) carboxyl,
   (g) carbamoyl optionally mono- or di-substituted by C₇₋₁₃ aralkyl (e.g., benzyl),
   (h) C₁₋₆ alkyl-sulfonyl (e.g., ethylsulfonyl), and
   (i) C₆₋₁₄ aryl-sulfonyl (e.g., benzenesulfonyl);
(6) hydroxy optionally substituted by a substituent selected from
   (a) C₆₋₁₄ aryl (e.g., phenyl) optionally substituted by 1 to 3 substituents selected from
      (i) C₁₋₆ alkyl (e.g., ethyl, propyl, butyl) optionally substituted by 1 to 3 substituents selected from
         (A) a halogen atom (e.g., fluorine atom),
         (B) amino optionally mono- or di-substituted by C₁₋₆ alkyl (e.g., methyl), and
         (C) an aromatic heterocyclic group (e.g., imidazolyl) optionally substituted by 1 to 3 C₁₋₆ alkyl (e.g., methyl) optionally substituted by 1 to 3 hydroxy (specific example, hydroxymethyl),
      (ii) aromatic heterocyclic group (e.g., imidazolyl, pyrazolyl) optionally substituted by 1 to 3 C₁₋₆ alkyl (e.g., methyl),
      (iii) a non-aromatic heterocyclic group (e.g., morpholinyl),
      (iv) C₁₋₆ alkoxy (e.g., methoxy) optionally substituted by 1 to 3 substituents selected from (A) a halogen atom (e.g., fluorine atom) and (B) carboxyl,
      (v) amino optionally mono- or di-substituted by substituent(s) selected from
         (A) C₁₋₆ alkyl (e.g., methyl, ethyl),
         (B) C₁₋₆ alkyl-carbonyl (e.g., acetyl, tert-butylcarbonyl),
         (C) C₃₋₁₀ cycloalkyl-carbonyl (e.g., cyclopropylcarbonyl, cyclohexylcarbonyl),
         (D) C₆₋₁₄ aryl-carbonyl (e.g., benzoyl) optionally substituted by 1 to 3 substituents selected from (1') C₁₋₆ alkyl (e.g., methyl) optionally substituted by 1 to 3 halogen atoms (e.g., fluorine atom) (specific example, trifluoromethyl), and (2') a halogen atom (e.g., fluorine atom, chlorine atom), and
         (E) aromatic heterocyclyl-carbonyl (e.g., thienylcarbonyl, furylcarbonyl, pyridylcarbonyl, pyrazolylcarbonyl, thiadiazolylcarbonyl, isoxazolylcarbonyl) optionally substituted by 1 to 3 C₁₋₆ alkyl (e.g., methyl),
      (vi) carboxyl, and
      (vii) non-aromatic heterocyclyl-carbonyl (e.g., pyrrolidinylcarbonyl) optionally substituted by 1 to 3 substituents selected from C₁₋₆ alkyl (e.g., methyl) and oxo,
   (b) an aromatic heterocyclic group (e.g., pyridyl, benzofuranyl) optionally substituted by 1 to 3 substituents selected from
      (i) C₁₋₆ alkyl (e.g., methyl),
      (ii) C₁₋₆ alkyl-carbonyl (e.g., acetyl), and
      (iii) carboxyl, and
   (c) a non-aromatic heterocyclic group (e.g., pyrrolidinyl, piperidinyl, chromenyl) optionally substituted by 1 to 3 substituents selected from C₁₋₆ alkyl (e.g., methyl) and oxo;
(7) mercapto optionally substituted by
   (a) an aromatic heterocyclic group (e.g., pyridyl, imidazolyl, thiazolyl, thiadiazolyl, triazolyl) optionally substituted by 1 to 3 substituents selected from
      (i) C₁₋₆ alkyl (e.g., methyl) optionally substituted by 1 to 3 halogen atoms (e.g., fluorine atom)
         (specific example, methyl, trifluoromethyl),
      (ii) C₆₋₁₄ aryl (e.g., phenyl), and
      (iii) an aromatic heterocyclic group (e.g., pyridyl);
(8) amino optionally mono- or di-substituted by substituent(s) selected from
   (a) C₁₋₆ alkyl (e.g., methyl, ethyl, propyl) optionally substituted by 1 to 3 substituents selected from
      (i) an aromatic heterocyclic group (e.g., pyridyl, imidazolyl),
      (ii) hydroxy,
      (iii) C₁₋₆ alkoxy (e.g., methoxy), and
      (iv) amino optionally mono- or di-substituted by C₁₋₆ alkyl (e.g., methyl),
   (b) C₃₋₁₀ cycloalkyl (e.g., cyclopropyl, cyclopentyl, cyclohexyl),
   (c) C₇₋₁₃ aralkyl (e.g., benzyl), and
   (d) a non-aromatic heterocyclic group (e.g., pyrrolidinyl, piperidinyl) optionally substituted by 1 to 3 C₁₋₆ alkyl (e.g., methyl);
(9) cyano;
(10) C₁₋₆ alkyl-carbonyl (e.g., acetyl) optionally substituted by 1 to 3 hydroxy;
(11) a halogen atom (e.g., fluorine atom, chlorine atom, bromine atom, iodine atom); or
(12) hydroxy substituted by C₆₋₁₄ aryl (e.g., phenyl) fused with C₃₋₁₀ cycloalkane (e.g., cycloheptane) optionally substituted by oxo (specific example, 5-oxotetrahydrobenzoannulen-2-yl);

X is =C(Z)-,
Z is a hydrogen atom or C₁₋₆ alkyl (e.g., methyl) optionally substituted by 1 to 3 hydroxy,
or, when X is =C(Z)-, Z and R⁶ are bonded to each other to optionally form, together with the carbon atom bonded thereto, an aromatic hydrocarbon (e.g., benzene) or heterocycle (e.g., dihydrofuran, imidazole), each being optionally substituted by 1 to 3 C₁₋₆ alkyl.

Specifically, preferable examples include
4-tert-butyl-N-(1,2-dihydroflo[2,3-e]imidazo[1,2-a]pyridin-7-yl)benzamide,
4-tert-butyl-N-[6-(1H-imidazol-1-yl)imidazo[1,2-a]pyridin-2-yl]benzamide,
6-tert-butyl-N-[6-(1H-imidazol-1-yl)imidazo[1,2-a]pyridin-2-yl]nicotinamide,
4-(2-hydroxy-1,1-dimethylethyl)-N-[6-(1H-imidazol-1-yl)imidazo[1,2-a]pyridin-2-yl]benzamide,
4-tert-butyl-N-[6-(1H-pyrazol-4-yl)imidazo[1,2-a]pyridin-2-yl]benzamide,
4-(1-cyano-1-methylethyl)-N-[6-(1H-imidazol-1-yl)imidazo[1,2-a]pyridin-2-yl]benzamide.

### (imidazopyridine derivative)

### (I-2)

A compound wherein ring A is aromatic hydrocarbon (preferably, benzene) or 5-membered aromatic heterocycle (preferably, furan, thiophene, isoxazole);

R¹ is
(1) a hydrogen atom,
(2) C₁₋₆ alkyl (e.g., methyl, propyl, isopropyl, tert-butyl, 2-methylbutan-2-yl) optionally substituted by 1 to 3 substituents selected from
   (a) an aromatic heterocyclic group (e.g., tetrazolyl, oxadiazolyl),
   (b) amino optionally mono- or di-substituted by substituent(s) selected from
      (i) C₁₋₆ alkyl (e.g., propyl),
      (ii) C₁₋₆ alkyl-carbonyl (e.g., acetyl, propionyl) optionally substituted by 1 to 3 substituents selected from hydroxy and aromatic heterocyclic group (e.g., imidazolyl),
      (iii) C₆₋₁₄ aryl-carbonyl (e.g., benzoyl),
      (iv) C₁₋₆ alkyl-sulfonyl (e.g., methylsulfonyl), and
      (v) aromatic heterocyclyl-carbonyl (e.g., imidazolylcarbonyl),
   (c) cyano,
   (d) carboxyl,
   (e) C₁₋₆ alkoxy-carbonyl (e.g., methoxycarbonyl, ethoxycarbonyl),
   (f) N-hydroxycarbamoyl,
   (g) carbamoyl optionally mono- or di-substituted by C₁₋₆ alkyl (e.g., methyl, ethyl, propyl) optionally substituted by 1 to 3 substituents selected from (i) cyano, (ii) hydroxy, (iii) an aromatic heterocyclic group (e.g., tetrazolyl), and (iv) a non-aromatic heterocyclic group (e.g., dioxolyl) optionally having 1 to 3 C₁₋₆ alkyl (e.g., methyl, propyl, isopropyl, tert-butyl, 2-methylbutan-2-yl),
   (h) a halogen atom (e.g., fluorine atom),
   (i) di-C₁₋₆ alkylphosphoryl (e.g., diethylphosphoryl),
   (j) hydroxy, and
   (k) a non-aromatic heterocyclic group (e.g., oxoxadiazolyl);
(3) C₃₋₁₀ cycloalkyl (e.g., cyclopropyl, cyclobutyl, cyclopentyl, cyclohexyl) optionally substituted by 1 to 3 substituents selected from
   (a) C₁₋₆ alkoxy-carbonyl (e.g., methoxycarbonyl), and
   (b) carboxyl;
(4) an aromatic heterocyclic group (e.g., pyridyl, furyl, imidazolyl, pyrazolyl);
(5) hydroxy optionally substituted by C₁₋₆ alkyl (e.g., methyl);
(6) amino optionally mono- or di-substituted by substituent(s) selected from
   (a) C₁₋₆ alkyl (e.g., methyl, ethyl), and
   (b) C₁₋₆ alkyl-sulfonyl (e.g., methylsulfonyl);
(7) cyano;
(8) carboxyl;
(9) C₁₋₆ alkoxy-carbonyl (e.g., methoxycarbonyl);
(10) C₁₋₆ alkyl-sulfonyl (e.g., methylsulfonyl);
(11) sulfamoyl optionally mono- or di-substituted by substituent(s) selected from
   (a) C₁₋₆ alkyl (e.g., methyl), and
   (b) C₁₋₆ alkyl-carbonyl (e.g., acetyl); or
(12) a halogen atom (e.g., chlorine atom);

R² is a hydrogen atom,
R³ is a hydrogen atom, C₁₋₆ alkyl (e.g., methyl) or a halogen atom (e.g., bromine atom),
R⁴ is a hydrogen atom,
R⁵ is a hydrogen atom,

R⁶ is
(1) a hydrogen atom;
(2) C₁₋₆ alkyl (e.g., methyl, ethyl) optionally substituted by 1 to 3 substituents selected from
   (a) hydroxy,
   (b) amino optionally mono- or di-substituted by C₁₋₆ alkyl (e.g., methyl), and
   (c) a halogen atom (e.g., fluorine atom);
(3) C₆₋₁₄ aryl (e.g., phenyl) optionally substituted by 1 to 3 substituents selected from
   (a) C₁₋₆ alkyl (e.g., methyl) optionally substituted by 1 to 3 halogen atoms (e.g., fluorine atom)
      (specific example, trifluoromethyl),
   (b) C₁₋₆ alkoxy (e.g., methoxy) optionally substituted by 1 to 3 halogen atoms (e.g., fluorine atom)
      (specific example, methoxy, trifluoromethoxy),
   (c) C₁₋₆ alkoxy-carbonyl (e.g., methoxycarbonyl, ethoxycarbonyl),
   (d) amino optionally mono- or di-substituted by substituent(s) selected from
      (i) C₁₋₆ alkyl (e.g., methyl), and
      (ii) C₁₋₆ alkyl-carbonyl (e.g., acetyl),
   (e) C₁₋₆ alkyl-sulfonyl (e.g., methylsulfonyl),
   (f) cyano, and
   (g) C₁₋₃ alkylenedioxy (e.g., methylenedioxy);
(4) an aromatic heterocyclic group (e.g., thienyl, furyl, pyrrolyl, imidazolyl, pyrazolyl, oxazolyl, triazolyl, pyridyl, pyridazinyl, indolyl) optionally substituted by 1 to 3 substituents selected from
   (a) C₁₋₆ alkyl (e.g., methyl, ethyl, isopropyl, tert-butyl) optionally substituted by 1 to 3 substituents selected from
      (i) hydroxy optionally substituted by a non-aromatic heterocyclic group (e.g., tetrahydropyranyl), and
      (ii) cyano,
   (b) C₆₋₁₄ aryl (e.g., phenyl),
   (c) an aromatic heterocyclic group (e.g., pyridyl),
   (d) C₁₋₆ alkoxy (e.g., methoxy), and
   (e) a halogen atom (e.g., fluorine atom, chlorine atom, bromine atom);
(5) a non-aromatic heterocyclic group (e.g., pyrrolidinyl, piperidinyl, homopiperidinyl, tetrahydropyridyl, piperazinyl, morpholinyl, thiomorpholinyl, tetrahydroisoquinolinyl, dihydroisoindolyl, tetrahydrobenzoazepinyl) optionally substituted by 1 to 3 substituents selected from
   (a) C₁₋₆ alkyl (e.g., methyl, ethyl) optionally substituted by 1 to 3 substituents selected from
      (i) C₆₋₁₄ aryl (e.g., phenyl),
      (ii) an aromatic heterocyclic group (e.g., pyridyl), and
      (iii) hydroxy optionally substituted by C₁₋₆ alkyl (e.g., methyl),
   (b) C₆₋₁₄ aryl (e.g., phenyl) optionally substituted by 1 to 3 substituents selected from C₁₋₆ alkoxy (e.g., methoxy), and a halogen atom (e.g., fluorine atom),
   (c) an aromatic heterocyclic group (e.g., pyridyl, pyrimidinyl),
   (d) hydroxy,
   (e) amino optionally mono- or di-substituted by C₁₋₆ alkyl-carbonyl (e.g., acetyl),
   (f) carboxyl,
   (g) carbamoyl optionally mono- or di-substituted by C₇₋₁₃ aralkyl (e.g., benzyl),
   (h) C₁₋₆ alkyl-sulfonyl (e.g., ethylsulfonyl), and
   (i) C₆₋₁₄ aryl-sulfonyl (e.g., benzenesulfonyl);
(6) hydroxy optionally substituted by a substituent selected from
   (a) C₆₋₁₄ aryl (e.g., phenyl) optionally substituted by 1 to 3 substituents selected from
      (i) C₁₋₆ alkyl (e.g., ethyl, propyl, butyl) optionally substituted by 1 to 3 substituents selected from
         (A) a halogen atom (e.g., fluorine atom),
         (B) amino optionally mono- or di-substituted by C₁₋₆ alkyl (e.g., methyl), and
         (C) an aromatic heterocyclic group (e.g.,
         imidazolyl) optionally substituted by 1 to 3 C₁₋₆ alkyl (e.g., methyl) optionally substituted by 1 to 3 hydroxy (specific example, hydroxymethyl),
      (ii) aromatic heterocyclic group (e.g., imidazolyl, pyrazolyl) optionally substituted by 1 to 3 C₁₋₆ alkyl (e.g., methyl),
      (iii) a non-aromatic heterocyclic group (e.g., morpholinyl),
      (iv) C₁₋₆ alkoxy (e.g., methoxy) optionally substituted by 1 to 3 substituents selected from (A) a halogen atom (e.g., fluorine atom) and (B) carboxyl,
      (v) amino optionally mono- or di-substituted by substituent(s) selected from
         (A) C₁₋₆ alkyl (e.g., methyl, ethyl),
         (B) C₁₋₆ alkyl-carbonyl (e.g., acetyl, tert-butylcarbonyl),
         (C) C₃₋₁₀ cycloalkyl-carbonyl (e.g., cyclopropylcarbonyl, cyclohexylcarbonyl),
         (D) C₆₋₁₄ aryl-carbonyl (e.g., benzoyl) optionally substituted by 1 to 3 substituents selected from (1') C₁₋₆ alkyl (e.g., methyl) optionally substituted by 1 to 3 halogen atoms (e.g., fluorine atom) (specific example, trifluoromethyl), and (2') a halogen atom (e.g., fluorine atom, chlorine atom), and
         (E) aromatic heterocyclyl-carbonyl (e.g., thienylcarbonyl, furylcarbonyl, pyridylcarbonyl, pyrazolylcarbonyl, thiadiazolylcarbonyl, isoxazolylcarbonyl) optionally substituted by 1 to 3 C₁₋₆ alkyl (e.g., methyl),
      (vi) carboxyl, and
      (vii) non-aromatic heterocyclyl-carbonyl (e.g., pyrrolidinylcarbonyl) optionally substituted by 1 to 3 substituents selected from C₁₋₆ alkyl (e.g., methyl) and oxo,
   (b) an aromatic heterocyclic group (e.g., pyridyl, benzofuranyl) optionally substituted by 1 to 3 substituents selected from
      (i) C₁₋₆ alkyl (e.g., methyl),
      (ii) C₁₋₆ alkyl-carbonyl (e.g., acetyl), and
      (iii) carboxyl, and
   (c) a non-aromatic heterocyclic group (e.g., pyrrolidinyl, piperidinyl, chromenyl) optionally substituted by 1 to 3 substituents selected from C₁₋₆ alkyl (e.g., methyl) and oxo;
(7) mercapto optionally substituted by
   (a) an aromatic heterocyclic group (e.g., pyridyl, imidazolyl, thiazolyl, thiadiazolyl, triazolyl) optionally substituted by 1 to 3 substituents selected from
      (i) C₁₋₆ alkyl (e.g., methyl) optionally substituted by 1 to 3 halogen atoms (e.g., fluorine atom)
         (specific example, methyl, trifluoromethyl),
      (ii) C₆₋₁₄ aryl (e.g., phenyl), and
      (iii) an aromatic heterocyclic group (e.g., pyridyl);
(8) amino optionally mono- or di-substituted by substituent(s) selected from
   (a) C₁₋₆ alkyl (e.g., methyl, ethyl, propyl) optionally substituted by 1 to 3 substituents selected from
      (i) an aromatic heterocyclic group (e.g., pyridyl, imidazolyl),
      (ii) hydroxy,
      (iii) C₁₋₆ alkoxy (e.g., methoxy), and
      (iv) amino optionally mono- or di-substituted by C₁₋₆ alkyl (e.g., methyl),
   (b) C₃₋₁₀ cycloalkyl (e.g., cyclopropyl, cyclopentyl, cyclohexyl),
   (c) C₇₋₁₃ aralkyl (e.g., benzyl), and
   (d) a non-aromatic heterocyclic group (e.g., pyrrolidinyl, piperidinyl) optionally substituted by 1 to 3 C₁₋₆ alkyl (e.g., methyl);
(9) cyano;
(10) C₁₋₆ alkyl-carbonyl (e.g., acetyl) optionally substituted by 1 to 3 hydroxy;
(11) a halogen atom (e.g., fluorine atom, chlorine atom, bromine atom, iodine atom); or
(12) hydroxy substituted by C₆₋₁₄ aryl (e.g., phenyl) fused with C₃₋₁₀ cycloalkane (e.g., cycloheptane) optionally substituted by oxo (specific example, 5-oxotetrahydrobenzoannulen-2-yl);

X is =N-,
Z is a hydrogen atom or C₁₋₆ alkyl (e.g., methyl) optionally substituted by 1 to 3 hydroxy,
or, when X is =C(Z)-, Z and R⁶ are bonded to each other to optionally form, together with the carbon atom bonded thereto, an aromatic hydrocarbon (e.g., benzene) or heterocycle (e.g., dihydrofuran, imidazole), each being optionally substituted by 1 to 3 C₁₋₆ alkyl.

Specifically, preferable examples include 4-tert-butyl-N-[6-(1H-imidazol-1-yl)imidazo[1,2-b]pyridazin-2-yl]benzamide.

As a salt of compound (I), for example, a pharmacologically acceptable salt and the like are preferable. For example, a salt with inorganic base, a salt with organic base, a salt with inorganic acid, a salt with organic acid, a salt with basic or acidic amino acid and the like can be mentioned.
Preferable examples of salts with inorganic base include alkali metal salt such as sodium salt, potassium salt and the like, alkaline earth metal salt such as calcium salt, magnesium salt and the like, and aluminum salt, ammonium salt and the like.
Preferable examples of salts with organic base include salts with trimethylamine, triethylamine, pyridine, picoline, ethanolamine, diethanolamine, triethanolamine, dicyclohexylamine, N,N'-dibenzylethylenediamine and the like.
Preferable examples of salts with inorganic acid include salts with hydrochloric acid, hydrobromic acid, nitric acid, sulfuric acid, phosphoric acid and the like.
Preferable examples of salts with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.
Preferable examples of salts with basic amino acid include salts with arginine, lysine, ornithine and the like, and preferable examples of salts with acidic amino acid include salts with aspartic acid, glutamic acid and the like.

A prodrug of compound (I) refers to a compound which is converted to compound (I) as a result of a reaction with an enzyme, gastric acid, etc. under physiological conditions in vivo, thus a compound that undergoes enzymatic oxidation, reduction, hydrolysis, etc. to convert into compound (I) and a compound that undergoes hydrolysis and the like by gastric acid, etc. to convert into compound (I). As a prodrug for compound (I), a compound obtained by subjecting an amino group in compound (I) to acylation, alkylation or phosphorylation (e.g., a compound obtained by subjecting an amino group in compound (I) to eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxy group in compound (I) to acylation, alkylation, phosphorylation and boration (e.g., a compound obtained by subjecting a hydroxy group in compound (I) to acetylation, palmitoylation, propanoylation, pivaloylation, succinylation, fumarylation, alanylation, dimethylaminomethylcarbonylation, etc.); a compound obtained by subjecting a carboxyl group in compound (I) to esterification or amidation (e.g., a compound obtained by subjecting a carboxyl group in compound (I) to ethylesterification, phenylesterification, carboxymethylesterification, dimethylaminomethylesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonylethylesterification and methylamidation, etc.) and the like can be mentioned. Any of these compounds can be produced from compound (I) by a method known per se.
A prodrug for compound (I) may also be one which is converted to compound (I) under physiological conditions as described in "IYAKUHIN no KAIHATSU (Development of Pharmaceuticals)", Vol. 7, Design of Molecules, p. 163-198, Published by HIROKAWA SHOTEN (1990).
Compound (I) may be labeled with an isotope (e.g., ³_{H}, ¹⁴_{C}, ³⁵S, ¹²⁵I and the like).
Compound (I) may be an anhydrate or a hydrate.

compound (I) or a prodrug thereof (hereinafter sometimes to be simply abbreviated as the compound of the present invention) has low toxicity, and can be used as-is or in the form of a pharmaceutical composition by mixing with a pharmacologically acceptable carrier etc. to mammals (e.g., human, mouse, rat, rabbit, dog, cat, bovine, horse, swine, monkey) as an agent for the prophylaxis or treatment of various diseases mentioned below.
As pharmacologically acceptable carriers, various organic or inorganic carrier substances conventionally used as preparation materials can be used. For example, suitable amounts of additives such as excipient, lubricant, binder and disintegrant for solid preparations, or solvent, solubilizing agent, suspending agent, isotonicity agent, buffer and soothing agent for liquid preparations, and where necessary, conventional preservative, antioxidant, colorant, sweetening agent and the like can be added as necessary.
Preferable examples of the excipient include lactose, sucrose, D-mannitol, D-sorbitol, starch, gelatinated starch, dextrin, crystalline cellulose, low-substituted hydroxypropylcellulose, sodium carboxymethylcellulose, gum arabic, pullulan, light anhydrous silicic acid, synthesis aluminum silicate, magnesium alumino metasilicate and the like.
Preferable examples of the lubricant include magnesium stearate, calcium stearate, talc, colloidal silica and the like.
Preferable examples of the binder include gelatinated starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose, polyvinylpyrrolidone and the like.
Preferable examples of the disintegrant include lactose, sucrose, starch, carboxymethylcellulose, calcium carboxymethylcellulose, croscarmellose sodium, sodium carboxymethyl starch, light anhydrous silicic acid, low-substituted hydroxypropylcellulose and the like.

Preferable examples of the solvent include water for injection, physiological brine, Ringer's solution, alcohol, propylene glycol, polyethylene glycol, sesame oil, corn oil, olive oil, cottonseed oil and the like.
Preferable examples of the solubilizing agents include polyethylene glycol, propylene glycol, D-mannitol, trehalose, benzyl benzoate, ethanol, trisaminomethane, cholesterol, triethanolamine, sodium carbonate, sodium citrate sodium salicylate, sodium acetate and the like.
Preferable examples of the suspending agent include surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like, polysorbates, polyoxyethylene hydrogenated castor oil and the like.
Preferable examples of the isotonicity agent include sodium chloride, glycerol, D-mannitol, D-sorbitol, glucose and the like.
Preferable examples of the buffer include buffers of phosphate, acetate, carbonate, citrate etc. and the like.
Preferable examples of the soothing agent include benzyl alcohol and the like.
Preferable examples of the preservative include p-oxybenzoates, chlorobutanol, benzyl alcohol, phenethyl alcohol, dehydroacetic acid, sorbic acid and the like.
Preferable examples of the antioxidant include sulfite, ascorbic acid salt and the like.
Preferable examples of the colorant include aqueous food tar color (e.g., Food Color Red Nos. 2 and 3, Food Color Yellow Nos. 4 and 5, Food Color Blue Nos. 1 and 2 and the like food colors), water insoluble lake dye (e.g., aluminum salt of the aforementioned aqueous food tar color), natural dye (e.g., β-carotene, chlorophyll, red iron oxide) and the like.
Preferable examples of the sweetening agent include saccharin sodium, dipotassium glycyrrhizinate, aspartame, stevia and the like.

Examples of the dosage form of the aforementioned pharmaceutical composition include oral preparations such as tablet (including sublingual tablet, orally disintegrating tablet), capsule (including soft capsule, microcapsule), granule, powder, troche, syrup, emulsion, suspension and the like; and parenteral agent such as injection (e.g., subcutaneous injection, intravenous injection, intramuscular injection, intraperitoneal injection, drip infusion), external preparation (e.g., dermal preparation, ointment), suppository (e.g., rectal suppository, vaginal suppository), pellet, nasal preparations, pulmonary preparation (inhalant), eye drop and the like, which can be respectively safely administered orally or parenterally.
These preparations may be a release control preparation (e.g., sustained-release microcapsule) such as an immediate-release preparation, a sustained-release preparation and the like.
The pharmaceutical composition can be produced a method conventionally used in the field of preparation formulation, for example, the method described in the Japanese Pharmacopoeia, and the like.
While the content of the compound of the present invention in the pharmaceutical composition varies depending on the dosage form, dose of the compound of the present invention and the like, it is, for example, about 0.1 - 100 wt%.

Since the compound of the present invention shows a superior ASKlinhibitory action, it is also useful as a safe pharmaceutical product based on such action.
For example, the pharmaceutical agent of the present invention containing compound (I) is useful as an agent for the prophylaxis or treatment of apoptosis or inflammation-associated diseases in mammals (e.g., mouse, rat, hamster, rabbit, cat, dog, bovine, sheep, monkey, human, etc.). Here, the "apoptosis or inflammation-associated diseases" refer to a disease or pathology resulting from insufficient or abnormal promotion of apoptosis induction or inflammatory cytokine production, or a disease or pathology consequently producing such conditions.

Examples of the "apoptosis or inflammation-associated diseases" include diseases caused by apoptosis induction or abnormal promotion of inflammatory cytokine production, such as virus infections (e.g., AIDS, influenza, fever of unknown origin etc.), endocrine diseases (e.g., hormone deficiency, cytokine deficiency etc.), blood diseases (e.g., hypocytosis, renal anemia etc.), organ hypoplasia (e.g., thyroid gland atrophy, cleft palate etc.), organ graft rejection, graft-versus-host disease, immunodeficiency, neurodegenerative diseases (e.g., polyglutamine disease, Alzheimer's disease, Parkinson's disease, amyotropic lateral sclerosis retinitis, prion disease, cerebellar degeneration, Creutzfeldt-Jakob disease, Huntington chorea, multiple sclerosis, spinocerebellar ataxia etc.), mental diseases (e.g., schizophrenia, depression, anxiety disorder etc.), cerebrovascular disorders (e.g., brain thrombosis, brain embolism, transient cerebral ischemic attack etc.), radiation disorder, ultraviolet injury (e.g., sunburns etc.), poisoning diseases (e.g., renal tubular cell injury by heavy metal, hepatocyte injury by alcohol etc.), malnutrition (e.g., thymus atrophy due to lack of vitamin, trace elements etc.), inflammatory diseases (e.g., acute pancreatitis, arthritis, periodontal disease, colitis, chronic obliterative pulmonary diseases (COPD) etc.), ischemic neuropathy, blood vessel system diseases (e.g., arteriosclerosis etc.), respiratory diseases (e.g., interstitial pneumonia, pulmonary fibrosis etc.), articular diseases (e.g., osteoarthritis etc.) and the like.

Other examples of the "apoptosis or inflammation-associated diseases" include diseases caused by apoptosis induction or insufficient inflammatory cytokine production such as cancer (e.g., leukemia, esophagus cancer, gastric cancer, colorectal cancer, rectal cancer, lung cancer, liver cancer, kidney cancer, breast cancer, uterine cancer, ovarian cancer, prostate cancer, melanoma, myeloma, osteosarcoma, brain tumor etc.), autoimmune diseases (e.g., systemic lupus erythematosus, scleroderma, chronic rheumatoid arthritis, Sjogren's syndrome, multiple sclerosis, type-1 diabetes, psoriasis, ulcerative colitis, idiopathic thrombocytopenic purpura, Crohn's disease, glomerulonephritis etc.), virus infections (e.g., hemorrhagic fever, T cell leukemia, Kaposi's sarcoma, infectious mononucleosis, lymphoma, epipharynx cancer, fallopian tube cancer, skin cancer, hepatitis, liver cancer etc.), endocrine diseases (e.g., hormone excess, hypercytokine disease etc.), blood diseases (e.g., polycythemia, B-cell lymphoma, polycythemia etc.), organ hyperplasia (e.g., hermaphroditism, undescended testis, teratoma, nephroblastoma, polycystic kidney, cardiac/aortic malformation, syndactyly etc.), post-angioplastic restenosis, recurrence after cancer resection and the like.

The compound of the present invention is particularly effective for the prophylaxis or treatment of diabetes (e.g., type-1 diabetes, type-2 diabetes, gestational diabetes) and complications thereof (e.g., diabetic nephropathy, diabetic retinopathy, diabetic neuropathy etc.), obesity, and impaired glucose tolerance. Moreover, the compound of the present invention is also effective for the prophylaxis or treatment of cardiac failure (congestive heart failure and the like), ischemic brain diseases (e.g., cerebral infarction, cerebral apoplexy etc.), ischemic cardiac diseases (e.g., angina pectoris, myocardial infarction etc.), anemia (Fanconi anemia and the like), and hypertension (essential hypertension, lung hypertension and the like).

As used herein, the "prophylaxis" of the above-mentioned diseases means, for example, administration of a pharmaceutical containing the compound of the present invention to patients before onset of a disease but having a high risk of the onset due to some factor associated with the disease or patients who developed the disease but without subjective symptoms, or administration of a pharmaceutical containing the compound of the present invention to patients having a risk of recurrence of disease after treatment of the disease.

The dose of the compound of the present invention varies depending on the administration subject, route of administration, target disease, symptoms, etc. For example, when it is administered orally to an adult patient with diabetes, its dose is, for example, 0.01 to 100 mg/kg body weight per day, preferably 0.05 to 30 mg/kg body weight per day, and more preferably 0.1 to 10 mg/kg body weight per day. This amount is desirably administered in one to 3 portions daily.

The compound of the present invention can be used in combination with a pharmaceutical agent such as therapeutic agent for diabetes, therapeutic agents for diabetic complications, therapeutic agent for hyperlipidemia, antihypertensive agent, antiobesity agent, diuretic, chemotherapeutic agent, immunotherapeutic agent, antithrombotic agent, therapeutic agent for osteoporosis, antidementia agent, erectile dysfunction improver, therapeutic agent for incontinence·frequent urination, therapeutic agent for dysuria and the like (hereinafter to be abbreviated as a concomitant drug). The time of administration of the compound of the present invention and that of the concomitant drug are not limited, and they may be administered simultaneously or in a staggered manner to the administration subject. Furthermore, the compound of the present invention and a concomitant drug may be administered as two kinds of preparations containing each active ingredient, or may be administered as a single preparation containing both active ingredients.
The dose of the concomitant drug can be appropriately determined based on the dose employed in clinical situations. The mixing ratio of the compound of the present invention and a concomitant drug can be appropriately determined depending on the administration subject, administration route, target disease, symptom, combination and the like. When the subject of administration is human, for example, a concomitant drug can be used in 0.01 - 100 parts by weight relative to 1 part by weight of the compound of the present invention.

Examples of the therapeutic agents for diabetes include insulin preparations (e.g., animal insulin preparations extracted from pancreas of bovine and swine; human insulin preparations genetically synthesized using *Escherichia coli,* yeast; zinc insulin; protamine zinc insulin; fragment or derivative of insulin (e.g., INS-1 etc.), oral insulin preparation and the like),insulin sensitizer (e.g., pioglitazone or a salt thereof (preferably hydrochloride), rosiglitazone or a salt thereof (preferably maleate), Netoglitazone, Edaglitazone (BM-13.1258), Rivoglitazone (CS-011), FK-614, WO01/38325 compounds described in, Tesaglitazar (AZ-242), Ragaglitazar (NN-622), Muraglitazar (BMS-298585), Metaglidasen (MBX-102), Naveglitazar (LY-519818), MX-6054, LY-510929, Balaglitazone (NN-2344), T-131 or a salt thereof, THR-0921), PPARγ agonist, PPARγ antagonist, PPARγ/α dual agonist, α-glucosidase inhibitor (e.g., voglibose, acarbose, miglitol, emiglitate), biguanide (e.g., phenformin, metformin, buformin or a salt thereof (e.g., hydrochloride, fumarate, succinate)), insulin secretagogue [sulfonylurea (e.g., tolbutamide, glibenclamide, gliclazide, chlorpropamide, tolazamide, acetohexamide, glyclopyramide, glimepiride, glipizide, glybuzole), repaglinide, senaglinide, nateglinide, mitiglinide or calcium hydrate thereof], GPR40 agonist, GLP-1receptor agonist [e.g., GLP-1, GLP-1MR agent, NN-2211, AC-2993 (exendin-4), BIM-51077, Aib(8,35)hGLP-1(7,37)NH₂, CJC-1131], amylin agonist (e.g., pramlintide), phosphotyrosine phosphatase inhibitor (e.g., sodium vanadate), dipeptidyl peptidase IV inhibitor (e.g., NVP-DPP-278, PT-100, P32/98, Alogliptin benzoate, Vildagliptin (LAF-237), P93/01, TS-021, Sitagliptin phosphate (MK-431), Saxagliptin (BMS-477118), E-3024, TA-6666, 823093, 825964, 815541), β3 agonist (e.g., AJ-9677), gluconeogenesis inhibitors (e.g., glycogen phosphorylase inhibitors, glucose-6-phosphatase inhibitors, glucagon antagonists etc.), SGLT (sodium-glucose cotransporter) inhibitors (e.g., T-1095 etc.),11β**-**HSDl inhibitor (e.g., BVT-3498 etc.), adiponectin or agonists thereof, IKK inhibitors (e.g., AS-2868 etc.), leptin resistance improving drugs, somatostatin receptor agonists (compounds described in WO01/25228, WO03/42204, compounds described in WO01/25228 WO03/42204, WO98/44921, WO98/45285, WO99/22735 etc.), and the like.

Examples of the therapeutic agents for diabetic complications include aldose reductase inhibitors (e.g., Tolrestat, Epalrestat, Zenarestat, Zopolrestat, minalrestat, fidarestat, CT-112), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole etc.)), nerve regeneration promoters (e.g., Y-128), PKC inhibitors (e.g., ruboxistaurin mesylate (ruboxistaurin mesylate)), AGE inhibitors (e.g., ALT946, pimagedine, N-phenacylthiazolium bromide (ALT766), ALT-711, EXO-226, Pyridorin, pyridoxamine), active oxygen scavengers (e.g., thioctic acid etc.), cerebral vasodilators (e.g., tiapuride, mexiletine), somatostatin receptor agonists (e.g., BIM23190), and apoptosis signal regulating kinase-1 (ASK-1) inhibitor.
Examples of the therapeutic agent for hyperlipidemia include HMG-CoA reductase inhibitors (e.g., pravastatin, simvastatin, lovastatin, atorvastatin, fluvastatin, pitavastatin, rosuvastatin or a salt thereof (e.g., sodium salt, calcium salt)), squalene synthase inhibitors (e.g., compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), ACAT inhibitors (e.g., Avasimibe, Eflucimibe etc.), anion exchange resins (e.g., colestyramine etc.), probucol, nicotinic acid drugs (e.g., nicomol, niceritrol etc.), ethyl icosapentate, plant sterols (e.g., soysterol, γ-oryzanol etc.) and the like.

Examples of the antihypertensive agents include angiotensin converting enzyme inhibitors (e.g., captopril, enalapril, delapril etc.), angiotensin II antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid etc.), calcium antagonists (e.g., manidipine, nifedipine, nicardipine, amlodipine, efonidipine, nicardipine etc.), potassium channel openers (e.g., levcromakalim, L-27152, AL 0671, NIP-121 etc.), clonidine and the like.
Examples of the antiobesity agents include antiobesity agents acting on the central nervous system (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds encompassed in WO01/829 and WO01/87834 etc.); neuropeptide Y antagonists (e.g., CP-422935 etc.); cannabinoid receptor antagonists (e.g., SR-141716, SR-147778 etc.); ghrelin antagonists), pancreatic lipase inhibitors (e.g., orlistat, ATL-962), β3 agonist (e.g., AJ-9677), peptide anorexiants (e.g., leptin, CNTF (Ciliary Neurotropic Factor) etc.), cholecystokinin agonists (e.g., lintitript, FPL-15849 etc.), feeding deterrent (e.g., P-57 etc.) and the like.

Examples of the diuretics include xanthine derivatives (e.g., sodium salicylate and theobromine, calcium salicylate and theobromine etc.), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide etc.), antialdosterone preparations (e.g., spironolactone, triamterene etc.), carbonate dehydratase inhibitors (e.g., acetazolamide and the like), chlorobenzenesulfonamide preparations (e.g., chlortalidone, mefruside, indapamide etc.), azosemide, isosorbide, etacrynic acid, piretanide, bumetanide, furosemide and the like.
Examples of the chemotherapeutic agents include alkylating agents (e.g., cyclophosphamide, ifosfamide etc.), metabolic antagonists (e.g., methotrexate, 5-fluorouracil or a derivative thereof), antitumor antibiotics (e.g., mitomycin, adriamycin etc.), plant-derived antitumor agent (e.g., vincristine, vindesine, Taxol etc.), cisplatin, carboplatin, etoposide and the like. Of these, Furtulon or NeoFurtulon, which are 5-fluorouracil derivatives, and the like are preferable.
Examples of the immunotherapeutic agents include microorganism or bacterial components (e.g., muramyl dipeptide derivative, Picibanil etc.), polysaccharides having immunity potentiating activity (e.g., lentinan, schizophyllan, krestin etc.), cytokines obtained by genetic engineering techniques (e.g., interferon, interleukin (IL) etc.), colony stimulating factors (e.g., granulocyte colony stimulating factor, erythropoietin etc.) and the like, with preference given to interleukins such as IL-1, IL-2, IL-12 and the like.

Examples of the antithrombotic agents include heparin (e.g., heparin sodium, heparin calcium, dalteparin sodium etc.), warfarin (e.g., warfarin potassium etc.), anti-thrombin drugs (e.g., aragatroban etc.), thrombolytic agents (e.g., urokinase, tisokinase, alteplase, nateplase, monteplase, pamiteplase etc.), platelet aggregation inhibitors (e.g., ticlopidine hydrochloride, cilostazol, ethyl icosapentate, beraprost sodium, sarpogrelate hydrochloride etc.) and the like.
Examples of the therapeutic agents for osteoporosis include alfacalcidol, calcitriol, elcatonin, calcitonin salmon, estriol, ipriflavone, risedronate disodium, pamidronate disodium, alendronate sodium hydrate, incadronate disodium and the like.
Examples of the antidementia agents include tacrine, donepezil, rivastigmine, galanthamine and the like.
Examples of the erectile dysfunction improver include apomorphine, sildenafil citrate and the like.
Examples of the incontinence·frequent urination therapeutic agent include flavoxate hydrochloride, oxybutynin hydrochloride, propiverine hydrochloride and the like.
Examples of the therapeutic agents for dysuria include acetylcholine esterase inhibitors (e.g., distigmine) and the like.

Furthermore, drugs having a cachexia-improving action established in animal models and clinical situations, such as cyclooxygenase inhibitors (e.g., indomethacin etc.), progesterone derivatives (e.g., megestrol acetate), glucosteroids (e.g., dexamethasone etc.), metoclopramide agents, tetrahydrocannabinol agents (publications are all as mentioned above), fat metabolism improving agents (e.g., eicosapentanoic acid etc.), growth hormones, IGF-1, or antibodies to a cachexia-inducing factor such as TNF-α, LIF, IL-6, oncostatin M and the like, can be used in combination with the compound of the present invention.

The concomitant drug is preferably an insulin preparation, an insulin sensitizer, an α-glucosidase inhibitor, biguanide, an insulin secretagogue (preferably sulfonylurea) and the like.
The above-mentioned concomitant drug may be used in a combination of two or more kinds at an appropriately ratio.
When the compound of the present invention and a combination drug are used in combination, the amount of each agent can be reduced within a safe range in consideration of the opposite effect of the agents. Particularly, the dose of insulin sensitizer, insulin secretagogue (preferably sulfonylurea) and biguanide can be reduced from the general level. As a result, the opposite effect caused by these agents can be prevented safely. In addition, the dose of therapeutic agents for diabetic complications, therapeutic agent for hyperlipidemia and antihypertensive agent can be reduced. As a result, the opposite effect possibly caused by these agents can be prevented effectively.

The production methods of compound (I) are explained by referring to the following schemes, which are not to be construed as limitative. In the synthesis of compound (I), the order of syntheses is not limited to the one explained below, and the steps may be appropriately exchanged.
Even when an explanation is not particularly presented, esterification, amidation, hydrolysis, reduction, oxidation and the like may be additionally performed besides the following steps, so as to convert a functional group.
Moreover, to promote the progress of the reaction, a reagent other than those exemplified may be used as appropriate.

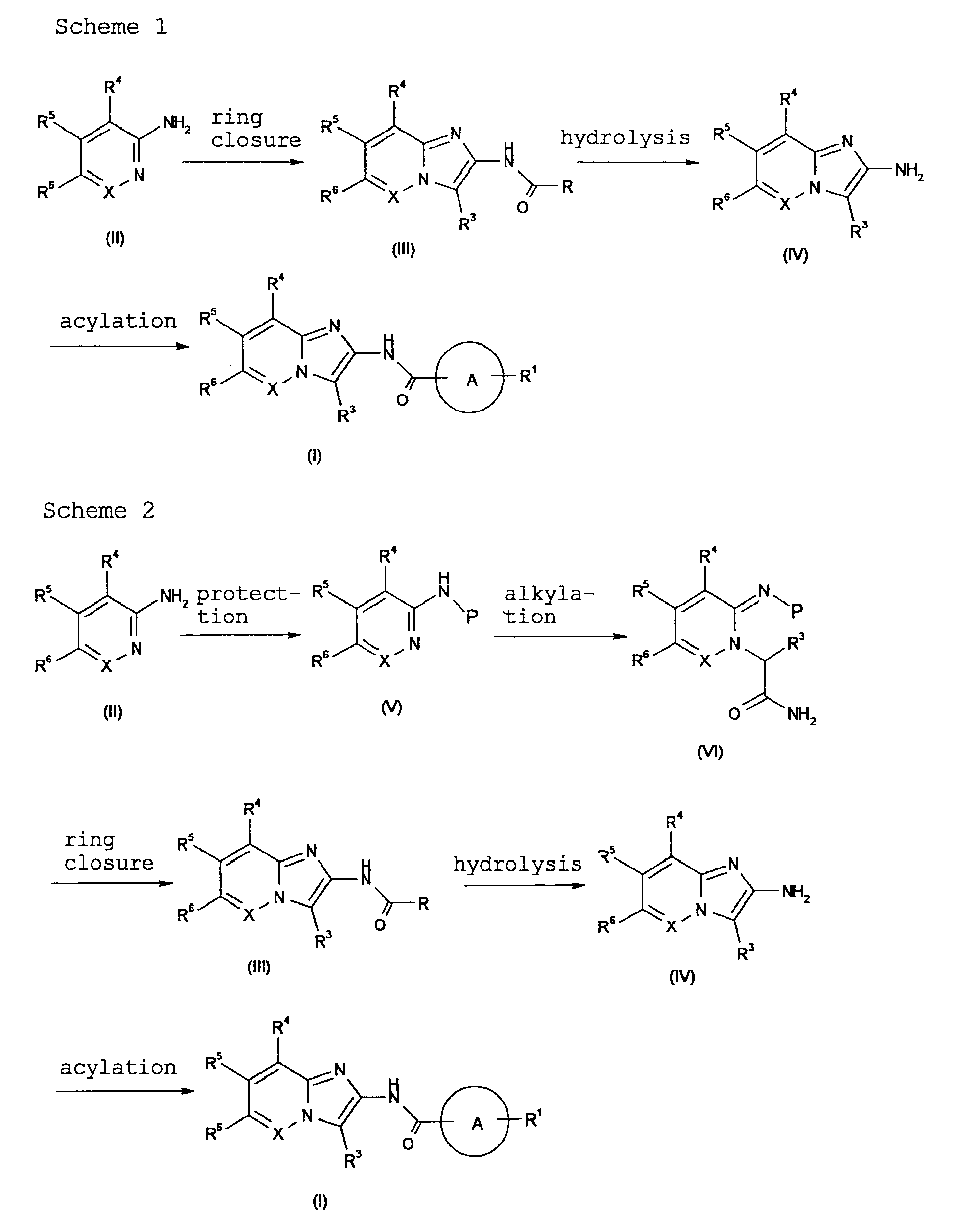

wherein R is C₁₋₆ alkyl (e.g., methyl, ethyl and the like) optionally substituted by a halogen atom, P is a protecting group such as toluenesulfonyl, methylsulfonyl and the like, and other symbols are as defined above.
In the following, the compounds represented by the formula (I), (II), (III), (IV), (V) and (VI) in the above-mentioned schemes are to be abbreviated as "compound (I)", "compound (II)", "compound (III)", "compound (IV)", "compound (V)" and "compound (VI)", respectively.

### (ring closure)

Compound (III) can be produced according to a method known per se, for example, the method described in J. Med. Chem., vol. 21, page 235 (1978) and the like, or a method analogous thereto. To be specific, compound (III) is produced by reacting compound (II) with (haloacetyl)carbamic acid ester (Scheme 1).

(Haloacetyl)carbamic acid ester is used in an amount of about 1.0 - 10.0 mol, preferably about 1.0 - 5.0 mol, per 1 mol of compound (II). Examples of the (haloacetyl)carbamic acid ester include methyl (chloroacetyl)carbamate, methyl (iodoacetyl)carbamate and the like.

This reaction is advantageously performed using a solvent inert to the reaction. Such solvent is not particularly limited as long as the reaction proceeds and, for example, halogenated hydrocarbons such as dichloromethane, chloroform, carbon tetrachloride, 1,2-dichloroethane and the like; aromatic hydrocarbons such as benzene, toluene and the like; ethers such as tetrahydrofuran, dioxane, 1,2-dimethoxyethane and the like; amides such as N,N-dimethylformamide, N,N-dimethylacetamide and the like; nitriles such as acetonitrile, propionitrile and the like; sulfoxides such as dimethyl sulfoxide and the like; phosphoric amides such as N,N,N',N',N",N"-hexamethylphosphorous triamide and the like; or a mixed solvent thereof and the like are preferable.

The reaction time is generally 1 hr - 60 hr, preferably 1 hr - 24 hr, and the reaction temperature is generally -10°C to 200°C, preferably 0°C to 150°C.

The resultant product (III) can also be used for the next reaction directly in the form of a reaction mixture or as a crude product. It can also be isolated from the reaction mixture according to a conventional method and can be easily purified by a separation means such as washing, recrystallization, distillation, chromatography and the like.

Compound (III) can be produced according to a method known per se, for example, the methods described in J. Med. Chem., vol. 46, page 4333 (2003), Synthesis, vol. 6, page 867 (1998) and the like or a method analogous thereto. To be specific, compound (III) is produced by reacting compound (VI) with an acid anhydride (Scheme 2). Acid anhydride is used in an amount of about 1.0 - 100.0 mol, preferably about 1.0 - 10.0 mol, per 1 mol of compound (VI). Examples of the acid anhydride include trifluoroacetic anhydride, acetic anhydride and the like.

This reaction is advantageously performed using a solvent inert to the reaction. Such solvent is not particularly limited as long as the reaction proceeds and, for example, halogenated hydrocarbons such as dichloromethane, chloroform, carbon tetrachloride, 1,2-dichloroethane and the like; aromatic hydrocarbons such as benzene, toluene and the like; ethers such as tetrahydrofuran, dioxane, 1,2-dimethoxyethane and the like; amides such as N,N-dimethylformamide, N,N-dimethylacetamide and the like; nitriles such as acetonitrile, propionitrile and the like; sulfoxides such as dimethyl sulfoxide and the like; or a mixed solvent thereof and the like are preferable.

The reaction time is generally 1 hr - 60 hr, preferably 1 hr - 24 hr, and the reaction temperature is generally -10°C to 200°C, preferably 0°C to 100°C.

The obtained compound (III) can also be used for the next reaction directly in the form of a reaction mixture or as a crude product. It can also be isolated from the reaction mixture according to a conventional method, and can be easily purified by a separation means such as washing, recrystallization, distillation, chromatography and the like.

### (hydrolysis)

Compound (IV) is produced by subjecting amide of compound (III) to acid hydrolysis or alkali hydrolysis (Schemes 1 and 2).
For acid hydrolysis, mineral acids such as hydrochloric acid, sulfuric acid and the like, Lewis acids such as boron trichloride, tribromide boron and the like, a combination of Lewis acid and thiol or sulfide, organic acids such as trifluoroacetic acid, p-toluenesulfonic acid and the like are generally used.
For alkali hydrolysis, inorganic bases such as sodium hydroxide, potassium hydroxide, barium hydroxide and the like, basic salts such as sodium carbonate, potassium carbonate and the like, metal alkoxides such as sodium methoxide, sodium ethoxide, potassium t-butoxide and the like, organic bases such as triethylamine, imidazole, formamidine and the like, and the like are used.
These acids and bases are used in an amount of about 0.5 - 10 mol, preferably about 0.5 - 6.0 mol, per 1 mol of compound (III).

This reaction is advantageous performed without a solvent or using a solvent inert to the reaction. Such solvent is not particularly limited as long as the reaction proceeds and, for example, alcohols such as methanol, ethanol, propanol and the like; aromatic hydrocarbons such as benzene, toluene and the like; saturated hydrocarbons such as cyclohexane, hexane and the like; organic acids such as formic acid, acetic acid and the like; ethers such as tetrahydrofuran, dioxane, 1,2-dimethoxyethane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; ketones such as acetone, methylethyl ketone and the like; sulfoxides such as dimethyl sulfoxide and the like; a solvent such as water and the like; or a mixed solvent thereof and the like are preferable.

The reaction time is generally 10 min - 60 hr, preferably 10 min - 12 hr, and the reaction temperature is generally -10°C to 200°C, preferably 0°C to 120°C.

The obtained compound (IV) can also be used for the next reaction directly in the form of a reaction mixture or as a crude product. It can also be isolated from the reaction mixture according to a conventional method, and can be easily purified by a separation means such as washing, recrystallization, distillation, chromatography and the like.

### (acylation)

Compound (I) is produced by reacting compound (IV) with carboxylic acid, a salt thereof or a reactive derivative (Schemes 1 and 2). As the carboxylic acid, for example, a compound represented by the following formula (VII)

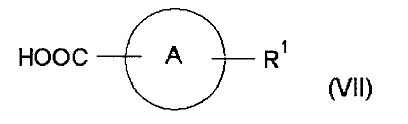

wherein ring A and R¹ are as defined above, can be mentioned. Hereinafter a compound represented by the formula (VII) and a salt thereof are abbreviated as "compound (VII)".

As the reactive derivative of compound (VII), for example, acid halides (e.g., acid chloride, acid bromide), acid amides (e.g., acid amide with pyrazole, imidazole, benzotriazole and the like), acid anhydride (e.g., C₁₋₆ aliphatic carbonic anhydride such as acetic anhydride, propionic anhydride, butyric anhydride and the like), acid azide, activated ester (e.g., diethoxyphosphate ester, diphenoxyphosphate ester, p-nitrophenyl ester, 2,4-dinitrophenyl ester, cyanomethyl ester, pentachlorophenyl ester, ester with N-hydroxysuccinimide, ester with N-hydroxyphthalimide, ester with 1-hydroxybenzotriazole, ester with 6-chloro-1-hydroxybenzotriazole, ester with 1-hydroxy-1H-2-pyridone), activated thioester (e.g., 2-pyridylthioester, 2-benzothiazolylthioester) and the like are used.

Instead of using the reactive derivative, compound (VII) may be directly reacted with compound (IV) in the presence of a suitable condensation agent. As such a condensation agent, for example, N,N'-disubstituted carbodiimides such as N,N'-dicyclohexylcarbodiimide, 1-ethyl-3-(3-dimethylaminopropyl)carbodiimide (WSC) hydrochloride and the like; azolides such as N,N'-carbonyldiimidazole and the like; a dehydrating agent such as N-ethoxycarbonyl-2-ethoxy-1,2-dihydroquinoline, phosphorus oxychloride, alkoxyacetylene and the like; 2-halogenopyridinium salt such as 2-chloromethylpyridinium iodide, 2-fluoro-1-methylpyridinium iodide and the like, and the like are used. When these condensation agents are used, the reaction can proceed via a reactive derivative of carboxylic acid.

Compound (VII) or a reactive derivative thereof is used in an amount of generally about 1.0 - 5.0 mol, preferably about 1.0 - 2.0 mol, per 1 mol of compound (IV). This reaction is advantageously performed using a solvent inert to the reaction. Such solvent is not particularly limited as long as the reaction proceeds and, for example, ethers such as diethyl ether, tetrahydrofuran, dioxane, 1,2-dimethoxyethane and the like; hydrocarbons such as benzene, toluene, cyclohexane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethyl sulfoxide and the like; a solvent such as water and the like; or a mixed solvent thereof and the like are preferable.

When acid halide is used as the reactive derivative of carboxylic acid, the reaction can be preformed in the presence of an acid scavenger to remove the released halogenated hydrogen from the reaction system. As such acid scavenger, for example, basic salts such as sodium carbonate, potassium carbonate, sodium hydrogen carbonate and the like; aromatic amines such as pyridine, lutidine and the like; tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like are desirably added.

While the reaction time varies depending on the reagent and solvent to be used, it is generally 30 min - 24 hr, preferably 30 min - 4 hr, and the reaction temperature is generally 0°C to 100°C, preferably 0°C to 70°C.

Alternatively, when R⁶ is 1-imidazolyl, compound (I) can be produced according to the methods described in J. Am. Chem. Soc., vol. 120, page 12459 (1998), J. Am. Chem. Soc., vol. 124, page 7421 (2002) and the like or a method analogous thereto. When R⁶ is a 5-membered heterocyclic group other than 1-imidazolyl, compound (I) can be produced according to the methods described in Chem. Rev, vol. 95, page 2457 (1995) and the like or a method analogous thereto.

The obtained compound (I) can also be used for the next reaction directly in the form of a reaction mixture or as a crude product. It can also be isolated from the reaction mixture according to a conventional method, and can be easily purified by a separation means such as washing, recrystallization, distillation, chromatography and the like.

### (alkylation)

Compound (VI) can be produced according to a method known per se, for example, the methods described in Synthesis, vol. 6, page 867 (1998) and the like or a method analogous thereto (Scheme 2). Compound (VI) can be produced from compound (V) and haloacetamide (e.g., bromoacetamide, iodoacetamide) in the presence of a base.

Haloacetamide is used in an amount of about 1.0 - 5.0 mol, preferably about 1.0 - 2.0 mol, per 1 mol of compound (V).

As the base, for example, inorganic bases such as sodium hydroxide, potassium hydroxide and the like; basic salts such as sodium carbonate, potassium carbonate, cesium carbonate, sodium hydrogen carbonate and the like; aromatic amines such as pyridine, lutidine and the like; tertiary amines such as triethylamine, diisopropylethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned. The amount of the base to be used is about 1.0 - 5.0 mol, preferably about 1.0 - 2.0 mol, per 1 mol of compound (V).

This reaction is advantageously performed using a solvent inert to the reaction. Such solvent is not particularly limited as long as the reaction proceeds and, for example, aromatic hydrocarbons such as benzene, toluene and the like; ethers such as tetrahydrofuran, dioxane, 1,2-dimethoxyethane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethyl sulfoxide and the like or a mixed solvent thereof and the like are preferable.

The reaction time is generally 1 hr - 60 hr, preferably 1 hr - 24 hr, and the reaction temperature is generally -10°C to 200°C, preferably 0°C to 100°C.

The resultant product (VI) can also be used for the next reaction directly in the form of a reaction mixture or as a crude product. It can also be isolated from the reaction mixture according to a conventional method, and can be easily purified by a separation means such as washing, recrystallization, distillation, chromatography and the like.

### (protection)

Compound (V) can be produced according to a method known per se, for example, the methods described in Synthesis, vol. 6, page 867 (1998) and the like or a method analogous thereto (Scheme 2). More particularly, compound (V) is produced from compound (II) and sulfonyl chloride (e.g., p-toluenesulfonyl chloride, methylsulfonyl chloride etc.) in the presence of a base.

Sulfonyl chloride is used in an amount of about 1.0 - 5.0 mol, preferably about 1.0 - 2.0 mol, per 1 mol of compound (V).

As the base, for example, inorganic bases such as sodium hydroxide, potassium hydroxide and the like; basic salts such as sodium carbonate, potassium carbonate, cesium carbonate, sodium hydrogen carbonate and the like; aromatic amines such as pyridine, lutidine and the like; tertiary amines such as triethylamine, diisopropylethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like and the like can be mentioned. The amount of the base to be used is about 1.0 - 5.0 mol, preferably about 1.0 - 2.0 mol, per 1 mol of compound (V).

This reaction is advantageously performed using a solvent inert to the reaction. Such solvent is not particularly limited as long as the reaction proceeds and, for example, aromatic hydrocarbons such as benzene, toluene and the like; ethers such as tetrahydrofuran, dioxane, 1,2-dimethoxyethane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethyl sulfoxide and the like; or a mixed solvent thereof and the like are preferable.

The reaction time is generally 1 hr - 60 hr, preferably 1 hr - 24 hr, and the reaction temperature is generally -10°C to 200°C, preferably 0°C to 100°C. The resultant product (VI) can also be used for the next reaction directly in the form of a reaction mixture or as a crude product. It can also be isolated from the reaction mixture according to a conventional method, and can be easily purified by a separation means such as washing, recrystallization, distillation, chromatography and the like.

In each reaction of the aforementioned production methods, when a starting compound or compound (I) has amino, carboxyl, hydroxy or mercapto, a protecting group generally used in peptide chemistry and the like may be introduced into these groups. Such protecting group can be removed at any step in each reaction scheme by a general deprotection method.
In addition, the compound of the present invention obtained by each of the above-mentioned production methods can be isolated and purified by a known means such as concentration, concentration under reduced pressure, solvent extraction, crystallization, recrystallization, phase transfer, chromatography and the like. Each starting compound used for each of the above-mentioned production methods can also be isolated and purified by a known means such as those mentioned above. It may also be used as a starting material in the form of a reaction mixture in the next step without isolation.
In the production of compound (I), when a starting material compound can form a salt, the compound may be used in the form of a salt. As such salt, for example, those exemplified as the salt of compound (I) can be mentioned.
When compound (I) contains optical isomer, stereoisomer, positional isomer, rotamer or tautomer, each of these can also be contained as compound (I), as well as can be obtained as a single product by a synthesis method and a separation a method known per se. For example, when compound (I) has an optical isomer, the optical isomer resolved from the compound is also encompassed in the compound of the present invention.
Compound (I) may be a crystal.
The crystal of compound (I) can be produced by crystallization of compound (I) by a crystallization method known per se.
The crystal of compound (I) is superior in the physicochemical properties (e.g., melting point, solubility, stability) and biological properties (e.g., in vivo kinetics (absorbability, distribution, metabolism, excretion), efficacy expression) and is extremely useful as a pharmaceutical agent.

### Examples

The present invention is explained in detail in the following by referring to Reference Examples, Examples, Formulation Examples and Experimental Examples, which are mere exemplifications and do not limit the present invention. In addition, the present invention may be modified without departing from the scope of the invention.
In the following Reference Examples and Examples, "room temperature" generally shows about 10°C to about 35°C and "%" means weight % unless otherwise specified. However, the yield is in mol/mol%.

Other abbreviations used in the specification mean the following.
s: singlet
d: doublet
t: triplet
q: quartet
dd: double doublet
ddd: double double doublet
dt: double triplet
br: broad
J: coupling constant
Hz: hertz
CDCl₃: deuterated chloroform
DMSO-d₆: deuterated dimethyl sulfoxide
CD₃OD: deuterated methanol
¹H-NMR: proton nuclear magnetic resonance
o: ortho
m: meta
p: para
n: normal
DMF: N,N-dimethylformamide
THF: tetrahydrofuran
NMP: 1-methyl-2-pyrrolidinone
DME: 1,2-dimethoxyethane
WSC: hydrochloric acid 1-ethyl-3-(3-dimethylaminopropyl)carbodiimide
HOBt: 1-hydroxybenzotriazole
DMAP: 4-dimethylaminopyridine

LC/MS analyses in the following Examples were performed under the following conditions.
measurement device: Waters LC/MS system
HPLC unit: Agilent HP1100
MS unit: Micromass ZMD
column: CAPCELL PAK c18UG120 S-3 µm, 1.5×35 mm (manufactured by Shiseido)
solvent: SOLUTION A ;0.05% trifluoroacetic acid aqueous solution, SOLUTION B ; 0.04% trifluoroacetic acid acetonitrile solution
gradient cycle: 0 min (SOLUTION A/SOLUTION B = 90/10), 2.00 min (SOLUTION A/SOLUTION B = 5/95), 2.75 min (SOLUTION A/SOLUTION B = 5/95), 2.76 min (SOLUTION A/SOLUTION B = 90/10), 3.60 min (SOLUTION A/SOLUTION B = 90/10)
injection volume: 2 µL, flow rate: 0.5 mL/min, detection
method: UV 220 nm
MS conditions ionization method: ESI

Purification by preparative HLPC in the following Examples were performed under the following conditions. equipment: GILSON high through-put purification system column: YMC CombiPrep ODS-A S-5 µm, 50×20 mm or YMC Hydrosphere C18 S-5 µm solvent: SOLUTION A; 0.1% aqueous trifluoroacetic acid solution, SOLUTION B; 0.1% trifluoroacetic acid acetonitrile solution, or SOLUTION A; water, SOLUTION B; acetonitrile, or SOLUTION A; 0.1% aqueous formic acid solution, SOLUTION B; 0.1% formic acid acetonitrile solution representative gradient cycle: 0 min (SOLUTION A /SOLUTION B =98/2), 1.00 min (SOLUTION A/SOLUTION B = 98/2), 5.20 min (SOLUTION A/SOLUTION B = 0/100), 6.40 min (SOLUTION A/SOLUTION B = 0/100), 6.50 min (SOLUTION A/SOLUTION B = 98/2), 6.60 min (SOLUTION A/SOLUTION B = 98/2)
flow rate: 25 mL/min, detection method: UV 220 nm

### Reference Example 1

4-(2-methoxy-1,1-dimethyl-2-oxoethyl)benzoic acid

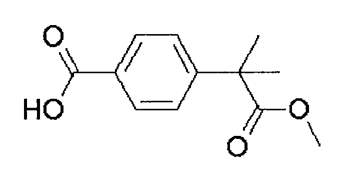

Potassium permanganate (94.5 g, 0.60 mol) was added to a mixed solution of methyl 2-methyl-2-(4-methylphenyl)propanoate (23.0 g, 0.12 mol, J. Am. Chem. Soc., 2002, 124, 12557.) in pyridine (60 mL)/water (575 mL), and the reaction mixture was stirred at 100°C for 3 hr. The reaction mixture was allowed to cool to room temperature, insoluble material was filtered off, and the filtrate was washed with ethyl acetate. The aqueous layer was acidified with 6N hydrochloric acid, and the mixture was extracted with ethyl acetate. The extract was dried over anhydrous magnesium sulfate and the solvent was evaporated under reduced pressure to give the title compound (yield 14.5 g, 55%).
¹H-NMR (DMSO-d₆) δ: 1.52 (6H, s), 3.60 (3H, s), 7.43 (2H, d, J = 8.3 Hz), 7.91 (2H, d, J = 8.3 Hz), 12.92 (1H, br). Elemental analysis: for C₁₂H₁₄O₄
Calculated: C, 64.85; H, 6.35.
Found: C, 64.57; H, 6.21.

### Reference Example 2

4-(1-cyano-1-methylethyl)benzoic acid

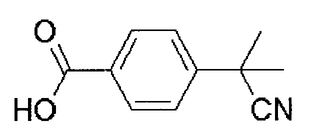

Using 2-methyl-2-(4-methylphenyl)propanenitrile (24.0 g, 0.15 mol, Tetrahedron, 2004, 60, 2843.) and in the same manner as in Reference Example 1, the reaction was performed to give the title compound (yield 7.8 g, 27%).
¹H-NMR (DMSO-d₆) δ: 1.71 (6H, s), 7.65 (2H, d, J = 8.7 Hz), 7.99 (2H, d, J = 8.7 Hz), 13.10 (1H, br).

### Reference Example 3

4-(3-cyano-1,1-dimethylpropyl)benzoic acid

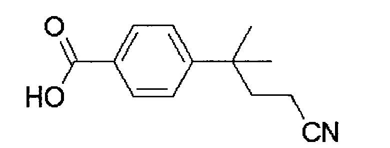

Using 4-methyl-4-(4-methylphenyl)pentanenitrile (12.7 g, 68 mmol, Tetrahedron Lett., 1977, 20, 1713.) and in the same manner as in Reference Example 1, the reaction was performed. The obtained crude crystals were recrystallized from diethyl ether/hexane to give the title compound (yield 7.3 g, 50%).
¹H-NMR (CDCl₃) δ: 1.40 (6H, s), 1.92-2.18 (4H, m), 7.42 (2H, d, J = 8.3 Hz), 8.08 (2H, d, J = 8.3 Hz), 13.10 (1H, br).

### Reference Example 4

ethyl 3-methyl-3-(4-methylphenyl)butanoate

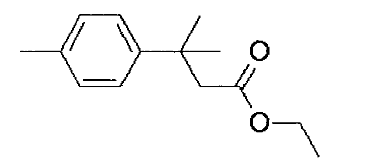

3-Methyl-3-(4-methylphenyl)butanoic acid (11.6 g, 60 mmol, Synth. Commun., 2001, 31, 679.) and concentrated sulfuric acid (100 µL) were stirred in ethanol (100 mL) at 60°C overnight. The reaction mixture was allowed to cool to room temperature and the solvent was evaporated under reduced pressure. Saturated aqueous sodium hydrogen carbonate solution was added to the residue and the mixture was extracted with ethyl acetate. The extract was dried over anhydrous magnesium sulfate and the solvent was evaporated under reduced pressure to give the title compound (yield 12.4 g, 93%).
¹H-NMR (CDCl₃) δ: 1.11 (3H, t, J = 7.2 Hz), 1.44 (6H, s), 2.31 (3H, s), 2.58 (2H, s), 3.99 (2H, q, J = 7.2 Hz), 7.11 (2H, d, J = 8.3 Hz), 7.25 (2H, d, J = 8.3 Hz).

### Reference Example 5

4-(3-ethoxy-1,1-dimethyl-3-oxopropyl)benzoic acid

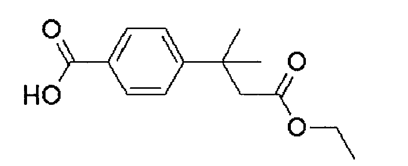

Using ethyl 3-methyl-3-(4-methylphenyl)butanoate (12.4 g, 56 mmol) obtained in Reference Example 4 and in the same manner as in Reference Example 1, the reaction was performed. The obtained residue was purified by silica gel column chromatography (30-80% ethyl acetate/hexane) to give the title compound (yield 3.8 g, 23%).
¹H-NMR (CDCl₃) δ: 1.08 (3H, t, J = 7.2 Hz), 1.48 (6H, s), 2.66 (2H, s), 3.97 (2H, q, J = 7.2 Hz), 7.47 (2H, d, J = 8.3 Hz), 8.04 (2H, d, J = 8.3 Hz), hidden (1H).

### Reference Example 6

4-(4-ethoxy-1,1-dimethyl-4-oxobutyl)benzoic acid

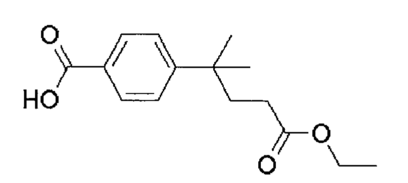

Using ethyl 4-methyl-4-(4-methylphenyl)pentanoate (5.4 g, 25 mmol, Tetrahedron Lett., 1994, 35, 3017.) and in the same manner as in Reference Example 1, the reaction was performed. The obtained residue was purified by silica gel column chromatography (25-30% ethyl acetate/hexane) to give the title compound (yield 4.6 g, 75%).
¹H-NMR (CDCl₃) δ: 1.21 (3H, t, J = 7.1 Hz), 1.36 (6H, s), 2.00-2.04 (4H, m), 4.05 (2H, q, J = 7.1 Hz), 7.44 (2H, d, J = 8.7 Hz), 8.05 (2H, d, J = 8.7 Hz), hidden (1H).

### Reference Example 7

methyl 1-(4-methylphenyl)cyclopropanecarboxylate

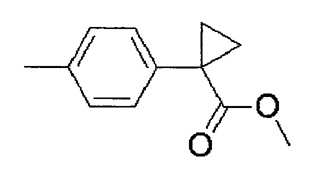

1-(4-Methylphenyl)cyclopropanecarboxylic acid (1.0 g, 5.6 mmol) and concentrated sulfuric acid (60 µL) were heated under reflux in methanol (60 mL) for 1 hr. The reaction mixture was allowed to cool to room temperature and the solvent was evaporated under reduced pressure. Saturated aqueous sodium hydrogen carbonate solution was added to the residue and the mixture was extracted with ethyl acetate. The extract was dried over anhydrous magnesium sulfate and the solvent was evaporated under reduced pressure to give the title compound (yield 0.80 g, 80%).
¹H-NMR (CDCl₃) δ: 1.13-1.19 (2H, m), 1.54-1.60 (2H, m), 2.34 (3H, s), 3.62 (3H, s), 7.12 (2H, d, J = 8.0 Hz), 7.23 (2H, d, J = 8.0 Hz).

### Reference Example 8

methyl 1-(4-methylphenyl)cyclobutanecarboxylate

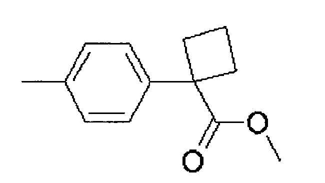

In the same manner as in Reference Example 7 and using 1-(4-methylphenyl)cyclobutanecarboxylic acid (0.45 g, 2.3 mmol, J. Org. Chem., 1998, 63, 3814.), the title compound (yield 0.53 g, 89%) was obtained.
¹H-NMR (DMSO-d₆) δ: 1.69-2.01 (2H, s), 2.27 (3H, s), 2.33-2.47 (2H, m), 2.63-2.78 (2H, m), 3.55 (3H, s), 7.15 (4H, s).

### Reference Example 9

methyl 1-(4-methylphenyl)cyclopentanecarboxylate

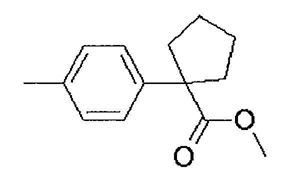

In the same manner as in Reference Example 7 and using 1-(4-methylphenyl)cyclopentanecarboxylic acid (1.6 g, 7.5 mmol), the title compound (yield 1.5 g, 93%) was obtained.
¹H-NMR (CDCl₃) δ: 1.63-1.81 (4H, m), 1.81-1.99 (2H, m), 2.32 (3H, s), 2.56-2.69 (2H, m), 3.60 (3H, s), 7.11 (2H, d, J = 8.3 Hz), 7.25 (2H, d, J = 8.3 Hz).

### Reference Example 10

methyl 1-(4-methylphenyl)cyclohexanecarboxylate

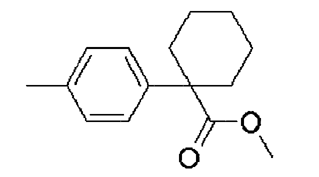

In the same manner as in Reference Example 7 and using 1-(4-methylphenyl)cyclohexanecarboxylic acid (1.5 g, 6.5 mmol), the title compound (yield 1.4 g, 91%) was obtained.
¹H-NMR (CDCl₃) δ: 1.17-1.36 (1H, m), 1.36-1.55 (2H, m), 1.55-1.79 (5H, m), 2.31 (3H, s), 2.39-2.53 (2H, m), 3.63 (3H, s), 7.13 (2H, d, J = 8.3 Hz), 7.27 (2H, d, J = 8.3 Hz).

### Reference Example 11

4-[1-(methoxycarbonyl)cyclopropyl]benzoic acid

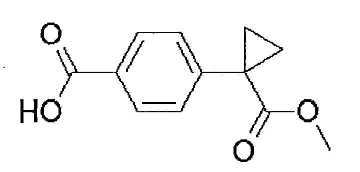

Using methyl 1-(4-methylphenyl)cyclopropanecarboxylate (0.84 g, 4.3 mmol) obtained in Reference Example 7 and in the same manner as in Reference Example 1, the reaction was performed. The obtained residue was purified by silica gel column chromatography (40-50% ethyl acetate/hexane) to give the title compound (yield 0.29 g, 31%).
¹H-NMR (DMSO-d₆) δ: 1.23-1.26 (2H, m), 1.51-1.54 (2H, m), 3.56 (3H, s), 7.46 (2H, d, J = 8.2 Hz), 7.88 (2H, d, J = 8.2 Hz), 12.92 (1H, br).

### Reference Example 12

4-[1-(methoxycarbonyl)cyclobutyl]benzoic acid

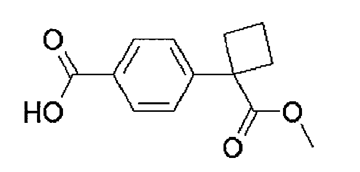

Using methyl 1-(4-methylphenyl)cyclobutanecarboxylate (0.58 g, 2.8 mmol) obtained in Reference Example 8 and in the same manner as in Reference Example 1, the reaction was performed. The obtained residue was purified by silica gel column chromatography (40% ethyl acetate/hexane) to give the title compound (yield 0.48 g, 73%).
¹H-NMR (DMSO-d₆) δ: 1.74-1.91 (1H, m), 1.91-2.07 (1H, m), 2.40-2.53 (2H, m), 2.68-2.84 (2H, m), 3.59 (3H, s), 7.39 (2H, d, J = 8.3 Hz), 7.92 (2H, d, J = 8.3 Hz), 12.93 (1H, br).

### Reference Example 13

4-[1-(methoxycarbonyl)cyclopentyl]benzoic acid

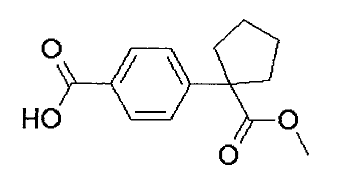

Using methyl 1-(4-methylphenyl)cyclopentanecarboxylate (1.5 g, 6.8 mmol) obtained in Reference Example 9 and in the same manner as in Reference Example 1, the reaction was performed. The obtained crude crystals were recrystallized from diisopropyl ether to give the title compound (yield 1.0 g, 59%).
¹H-NMR (CDCl₃) δ: 1.68-1.85 (4H, m), 1.85-2.04 (2H, m), 2.60-2.77 (2H, m), 3.63 (3H, s), 7.47 (2H, d, J = 8.3 Hz), 8.05 (2H, d, J = 8.3 Hz).

### Reference Example 14

4-[1-(methoxycarbonyl)cyclohexyl]benzoic acid

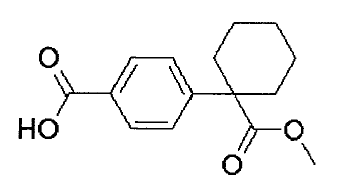

Using methyl 1-(4-methylphenyl)cyclohexanecarboxylate (1.4 g, 5.8 mmol) obtained in Reference Example 10 and in the same manner as in Reference Example 1, the reaction was performed. The obtained crude crystals were recrystallized from diisopropyl ether to give the title compound (yield 0.65 g, 43%).
¹H-NMR (DMSO-d₆) δ: 1.16-1.50 (3H, m), 1.50-1.82 (5H, m), 2.28-2.45 (2H, m), 3.60 (3H, s), 7.47 (2H, d, J = 8.3 Hz), 7.91 (2H, d, J = 8.3 Hz), 12.91 (1H, br).

### Reference Example 15

N-(2,3-dihydrofuro[3,2-b]pyridin-5-yl)-4-methylbenzenesulfonamide

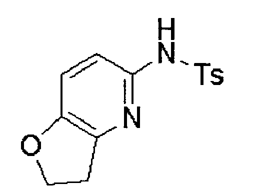

To a solution of 2,3-dihydrofuro[3,2-b]pyridine-5-amine (5.1 g, 37 mmol, Chem. Pharm. Bull., 1984, 32, 4914.) in pyridine (100 mL) was added 4-methylbenzenesulfonyl chloride (7.4 g, 39 mmol), and the reaction mixture was stirred at room temperature for 1 hr. Water was added to the reaction mixture, and the mixture was extracted with ethyl acetate. The extract was washed with saturated aqueous sodium hydrogen carbonate solution and saturated brine, dried over anhydrous magnesium sulfate, and the solvent was evaporated under reduced pressure. The residue was washed with methanol and ethyl acetate to give the title compound (yield 9.4 g, 87%).
¹H-NMR (DMSO-d₆) δ: 2.34 (3H, s), 3.10 (2H, t, J = 8.8 Hz), 4.54 (2H, t, J = 8.8 Hz), 6.86 (2H, d, J = 8.5 Hz), 7.08 (2H, d, J = 8.5 Hz), 7.35 (2H, d, J = 8.3 Hz), 7.71 (2H, d, J = 8.3 Hz), 10.54 (1H, br).

### Reference Example 16

2-[5-{[(4-methylphenyl)sulfonyl]imino}-3,5-dihydrofuro[3,2-b]pyridin-4(2H)-yl]acetamide

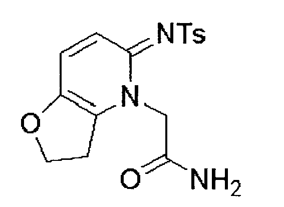

N-(2,3-Dihydrofuro[3,2-b]pyridin-5-yl)-4-methylbenzene sulfonamide (9.4 g, 32 mmol) obtained in Reference Example 15, 2-iodoacetamide (12.0 g, 65 mmol), and diisopropylethylamine (11.3 mL, 65 mmol) were dissolved in DMF (150 mL), and the mixture was stirred at 60°C for 24 hr. The solvent was evaporated under reduced pressure, and the residue was washed with methanol to give the title compound (yield 6.7 g, 60%).
¹H-NMR (DMSO-d₆) δ: 2.32 (3H, s), 3.35 (2H, t, J = 8.9 Hz), 4.60 (2H, t, J = 8.9 Hz), 4.86 (2H, s), 7.12 (1H, d, J = 9.4 Hz), 7.24 (2H, d, J = 8.1 Hz), 7.39-7.53 (2H, m), 7.62 (2H, d, J = 8.1 Hz), 7.85 (1H, s).

### Reference Example 17

1,2-dihydrofuro[2,3-e]imidazo[1,2-a]pyridine-7-amine

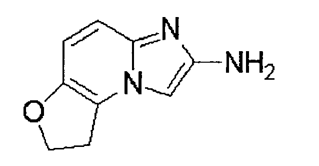

To a solution of 2-[5-{[(4-methylphenyl)sulfonyl]imino}-3,5-dihydrofuro[3,2-b]pyridin-4(2H)-yl]acetamide (6.7 g, 19 mmol) obtained in Reference Example 16 in dichloromethane (100 mL) was added trifluoroacetic anhydride (50 mL), and the reaction mixture was stirred at room temperature for 1 hr. The solvent was evaporated under reduced pressure, saturated aqueous sodium hydrogen carbonate solution was added to the residue, and the mixture was extracted with ethyl acetate and THF. The extract was dried over anhydrous magnesium sulfate and the solvent was evaporated under reduced pressure. The obtained residue was dissolved in ethanol (100 mL). n-Butylamine (39 mL, 340 mmol) was added to the reaction mixture, and the mixture was stirred at 100°C for 3 hr. The reaction mixture was allowed to cool to room temperature and the solvent was evaporated under reduced pressure. The obtained residue was purified by silica gel column chromatography (5-20% methanol/ethyl acetate) to give the title compound (yield 0.66 g, 19%).
¹H-NMR (DMSO-d₆) δ: 3.35 (2H, t, J = 9.0 Hz), 4.66 (2H, t, J = 9.0 Hz), 5.03 (2H, br), 6.69 (1H, s), 6.76 (1H, d, J = 9.2 Hz), 6.99 (1H, d, J = 9.2 Hz).

### Reference Example 18

4-methyl-N-(6-methylpyridin-2-yl)benzenesulfonamide

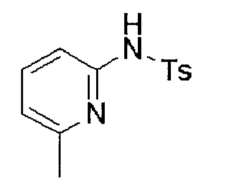

In the same manner as in Reference Example 15 and using 6-methylpyridine-2-amine (6.1 g, 55 mmol), the title compound (yield 13.5 g, 91%) was obtained.

### Reference Example 19

2-[6-methyl-2-{[(4-methylphenyl)sulfonyl]imino}pyridin-1(2H)-yl]acetamide

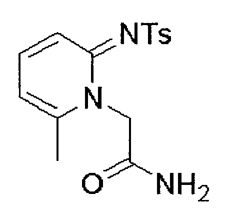

In the same manner as in Reference Example 16 and using 4-methyl-N-(6-methylpyridin-2-yl)benzenesulfonamide (13.5 g, 50 mmol), the title compound (yield 10.1 g, 63%) was obtained. ¹H-NMR (DMSO-d₆) δ: 2.33 (3H, s), 2.39 (3H, s), 4.96 (2H, br), 6.66 (1H, d, J = 7.2 Hz), 7.19 (1H, d, J = 9.0 Hz), 7.26 (2H, d, J = 8.3 Hz), 7.38 (1H, s), 7.59 (1H, dd, J = 9.0, 7.2 Hz), 7.65 (2H, d, J = 8.3 Hz), 7.82 (1H, s).

### Reference Example 20

5-methylimidazo[1,2-a]pyridine-2-amine

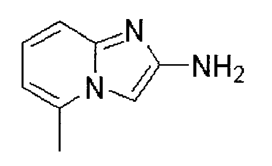

In the same manner as in Reference Example 17 and using 2-[6-methyl-2-{[(4-methylphenyl)sulfonyl]imino}pyridin-1(2H)-yl]acetamide (0.97 g, 3.0 mmol), the title compound (yield 0.35 g, 81%) was obtained.
¹H-NMR (DMSO-d₆) δ: 2.44 (3H, s), 5.04 (2H, s), 6.58 (1H, d, J = 7.0 Hz), 6.71 (1H, s), 6.94-7.01 (1H, m), 7.08 (1H, d, J = 8.7 Hz).

### Reference Example 21

6-chloroimidazo[1,2-a]pyridine-2-amine

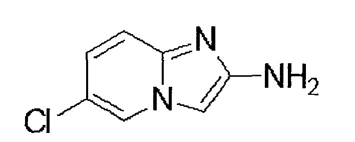

In the same manner as in Reference Example 17 and using 2-[5-chloro-2-{[(4-methylphenyl)sulfonyl]imino}pyridin-1(2H)-yl]acetamide (78.0 g, 0.23 mol, Synthesis, 1998, 6, 867.), the title compound (yield 14.0 g, 36%) was obtained.
¹H-NMR (DMSO-d₆) δ: 5.19 (2H, s), 6.99-7.02 (1H, m), 7.18 (1H, d, J = 9.5 Hz), 8.53 (1H, dd, J = 1.9, 0.8 Hz).

### Reference Example 22

6-bromoimidazo[1,2-a]pyridine-2-amine

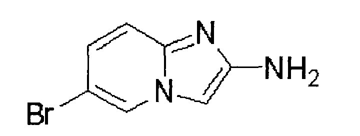

In the same manner as in Reference Example 17 and using 2-[5-bromo-2-{[(4-methylphenyl)sulfonyl]imino}pyridin-1(2H)-yl]acetamide (23.6 g, 61 mmol, Tetrahedron Lett., 2002, 43, 9051.), the title compound (yield 14 g, 90%) was obtained.
¹H-NMR (DMSO-d₆) δ: 5.20 (2H, br), 7.00 (1H, s), 7.04-7.18 (2H, m), 8.60 (1H, d, J = 0.9 Hz).

### Reference Example 23

6-iodoimidazo[1,2-a]pyridine-2-amine

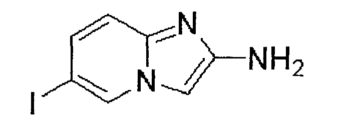

In the same manner as in Reference Example 17 and using 2-[5-iodo-2-{[(4-methylphenyl)sulfonyl]imino}pyridin-1(2H)-yl]acetamide (78 g, 0.23 mol, Synthesis, 1998, 6, 867.), the title compound (yield 14.0 g, 36%) was obtained.
¹H-NMR (DMSO-d₆) δ: 5.17 (2H, s), 6.97 (1H, s), 7.02 (1H, d, J = 9.1 Hz), 7.16 (1H, dd, J = 9.1, 1.5 Hz), 8.63 (1H, d, J = 1.5 Hz).

### Reference Example 24

4-methyl-N-(5-methylpyridin-2-yl)benzenesulfonamide

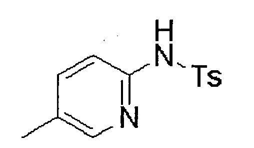

In the same manner as in Reference Example 15 and using 5-methylpyridine-2-amine (5.0 g, 46 mmol), the title compound (yield 11.6 g, 86%) was obtained.
¹H-NMR (DMSO-d₆) δ: 2.13 (3H, s), 2.33 (3H, s), 7.05 (1H, d, J = 8.7 Hz), 7.32 (2H, t, J = 8.3 Hz), 7.53 (1H, dd, J = 8.7, 2.3 Hz), 7.73 (2H, d, J = 8. 3 Hz), 7.87 (1H, s), 11.53 (1H, s).

### Reference Example 25

2-[5-methyl-2-{[(4-methylphenyl)sulfonyl]imino}pyridin-1(2H)-yl]acetamide

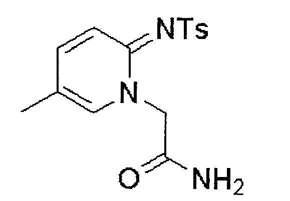

In the same manner as in Reference Example 16 and using 4-methyl-N-(5-methylpyridin-2-yl)benzenesulfonamide (5.0 g, 19 mmol), the title compound (yield 5.4 g, 89%) was obtained.
¹H-NMR (DMSO-d₆) δ: 2.10 (3H, s), 2.32 (3H, s), 4.79 (2H, s), 7.23-7.26 (3H, m), 7.35 (1H, s), 7.59 (1H, dd, J = 9.3, 2.1 Hz), 7.64 (2H, d, J = 8.3 Hz), 7.76 (1H, s), 7.84 (1H, s).

### Reference Example 26

6-methylimidazo[1,2-a]pyridine-2-amine

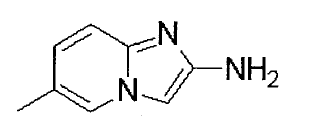

In the same manner as in Reference Example 17 and using 2-[5-methyl-2-{[(4-methylphenyl)sulfonyl]imino}pyridin-1(2H)-yl]acetamide (6.6 g, 0.21 mol), the title compound (yield 2.6 g, 84%) was obtained.
¹H-NMR (CDCl₃) δ: 2.28 (3H, s), 4.12 (2H, br), 6.83 (1H, s), 6.93 (1H, d, J = 9.1 Hz), 7.26 (1H, d, J = 9.1 Hz), 7.73 (1H, s).

### Reference Example 27

4-methyl-N-quinolin-2-ylbenzenesulfonamide

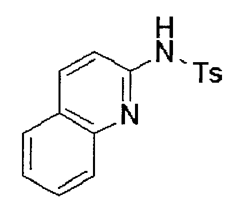

To a solution of quinoline-2-amine (0.34 g, 2.4 mmol) and pyridine (10 mL) was added 4-methylbenzenesulfonyl chloride (0.67 g, 3.5 mmol), and the reaction mixture was stirred at room temperature for 2 hr. Water was added to the reaction mixture, and the mixture was extracted with ethyl acetate. The extract was washed with saturated aqueous sodium hydrogen carbonate solution and saturated brine, dried over anhydrous magnesium sulfate, and the solvent was evaporated under reduced pressure. The obtained residue was purified by silica gel column chromatography (20-100% ethyl acetate/hexane) to give the title compound (yield 0.31 g, 44%).
MS (ESI+): 299 (M+H).
¹H-NMR (CDCl₃) δ: 2.40 (3H, s), 6.85 (1H, d, J = 9.4 Hz), 7.27 (2H, s), 7.32-7.44 (2H, m), 7.57-7.67 (2H, m), 7.81-7.94 (3H, m), 11.78 (1H, s).

### Reference Example 28

imidazo[1,2-a]quinoline-2-amine

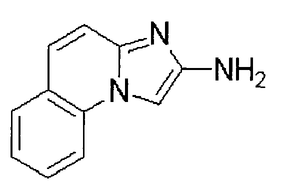

4-Methyl-N-quinolin-2-ylbenzenesulfonamide (0.31 g, 1.0 mmol) obtained in Reference Example 27, 2-iodoacetamide (0.21 g, 1.1 mmol) and diisopropylethylamine (0.16 mg, 1.2 mmol) were dissolved in DMF (150 mL), and the reaction mixture was stirred at 60°C for 24 hr. The solvent was evaporated under reduced pressure. The obtained residue was purified by silica gel column chromatography (20-100% ethyl acetate/hexane). The obtained residue was dissolved in a solution of trifluoroacetic anhydride (10 mL) in dichloromethane (10 mL), and the mixture was stirred at room temperature for 30 min. The solvent was evaporated under reduced pressure, saturated aqueous sodium hydrogen carbonate solution was added to the residue, and the mixture was extracted with ethyl acetate. The extract was dried over anhydrous magnesium sulfate and the solvent was evaporated under reduced pressure. The obtained residue was dissolved in a solution of 2N ammonia·methanol, the mixture was stirred for 30 min under microwave irradiation at 80°C. The solvent was evaporated under reduced pressure and the obtained residue was purified by silica gel column chromatography to give the title compound (yield 0.12 g, 66%).
¹H-NMR (DMSO-d₆) δ: 5.01 (2H, s), 7.30 (1H, d, J = 9.2 Hz), 7.41 (1H, t, J = 7.5 Hz), 7.49 (1H, s), 7.52 (1H, d, J = 9.2 Hz), 7.59-7.67 (1H, m), 7.89 (1H, dd, J = 7.9, 1.3 Hz), 8.06 (1H, d, J = 8.5 Hz).

### Reference Example 29

N-(6-chloropyridazin-3-yl)-4-methylbenzenesulfonamide

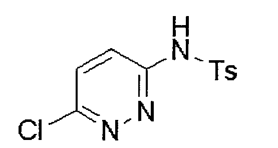

In the same manner as in Reference Example 27 and using 6-chloropyridazine-3-amine (10.0 g, 77 mmol), the title compound (yield 8.3 g, 38%) was obtained.
¹H-NMR (CDCl₃) δ: 2.41 (3H, s), 7.29 (2H, d, J = 7.9 Hz), 7.35 (1H, d, J = 9.4 Hz), 7.47 (1H, d, J = 9.4 Hz), 7.81 (2H, d, J = 8.5 Hz), 10.35-10.42 (1H, m).

### Reference Example 30

6-chloro-3-methylimidazo[1,2-b]pyridazine-2-amine

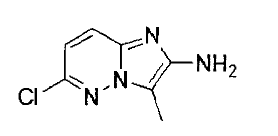

In the same manner as in Reference Example 28 and using N-(6-chloropyridazin-3-yl)-4-methylbenzenesulfonamide (2.0 g, 7.1 mmol) obtained in Reference Example 29 and 2-bromopropaneamide (1.2 g, 7.8 mmol), the title compound (yield 1.0 g, 77%) was obtained.
¹H-NMR (CDCl₃) δ: 2.33 (3H, s), 5.67 (2H, s), 6.94 (1H, d, J = 9.0 Hz), 7.63 (1H, d, J = 9.0 Hz).

### Reference Example 31

N-(5-fluoropyridin-2-yl)-4-methylbenzenesulfonamide

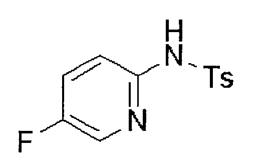

In the same manner as in Reference Example 27 and using 5-fluoropyridine-2-amine (4.8 g, 42 mmol), the title compound (yield 8.5 g, 75%) was obtained.
¹H-NMR (CDCl₃) δ: 2.38 (3H, s), 7.22 (2H, d, J = 8.1 Hz), 7.35-7.51 (2H, m), 7.68 (2H, d, J = 8.3 Hz), 8.31 (1H, d, J = 2.6 Hz), 10.04 (1H, s).

### Reference Example 32

6-fluoroimidazo[1,2-a]pyridine-2-amine

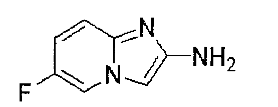

In the same manner as in Reference Example 28 and using N-(5-fluoropyridin-2-yl)-4-methylbenzenesulfonamide (7.4 g, 28 mmol) obtained in Reference Example 31, the title compound (yield 0.74 g, 18%) was obtained.
¹H-NMR (CDCl₃) δ: 5.10 (2H, s), 6.97-7.04 (2H, m), 7.17 (1H, dd, J = 9.6, 5.3 Hz), 8.84 (1H, dd, J = 5.3, 2.0 Hz).

### Reference Example 33

ethyl (6-chloroimidazo[1,2-b]pyridazin-2-yl)carbamate

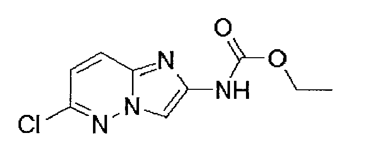

6-Chloropyridazine-3-amine (40 g, 0.31 mol, J. Med. Chem. 1981, 24, 59) was dissolved in N,N-dimethylacetamide (310 ml), ethyl (chloroacetyl)carbamate (51 g, 0.31 mol) and disodium hydrogenphosphate (66 g, 0.46 mol) were added, and the mixture was stirred at 110°C for 3 hr. The reaction mixture was cooled, water (930 ml) was added and the precipitated brown powder was collected by filtration, which was successively washed with water (200 ml), acetonitrile (200 ml) and diethyl ether (200 ml) and dried to give a white brown title compound (yield 38.5 g, 52%).

### Reference Example 34

6-chloroimidazo[1,2-b]pyridazine-2-amine

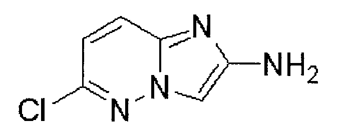

Barium hydroxide octahydrate was dissolved in water (750 ml), N-methylpyrrolidinone (250 ml) and ethyl (6-chloroimidazo[1,2-b]pyridazin-2-yl)carbamate (38.5 g, 0.16 mol) obtained in Reference Example 33 were added and the mixture was stirred at 120°C for 11 hr. The reaction mixture was cooled, extracted 3 times with a mixed solvent of tetrahydrofuran (500 ml)/ethyl acetate (500 ml), and the organic layer was washed with saturated brine, dried over magnesium sulfate and filtered. The solvent was evaporated under reduced pressure to give a green oily crude product containing a yellow needle product. The crude product was azeotroped with toluene, and the residual needle product was washed with diethyl ether to give the title compound (yield 21.7 g, 81%) as yellow needles.
¹H-NMR (DMSO-d₆) δ: 5.67 (2H, s), 7.04 (1H, d, J = 9.2 Hz), 7.37 (1H, s), 7.69 (1H, d, J = 9.2 Hz).

### Reference Example 35

ethyl (6-iodoimidazo[1,2-b]pyridazin-2-yl)carbamate

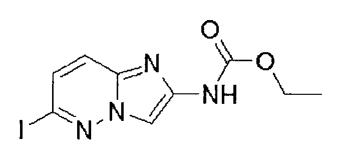

In the same manner as in Reference Example 33 and using 6-iodopyridazine-3-amine (10.5 g, 48 mmol, Tetrahedron, 2000, 56, 1777), the title compound (yield 11.6 g, 74%) was obtained. ¹H-NMR (DMSO-d₆) δ: 1.26 (3H, t, J = 7.2 Hz), 4.18 (2H, q, J = 7.2 Hz), 7.48 (1H, d, J = 9.2 Hz), 7.71 (1H, dd, J = 9.2, 0.6 Hz), 8.08 (1H, s), 10.52 (1H, s).

### Reference Example 36

6-iodoimidazo[1,2-b]pyridazine-2-amine

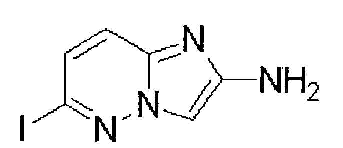

In the same manner as in Reference Example 34 and using ethyl (6-iodoimidazo[1,2-b]pyridazin-2-yl)carbamate (10.2 g, 31 mmol) obtained in Reference Example 35, the title compound (yield 7.0 g, 87%) was obtained.
¹H-NMR (DMSO-d₆) δ: 5.61 (2H, s), 7.23 (1H, d, J = 9.0 Hz), 7.37 (1H, s), 7.39 (1H, d, J = 9.0 Hz).

### Reference Example 37

6-tert-butylnicotinic acid

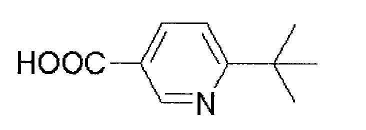

6-tert-Butylnicotinonitrile (10.5 g, 65.5 mmol and Tetrahedron, 1974, 30, 4201) was dissolved in concentrated hydrochloric acid (55 ml) and the mixture was stirred at 100°C for 20 min. After cooling the reaction solution, the solvent was evaporated under reduced pressure, and acetonitrile (100 ml) was added to the obtained crude product. Precipitated white solid was collected by filtration, and washed with acetonitrile and diethyl ether. The organic layers were combined, concentrated again under reduced pressure and a white solid was obtained in the same manner. This was repeated several times and the obtained white solids were combined, dissolved in water (50 ml) and pH was adjusted to weak acidic with 8N aqueous sodium hydroxide solution (pH=3-4) to give a white precipitate. The suspension was concentrated under reduced pressure and azeotroped with acetonitrile to dryness by evaporation. Methylene chloride (500 ml) was added to a mixture of the white solid, and the mixture was dried over anhydrous magnesium sulfate and filtered. The filtrate was concentrated under reduced pressure to give a white oily crude product. This was dissolved in ethyl acetate and concentrated under reduced pressure to give the title compound (yield 11.4 g, yield 97%) as a white solid.
¹H-NMR (DMSO-d₆) δ: 1.33 (9H, s), 7.57 (1H, d, J = 8.3 Hz), 8.19 (1H, dd, J = 8.3, 2.3 Hz), 9.01 (1H, d, J = 2.3 Hz), 12.50-14.20(1H, brs).

### Reference Example 38

1-[2-(tetrahydro-2H-pyran-2-yloxy)ethyl]-4-(4,4,5,5-tetramethyl-1,3,2-dioxaborolan-2-yl)-1H-pyrazole

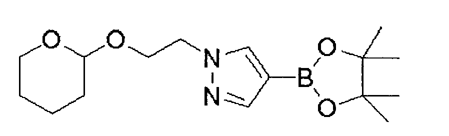

4-(4,4,5,5-Tetramethyl-1,3,2-dioxaborolan-2-yl)-1H-pyrazole (400 mg, 2.06 mmol) was dissolved in N,N-dimethylformamide (10 ml), sodium hydride (60% in mineral oil, 123.7 mg, 3.09 mmol) was added at room temperature and the mixture was stirred for 5 min. 2-(2-Bromoethoxy)tetrahydro-2H-pyrane was added and the mixture was further stirred at room temperature for 1 hr. Thereafter, the reaction solution was poured into water (10 ml), and the mixture was extracted 3 times with diethyl ether (50 ml). The organic layers were combined, washed with saturated brine, and dried over anhydrous magnesium sulfate. After filtration, the solvent was evaporated under reduced pressure to give a colorless oil crude product. This was purified by silica gel chromatography to give a colorless oily title compound (yield 126 mg, 19.0%).
¹H-NMR (CDCl₃) δ: 1.32 (12H, s), 1.40-1.85(6H, m), 3.39-3.48 (1H, m), 3.57-3.68 (1H, m), 3.73-3.83 (1H, m), 4.00-4.08 (1H, m), 4.30-4.37 (2H, m), 4.49-4.54 (1H, m), 7.78 (1H, s), 7.79 (1H, s).

### Reference Example 39

4-tert-butyl-N-(4-tert-butylbenzoyl)-N-(5-methylimidazo[1,2-a]pyridin-2-yl)benzamide

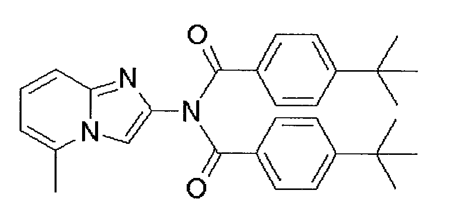

4-tert-Butyl-N-(5-methylimidazo[1,2-a]pyridin-2-yl)benzamide (1.0 g, 3.25 mmol) obtained in Example 65 was placed in acetonitrile (30 ml) and the mixture was heated to 80°C for complete dissolution. 4-tert-Butylbenzoyl chloride (783 mg, 3.90 mmol) and triethylamine (672 mg, 6.64 mmol) were added and the mixture was stirred for 5 min. Disappearance of the starting material was confirmed by thin layer chromatography. After cooling, the reaction mixture was poured into saturated aqueous sodium hydrogen carbonate solution (30 ml), and the mixture was extracted 3 times with ethyl acetate (50 ml). The organic layers were combined, washed with saturated brine, and dried over anhydrous magnesium sulfate. After filtration, the solvent was evaporated under reduced pressure to give an orange crude solid. This was purified by silica gel chromatography (25-30% ethyl acetate/hexane) to give a yellow-green solid. The solid was washed with diethyl ether to give the title compound (yield 1.06 g, 69.7%) as a white solid.
¹H-NMR (CDCl₃) δ: 1.27 (18H, s), 2.49(3H, s), 6.62 (1H, d, J = 6.8 Hz), 7.15 (1H, dd, J = 9.1, 6.8 Hz), 7.34 (4H, d, J = 8.7 Hz), 7.39 (1H, s), 7.49(1H, d, J = 9.1 Hz), 7.80 (4H, d, J = 8.7 Hz).

### Reference Example 40

N-[3-bromo-5-(bromomethyl)imidazo[1,2-a]pyridin-2-yl]-4-tert-butyl-N-(4-tert-butylbenzoyl)benzamide

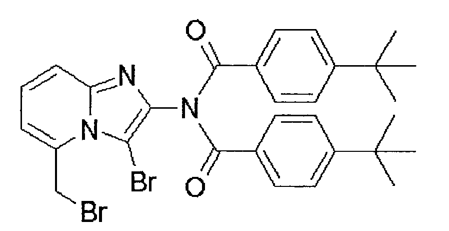

4-tert-Butyl-N-(4-tert-butylbenzoyl)-N-(5-methylimidazo[1,2-a]pyridin-2-yl)benzamide (1.06 g, 2.22 mmol) obtained in Reference Example 39 and N-bromosuccinimide (928 mg, 5.21 mmol) were suspended in carbon tetrachloride (100 ml) and the suspension was stirred under reflux for 5 min to give a yellow solution. The reaction starting material disappeared completely. After cooling the reaction mixture, diphenyl peroxyanhydride (71.8 mg, 0.30 mmol) was added and the mixture was stirred under reflux for 15 min. After cooling, the reaction mixture was poured into saturated aqueous sodium hydrogen carbonate solution (100 ml), and the mixture was extracted 3 times with methylene chloride (100 ml). The organic layers were combined, washed with saturated brine, and dried over anhydrous magnesium sulfate. After filtration, the solvent was evaporated under reduced pressure to give an orange crude solid. This was purified by silica gel chromatography (25-30% ethyl acetate/hexane) to give the title compound (yield 1.10 g, 79.2%) as a yellow white solid.
¹H-NMR (CDCl₃)δ: 1.27 (18H, s), 5.11(3H, s), 6.90 (1H, dd, J = 9.0, 1.3 Hz), 7.17 (1H, dd, J = 9.0, 7.0 Hz), 7.35 (4H, d, J = 8.5 Hz), 7.59(1H, dd, J = 7.0, 1.3 Hz), 7.79 (4H, d, J = 8.5 Hz).

### Reference Example 41

2-amino-6-phenylimidazo[1,2-b]pyridazine

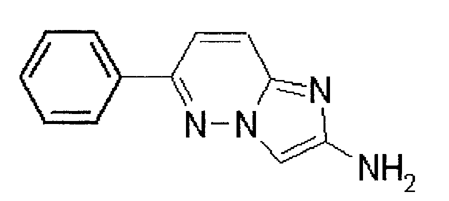

6-Iodoimidazo[1,2-b]pyridazine-2-amine (1.56 g, 6.0 mmol) obtained in Reference Example 36, phenylboronic acid (1.44 g, 12.0 mmol), potassium carbonate (2.46 g, 18.0 mmol), and tetrakis(triphenylphosphine)palladium (0.69 g, 0.6 mmol) were suspended in a mixture of 1,2-dimethoxyethane (30 mL) and water (30 mL), and the suspension was stirred with heating in a microwave reactor at 160°C for 300 sec. The reaction mixture was diluted with water and extracted with ethyl acetate. The ethyl acetate layer was dried over anhydrous magnesium sulfate and concentrated. The residue was purified by silica gel column chromatography to give the title compound (1.03 g, yield 87%) from a fraction eluted with methanol-ethyl acetate (1:9, volume ratio).
¹H-NMR (CDCl₃) δ: 4.09 (2H, s), 7.35 (1H, d, J = 9.3 Hz), 7.41 (1H, s), 7.44 - 7.54(3H, m), 7.69 (1H, d, J = 9.3 Hz), 7.91 (1H, d, J = 9.3 Hz).

In the same manner as in Reference Example 41, the following compounds were synthesized.

### Reference Example 42

2-amino-6-(3-pyridinyl)imidazo[1,2-b]pyridazine

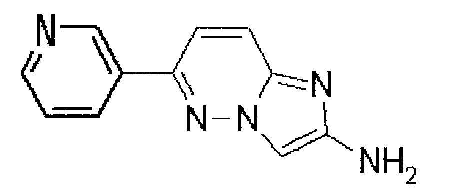

yield61%
¹H-NMR (CDCl₃) δ: 4.14 (2H, s), 7.35 (1H, dd, J = 9.2, 1.6 Hz), 7.38-7.49 (2H, m), 7.73 (1H, d, J = 9.3 Hz), 8.24(1H, dd, J = 8.0, 1.8 Hz), 8.59-8.77 (1H, m), 9.15 (1H, s).

### Reference Example 43

2-amino-6-(4-methoxyphenyl)imidazo[1,2-b]pyridazine

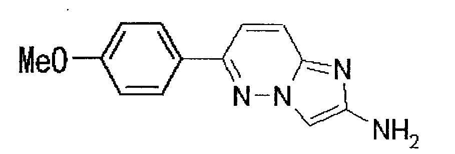

yield 21%
¹H-NMR (CDCl₃) δ: 3.88 (3H, s), 4. 05 (2H, s), 7.01 (2H, d, J = 8.6 Hz), 7.30 (1H, d, J = 9.3 Hz), 7.37 (1H, s), 7.65 (1H, d, J = 9.3 Hz), 7.86 (2H, d, J = 8.6 Hz).

### Reference Example 44

2-tert-butyl-5-methylpyridine

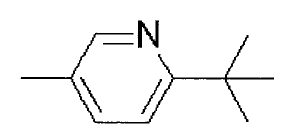

2N-tert-Butylmagnesium chloride - THF solution (120 mmol, 60 mL) was added dropwise to a suspension (200 mL) of copper cyanide (60 mmol, 5400 mg) in THF, and the mixture was stirred at -78°C for 20 min. Then, 2-bromo-5-methylpyridine (12 mmol, 2060 mg) was added and the mixture was stirred at -78°C for 2 hr, and further stirred at room temperature overnight. 25% Aqueous ammonia solution (100 mL) was added, and the mixture was stirred for 30 min and extracted with ethyl acetate. The extract was dried (MgSO₄), and the solvent was evaporated under reduced pressure. The residue was purified by silica gel chromatography (ethyl acetate:hexane=0:10 - 1:9) to give the title compound (1.6 g, 89%).

### Reference Example 46

5-tert-butylpyridine-2-carboxylic acid

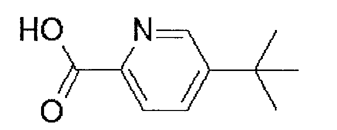

According to Reference Example 44 and then Reference Example 1, the title compound was synthesized from 5-bromo-2-methylpyridine.

### Reference Example 47

6-(trifluoromethyl)imidazo[1,2-a]pyridine-2-amine

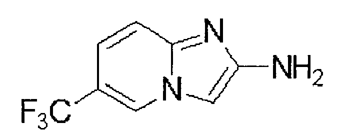

N-[5-(Trifluoromethyl)pyridin-2-yl]methanesulfonamide (22.5 g, 91.8 mmol, Chem. Pharm. Bull. 1995, 43, 1696), 2-iodoacetamide (22.50 g, 119.3 mmol) and diisopropylethylamine (21.2 mL, 119.3 mmol) were dissolved in DMF (180 mL) and the mixture was stirred at room temperature for 12 hr. Then, the solvent was evaporated under reduced pressure and water (500 mL) was added to the obtained oil. The precipitated solid was collected by filtration, washed with water, acetonitrile and diethyl ether, and dried to give a yellow white solid (yield 21.6 g). The solid (20 g) was suspended in methylene chloride (134 mL), trifluoroacetic anhydride (47.4 mL, 336 mmol) was gradually added at room temperature, and the mixture was stirred for 10 min to give a yellow solution. Then, the mixture was further stirred at room temperature for 30 min. The solvent was evaporated under reduced pressure, and the residue was azeotroped with a mixed solvent of 50% ethanol/toluene to give a yellow white solid. Saturated aqueous sodium hydrogen carbonate solution (60 mL) was added thereto, and the mixture was extracted with ethyl acetate, and the organic layer was washed with saturated brine, and dried over anhydrous magnesium sulfate. The solvent was evaporated under reduced pressure to give a yellow white solid (yield 21.0 g) as a crude product. The crude product was dissolved in 20%n-butylamine/ethanol solution (350 mL) and heated under reflux for 6 hr. After cooling the reaction mixture, toluene (100 mL) was added and the reaction solution was concentrated under reduced pressure to give an orange oily crude product. This was purified by silica gel chromatography (80-100% ethyl acetate/hexane) to give the title compound (yield 9.8 g, 64%). ¹H-NMR (DMSO-d₆) δ:4.04 (2H, brs), 6.99 (1H, s), 7.21 (1H, dd, J = 9.5, 1.9 Hz), 7.41 (1H, dd, J = 9.5, 0.8 Hz), 8.27-8.31 (1H, m).

### Reference Example 48

ethyl 4-(2-hydroxy-1,1-dimethylethyl)benzoate

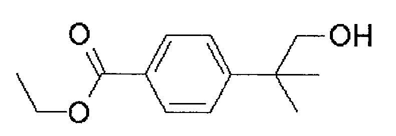

Isobutylaldehyde (200 mmol, 14.4 g), ethyl 4-bromobenzoate (100 mmol, 22.9 g), palladium acetate (2.0 mmol, 450 mg), tri-tert-butylphosphine (4.0 mmol, 809 mg), and cesium carbonate (120 mmol, 39 g) were suspended in dioxane (200 mL), and the mixture was stirred under nitrogen at 110°C for 4 hr. A dioxane insoluble material was removed by silica gel chromatography, and the solvent and isobutylaldehyde were evaporated under reduced pressure. The residue (ethyl 4-(1,1-dimethyl-2-oxoethyl)benzoate) was dissolved in THF (100 mL), sodium borohydride (200 mmol, 7.5 g) was added, and the mixture was stirred for 1 hr. Aqueous saturated ammonium chloride was added, and the mixture was extracted with ethyl acetate.
The extract was dried (MgSO₄), and the solvent was evaporated under reduced pressure. The residue was purified by silica gel chromatography (ethyl acetate:hexane=1:9 - 3:7) to give the title compound (9.8 g, 22%).
¹H-NMR (CDCl₃) δ: 1.34-1.42 (9H, m), 3.65 (2H, d, J = 6.2 Hz), 4.37 (2H, q, J = 7.2 Hz), 7.44-7.49 (2H, m), 7.98-8.04 (2H, m), hidden (1H).

### Reference Example 49

ethyl 4-(2-{[tert-butyl(dimethyl)silyl]oxy}-1,1-dimethylethyl)benzoate

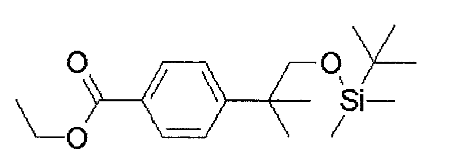

Ethyl 4-(2-hdroxy-1,1-dimethylethyl)benzoate (44.1 mmol, 9.8 g) obtained in Reference Example 48 and 2,6-lutidine (88.2 mmol, 9.5 g) were dissolved in dichloromethane (200 mL), TBDMSOTf (66.1 mmol, 17.5 g) was added, and the mixture was stirred for 1 hr. Water was added and the mixture was extracted with dichloromethane. The extract was dried (MgSO₄), and the solvent was evaporated under reduced pressure. The residue was purified by silica gel chromatography (ethyl acetate:hexane=0.5:9.5 - 2:8) to give the title compound (13.3 g, 90%).
¹H-NMR (CDCl₃) δ: -0.07 (6H, s), 0.83 (9H, s), 1.31 (6H, s), 1.38 (3H, t, J = 7.1 Hz), 3.55 (2H, s), 4.36 (2H, q, J = 7.1 Hz), 7.44 (2H, d, J= 8.4 Hz), 7.96 (2H, d, J = 8.4Hz).

### Reference Example 50

4-(2-{[tert-butyl(dimethyl)silyl]oxy}-1,1-dimethylethyl)benzoic acid

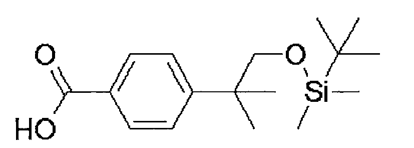

Potassium hydroxide (9.0 mmol, 505 mg), water (10 mL), THF (10 mL) and methanol (10 mL) were added to ethyl 4-(2-{[tert-butyl(dimethyl)silyl]oxy}-1,1-dimethylethyl)benzoate (3.0 mmol, 1040 mg) obtained in Reference Example 49, and the mixture was stirred at 70°C for 1 hr. After evaporation of the solvent, diluted hydrochloric acid was added, and the mixture was extracted with dichloromethane. The extract was dried (MgSO₄), and the solvent was evaporated under reduced pressure to give the title compound (770 mg, 82%).
¹H-NMR (CDCl₃) δ: -0.07 (6H, s), 0.82 (9H, s), 1.32 (6H, s), 3.56 (2H, s), 7.48 (2H, d, J = 8.5 Hz), 8.02 (2H, d, J = 8.5 Hz), hidden (1H).

### Reference Example 51

ethyl 4-(3-hydroxy-1,1-dimethylpropyl)benzoate

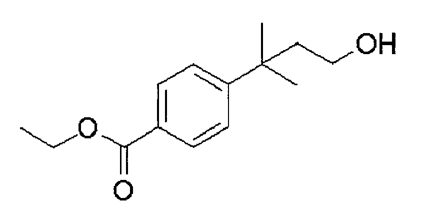

To a suspension of (methoxymethyl)triphenylphosphonium chloride (114.4 mmol, 39.2 g) in THF (300 mL) was added tert-butoxy potassium, and the mixture was stirred at 0°C for 30 min. Then, intermediate ethyl 4-(1,1-dimethyl-2-oxoethyl)benzoate (95.3 mmol, 21.0 g) obtained in Reference Example 48 was added, and the mixture was stirred at room temperature overnight. Saturated brine was added, and the mixture was extracted with ethyl acetate. The extract was dried (MgSO₄), and the solvent was evaporated under reduced pressure. Aqueous 2N-HCl (50 mL) and THF (150 mL) were added to the residue, and the mixture was stirred at 40°C for 1 hr. Saturated brine was added, and the mixture was extracted with ethyl acetate. The extract was dried (MgSO₄), and the solvent was evaporated under reduced pressure. The residue was purified by silica gel chromatography (ethyl acetate:hexane=0.5:9.5 - 3:7) to give an aldehyde form (11.7 g), which was dissolved in THF (200 mL), NaBH₄ (50 mmol, 1.89 g) was added and the mixture was stirred at room temperature overnight. Diluted hydrochloric acid was added and the mixture was extracted with ethyl acetate. The extract was dried (MgSO₄), and the solvent was evaporated under reduced pressure. The residue was purified by silica gel chromatography (ethyl acetate:hexane=1:9 - 1:1) to give the title compound (9.9 g, 41%) as a colorless liquid.
¹H-NMR (DMSO-d₆) δ: 1.22-1.39 (9H, m), 1.78-1.86 (2H, m), 1.99 (1H, s), 3.13-3.22 (2H, m), 4.30 (2H, q, J = 6.7 Hz), 7.49 (2H, d, J = 8.3 Hz), 7.89 (2H, d, J = 8.3 Hz).

### Reference Example 52

4-(3-{[tert-butyl(dimethyl)silyl]oxy}-1,1-dimethylpropyl)benzoic acid

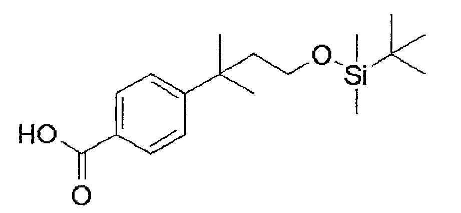

According to Reference Example 49 and Reference Example 50 and using ethyl 4-(3-hydroxy-1,1-dimethylpropyl)benzoate obtained in Reference Example 51, the title compound was synthesized.
¹H-NMR (DMSO-d₆) δ: -0.04 (6H, s), 0.83 (9H, s), 1.35 (6H, s), 1.90 (2H, t, J = 7.4 Hz), 3.41 (2H, t, J = 7.4 Hz), 7.51 (2H, d, J = 8.5 Hz), 7.91 (2H, d, J = 8.5 Hz), hidden (1H).

### Reference Example 53

4-[ethyl(methylsulfonyl)amino]benzoic acid

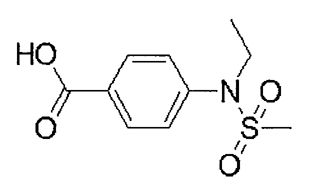

A suspension of 4-methylsulfonylaminobenzoic acid (5 mmol, 1.07 g), potassium carbonate (20 mmol, 2.76 g) and ethyl iodide (20 mmol, 3.12 g) in DMF (50 mL) was stirred at 60°C overnight. Volatile components were evaporated under reduced pressure, saturated brine was added and the mixture was extracted with ethyl acetate. The extract was washed with water, dried (MgSO₄), and the solvent was evaporated. The residue was purified by silica gel chromatography (ethyl acetate:hexane=5:5 - 10:0) to give ethyl ester of the title compound (1.19 g, 88%). Then, the title compound was obtained according to Reference Example 50.
¹H-NMR (DMSO-d₆) δ: 1.02 (3H, t, J = 7.1 Hz), 3.74 (2H, q, J = 7.1 Hz), 7.47-7.53 (2H, m), 7.94-8.01 (2H, m), 13.05 (1H, s).

### Reference Example 54

6-(3-aminophenoxy)imidazo[1,2-b]pyridazine-2-amine

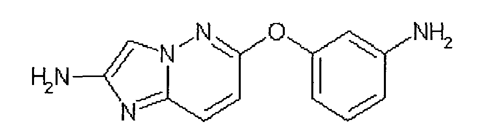

A mixture of 6-iodoimidazo[1,2-b]pyridazine-2-amine (390 mg, 1.5 mmol), 3-aminophenol (491 mg, 4.5 mmol), potassium carbonate (415 mg, 3.0 mmol) and N,N-dimethylformamide (3 mL) was stirred in a microwave reaction apparatus at 180°C for 40 min. The reaction mixture was diluted with water, and extracted with a mixed solvent of ethyl acetate/tetrahydrofuran (1:1). The organic layer was concentrated under reduced pressure, and the residue was subjected to column chromatography (silica gel, ethyl acetate/methanol=100/0→60/40). The object fraction was concentrated under reduced pressure, and the residue was purified by column chromatography (NH silica gel, ethyl acetate/methanol=100/0→80/20), and precipitated from ethyl acetate to give the title compound (133 mg, 37%) as a purple solid.
¹H-NMR (DMSO-d₆, 300 MHz) δ 5.27 (2H, s), 5.31 (2H, s), 6.23 (1H, ddd, J= 7.8, 2.4, 0.9 Hz), 6.27 (1H, t, J = 2.1 Hz), 6.38 (1H, ddd, J = 8.1, 2.1, 0.9 Hz), 6.69 (1H, d, J = 9.3 Hz), 7.02 (1H, t, J = 7.9 Hz), 7.14 (1H, s), 7.68 (1H, dd, J = 9.3, 0.6 Hz).

### Reference Example 55

ethyl 6-(3-aminophenoxy)imidazo[1,2-b]pyridazine-2-carboxylate

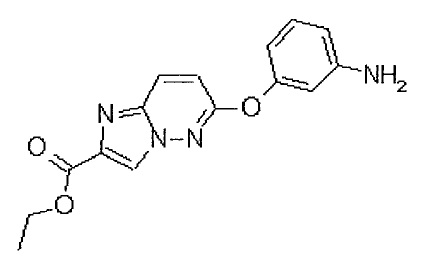

A mixture of ethyl 6-iodoimidazo[1,2-b]pyridazine-2-carboxylate (951 mg, 3.00 mmol), 3-aminophenol (655 mg,6.00 mmol), potassium carbonate (622 mg, 4.50 mmol) and N,N-dimethylformamide (9.0 mL) was stirred at 150°C for 6 hr. The reaction mixture was cooled to room temperature, ethyl acetate and water were added, and insoluble material was filtered off through celite. The organic layer was collected from the filtrate, washed with saturated aqueous sodium hydrogen carbonate, and dried over anhydrous magnesium sulfate. The solvent was evaporated under reduced pressure, and the residue was purified by silica gel column chromatography (ethyl acetate/hexane=1/19→ethyl acetate alone) to give the title compound (350 mg, 39%).
¹H-NMR (DMSO-d₆, 300 MHz) δ 1.30 (3H, t, J = 7.2 Hz), 4.29 (2H, q, J = 7.2 Hz), 5.34 (2H, brs), 6.35-6.48 (3H, m), 7.07 (1H, t, J = 8.1 Hz), 7.17 (1H, d, J = 9.9 Hz), 8.20 (1H, d, J = 9.6 Hz), 8.61 (1H, s).

### Reference Example 56

ethyl 6-(3-{[3-(trifluoromethyl)benzoyl]amino}phenoxy)imidazo[1,2-b]pyridazine-2-carboxylate

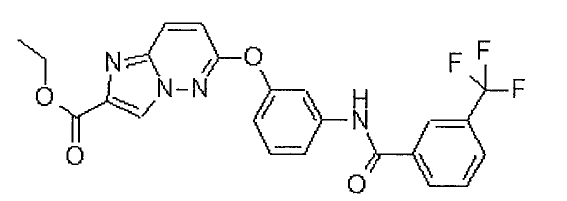

A mixture of ethyl 6-(3-aminophenoxy)imidazo[1,2-b]pyridazine-2-carboxylate (350 mg, 1.17 mmol), 3-(trifluoromethyl)benzoic acid (342 mg, 1.80 mmol), 1-hydroxybenzotriazole (243 mg, 1.80 mmol), 1-ethyl-3-(3-dimethylaminopropyl)carbodiimide hydrochloride (345 mg, 1.80 mmol) and N,N-dimethylformamide (5.0 mL) was stirred at room temperature overnight. The reaction mixture was poured into saturated aqueous sodium hydrogen carbonate, and extracted with ethyl acetate. The extract was dried over magnesium sulfate, and concentrated under reduced pressure. The residue was purified by aminosilica gel column chromatography (hexane-ethyl acetate=19:1 to ethyl acetate alone), and the obtained solid was washed with ethyl acetate-hexane to give the title compound (496 mg, 90%) as a white powder.
¹H-NMR (DMSO-d₆, 300 MHz) δ 1.29 (3H, t, J = 7.1 Hz), 4.29 (2H, q, J = 7.1 Hz), 7.10 (1H, dd, J = 1.5, 7.5 Hz), 7.29 (1H, d, J = 9.9 Hz), 7.49 (1H, t, J = 8.4 Hz), 7.68-7.82 (3H, m), 7.97-8.00 (1H, m), 8.25-8.28 (3H, m), 8.62 (1H, s), 10.63 (1H, s).

### Reference Example 57

6-(3-{[3-(trifluoromethyl)benzoyl]amino}phenoxy)imidazo[1,2-b]pyridazine-2-carboxylic acid

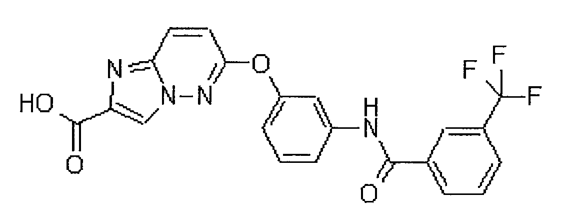

A mixture of ethyl 6-(3-{[3-(trifluoromethyl)benzoyl]amino}phenoxy)imidazo[1,2-b]pyridazine-2-carboxylate (470 mg, 1.00 mmol), 8N aqueous sodium hydroxide solution (2.0 mL) and methanol (6.0 mL) was stirred at room temperature for 4 hr. 6N Hydrochloric acid (2.7 mL) and water (25 mL) were added, and the mixture was extracted with ethyl acetate (50 mL). The extract was dried over anhydrous magnesium sulfate, and the solvent was evaporated under reduced pressure to give the title compound (425 mg, 96%) as a white powder.
¹H-NMR (DMSO-d₆, 300 MHz) δ 7.10 (1H, dd, J = 2.1, 7.8 Hz), 7.27 (1H, d, J = 9.9 Hz), 7.48 (1H, t, J = 7.8 Hz), 7.69-7.82 (3H, m), 7.98 (1H, d, J = 7.5 Hz), 8.23-8.29 (3H, m), 8.52 (1H, s), 10.62 (1H, s), 12.85 (1H, brs).

### Reference Example 58

tert-butyl[6-(3-{[3-(trifluoromethyl)benzoyl]amino}phenoxy)imidazo[1,2-b]pyridazin-2-yl]carbamate

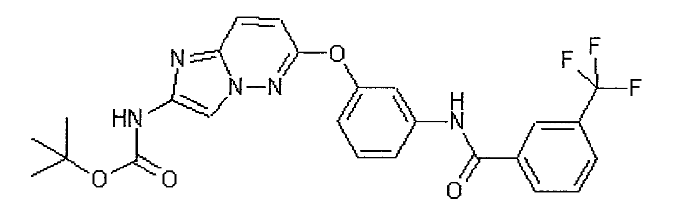

A suspension of 6-(3-{[3-(trifluoromethyl)benzoyl]amino}phenoxy)imidazo[1,2-b]pyridazine-2-carboxylic acid (111 mg, 0.25 mmol), diphenylphosphorylazide (103 mg, 0.375 mmol), triethylamine (51 mg, 0.5 mmol) and t-butanol (5.0 mL) was stirred at room temperature for 5 min, heated to 100°C, and stirred overnight. The reaction mixture was cooled to room temperature, ethyl acetate was added, and the mixture was washed with saturated aqueous sodium hydrogen carbonate, dried over anhydrous magnesium sulfate and filtered. The solvent was evaporated under reduced pressure, and the residue was purified by silica gel column chromatography (ethyl acetate/hexane=1/19→ethyl acetate alone) to give the title compound (79 mg, 39%) as a white solid.
¹H-NMR (DMSO-d₆, 300 MHz) δ 1.47 (9H, s), 7.00-7.05 (2H, m), 7.45 (1H, t, J = 8.1 Hz), 7.65-7.81 (4H, m), 7.96-8.03 (2H, m), 8.24-8.28 (2H, m), 10.04 (1H, brs), 10.58 (1H, brs).

### Reference Example 59

N-(3-[(2-aminoimidazo[1,2-b]pyridazin-6-yl)oxy]phenyl}-3-(trifluoromethyl)benzamide

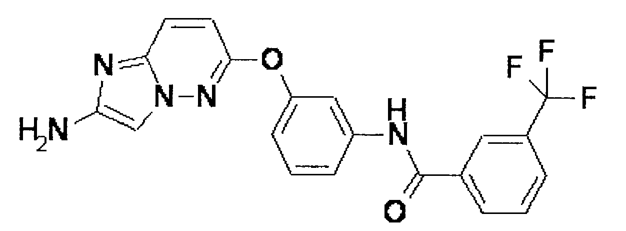

A mixture of tert-butyl [6-(3-{[3-(trifluoromethyl)benzoyl]amino}phenoxy)imidazo[1,2-b]pyridazin-2-yl]carbamate (2.37 g, 4.62 mmol), 4N hydrochloric acid-ethyl acetate solution (50 mL) and methanol (50 mL) was stirred at room temperature for 6 hr. The solvent was evaporated under reduced pressure, and saturated aqueous sodium hydrogen carbonate was added to the residue. The mixture was extracted with ethyl acetate. The extract was dried over anhydrous magnesium sulfate, evaporated under reduced pressure, and the residue was purified by silica gel column chromatography (ethyl acetate alone→methyl acetate/methanol=4:1) to give the title compound (1.57 g, 82%) as a green solid.
¹H-NMR (DMSO-d₆, 300 MHz) δ5.33 (2H, brs), 6.80 (1H, d, J = 9.0 Hz), 6.94 (1H, m), 7.15 (1H, s), 7.42 (1H, t, J = 7.8 Hz), 7.63-7.80 (4H, m), 7.97 (1H, d, J = 7.5 Hz), 8.23-8.27 (2H, m), 10.54 (1H, s).

### Reference Example 60

N-(6-iodoimidazo[1,2-b]pyridazin-2-yl)acetamide

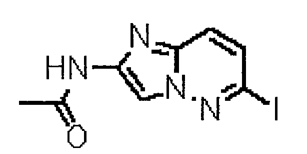

To a solution of 6-iodoimidazo[1,2-b]pyridazine-2-amine (5.0 g, 19.2 mmol) in N,N-dimethylacetamide (60 mL) was added a solution of acetyl chloride (1.64 mL, 23.0 mmol) in N,N-dimethylacetamide (30 mL), and the mixture was stirred at room temperature for 16 hr. Water was added to the reaction mixture and the precipitate was collected by filtration and washed with water to give the title compound (5.21 g, 90%) as a pale-yellow powder.
¹H-NMR (DMSO-d₆, 300 MHz) δ 2.09 (3H, s), 7.49 (1H, d, J = 9.2 Hz), 7.73 (1H, d, J = 9.2 Hz), 8.25 (1H, s), 10.90 (1H, s).

### Reference Example 61

N-[6-(3-aminophenoxy)imidazo[1,2-b]pyridazin-2-yl]acetamide

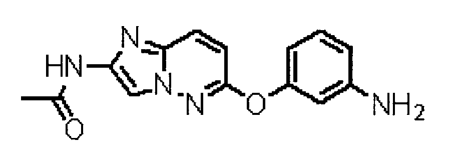

A mixture of N-(6-iodoimidazo[1,2-b]pyridazin-2-yl)acetamide (5.10 g, 16.8 mmol), 3-aminophenol (3.68 g, 33.7 mmol), potassium carbonate (5.34 g, 33.7 mmol) and N,N-dimethylformamide (70 mL) was stirred at 150°C for 6 hr. After cooling to room temperature, ethyl acetate and water were added, and insoluble material was filtered off on celite. The filtrate was extracted with ethyl acetate, and the organic layer was washed with saturated aqueous sodium hydrogen carbonate, dried over anhydrous magnesium sulfate and filtered. The solvent was evaporated under reduced pressure, and the residue was purified by silica gel column chromatography (ethyl acetate/hexane=35/65→100/0) to give the title compound (3.73 g, 78%) as a pale-orange powder.
¹H-NMR (DMSO-d₆, 300 MHz) δ 2.07 (3H, s), 5.30 (2H, s), 6.28-6.33 (1H, m), 6.36 (1H, t, J = 2.2 Hz), 6.41-6.46 (1H, m), 6.95 (1H, d, J = 9.6 Hz), 7.05 (1H, t, J = 8.0 Hz), 7.96-8.02 (2H, m), 10.78 (1H, s).

### Reference Example 62

N-{3-[(2-acetamideimidazo[1,2-b]pyridazin-6-yl)oxy]phenyl}-3-(1-cyanocyclopropyl)benzamide

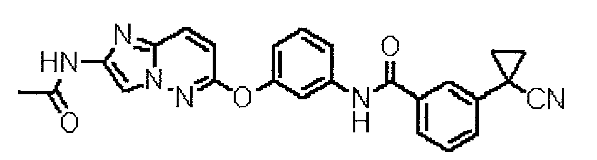

To a solution of 3-(1-cyanocyclopropyl)benzoic acid (1.50 g, 8.01 mmol) in tetrahydrofuran (30 mL) were added N,N-dimethylformamide (2 drops), oxalyl chloride (1.16 mL, 13.3 mmol), and the mixture was stirred at room temperature for 1.5 hr. The solvent was evaporated under reduced pressure, and the residue and N-[6-(3-aminophenoxy)imidazo[1,2-b]pyridazin-2-yl]acetamide (1.89 g, 6.67 mmol) were dissolved in N-methylpyrrolidone (30 mL), and the mixture was stirred at room temperature for 3 hr. Saturated aqueous sodium hydrogen carbonate was added to the reaction mixture, and the mixture was extracted with ethyl acetate. The extract was washed with saturated brine, dried over anhydrous magnesium sulfate and filtered. The residue was purified by silica gel column chromatography (ethyl acetate/hexane=0/100→100/0) to give the title compound (2.25 g, 75%) as a pale-brown powder.
¹H-NMR (DMSO-d₆, 300 MHz) δ 1.58-1.65 (2H, m), 1.77-1.84 (2H, m), 2.07 (3H, s), 6.99-7.11 (2H, m), 7.45 (1H, t, J = 8.2 Hz), 7.51-7.60 (2H, m), 7.62-7.68 (1H, m), 7.72 (1H, t, J = 2.1 Hz), 7.82 (1H, s), 7.84-7.90 (1H, m), 8.00 (1H, s), 8.05 (1H, d, J = 9.6 Hz), 10.42 (1H, s), 10.80 (1H, s).

### Reference Example 63

N-{3-[(2-aminoimidazo[1,2-b]pyridazin-6-yl)oxy]phenyl}-3-(1-cyanocyclopropyl)benzamide

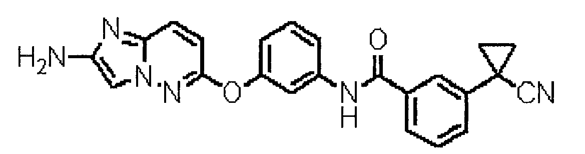

A mixture of N-{3-[(2-acetamideimidazo[1,2-b]pyridazin-6-yl)oxy]phenyl}-3-(1-cyanocyclopropyl)benzamide (2.20 g, 4.86 mmol), 4N hydrochloric acid-ethyl acetate solution (35 mL) and methanol (35 mL) was stirred at room temperature for 14 hr.
The mixture was neutralized with 8N aqueous sodium hydroxide solution, and extracted with ethyl acetate. The extract was washed with saturated brine, dried over anhydrous magnesium sulfate and filtered. The solvent was evaporated under reduced pressure, and the residue was washed with diisopropyl ether to give the title compound (1.63 g, 82%) as a yellow powder.
¹H-NMR (DMSO-d₆, 300 MHz) δ 1.58-1.64 (2H, m), 1.77-1.84 (2H, m), 5.34 (2H, brs), 6.80 (1H, d, J = 9.3 Hz), 6.91-6.97 (1H, m), 7.15 (1H, s), 7.41 (1H, t, J = 8.3 Hz), 7.51-7.67 (4H, m), 7.73 (1H, d, J = 9.3 Hz), 7.81 (1H, s), 7.84-7.89 (1H, m), 10.38 (1H, s).

### Reference Example 64

N-(3-{[2-(acetylamino)imidazo[1,2-b]pyridazin-6-yl]oxy}phenyl)cyclopropanecarboxamide

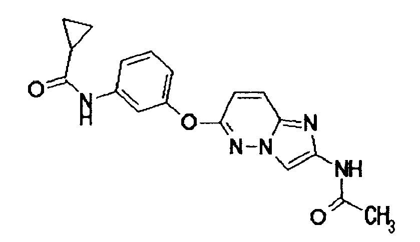

A mixture of N-[6-(3-aminophenoxy)imidazo[1,2-b]pyridazin-2-yl]acetamide (17 mg, 0.06 mmol), cyclopropanecarbonyl chloride (9.5 mg, 0.09 mmol) and N,N-dimethylacetamide (0.5 mL) was stirred at room temperature for 14 hr. The reaction mixture was purified by preparative HPLC to give the title compound (10.8 mg, 51%).
LC-MS 352 (M+H) .

### Reference Example 65

N-{3-[(2-aminoimidazo[1,2-b]pyridazin-6-yl)oxy]phenyl}cyclopropanecarboxamide

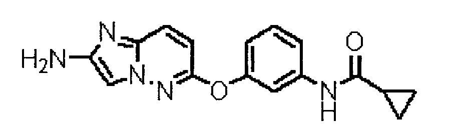

A mixture of N-{3-[(2-acetamideimidazo[1,2-b]pyridazin-6-yl)oxy]phenyl}cyclopropanecarboxamide (465 mg, 1.32 mmol), 4N hydrochloric acid-ethyl acetate solution (10 mL) and methanol (10 mL) was stirred at room temperature for 14 hr. The mixture was neutralized with 8N aqueous sodium hydroxide solution, and the mixture was extracted with ethyl acetate. The extract was washed with saturated brine, dried over anhydrous magnesium sulfate and filtered. The solvent was evaporated under reduced pressure, and the residue was washed with ethyl acetate/diisopropyl ether to give the title compound (376 mg, 92%) as a pale-green powder.
¹H-NMR (DMSO-d₆, 300 MHz) δ 0.75-0.81 (4H, m), 1.69-1.79 (1H, m), 5.33 (2H, s), 6.76 (1H, d, J = 9.3 Hz), 6.80-6.86 (1H, m), 7.14 (1H, s), 7.28-7.39 (2H, m), 7.47 (1H, t, J = 1.9 Hz), 7.71 (1H, d, J = 9.3 Hz), 10.30 (1H, s).

### Example 1

4-cyano-N-(6-phenylimidazo[1,2-b]pyridazin-2-yl)benzamide trifluoroacetate

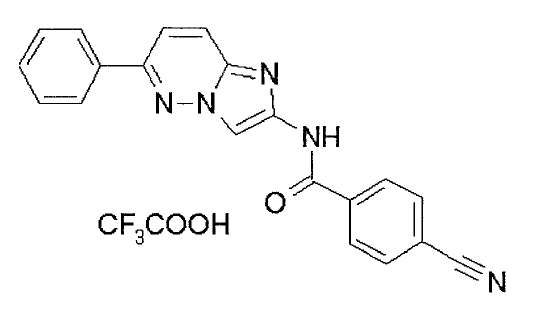

A mixture of 2-amino-6-phenylimidazo[1,2-b]pyridazine (11 mg, 0.05 mmol) obtained in Reference Example 41, 4-cyanobenzoyl chloride (10 mg, 0.06 mmol), N,N-dimethylacetamide (0.5 mL) was stirred at room temperature for 14 hr. Water was added to the reaction mixture, and the mixture was extracted with dichloromethane. The organic layer was filtered with Teflon (registered trade mark) filter, and concentrated. The residue was purified by preparative HPLC to give the title compound (10.1 mg, yield 44%).
LC-MS 340(M+H).

### Examples 2 - 13

In the same manner as in Example 1, various amine forms were reacted with acid chloride to give the objective resultant products at a purity of not less than 80% (LC/MS). The structural formulas of the compounds obtained in Examples 2 - 13 and mass spectrum data are shown in Table 1 and Table 2.

**Table 1**

| Ex. No. | Chemical formula | MS (m/Z) |
|---|---|---|
| 2 | 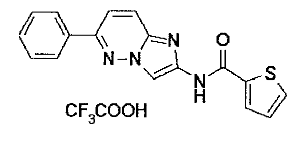 | 321 |
| 3 | 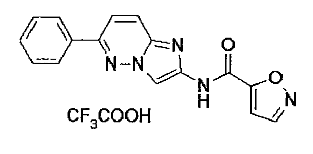 | 306 |
| 4 | 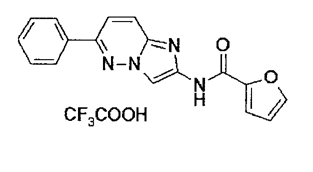 | 305 |
| 5 | 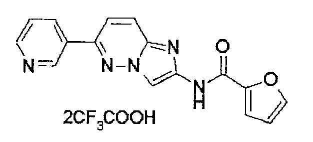 | 306 |
| 6 | 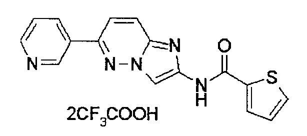 | 322 |
| 7 | 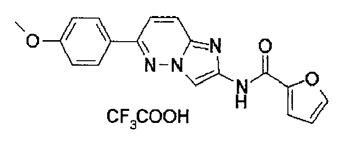 | 335 |
| 8 | 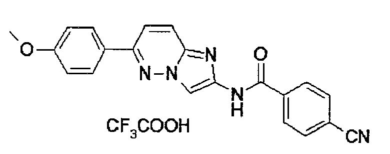 | 370 |
| 9 | 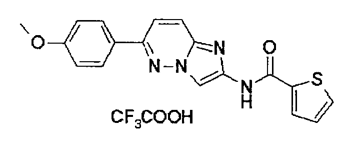 | 351 |
| 10 | 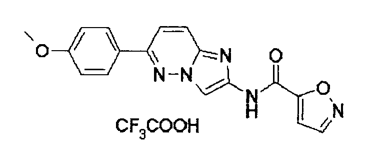 | 336 |

**Table 2**

| Ex. No. | Chemical formula | MS (m/Z) |
|---|---|---|
| 11 | 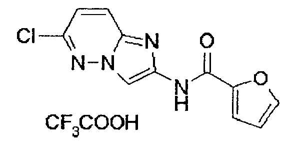 | 263 |
| 12 | 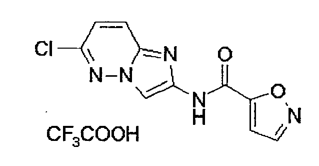 | 264 |
| 13 | 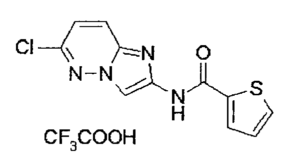 | 279 |

### Example 14

4-tert-butyl-N-(1,2-dihydrofuro[2,3-e]imidazo[1,2-a]pyridin-7-yl)benzamide

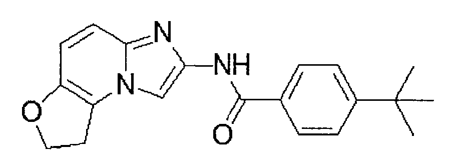

1,2-Dihydrofuro[2,3-e]imidazo[1,2-a]pyridine-7-amine (120 mg, 0.69 mmol) synthesized in Reference Example 17, 4-tert-butylbenzoyl chloride (0.14 mL, 0.73 mmol) and triethylamine (0.24 mL, 1.7 mmol) were dissolved in THF (30 mL) and the mixture was stirred at room temperature for 2 hr. The reaction mixture was diluted with water, and extracted with ethyl acetate/THF. The extract was washed with saturated aqueous sodium hydrogen carbonate solution and saturated brine, dried over anhydrous magnesium sulfate, and the solvent was evaporated under reduced pressure. The obtained residue was purified by basic silica gel column chromatography (50-100% ethyl acetate/hexane). The obtained crude crystal was recrystallized from acetonitrile to give the title compound (yield 49 mg, 21%).
melting point 266-268°C
MS (ESI+): 336 (M+H).
¹H-NMR (DMSO-d₆) δ: 1.32 (9H, s), 3.53 (2H, t, J = 8.9 Hz), 4.75 (2H, t, J = 8.9Hz), 7.06 (1H, d, J = 9.1 Hz), 7.31 (1H, d, J = 9.1 Hz), 7.53 (2H, d, J = 8.3 Hz), 7.97-8.06 (3H, m), 11.13 (1H, s).
Elemental analysis: for C₂₀H₂₁N₃O₂
Calculated: C, 71.62; H, 6.31; N, 12.53.
Found: C, 71.58; H, 6.35; N, 12.58.

### Example 15

methyl 2-[4-(1,2-dihydrofuro[2,3-e]imidazo[1,2-a]pyridin-7-ylcarbamoyl)phenyl]-2-methylpropanoate

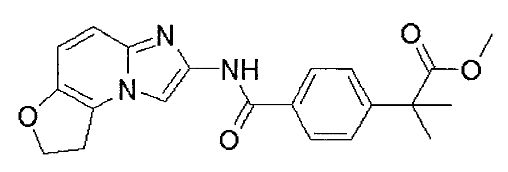

4-(2-Methoxy-1,1-dimethyl-2-oxoethyl)benzoic acid (0.35 g, 1.6 mmol) synthesized in Reference Example 1 was suspended in dichloromethane (10 mL), oxalyl dichloride (0.20 mL, 2.3 mmol) and a catalytic amount of DMF were added, and the mixture was stirred at room temperature for 30 min. The solvent was evaporated under reduced pressure, toluene was added to the residue, and the solvent was evaporated again under reduced pressure. The obtained residue was dissolved in THF (20 mL), 1,2-dihydrofuro[2,3-e]imidazo[1,2-a]pyridine-7-amine (0.24 g, 1.4 mmol) synthesized in Reference Example 17 and triethylamine (0.59 mL, 2.3 mmol) were added, and the mixture was stirred at room temperature for 3 hr. The reaction mixture was diluted with water, and extracted with ethyl acetate/THF. The extract was washed with saturated aqueous sodium hydrogen carbonate solution and saturated brine, dried over anhydrous magnesium sulfate, and the solvent was evaporated under reduced pressure. The residue was purified by basic silica gel column chromatography (50-100% ethyl acetate/hexane) to give the title compound (yield 0.16 g, 30%).
melting point 235-237°C
¹H-NMR (DMSO-d₆) δ: 1.55 (6H, s), 3.53 (2H, t, J = 8.9 Hz), 3.62 (3H, s), 4.75 (2H, t, J = 8.9 Hz), 7.07 (1H, d, J = 9.5 Hz), 7.32 (1H, d, J = 9.5 Hz), 7.44 (2H, d, J = 8.3 Hz), 8.00 (1H, s), 8.04 (2H, d, J = 8.3 Hz), 11.20 (1H, s).
Elemental analysis: for C₂₁H₂₁N₃O₄
Calculated: C, 66.48; H, 5.58; N, 11.08.
Found: C, 66.30; H, 5.50; N, 11.20.

### Example 16

2-[4-(1,2-dihydrofuro[2,3-e]imidazo[1,2-a]pyridin-7-ylcarbamoyl)phenyl]-2-methylpropanoic acid

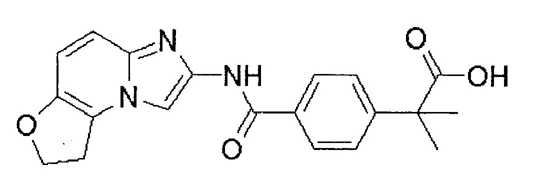

To a mixed solution of methyl 2-[4-(1,2-dihydrofuro[2,3-e]imidazo[1,2-a]pyridin-7-ylcarbamoyl)phenyl]-2-methylpropanoate (0.14 g, 0.36 mmol) synthesized in Example 15 in methanol (10 mL)/THF (10 mL)/water (10 mL) was added 8N aqueous potassium hydroxide solution (0.45 mL, 3.6 mmol), and the mixture was stirred at 50°C for 3 hr. The reaction mixture was concentrated under reduced pressure, and water and ethyl acetate were added thereto. The aqueous layer was acidified with 1N hydrochloric acid, and the resulting white precipitate was collected by filtration. The obtained white precipitate was washed with acetonitrile and diethyl ether and recrystallized from DMSO/ethanol to give the title compound (yield 0.67 g, 51%).
melting point 293-295°C
MS (ESI+): 366 (M+H).
¹H-NMR (DMSO-d₆) δ: 1.51 (6H, s), 3.53 (2H, t, J = 9.1 Hz), 4.75 (2H, t, J = 9.1 Hz), 7.06 (1H, d, J = 9.5 Hz), 7.31 (1H, d, J = 9.5 Hz), 7.47 (2H, d, J = 8.3 Hz), 8.00 (1H, s), 8.04 (2H, d, J = 8.3 Hz), 11.18 (1H, s), 12.52 (1H, br).
Elemental analysis: for C₂₀H₁₉N₃O₄
Calculated: C, 65.74; H, 5.24; N, 11.50.
Found: C, 65.52; H, 5.11; N, 11.41.

### Example 17

6-tert-butyl-N-(1,2-dihydrofuro[2,3-e]imidazo[1,2-a]pyridin-7-yl)nicotinamide

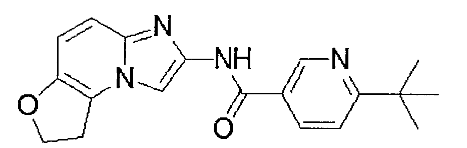

In the same manner as in Example 15 and using 6-tert-butylnicotinic acid (0.11 g, 0.63 mmol) synthesized in Reference Example 37 and 1,2-dihydrofuro[2,3-e]imidazo[1,2-a]pyridine-7-amine (0.10 g, 0.57 mmol) synthesized in Reference Example 17, the title compound (yield 0.10 g, 52%) was obtained.
melting point 246-248°C
MS (ESI+): 337 (M+H).
¹H-NMR (DMSO-d₆) δ: 1.35 (9H, s), 3.53 (2H, t, J = 9.1 Hz), 4.76 (2H, t, J = 9.1 Hz), 7.08 (1H, d, J = 9.5 Hz), 7.33 (1H, d, J = 9.5 Hz), 7.58 (1H, d, J = 8.3 Hz), 8.01 (1H, s), 8.35 (1H, dd, J = 8.3, 1.9 Hz), 9.13 (1H, d, J = 1.9 Hz), 11.39 (1H, s).
Elemental analysis: for C₁₉H₂₀N₄O₂
Calculated: C, 67.84; H, 5.99; N, 16.66.
Found: C, 67.69; H, 5.99; N, 16.66.

### Example 18

6-tert-butyl-N-(1,2-dihydrofuro[2,3-e]imidazo[1,2-a]pyridin-7-yl)nicotinamide dihydrochloride

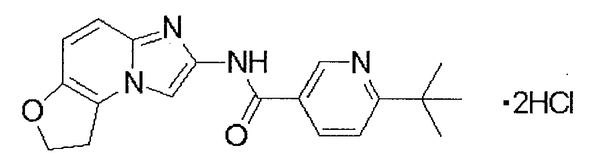

6-tert-Butyl-N-(1,2-dihydrofuro[2,3-e]imidazo[1,2-a]pyridin-7-yl)nicotinamide (50 mg, 0.15 mmol) synthesized in Example 17 was dissolved in methanol, 10% HCl solution in methanol was added, and the mixture was stirred at room temperature for 30 min. The solvent was evaporated under reduced pressure, and the residue was recrystallized from methanol/diethyl ether to give the title compound (yield 50 mg, 81%).
melting point 199-200°C
MS (ESI+): 337 (M+H).
¹H-NMR (DMSO-d₆) δ: 1.39 (9H, s), 3.68 (2H, t, J = 9.1 Hz), 4.86 (2H, t, J = 9.1 Hz), 7.54 (1H, d, J = 9.5 Hz), 7.72-7.76 (2H, m), 8.12 (1H, s), 8.51 (1H, dd, J = 8.3, 1.9 Hz), 9.24 (1H, d, J = 1.9 Hz), 12.50 (1H, s).
Elemental analysis: for C₁₉H₂₀N₄O₂·2HCl+0.1H₂O
Calculated: C, 55.51; H, 5.44; N, 13.63; Cl, 17.25.
Found: C, 55.15; H, 5.29; N, 13.67; Cl. 17.23.

### Example 19

methyl 2-methyl-2-{4-[(6-methylimidazo[1,2-a]pyridin-2-yl)carbamoyl]phenyl}propanoate

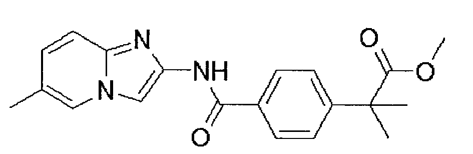

In the same manner as in Example 15 and using 4-(2-methoxy-1,1-dimethyl-2-oxoethyl)benzoic acid (1.9 g, 8.7 mmol) synthesized in Reference Example 1 and 6-methylimidazo[1,2-a]pyridine-2-amine (1.1 g, 7.3 mmol) synthesized in Reference Example 26, the title compound (yield 0.32 g, 12%) was obtained.
melting point 228-230°C
MS (ESI+): 352 (M+H).
¹H-NMR (DMSO-d₆) δ: 1.54 (6H, s), 2.28 (3H, s), 3.61 (3H, s), 7.10 (1H, dd, J = 9.1, 1.7 Hz), 7.36 (1H, d, J = 9.1 Hz), 7.43 (2H, d, J = 8.3 Hz), 8.03 (2H, d, J = 8.3 Hz), 8.19 (1H, s), 8.38 (1H, s), 11.12 (1H, s).
Elemental analysis: for C₂₀H₂₁N₃O₃
Calculated: C, 68.36; H, 6.02; N, 11.96.
Found: C, 68.24; H, 6.04; N, 11.96.

### Example 20

2-[4-(1,2-dihydrofuro[2,3-e]imidazo[1,2-a]pyridin-7-ylcarbamoyl)phenyl]-2-methylpropanoic acid

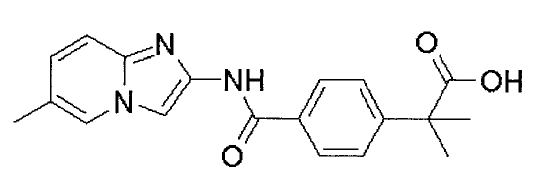

In the same manner as in Example 16 and using methyl 2-methyl-2-{4-[(6-methylimidazo[1,2-a]pyridin-2-yl)carbamoyl]phenyl}propanoate (0.20 g, 0.57 mmol) synthesized in Example 19, the title compound (yield 0.12 g, 62%) was obtained.
melting point 275-277°C
MS (ESI+): 338 (M+H).
¹H-NMR (DMSO-d₆) δ: 1.51 (6H, s), 2.28 (3H, s), 7.10 (1H, dd, J = 9.0, 1.6 Hz), 7.36 (1H, d, J = 9.0 Hz), 7.46 (2H, d, J = 8.7 Hz), 8.03 (2H, d, J = 8.7 Hz), 8.19 (1H, s), 8.39 (1H, s), 11.11 (1H, s), 12.51 (1H, br).
Elemental analysis: for C₁₉H₁₉N₃O₃+0.1H₂O
Calculated: C, 67.28; H, 5.71; N, 12.39.
Found: C, 67.11; H, 5.69; N, 12.24.

### Example 21

ethyl 4-methyl-4-{4-[(6-methylimidazo[1,2-a]pyridin-2-yl)carbamoyl]phenyl}pentanoate

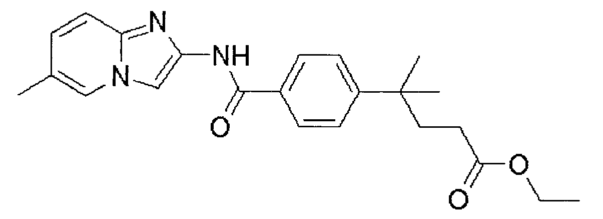

In the same manner as in Example 15 and using 4-(4-ethoxy-1,1-dimethyl-4-oxobutyl)benzoic acid (0.99 g, 3.7 mmol) synthesized in Reference Example 6 and 6-methylimidazo[1,2-a]pyridine-2-amine (0.50 g, 3.4 mmol) synthesized in Reference Example 26, the title compound (yield 0.24 g, 18%) was obtained.
melting point 152-154°C
MS (ESI+): 394 (M+H).
¹H-NMR (DMSO-d₆) δ: 1.12 (3H, t, J = 6.9 Hz), 1.30 (6H, s), 1.81-2.08 (4H, m), 2.28 (3H, s), 3.97 (2H, q, J = 6.9 Hz), 7.10 (1H, dd, J = 9.1, 1.5 Hz), 7.36 (1H, d, J = 9.1 Hz), 7.47 (2H, d, J = 8.7 Hz), 8.03 (2H, d, J = 8.7 Hz), 8.19 (1H, s), 8.38 (1H, s), 11.07 (1H, s).
Elemental analysis: for C₂₃H₂₇N₃O₃
Calculated: C, 70.21; H, 6.92; N, 10.68.
Found: C, 70.21; H, 6.92; N, 10.76.

### Example 22

4-methyl-4-{4-[(6-methylimidazo[1,2-a]pyridin-2-yl)carbamoyl]phenyl}pentanoic acid

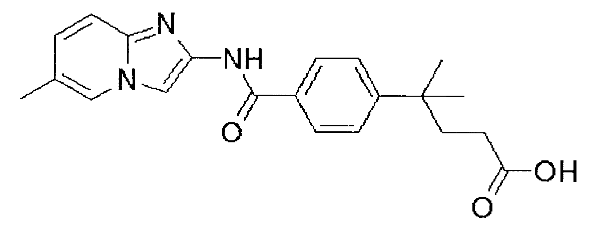

In the same manner as in Example 16 and using ethyl 4-methyl-4-{4-[(6-methylimidazo[1,2-a]pyridin-2-yl)carbamoyl]phenyl}pentanoate (0.21 g, 0.54 mmol) synthesized in Example 21, the title compound (yield 0.15 g, 76%) was obtained.
melting point 280-282°C
MS (ESI+): 366 (M+H).
¹H-NMR (DMSO-d₆) δ: 1.30 (6H, s), 1.88 (4H, s), 2.28 (3H, s), 7.08-7.11 (1H, m), 7.36 (1H, d, J = 9.1 Hz), 7.47 (2H, d, J = 8.3 Hz), 8.02 (2H, d, J = 8.3 Hz), 8.19 (1H, s), 8.39 (1H, s), 11.07 (1H, s).
Elemental analysis: for C₂₁H₂₃N₃O₃
Calculated: C, 69.02; H, 6.34; N, 11.50.
Found: C, 68.93; H, 6.26; N, 11.55.

### Example 23

methyl 2-{4-[(6-iodoimidazo[1,2-a]pyridin-2-yl)carbamoyl]phenyl}-2-methylpropanoate

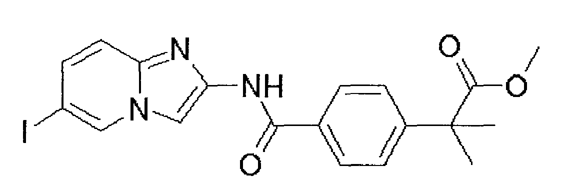

In the same manner as in Example 15 and using 4-(2-methoxy-1,1-dimethyl-2-oxoethyl)benzoic acid (4.7 g, 21 mmol) synthesized in Reference Example 1 and 6-iodoimidazo[1,2-a]pyridine-2-amine (5.0 g, 19 mmol) synthesized in Reference Example 23, the title compound (yield 3.3 g, 37%) was obtained.
melting point 234-235°C
MS (ESI+): 464 (M+H).
¹H-NMR (DMSO-d₆) δ: 1.54 (6H, s), 3.62 (3H, s), 7.32 (1H, d, J = 9.1 Hz), 7.40-7.45 (3H, m), 8.03 (2H, d, J = 8.3 Hz), 8.28 (1H, s), 8.97 (1H, s), 11.23 (1H, s).
Elemental analysis: for C₁₉H₁₈N₃O₃I
Calculated: C, 49.26; H, 3.92; N, 9.07.
Found: C, 49.20; H, 3.86; N, 9.28.

### Example 24

methyl 2-methyl-2-(4-{[6-(3-thienyl)imidazo[1,2-a]pyridin-2-yl]carbamoyl}phenyl)propanoate

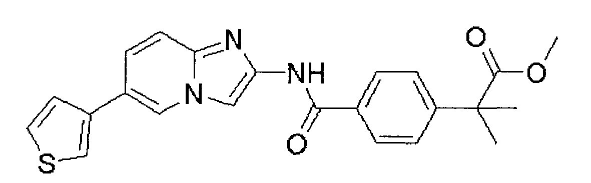

Methyl 2-{4-[(6-iodoimidazo[1,2-a]pyridin-2-yl)carbamoyl]phenyl}-2-methylpropanoate (0.50 g, 1.1 mmol) synthesized in Example 23, 3-thienylboronic acid (0.41 g, 3.2 mmol), tetrakis(triphenylphosphine)palladium(0) (0.12 g, 0.11 mmol) and sodium carbonate (0.69 g, 6.5 mmol) were dissolved in a mixed solution of 1,2-dimethoxyethane (25 mL)/water (5 mL), and the mixture was stirred under a nitrogen atmosphere at 100°C overnight. The reaction mixture was allowed to cool to room temperature, diluted with water, and extracted with ethyl acetate/THF. The extract was washed with saturated brine, dried over anhydrous magnesium sulfate, and the solvent was evaporated under reduced pressure. The residue was purified by silica gel column chromatography (20-50% ethyl acetate/hexane). The obtained crude crystals were recrystallized from acetonitrile to give the title compound (yield 0.28 g, 67%). melting point 200-201°C
MS (ESI+): 420 (M+H).
¹H-NMR (DMSO-d₆) δ: 1.55 (6H, s), 3.62 (3H, s), 7.45 (2H, d, J = 8.3 Hz), 7.51 (1H, d, J = 9.1 Hz), 7.59 (1H, d, J = 4.9 Hz), 7.66-7.73 (2H, m), 7.91-7.92 (1H, m), 8.05 (2H, d, J = 8.3 Hz), 8.29 (1H, s), 9.04 (1H, s), 11.22 (1H, s).
Elemental analysis: for C₂₃H₂₁N₃O₃S
Calculated: C, 65.85; H, 5.05; N, 10.02.
Found: C, 65.89; H, 5.06; N, 10.09.

### Example 25

2-methyl-2-(4-{[6-(3-thienyl)imidazo[1,2-a]pyridin-2-yl]carbamoyl}phenyl)propanoic acid

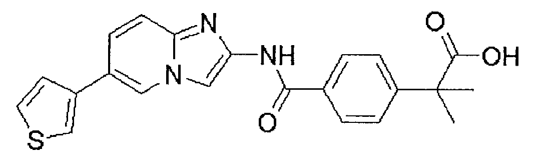

In the same manner as in Example 16 and using methyl 2-methyl-2-(4-{[6-(3-thienyl)imidazo[1,2-a]pyridin-2-yl]carbamoyl}phenyl)propanoate (0.20 g, 0.47 mmol) synthesized in Example 24, the title compound (yield 0.10 g, 53%) was obtained.
melting point 304-306°C
MS (ESI+): 406 (M+H).
¹H-NMR (DMSO-d₆) δ: 1.51 (6H, s), 7.46-7.52 (3H, m), 7.59 (1H, dd, J = 4.9, 1.3 Hz), 7.67 (1H, dd, J = 9.3, 1.7 Hz), 7.71 (1H, dd, J = 4.9, 2.8 Hz), 7.91 (1H, dd, J = 2.8, 1.3 Hz), 8.05 (2H, d, J = 8.3 Hz), 8.29 (1H, s), 9.04 (1H, s), 11.21 (1H, s), 12.51 (1H, br).
Elemental analysis: for C₂₂H₁₉N₃O₃S
Calculated: C, 65.17; H, 4.72; N, 10.36.
Found: C, 64.93; H, 4.80; N, 10.32.

### Example 26

methyl 2-methyl-2-(4-{[6-(3-furyl)imidazo[1,2-a]pyridin-2-yl]carbamoyl}phenyl)propanoate

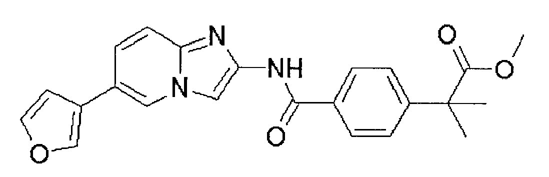

In the same manner as in Example 24 and using methyl 2-{4-[(6-iodoimidazo[1,2-a]pyridin-2-yl)carbamoyl]phenyl}-2-methylpropanoate (0.50 g, 1.1 mmol) synthesized in Example 23 and 3-furylboronic acid (0.36 g, 3.2 mmol), the title compound (yield 0.26 g, 61%) was obtained.
melting point 202-203°C
MS (ESI+): 404 (M+H).
¹H-NMR (DMSO-d₆) δ: 1.55 (6H, s), 3.62 (3H, s), 6.97 (1H, s), 7.43-7.57 (4H, m), 7.79 (1H, d, J = 1.5 Hz), 8.05 (2H, d, J = 8.3 Hz), 8.23-8.25 (2H, m), 8.91 (1H, s), 11.21 (1H, s). Elemental analysis: for C₂₃H₂₁N₃O₄
Calculated: C, 68.47; H, 5.25; N, 10.42.
Found: C, 68.47; H, 5.32; N, 10.52.

### Example 27

2-methyl-2-(4-{[6-(3-furyl)imidazo[1,2-a]pyridin-2-yl]carbamoyl}phenyl)propanoic acid

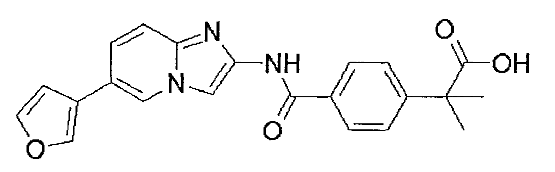

In the same manner as in Example 16 and using methyl 2-methyl-2-(4-{[6-(3-furyl)imidazo[1,2-a]pyridin-2-yl]carbamoyl}phenyl)propanoate (0.18 g, 0.43 mmol) synthesized in Example 26, the title compound (yield 76 mg, 45%) was obtained.
melting point 307-309°C
MS (ESI+): 390 (M+H).
¹H-NMR (DMSO-d₆) δ: 1.51 (6H, s), 6.98 (1H, s), 7.46-7.50 (3H, m), 7.79 (1H, d, J = 1.5 Hz), 8.05 (2H, d, J = 8.3 Hz), 8.23-8.26 (2H, m), 8.91 (1H, s), 11.20 (1H, s), 12.51 (1H, br). Elemental analysis: for C₂₂H₁₉N₃O₄+0.1H₂O
Calculated: C, 67.54; H, 4.95; N, 10.74.
Found: C, 67.31; H, 4.94; N, 10.58.

### Example 28

methyl 2-methyl-2-(4-{[6-(1H-pyrazol-4-yl)imidazo[1,2-a]pyridin-2-yl]carbamoyl}phenyl)propanoate

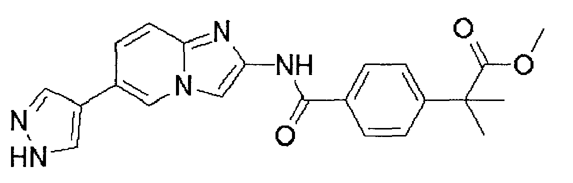

In the same manner as in Example 24 and using methyl 2-{4-[(6-iodoimidazo[1,2-a]pyridin-2-yl)carbamoyl]phenyl}-2-methylpropanoate (0.50 g, 1.1 mmol) synthesized in Example 23 and 1H-pyrazol-4-ylboronic acid (0.36 g, 3.2 mmol), the title compound (yield 90 mg, 21%) was obtained.
melting point 248-249°C
¹H-NMR (DMSO-d₆) δ: 1.55 (6H, s), 3.62 (3H, s), 7.43-7.56 (4H, m), 8.05 (2H, d, J = 8.7 Hz), 8.16 (2H, br), 8.23 (1H, s), 8.89 (1H, s), 11.20 (1H, s), 13.02 (1H, br).

### Example 29

2-methyl-2-(4-{[6-(1H-pyrazol-4-yl)imidazo[1,2-a]pyridin-2-yl]carbamoyl}phenyl)propanoic acid

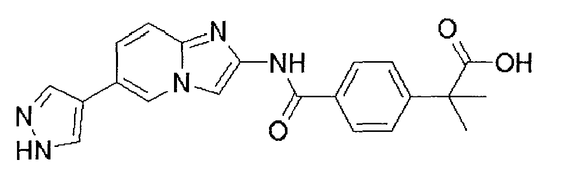

In the same manner as in Example 16 and using methyl 2-methyl-2-(4-{[6-(1H-pyrazol-4-yl)imidazo[1,2-a]pyridin-2-yl]carbamoyl}phenyl)propanoate (0.11 g, 0.27 mmol) synthesized in Example 28, the title compound (yield 76 mg, 45%) was obtained.
melting point 306-307°C
MS (ESI+): 390 (M+H).
¹H-NMR (DMSO-d₆) δ: 1.51 (6H, s), 7.46-7.49 (3H, m), 7.54 (1H, dd, J = 9.1, 1.9Hz), 7.96-8.10 (4H, m), 8.23 (1H, s), 8.88 (1H, s), 11.17 (1H, s), 12.76 (2H, br).
Elemental analysis: for C₂₁H₁₉N₅O₃+0.3H₂O
Calculated: C, 63.88; H, 5.00; N, 17.74.
Found: C, 63.96; H, 4.99; N, 17.40.

### Example 30

methyl 2-{4-[(6-chloroimidazo[1,2-a]pyridin-2-yl)carbamoyl]phenyl}-2-methylpropanoate

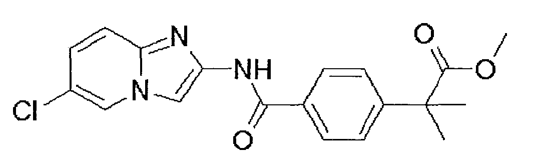

In the same manner as in Example 15 and using 4-(2-methoxy-1,1-dimethyl-2-oxoethyl)benzoic acid (0.36 g, 1.7 mmol) synthesized in Reference Example 1 and 6-chloroimidazo[1,2-a]pyridine-2-amine (0.23 g, 1.4 mmol) synthesized in Reference Example 21, the title compound (yield 0.21 g, 41%) was obtained.
melting point 245-246°C
¹H-NMR (DMSO-d₆) δ: 1.54 (6H, s), 3.61 (3H, s), 7.28 (1H, dd, J = 9.3, 2.0 Hz), 7.43 (2H, d, J = 8.1 Hz), 7.50 (1H, d, J = 9.3 Hz), 8.03 (2H, d, J = 8.1 Hz), 8.32 (1H, s), 8.87-8.88 (1H, m), 11.27 (1H, s).
Elemental analysis: for C₁₉H₁₈N₃O₃Cl
Calculated: C, 61.38; H, 4.88; N, 11.30.
Found: C, 61.30; H, 4.99; N, 11.43.

### Example 31

2-{4-[(6-chloroimidazo[1,2-a]pyridin-2-yl)carbamoyl]phenyl}-2-methylpropanoic acid

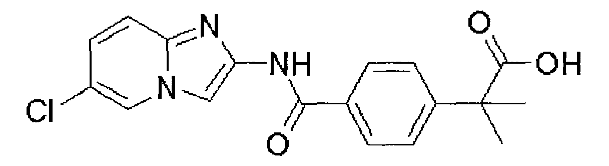

In the same manner as in Example 16 and using methyl 2-{4-[(6-chloroimidazo[1,2-a]pyridin-2-yl)carbamoyl]phenyl}-2-methylpropanoate (1.2 g, 0.27 mmol) synthesized in Example 30, the title compound (yield 0.73 g, 64%) was obtained.
melting point 311-313°C
MS (ESI+): 358 (M+H).
¹H-NMR (DMSO-d₆) δ: 1.51 (6H, s), 7.28 (1H, dd, J = 9.5, 2.2 Hz), 7.46-7.52 (3H, m), 8.03 (2H, d, J = 8.5 Hz), 8.33 (1H, s), 8.88 (1H, dd, J = 2.2, 0.9 Hz), 11.27 (1H, s), 12.52 (1H, br). Elemental analysis: for C₁₈H₁₆N₃O₃Cl+0.2H₂O
Calculated: C, 59.82; H, 4.57; N, 11.63.
Found: C, 59.81; H, 4.63; N, 11.41.

### Example 32

N-(6-chloroimidazo[1,2-a]pyridin-2-yl)-4-(3-cyano-1,1-dimethylpropyl)benzamide

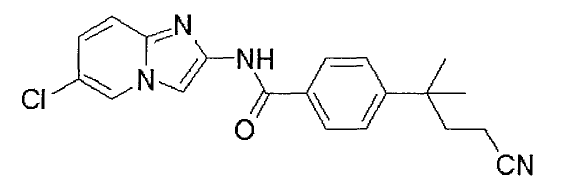

In the same manner as in Example 15 and using 4-(3-cyano-1,1-dimethylpropyl)benzoic acid (0.71 g, 3.3 mmol) synthesized in Reference Example 3 and 6-chloroimidazo[1,2-a]pyridine-2-amine (0.50 g, 3.0 mmol) synthesized in Reference Example 21, the title compound (yield 0.60 g, 55%) was obtained.
melting point 198-199°C
MS (ESI+): 367 (M+H).
¹H-NMR (DMSO-d₆) δ: 1.32 (6H, s), 2.00 (2H, t, J = 8.3 Hz), 2.18 (2H, t, J = 8.3 Hz), 7.29 (1H, dd, J = 9.5, 1.9 Hz), 7.49-7.52 (3H, m), 8.05 (2H, d, J = 8.3 Hz), 8.33 (1H, s), 8.88 (1H, d, J = 1.9 Hz), 11.24 (1H, s).
Elemental analysis: for C₂₀H₁₉N₄OCl+0.5H₂O
Calculated: C, 63.91; H, 5.36; N, 14.91.
Found: C, 63.94; H, 5.24; N, 15.17.

### Example 33

N-(6-chloroimidazo[1,2-a]pyridin-2-yl)-4-[2-(hydroxyamino)-1,1-dimethyl-2-oxoethyl]benzamide

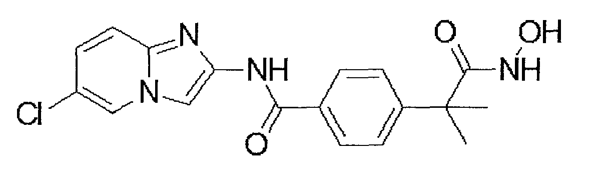

Using 2-{4-[(6-chloroimidazo[1,2-a]pyridin-2-yl)carbamoyl]phenyl}-2-methylpropanoic acid (0.10 g, 0.28 mmol) synthesized in Example 31 and hydroxylamine hydrochloride (53 mg, 0.42 mmol) and in the same manner as in Example 15, the reaction was performed. The obtained crude crystals were recrystallized from acetonitrile to give the title compound (yield 40 mg, 12%).
melting point 212-213°C
MS (ESI+): 373 (M+H).
¹H-NMR (DMSO-d₆) δ: 1.48 (6H, s), 7.29 (1H, dd, J = 9.5, 1.9 Hz), 7.44 (2H, d, J = 8.3 Hz), 7.50 (1H, d, J = 9.5 Hz), 8.03 (2H, d, J = 8.3 Hz), 8.33 (1H, s), 8.74 (1H, s), 8.89 (1H, d, J = 1.9 Hz), 10.34 (1H, s), 11.25 (1H, s).
Elemental analysis: for C₁₈H₁₇N₄O₃Cl
Calculated: C, 57.99; H, 4.60; N, 15.03.
Found: C, 58.36; H, 4.71; N, 14.82.

### Example 34

N-(6-chloroimidazo[1,2-a]pyridin-2-yl)-4-{2-[(cyanomethyl)amino]-cyano-1,1-dimethyl-2-oxoethyl}benzamide

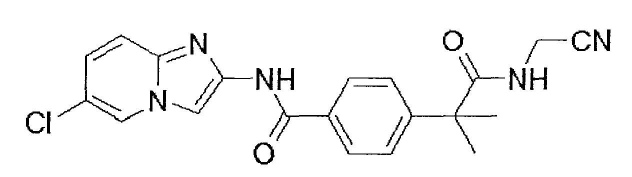

2-{4-[(6-Chloroimidazo[1,2-a]pyridin-2-yl)carbamoyl]phenyl}-2-methylpropanoic acid (0.25 g, 0.70 mmol) synthesized in Example 31, aminoacetonitrile (0.12 g, 2.1 mmol), WSC (0.16 g, 0.84 mmol) and HOBt (0.11 g, 0.84 mmol) were dissolved in DMF (10 mL), and the mixture was stirred at room temperature for 24 hr. The reaction mixture was diluted with water, and extracted with ethyl acetate/THF. The extract was dried over anhydrous magnesium sulfate and the solvent was evaporated under reduced pressure. The residue was recrystallized from acetonitrile to give the title compound (yield 0.13 g, 45%).
melting point 268-269°C
MS (ESI+): 396 (M+H).
¹H-NMR (DMSO-d₆) δ: 1.50 (6H, s), 4.07 (2H, d, J = 5.5 Hz), 7.29 (1H, dd, J = 9.5, 1.5 Hz), 7.42 (2H, d, J = 8.3 Hz), 7.50 (1H, d, J = 9.5 Hz), 8.06 (2H, d, J = 8.3 Hz), 8.20 (1H, t, J = 5.5 Hz), 8.33 (1H, s), 8.88 (1H, d, J = 1.5 Hz), 11.27 (1H, s).
Elemental analysis: for C₂₀H₁₈N₅O₂Cl
Calculated: C, 60.68; H, 4.58; N, 17.69.
Found: C, 60.41; H, 4.48; N, 17.67.

### Example 35

N-(6-chloroimidazo[1,2-a]pyridin-2-yl)-4-{2-[(2-cyanoethyl)amino]-1,1-dimethyl-2-oxoethyl}benzamide

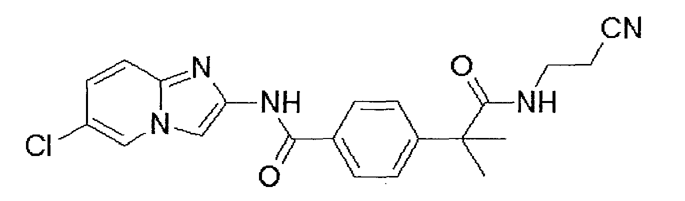

In the same manner as in Example 34 and using 2-{4-[(6-chloroimidazo[1,2-a]pyridin-2-yl)carbamoyl]phenyl}-2-methylpropanoic acid (0.20 g, 0.56 mmol) synthesized in Example 31 and 3-aminopropanenitrile (78 mg, 1.1 mmol), the title compound (yield 0.19 g, 84%) was obtained.
melting point 208-209°C
MS (ESI+): 410 (M+H).
¹H-NMR (DMSO-d₆) δ: 1.49 (6H, s), 2.64 (2H, t, J = 6.4 Hz), 3.27-3.30 (2H, m), 7.29 (1H, dd, J = 9.5, 2.3 Hz), 7.44 (2H, d, J = 8.3 Hz), 7.50 (1H, d, J = 9.5 Hz), 7.80 (1H, t, J = 5.7 Hz), 8.04 (2H, d, J = 8.3 Hz), 8.33 (1H, s), 8.88 (1H, d, J = 2.3 Hz), 11.25 (1H, s).

### Example 36

N-(6-chloroimidazo[1,2-a]pyridin-2-yl)-4-[1,1-dimethyl-3-(1H-tetrazol-5-yl)propyl]benzamide

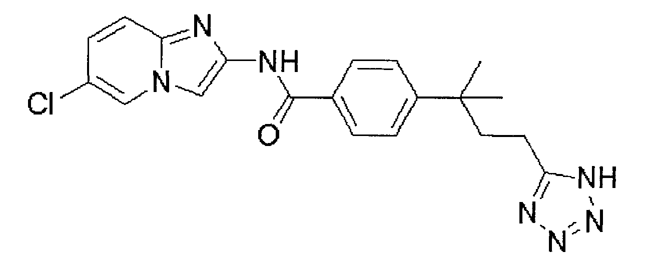

N-(6-Chloroimidazo[1,2-a]pyridin-2-yl)-4-(3-cyano-1,1-dimethylpropyl)benzamide (0.30 g, 0.82 mmol) synthesized in Example 32, azidotrimethylsilane (3.0 mL, 22.6 mmol) and dibutyltin oxide (IV) (40 mg, 0.16 mmol) were heated under reflux in toluene (30 mL) for 24 hr, and the title compound (yield 0.60 g, 55%) was obtained using 6-chloroimidazo[1,2-a]pyridine-2-amine (0.50 g, 3.0 mmol) synthesized in Reference Example 21. The solvent was evaporated under reduced pressure, and methanol was added to the residue. The solvent was evaporated under reduced pressure. 1N Aqueous sodium hydroxide solution (5 mL) was added to the residue, and the mixture was diluted with water and washed with ethyl acetate. The obtained aqueous layer was acidified with 1N hydrochloric acid, and the mixture was extracted with ethyl acetate. The extract was dried over anhydrous magnesium sulfate and the solvent was evaporated under reduced pressure. The residue was recrystallized from acetonitrile to give the title compound (yield 0.15 g, 45%).
melting point 250-251°C
MS (ESI+): 410 (M+H).
¹H-NMR (DMSO-d₆) δ: 1.37 (6H, s), 2.06-2.11 (2H, m), 2.57-2.62 (2H, m), 7.33 (1H, dd, J = 9.5, 1.9 Hz), 7.52-7.57 (3H, m), 8.06 (2H, d, J = 8.3 Hz), 8.33 (1H, s), 8.90 (1H, d, J = 1.9 Hz), 11.27 (1H, s).
Elemental analysis: for C₂₀H₂₀N₇OCl
Calculated: C, 58.61; H, 4.92; N, 23.92.
Found: C, 58.48; H, 4.87; N, 23.95.

### Example 37

N-(6-chloroimidazo[1,2-a]pyridin-2-yl)-4-[1,1-dimethyl-dimethyl-3-(5-oxo-4,5-dihydro-1,2,4-oxadiazol-3-yl)propyl]benzamide

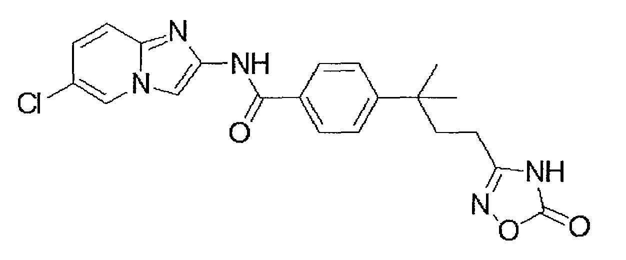

N-(6-Chloroimidazo[1,2-a]pyridin-2-yl)-4-(3-cyano-1,1-dimethylpropyl)benzamide (0.15 g, 0.41 mmol) synthesized in Example 32, hydroxylamine hydrochloride (0.28 g, 4.1 mmol) and sodium hydrogen carbonate (0.41 g, 4.9 mmol) were stirred in DMSO (7 mL) at 60°C overnight. The reaction mixture was diluted with water, and extracted with ethyl acetate/THF. The extract was dried over anhydrous magnesium sulfate and the solvent was evaporated under reduced pressure. The obtained residue was dissolved in THF (10 mL), N,N'-carbonyldiimidazole (0.10 g, 0.61 mmol) and 1,8-diazabicyclo[5.4.0]undec-7-ene (93 mg, 0.61 mmol) were added, and the mixture was stirred at room temperature overnight. The reaction mixture was diluted with water, and extracted with ethyl acetate/THF. The extract was dried over anhydrous magnesium sulfate and the solvent was evaporated under reduced pressure. The obtained residue was purified by silica gel column chromatography (0-5% methanol/ethyl acetate). The obtained crude crystal was recrystallized from acetonitrile to give the title compound (yield 24 mg, 15%).
MS (ESI+): 426 (M+H).
¹H-NMR (DMSO-d₆) δ: 1.34 (6H, s), 1.95-2.00 (2H, m), 2.17-2.20 (2H, m), 7.28 (1H, dd, J = 9.5, 2.1 Hz), 7.48-7.52 (3H, m), 8.05 (2H, d, J = 8.5 Hz), 8.32 (1H, s), 8.88 (1H, d, J = 1.9 Hz), 11.23 (1H, s), 12.10 (1H, br).
Elemental analysis: for C₂₁H₂₀N₅O₃Cl
Calculated: C, 59.23; H, 4.73; N, 16.44.
Found: C, 59.18; H, 4.88; N, 16.65.

### Example 38

N-(6-chloroimidazo[1,2-a]pyridin-2-yl)-4-(1-cyano-1-methylethyl)benzamide

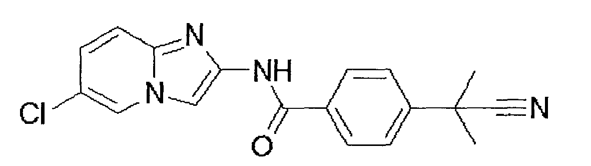

In the same manner as in Example 15 and using 4-(1-cyano-1-methylethyl)benzoic acid (1.2 g, 6.6 mmol) synthesized in Reference Example 2 and 6-chloroimidazo[1,2-a]pyridine-2-amine (1.0 g, 6.0 mmol) synthesized in Reference Example 21, the title compound (yield 0.95 g, 47%) was obtained.
melting point 248-250°C
MS (ESI+): 339 (M+H).
¹H-NMR (DMSO-d₆) δ: 1.73 (6H, s), 7.29 (1H, d, J = 9.4 Hz), 7.51 (1H, d, J = 9.4 Hz), 7.66 (2H, d, J = 8.6 Hz), 8.13 (2H, d, J = 8.6 Hz), 8.34 (1H, s), 8.89 (1H,s), 11.35 (1H,s). Elemental analysis: for C₁₈H₁₅N₄OCl+0.7H₂O
Calculated: C, 61.52; H, 4.70; N, 15.94.
Found: C, 61.31; H, 4.40; N, 16.09.

### Example 39

N-(6-chloroimidazo[1,2-a]pyridin-2-yl)-4-[1-methyl-1-(1H-tetrazol-5-yl)ethyl]benzamide

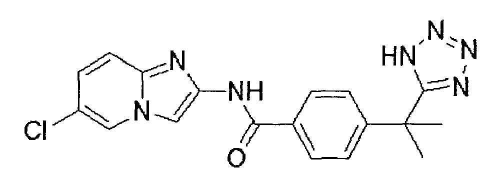

In the same manner as in Example 36 and using N-(6-chloroimidazo[1,2-a]pyridin-2-yl)-4-(1-cyano-1-methylethyl)benzamide (0.20 g, 0.59 mmol) synthesized in Example 38, the title compound (yield 0.10 g, 43%) was obtained.
melting point 266-267°C
MS (ESI+): 382 (M+H).
¹H-NMR (DMSO-d₆) δ: 1.81 (6H, s), 7.27-7.33 (3H, m), 7.50 (1H, d, J = 9.5 Hz), 8.03 (2H, d, J = 8.7 Hz), 8.32 (1H, s), 8.88 (1H, d, J = 1.5 Hz), 11.27 (1H, s).
Elemental analysis: for C₁₈H₁₆N₇OCl
Calculated: C, 56.09; H, 4.29; N, 25.44.
Found: C, 55.95; H, 4.42; N, 25.27.

### Example 40

N-(6-chloroimidazo[1,2-a]pyridin-2-yl)-4-[1-methyl-1-(5-oxo-4,5-dihydro-1,2,4-oxadiazol-3-yl)ethyl]benzamide

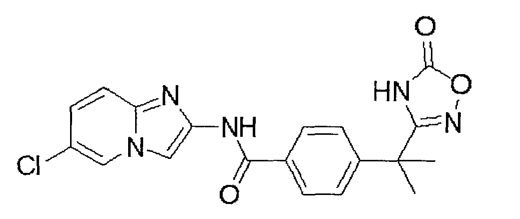

In the same manner as in Example 37 and using N-(6-chloroimidazo[1,2-a]pyridin-2-yl)-4-(1-cyano-1-methylethyl)benzamide (68 mg, 0.20 mmol) synthesized in Example 38, the title compound (yield 13 mg, 16%) was obtained. melting point 310-312°C
MS (ESI+): 397 (M+H).
¹H-NMR (DMSO-d₆) δ: 1.63 (6H, s), 7.26-7.32 (1H, m), 7.43-7.54 (3H, m), 8.07 (2H, d, J = 8.5 Hz), 8.33 (1H, s), 8.87-8.90 (1H, m), 11.31 (1H, s), 12.29 (1H, m).

### Example 41

N-(6-chloroimidazo[1,2-a]pyridin-2-yl)-4-{1,1-dimethyl-2-oxo-2-[(1H-tetrazol-5-ylmethyl)amino]ethyl}benzamide

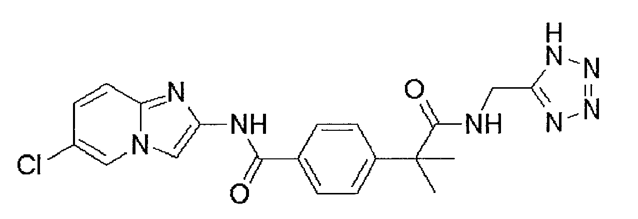

In the same manner as in Example 36 and using N-(6-chloroimidazo[1,2-a]pyridin-2-yl)-4-{2-[(cyanomethyl)amino]-cyano-1,1-dimethyl-2-oxoethyl}benzamide (0.50 mg, 0.13 mmol) synthesized in Example 34, the title compound (yield 9.0 mg, 16%) was obtained.
MS (ESI+): 439 (M+H).
¹H-NMR (DMSO-d₆) δ: 1.51 (6H, s), 4.51 (2H, d, J = 5.3 Hz), 7.28 (1H, dd, J = 9.5, 1.9 Hz), 7.44 (2H, d, J = 8.3 Hz), 7.50 (1H, d, J = 9.5 Hz), 8.03 (2H, d, J = 8.3 Hz), 8.23 (1H, t, J = 5.3 Hz), 8.33 (1H, s), 8.88 (1H, d, J = 1.9 Hz), 11.24 (1H, s).

### Example 42

N-(6-chloroimidazo[1,2-a]pyridin-2-yl)-4-(1,1-dimethyl-2-oxo-2-{[2-(1H-tetrazol-5-yl)ethyl]amino}ethyl)benzamide

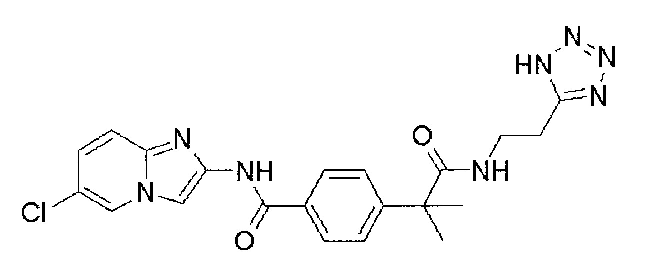

In the same manner as in Example 36 and using N-(6-chloroimidazo[1,2-a]pyridin-2-yl)-4-{2-[(2-cyanoethyl)amino]-1,1-dimethyl-2-oxoethyl}benzamide (0.50 mg, 0.12 mmol) synthesized in Example 35, the title compound (yield 9.0 mg, 17%) was obtained.
MS (ESI+): 453 (M+H).
¹H-NMR (DMSO-d₆) δ: 1.44 (6H, s), 3.02 (2H, t, J = 7.0 Hz), 3.38-3.46 (2H, m), 7.29 (1H, dd, J = 9.7, 1.9 Hz), 7.36 (2H, d, J = 8.7 Hz), 7.51 (1H, d, J=9.5Hz), 7.63 (1H, t, J=5.7Hz), 8.00 (2H, d, J=8.7Hz), 8.33 (1H, s), 8.88 (1H, d, J=1.9Hz), 11.24 (1H, s), 15.98 (1H, br).

### Example 43

2-(4-{[6-(1H-imidazol-1-yl)imidazo[1,2-a]pyridin-2-yl]carbamoyl}phenyl)-2-methylpropanoic acid

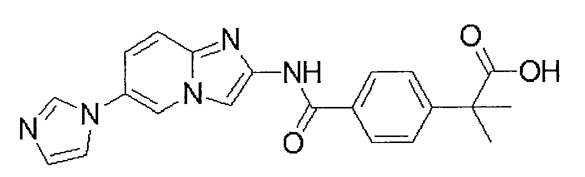

Methyl 2-{4-[(6-iodoimidazo[1,2-a]pyridin-2-yl)carbamoyl]phenyl}-2-methylpropanoate (1.5 g, 3.2 mmol) synthesized in Example 23, imidazole (0.44 g, 6.5 mmol), potassium carbonate (0.90 g, 6.5 mmol) and copper iodide(I) (62 mg, 0.32 mmol) were dissolved in DMF (50 mL), and the mixture was stirred under a nitrogen atmosphere at 160°C overnight. The reaction mixture was allowed to cool to room temperature and the solvent was evaporated under reduced pressure. The obtained residue was purified by silica gel column chromatography (0-10% methanol/ethyl acetate). The obtained solid was dissolved in a mixed solution of methanol (10 mL)/THF (10 mL)/aqueous potassium hydroxide solution (1.6N, 10 mL), and the mixture was stirred at 50°C for 2 hr. The solvent was evaporated under reduced pressure, water was added, and the mixture was washed with ethyl acetate. The aqueous layer was acidified with 1N hydrochloric acid. The precipitated solid was collected by filtration, and washed with water, acetonitrile and diethyl ether. The obtained crude crystals were recrystallized from DMSO/ethanol to give the title compound (yield 0.37 g, 30%).
¹H-NMR (DMSO-d₆) δ: 1.51 (6H, s), 7.15 (1H, dd, J = 1.4, 1.0 Hz), 7.48 (2H, d, J = 8.7 Hz), 7.58 (1H, dd, J = 9.5, 2.0 Hz), 7.63 (1H, d, J = 9.5 Hz), 7.70 (1H, t, J = 1.4 Hz), 8.05 (2H, d, J = 8.7 Hz), 8.20 (1H, dd, J = 1.4, 1.0 Hz), 8.35 (1H, s), 9.07 (1H, dd, J = 2.0, 1.0 Hz), 11.28 (1H, s), 12.52 (1H, br). Elemental analysis: for C₂₁H₁₉N₅O₃
Calculated: C, 64.77; H, 4.92; N, 17.98.
Found: C, 64.75; H, 4.83; N, 17.91.

### Example 44

N-(6-iodoimidazo[1,2-a]pyridin-2-yl)-4-(3-cyano-1,1-dimethylpropyl)benzamide

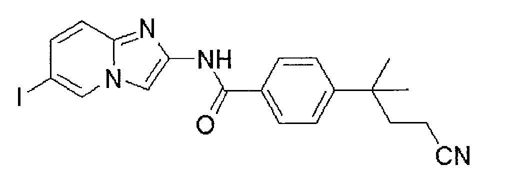

In the same manner as in Example 15 and using 4-(3-cyano-1,1-dimethylpropyl)benzoic acid (2.8 g, 12.7 mmol) synthesized in Reference Example 3 and 6-iodoimidazo[1,2-a]pyridine-2-amine (3.0 g, 11.6 mmol) synthesized in Reference Example 23, the title compound (yield 1.7 g, 30%) was obtained.
¹H-NMR (DMSO-d₆) δ: 1.32 (6H, s), 1.95-2.06 (2H, m), 2.13-2.23 (2H, m), 7.32 (1H, d, J = 9.1 Hz), 7.42 (1H, dd, J = 9.1, 1.9 Hz), 7.51 (2H, d, J = 8.3 Hz), 8.05 (2H, d, J = 8.3 Hz), 8.28 (1H, s), 8.97-8.98 (1H, m), 11.19 (1H, s).

### Example 45

4-(3-cyano-1,1-dimethylpropyl)-N-[6-(1H-imidazol-1-yl)imidazo[1,2-a]pyridin-2-yl]benzamide

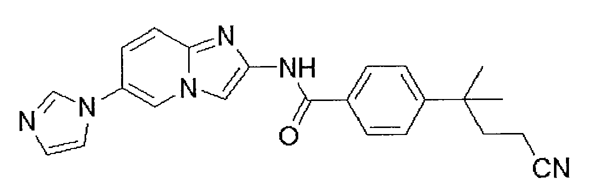

N-(6-Iodoimidazo[1,2-a]pyridin-2-yl)-4-(3-cyano-1,1-dimethylpropyl)benzamide (0.20 g, 0.45 mmol) synthesized in Example 44, imidazole (59 mg, 0.90 mmol), potassium carbonate (0.12 g, 0.90 mmol) and copper iodide(I) (10 mg, 0.050 mmol) were dissolved in DMF (10 mL), and the mixture was stirred under a nitrogen atmosphere at 160°C for 6 hr. The reaction mixture was allowed to cool to room temperature and the solvent was evaporated under reduced pressure. The obtained residue was purified by silica gel column chromatography (0-10% methanol/ethyl acetate). The obtained residue was recrystallized from ethyl acetate/hexane to give the title compound (yield 75 mg, 42%).
melting point 233-234°C
MS (ESI+): 399 (M+H).
¹H-NMR (DMSO-d₆) δ: 1.33 (6H, s), 1.99-2.03 (2H, m), 2.16-2.21 (2H, m), 7.15 (1H, s), 7.52 (2H, d, J = 8.3 Hz), 7.57 (1H, dd, J = 9.5, 1.9 Hz), 7.70-7.71 (1H, m), 8.07 (2H, d, J = 8.3 Hz), 8.19 (1H, s), 8.35 (1H, s), 9.07 (1H, s), 11.26 (1H, s). Elemental analysis: for C₂₃H₂₂N₆O
Calculated: C, 69.33; H, 5.57; N, 21.09.
Found: C, 69.17; H, 5.50; N, 21.06.

### Example 46

4-[1,1-dimethyl-3-(1H-tetrazol-5-yl)propyl]-N-[6-(1H-imidazol-1-yl)imidazo[1,2-a]pyridin-2-yl]benzamide

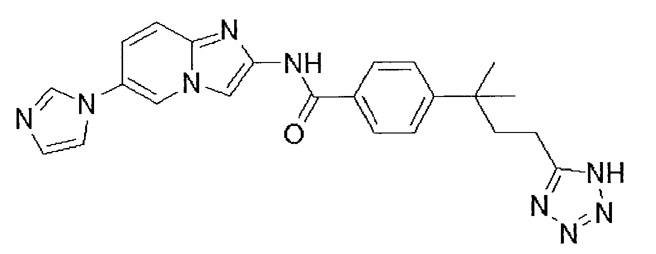

In the same manner as in Example 36 and using 4-(3-cyano-1,1-dimethylpropyl)-N-[6-(1H-imidazol-1-yl)imidazo[1,2-a]pyridin-2-yl]benzamide (0.20 g, 0.50 mmol) synthesized in Example 45, the title compound (yield 50 mg, 23%) was obtained. melting point 266-267°C
MS (ESI+) : 442 (M+H).
¹H-NMR (DMSO-d₆) δ: 1.38 (6H, s), 2.06-2.12 (2H, m), 2.57-2.63 (2H, m), 7.16 (1H, s), 7.54-7.64 (4H, m), 7.70 (1H, t, J = 1.3 Hz), 8.06 (2H, d, J = 8.7 Hz), 8.19 (1H, s), 8.35 (1H, s), 9.06-9.07 (1H, m), 11.26 (1H, s), 12.92 (1H, br).
Elemental analysis: for C₂₃H₂₃N₉O
Calculated: C, 62.57; H, 5.25; N, 28.55.
Found: C, 62.46; H, 5.27; N, 28.55.

### Example 47

4-tert-butyl-N-(6-chloroimidazo[1,2-a]pyridin-2-yl)benzamide

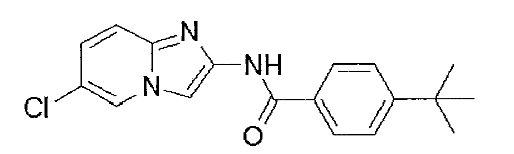

According to Example 49 and using 6-chloroimidazo[1,2-a]pyridine-2-amine obtained in Reference Example 21, the title compound was synthesized.
melting point 250-251°C
MS (ESI+): 328 (M+H).
¹H-NMR (CDCl₃) δ: 1.36(9H, s), 7.13 (1H, dd, J = 9.4, 2.0 Hz), 7.31 (1H, d, J = 9.4 Hz), 7.52 (2H, d, J = 8.8 Hz), 7.87 (2H, d, J = 8.8 Hz), 8.15-8.16 (1H, m), 8.22(1H, s), 8.92 (1H,s). Elemental analysis: for C₁₈H₁₈ClN₃O
Calculated: C, 65.95; H, 5.53; N, 12.82.
Found: C, 65.96; H, 5.56; N, 12.86.

### Example 48

4-tert-butyl-N-(6-bromoimidazo[1,2-a]pyridin-2-yl)benzamide

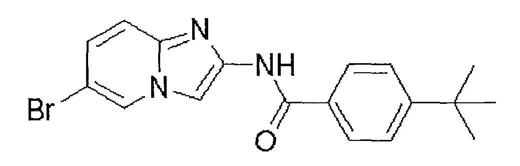

According to Example 49 and using 6-bromoimidazo[1,2-a]pyridine-2-amine obtained in Reference Example 22, the title compound was synthesized.
melting point 249-250°C
MS (ESI+): 372, 374 (M+H).
¹H-NMR (DMSO-d₆) δ: 1.32(9H, s), 7.32-7.38 (1H, m), 7.42-7.47 (1H, m), 7.53 (2H, d, J = 8.3 Hz), 8.01 (2H, d, J = 8.3 Hz), 8.32 (1H, s), 8.95 (1H, s), 11.19 (1H, s).

### Example 49

4-tert-butyl-N-(6-iodoimidazo[1,2-a]pyridin-2-yl)benzamide

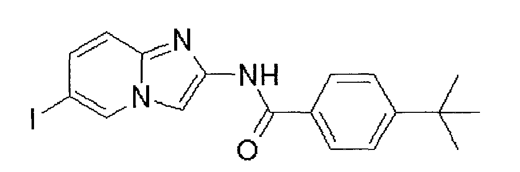

4-tert-Butylbenzoyl chloride (28.0 g, 108 mmol) was added dropwise to a solution of 6-iodoimidazo[1,2-a]pyridine-2-amine obtained in Reference Example 23 and triethylamine in THF, and the mixture was stirred at room temperature for 1 hr. Then, volatile components were evaporated under reduced pressure, and the residue was washed with dichloromethane-hexane (1/1) solution to give the title compound (31.2 g, yield 69%).
MS (ESI+) : 419 (M).
¹H-NMR (DMSO-d₆) δ: 1.31(9H, s), 7.31 (1H, d, J = 9.3 Hz), 7.42 (1H, d, J = 9.3 Hz), 7.53 (2H, d, J = 8.5 Hz), 8.01 (2H, d, J = 8.5 Hz), 8.28 (1H, s), 8.97 (1H, s), 11.17 (1H,s).

### Example 50

4-tert-butyl-N-(imidazo[1,2-a]quinolin-3-yl)benzamide

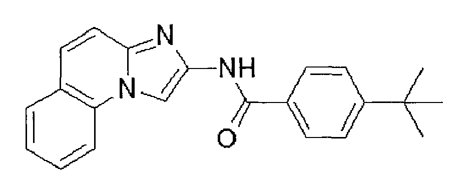

According to Example 49 and using imidazo[1,2-a]quinoline-2-amine obtained in Reference Example 28, the title compound was synthesized.
melting point 204-205°C
MS (ESI+): 344 (M+H).
¹H-NMR (DMSO-d₆) δ: 1.33(9H, s), 7.50-7.57 (4H, m), 7.70-7.77 (2H, m), 8.00 (1H, dd, J = 7.9, 1.1 Hz), 8.03-8.08 (2H, m), 8.34 (1H, d, J = 8.5 Hz), 8.78 (1H, s), 11.18 (1H,s).
Elemental analysis: for C₂₂H₂₁N₃O
Calculated: C, 76.94; H, 6.16; N, 12.24.
Found: C, 76.78; H, 6.15; N, 12.28.

### Example 51

4-tert-butyl-N-[6-(1H-imidazol-1-yl)imidazo[1,2-a]pyridin-2-yl]benzamide

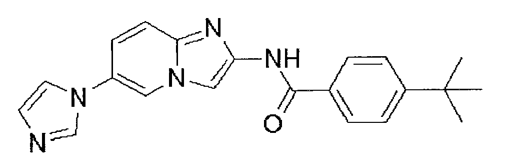

4-tert-Butyl-N-(6-iodoimidazo[1,2-a]pyridin-2-yl)benzamide (20.0 g, 4.77 mmol) obtained in Example 49, imidazole (6.5 g, 95.4 mmol) and CuI (908 mg, 47.7 mmol) were stirred in N,N-dimethylformamide (500 mL) under a nitrogen atmosphere at 160°C for 8 hr. The reaction mixture was allowed to cool to room temperature, filtered, and volatile components were evaporated under reduced pressure. The residue was purified by silica gel chromatography (ethyl acetate:methanol=10:0 - 7:3), and recrystallized from methanol to give the title compound (yield 7.3 g, 42%).
melting point 289-291°C
MS (ESI+): 360 (M+H).
¹H-NMR (DMSO-d₆) δ: 1.33 (9H, s), 7.15 (1H, s), 7.55 (2H, d, J = 8.4 Hz), 7.58-7.64 (2H, m), 7.70 (1H, s), 8.03 (2H, d, J = 8.4 Hz), 8.19 (1H, s), 8.34 (1H,s), 9.07 (1H,s), 11.23 (1H,s).
Elemental analysis: for C₂₁H₂₁N₅O
Calculated: C, 70.17; H, 5.89; N, 19.48.
Found: C, 70.08; H, 5.81; N, 19.39.

### Example 52

4-tert-butyl-N-[6-(1H-imidazol-1-yl)imidazo[1,2-a]pyridin-2-yl]benzamide dihydrochloride

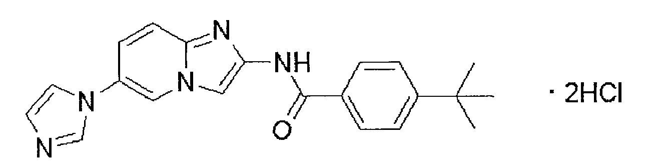

4-tert-Butyl-N-[6-(1H-imidazol-1-yl)imidazo[1,2-a]pyridin-2-yl]benzamide (900 mg, 2.5 mmol) obtained in Example 51 was dissolved in methanol, 4N hydrochloric acid gas-containing ethyl acetate (5 ml) was added and the mixture was stirred for 10 min. Volatile components were evaporated under reduced pressure and the residue was recrystallized from methanol-ethyl acetate to give the title compound (850 mg).
MS (ESI+): 360 (M+H).
¹H-NMR (DMSO-d₆) δ: 1.33 (9H, s), 7.56 (2H, d, J = 8.5 Hz), 7.72-7.83 (2H, m), 7.98 (1H, s), 8.05 (2H, d, J = 8.5 Hz), 8.25-8.29 (1H, m), 8.43 (1H,s), 9.35 (1H,d, J = 1.9 Hz), 9.74 (1H, s), 11.46 (1H,s).
Elemental analysis: for C₂₁H₂₁N₅O·2HCl·H₂O
Calculated: C, 56.00; H, 5.60; N, 15.55.
Found: C, 56.08; H, 5.64; N, 15.55.

### Example 53

4-tert-butyl-N-[6-(1H-imidazol-1-yl)imidazo[1,2-a]pyridin-2-yl]benzamide dimethanesulfonate

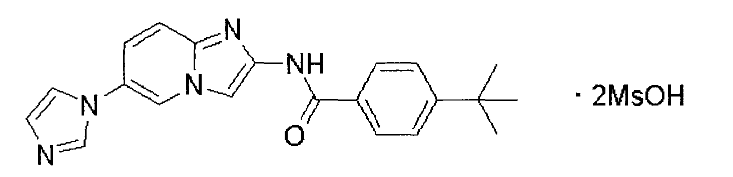

4-tert-Butyl-N-[6-(1H-imidazol-1-yl)imidazo[1,2-a]pyridin-2-yl]benzamide (180 mg, 0.5 mmol) obtained in Example 51 was dissolved in methanol, methanesulfonic acid (115 mg,1.2 mmol) was added and the mixture was stirred for 10 min. Volatile components were evaporated under reduced pressure and the residue was recrystallized from methanol-ethyl acetate to give the title compound (120 mg).
MS (ESI+): 360 (M+H).
¹H-NMR (DMSO-d₆) δ: 1.33 (9H, s), 2.32 (6H, s), 7.55 (2H, d, J = 8.5 Hz), 7.67 (1H, dd, J = 9.6, 2.1 Hz), 7.77 (1H, d, J = 9.6 Hz), 7.97 (1H, s), 8.04 (2H, d, J = 8.5 Hz), 8.25-8.29 (1H, m), 8.43 (1H,s), 9.26 (1H,d, J = 1.3 Hz), 9.66 (1H, s), 11.34 (1H,s).
Elemental analysis: for C₂₁H₂₁N₅O-2MsOH·H₂O
Calculated: C, 48.49; H, 5.49; N, 12.29.
Found: C, 48.33; H, 5.45; N, 12.33.

### Example 54

4-tert-butyl-N-(imidazo[1,2-a]pyridin-2-yl)benzamide

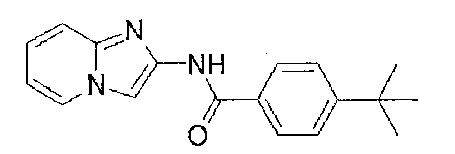

According to Example 51, the title compound was obtained as a byproduct (yield 3%) of the reaction of 4-tert-butyl-N-(6-bromoimidazo[1,2-a]pyridin-2-yl)benzamide obtained in Example 48 and imidazole.
melting point 230-231°C
MS (ESI+): 294 (M+H).
¹H-NMR (CDCl₃) δ: 1.35 (9H, s), 6.75-6.83 (1H, m), 7.06-7.15 (1H, m), 7.17-7.22 (1H, m), 7.48 (2H, d, J = 8.7 Hz), 7.89 (2H, d, J = 8.7 Hz), 8.09-8.15 (1H, m), 8.25 (1H, s), 9.55 (1H,s).
Elemental analysis: for C₁₈H₁₉N₃O· 0.2H₂O
Calculated: C, 72.80; H, 6.58; N, 14.15.
Found: C, 72.78; H, 6.56; N, 14.18.

### Example 55

4-tert-butyl-N-(6-chloro-3-methylimidazo[1,2-b]pyridazin-2-yl)benzamide

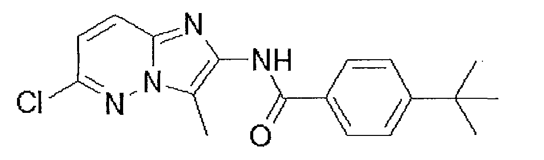

According to Example 49 and using 6-chloro-3-methylimidazo[1,2-b]pyridazine-2-amine obtained in Reference Example 30, the title compound was synthesized.
melting point 269-270°C
MS (ESI+): 342 (M).
¹H-NMR (DMSO-d₆) δ: 1.33 (9H, s), 2.41 (3H, s), 7.34 (1H, d, J = 9.4 Hz), 7.55 (2H, d, J = 8.5 Hz), 7.99 (2H, d, J = 8.5 Hz), 8.15 (1H, d, J = 9.4 Hz), 10.66 (1H,s).
Elemental analysis: for C₁₈H₁₉N₄OCl
Calculated: C, 63.06; H, 5.59; N, 16.34.
Found: C, 62.93; H, 5.53; N, 16.34.

### Example 56

4-tert-butyl-N-[6-(1H-imidazol-1-yl)-3-methylimidazo[1,2-b]pyridazin-2-yl]benzamide

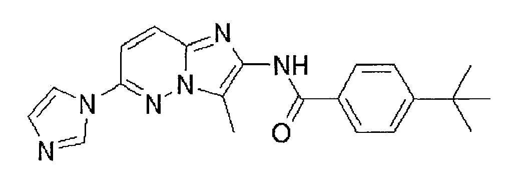

4-tert-Butyl-N-(6-chloro-3-methylimidazo[1,2-b]pyridazin-2-yl)benzamide (0.5 mmol, 171 mg) obtained in Example 55, imidazole (1.0 mmol, 68 mg) and cesium carbonate (1.2 mmol, 391 mg) were stirred in N,N-dimethylformamide (5 mL) using microwave at 160°C for 30 min. The reaction mixture was allowed to cool to room temperature and the solvent was evaporated under reduced pressure. The residue was purified by silica gel chromatography (ethyl acetate:methanol=8:2), and recrystallized from ethyl acetate to give the title compound (yield 10 mg, 5%).
melting point 302-303°C
MS (ESI+) : 375 (M+H).
¹H-NMR (DMSO-d₆) δ: 1.33 (9H, s), 2.47 (3H, s), 7.21 (1H, s), 7.56 (2H, d, J = 8.5 Hz), 7.76 (1H, d, J = 9.6 Hz), 8.00 (2H, d, J = 8.5 Hz), 8.05 (1H, s), 8.29 (1H, d, J = 9.6 Hz), 8.63 (1H, s), 10.65 (1H,s).

### Example 57

4-tert-butyl-N-[6-(pyridin-3-yl)imidazo[1,2-a]pyridin-2-yl]benzamide

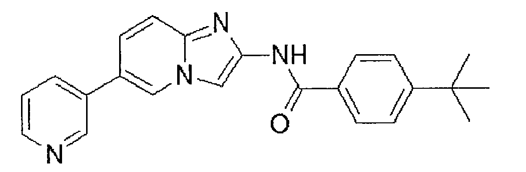

4-tert-Butyl-N-(6-bromoimidazo[1,2-a]pyridin-2-yl)benzamide (0.5 mmol, 186 mg) obtained in Example 48, pyridine-3-boronic acid (1.0 mmol, 123 mg), Pd(PPh₃)₄ (0.05 mmol, 58 mg) and sodium carbonate (2.0 mmol, 212 mg) were stirred in 1,2-dimethoxyethane (7 mL)-water (3 mL) solution under a nitrogen atmosphere at 100°C for 3 hr. The reaction mixture was allowed to cool to room temperature and the solvent was evaporated under reduced pressure. Saturated brine was added and the mixture was extracted with ethyl acetate. The extract was washed with water, dried (MgSO₄), and the solvent was evaporated under reduced pressure. The residue was purified by silica gel chromatography (ethyl acetate:hexane=6:4 - 10:0), and recrystallized from dichloromethane-hexane to give the title compound (yield 70 mg, 38%).
melting point 288-289°C
MS (ESI+): 371 (M+H).
¹H-NMR (CDCl₃) δ: 1.37 (9H, s), 7.36-7.56 (5H, m), 7.84-7.93 (3H, m), 8.30-8.35 (2H, m), 8.66 (1H, dd, J = 4.8, 1.4 Hz), 8.85 (1H, d, J = 1.9 Hz), 8.94 (1H,s).
Elemental analysis: for C₂₃H₂₂N₄O
Calculated: C, 74.57; H, 5.99; N, 15.12.
Found: C, 74.44; H, 5.99; N, 15.06.

### Example 58

4-tert-butyl-N-[6-(pyridin-3-yl)imidazo[1,2-a]pyridin-2-yl]benzamide dihydrochloride

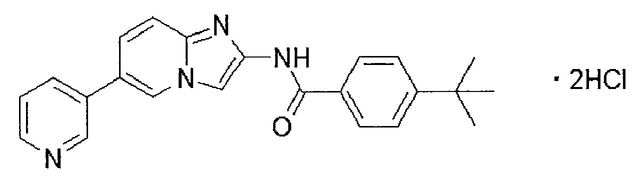

According to Example 52, the title compound was synthesized from 4-tert-butyl-N-[6-(pyridin-3-yl)imidazo[1,2-a]pyridin-2-yl]benzamide obtained in Example 57.
MS (ESI+): 371 (M+H).
¹H-NMR (DMSO-d₆) δ: 1.33 (9H, s), 7.58 (2H, d, J = 8.4 Hz), 7.78-7.97 (3H, m), 8.05 (2H, d, J = 8.4 Hz), 8.37 (1H, s), 8.57 (1H, d, J = 7.7 Hz), 8.81 (1H, d, J = 5.1 Hz), 9.17 (1H, s), 9.29 (1H, s), 11.54 (1H, s).

### Example 59

4-tert-butyl-N-[6-(pyridin-4-yl)imidazo[1,2-a]pyridin-2-yl]benzamide

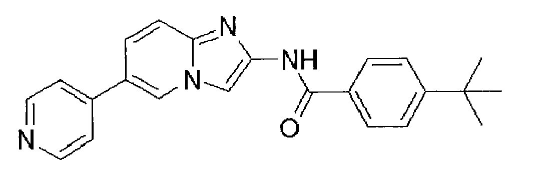

According to Example 57 and using pyridine-4-boronic acid, the title compound was synthesized.
melting point 293-294°C
MS (ESI+): 371 (M+H).
¹H-NMR (CDCl₃) δ: 1.36 (9H, s), 7.35-7.46 (2H, m), 7.47-7.55 (4H, m), 7.87-7.96 (2H, m), 8.34 (1H, s), 8.42 (1H, s), 8.68-8.75 (2H, m), 9.27 (1H, s).

### Example 60

4-tert-butyl-N-(6-fluoroimidazo[1,2-a]pyridin-2-yl)benzamide

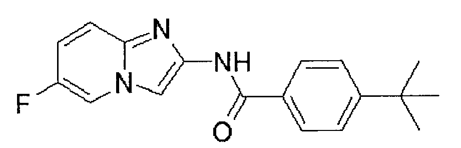

According to Example 49 and using 6-fluoroimidazo[1,2-a]pyridine-2-amine obtained in Reference Example 32, the title compound was synthesized.
melting point 250-251°C
MS (ESI+): 312 (M+H).
¹H-NMR (CDCl₃) δ: 1.36 (9H, s), 7.13 (1H, dd, J = 9.4, 2.0 Hz), 7.31 (1H, d, J = 9.4 Hz), 7.52 (2H, d, J = 8.8 Hz), 7.87 (2H, d, J = 8.8 Hz), 8.15-8.16 (1H, m), 8.22 (1H, s), 8.92 (1H,s). Elemental analysis: for C₁₈H₁₉ClN₃O
Calculated: C, 65.95; H, 5.53; N, 12.82.
Found: C, 65.96; H, 5.56; N, 12.86.

### Example 61

4-tert-butyl-N-[6-(3-furyl)imidazo[1,2-a]pyridin-2-yl]benzamide

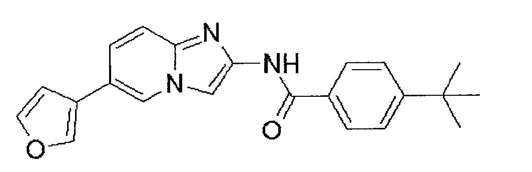

According to Example 57 and using 3-furylboronic acid, the title compound was synthesized.
melting point 258-259°C
MS (ESI+): 360 (M+H).
¹H-NMR (DMSO-d₆) δ: 1.32 (9H, s), 6.98 (1H, s), 7.45-7.58 (4H, m), 7.80 (1H, s), 8.03 (2H, d, J = 8.5 Hz), 8.23 (1H, s), 8.25 (1H, s), 8.91 (1H, s), 11.15 (1H, s).

### Example 62

4-tert-butyl-N-[6-(1H-pyrrol-2-yl)-imidazo[1,2-a]pyridazin-2-yl]benzamide

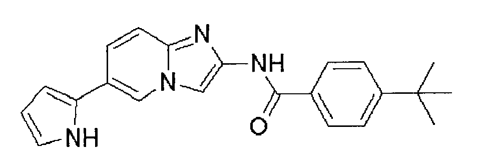

According to Example 57, the reaction was performed using 1-tert-(butoxycarbonyl)pyrrole-2-boronic acid to synthesize tert-butyl 2-{2-[(4-tert-butylbenzoyl)amino]imidazo[1,2-a]pyridin-6-yl}-1H-pyrrole-1-carboxylate. Then, 4N hydrochloric acid gas-containing ethyl acetate (5 ml) was added and the mixture was stirred in methanol overnight. Volatile components were evaporated under reduced pressure, saturated brine and aqueous sodium bicarbonate were added, and the mixture was extracted with ethyl acetate. The extract was washed with water, dried (MgSO₄), and the solvent was evaporated. The residue was purified by silica gel chromatography (ethyl acetate:hexane=8:2 - 10:0), and recrystallized from ethyl acetate to give the title compound.
melting point 279-280°C
¹H-NMR (DMSO-d₆) δ: 1.32 (9H, s), 6.15 (1H, s), 6.50 (1H, s), 6.89 (1H, s), 7.40-7.61 (4H, m), 8.03 (2H, d, J = 8.3 Hz), 8.21 (1H, s), 8.78 (1H, s), 11.13 (1H,s), 11.32 (1H, s). Elemental analysis: for C₂₂H₂₂N₄O
Calculated: C, 73.72; H, 6.19; N, 15.63.
Found: C, 73.61; H, 6.22; N, 15.61.

### Example 63

4-tert-butyl-N-[6-(1H-pyrrol-2-yl)-imidazo[1,2-a]pyridazin-2-yl]benzamide hydrochloride

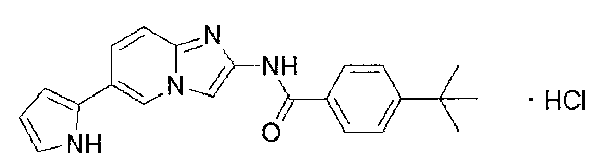

According to Example 52 and using 4-tert-butyl-N-[6-(1H-pyrrol-2-yl)-imidazo[1,2-a]pyridazin-2-yl]benzamide obtained in Example 62, the title compound was synthesized.
¹H-NMR (DMSO-d₆) δ: 1.33 (9H, s), 6.16-6.20 (1H, m), 6.55-6.60 (1H, m), 6.92-6.96 (1H, m), 7.59 (2H, d, J = 8.7 Hz), 7.69 (1H, d, J = 9.1 Hz), 7.85 (1H, d, J = 9.1 Hz), 8.03 (2H, d, J = 8.7 Hz), 8.21 (1H, s), 8.94 (1H, s), 11.50 (2H, br).
Elemental analysis: for C₂₂H₂₂N₄O·HCl·0.5H₂O
Calculated: C, 65.42; H, 5.99; N, 13.87.
Found: C, 65.45; H, 5.96; N, 13.72.

### Example 64

4-tert-butyl-N-[6-(1H-pyrrol-1-yl)-imidazo[1,2-a]pyridin-2-yl]benzamide

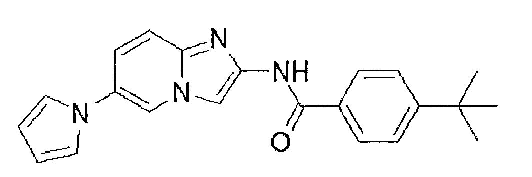

According to Example 51, 4-tert-butyl-N-(6-iodoimidazo[1,2-a]pyridin-2-yl)benzamide obtained in Example 49 was reacted with pyrrole to synthesize the title compound. melting point 274-275°C
MS (ESI+): 359 (M+H).
1H-NMR (DMSO-d₆) δ: 1.32 (9H, s), 6.31 (2H, t, J = 2.2 Hz), 7.32 (2H, t, J = 2.2 Hz), 7.51-7.59 (4H, m), 8.03 (2H, d, J = 8.5 Hz), 8.32 (1H, s), 8.96 (1H, t, J = 1.5 Hz), 11.18 (1H, s). Elemental analysis: for C₂₂H₂₂N₄O
Calculated: C, 73.72; H, 6.19; N, 15.63.
Found: C, 73.61; H, 6.22; N, 15.66.

### Example 65

4-tert-butyl-N-[5-methylimidazo[1,2-a]pyridin-2-yl]benzamide

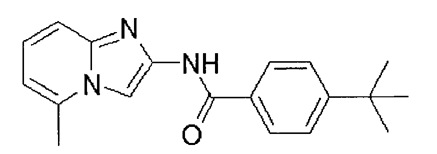

According to Example 49 and using 5-methylimidazo[1,2-a]pyridine-2-amine obtained in Reference Example 20, the title compound was synthesized.
melting point 204-205°C
MS (ESI+): 308 (M+H).
¹H-NMR (DMSO-d₆) δ: 1.32 (9H, s), 2.62 (3H, s), 6.80 (1H, d, J = 7.0 Hz), 7.23 (1H, dd, J = 8.9, 7.0 Hz), 7.37 (1H, d, J = 8.9 Hz), 7.53 (2H, d, J = 8.5 Hz), 8.04 (2H, d, J = 8.5 Hz), 8.07 (1H, s), 11.16 (1H, s).
Elemental analysis: for C₁₉H₂₁N₃O
Calculated: C, 74.24; H, 6.89; N, 13.67.
Found: C, 74.14; H, 6.83; N, 13.65.

### Example 66

4-tert-butyl-N-[5-methylimidazo[1,2-a]pyridin-2-yl]benzamide hydrochloride

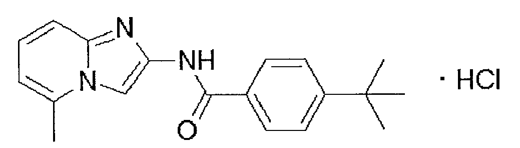

According to Example 52 and using 4-tert-butyl-N-[5-methylimidazo[1,2-a]pyridin-2-yl]benzamide obtained in Example 65, the title compound was synthesized.
melting point 204-205°C
MS (ESI+): 308 (M+H).
¹H-NMR (DMSO-d₆) δ: 1.33 (9H, s), 2.71 (3H, s), 7.16 (1H, d, J = 6.8 Hz), 7.57-7.70 (4H, m), 8.02-8.07 (3H, m), 11.69 (1H, s).

### Example 67

4-tert-butyl-N-[6-methylimidazo[1,2-a]pyridin-2-yl]benzamide

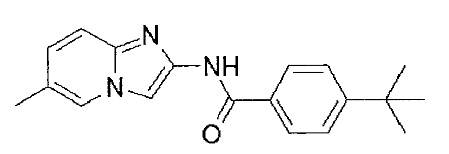

According to Example 49 and using 6-methylimidazo[1,2-a]pyridine-2-amine obtained in Reference Example 26, the title compound was synthesized.
melting point 259-260°C
MS (ESI+): 308 (M+H).
¹H-NMR (CDCl₃) δ: 1.35 (9H, s), 2.33 (3H, s), 7.00 (1H, d, J = 9.1 Hz), 7.20-7.29 (1H, m), 7.51 (2H, d, J = 8.7 Hz), 7.87 (2H, d, J = 8.7 Hz), 7.89-7.91 (1H, m), 8.14 (1H, s), 9.01 (1H, s). Elemental analysis: for C₁₉H₂₁N₃O
Calculated: C, 74.24; H, 6.89; N, 13.67.
Found: C, 74.03; H, 6.95; N, 13.69.

### Example 68

4-tert-butyl-N-[6-phenylimidazo[1,2-a]pyridin-2-yl]benzamide

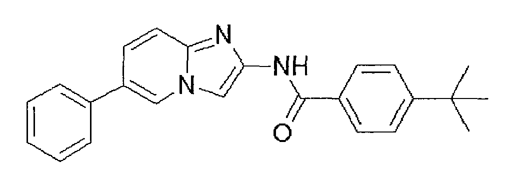

According to Example 57 and using phenylboronic acid, the title compound was synthesized.
melting point 252-253°C
MS (ESI+): 370 (M+H).
¹H-NMR (DMSO-d₆) δ: 1.33 (9H, s), 7.36-7.44 (1H, m), 7.47-7.57 (5H, m), 7.61 (1H, dd, J = 9.4, 1.7 Hz), 7.70-7.75 (2H, m), 8.04 (2H, d, J = 8.7 Hz), 8.35 (1H, s), 8.98 (1H, s), 11.17 (1H, s).
Elemental analysis: for C₂₄H₂₃N₃O
Calculated: C, 78.02; H, 6.27; N, 11.37.
Found: C, 77.96; H, 6.14; N, 11.34.

### Example 69

4-tert-butyl-N-[6-phenylimidazo[1,2-a]pyridin-2-yl]benzamide hydrochloride

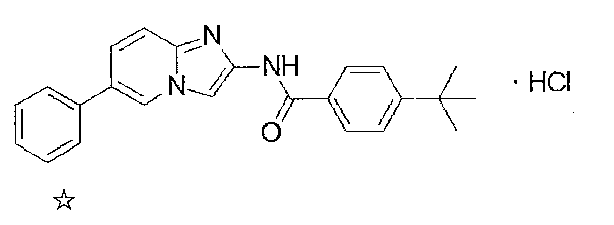

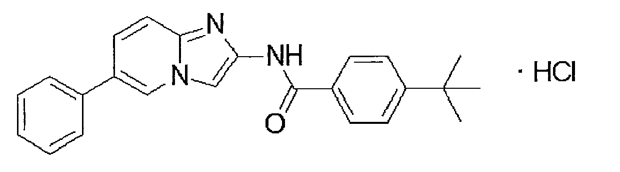

According to Example 52, the title compound was synthesized from 4-tert-butyl-N-[6-phenylimidazo[1,2-a]pyridin-2-yl]benzamide obtained in Example 68.
MS (ESI+) : 370 (M+H).
¹H-NMR (DMSO-d₆) δ: 1.33 (9H, s), 7.41-7.49 (1H, m), 7.51-7.62 (4H, m), 7.72-7.81 (3H, m), 7.91 (1H, d, J = 8.7 Hz), 8.04 (2H, d, J = 8.5 Hz), 8.33 (1H, s), 9.14 (1H, s), 11.58 (1H, s).
Elemental analysis: for C₂₄H₂₃N₃O·1HCl
Calculated: C, 71.01; H, 5.96; N, 10.35.
Found: C, 70.96; H, 5.88; N, 10.43.

### Example 70

4-tert-butyl-N-[6-hydroxymethylimidazo[1,2-a]pyridin-2-yl]benzamide

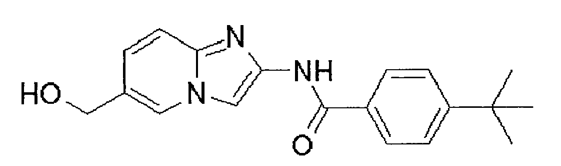

4-tert-Butyl-N-(6-iodoimidazo[1,2-a]pyridin-2-yl)benzamide (2.0 g, 4.77 mmol) synthesized in Example 49 was suspended in THF (60 mL) under a nitrogen atmosphere, isopropylmagnesium chloride (1M THF solution) (24 mL, 24.0 mmol) was added, and the mixture was stirred at -20°C for 2 hr. Then, N,N-dimethylformamide (1.75 g, 24 mmol) was added, and the mixture was stirred at 10°C for 3 hr. Saturated aqueous ammonium chloride solution was added, and the mixture was stirred at room temperature for 30 min and extracted with ethyl acetate. The extract was dried (MgSO₄) and the solvent was evaporated under reduced pressure. The residue was purified by silica gel chromatography to give 4-tert-butyl-N-(6-formylimidazo[1,2-a]pyridin-2-yl)benzamide (1.4 g, purity about 50%, a mixture with 4-tert-butyl-N-imidazo[1,2-a]pyridin-2-yl)benzamide (Example 54)). The compound (168 mg, purity about 50%, about 0.25 mmol) was dissolved in THF (10 mL), lithium tetrahydroborate (5.7 mg, 0.26 mmol) was added, and the mixture was stirred at 5°C for 10 min. Water was added, and the mixture was extracted with ethyl acetate. The extract was dried (MgSO₄), and the solvent was evaporated under reduced pressure. The residue was purified by silica gel chromatography (ethyl acetate:hexane=6:4 - 10:0), and recrystallized from ethyl acetate-hexane to give the title compound (21 mg, yield 50%).
melting point 249-250°C
MS (ESI+): 324 (M+H).
¹H-NMR (DMSO-d₆) δ: 1.32 (9H, s), 4.49 (2H, d, J = 5.7 Hz), 5.32 (1H, t, J = 5.7 Hz), 7.20 (1H, d, J = 9.2 Hz), 7.42 (1H, d, J = 9.2 Hz), 7.52 (2H, d, J = 8.5 Hz), 8.01 (2H, d, J = 8.5 Hz), 8.26 (1H, s), 8.49 (1H, s), 11.07 (1H, s).
Elemental analysis: for C₁₉H₂₁N₃O₂
Calculated: C, 70.57; H, 6.55; N, 12.99.
Found: C, 70.43; H, 6.66; N, 13.05.

### Example 71

4-tert-butyl-N-[6-(1-hydroxyethyl)imidazo[1,2-a]pyridin-2-yl]benzamide

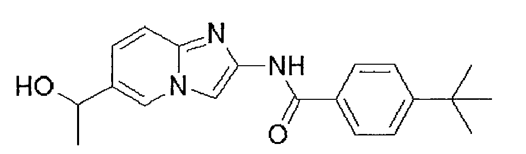

4-tert-Butyl-N-(6-formylimidazo[1,2-a]pyridin-2-yl)benzamide (168 mg, purity about 50%) obtained in Example 70, about 0.25 mmol) was dissolved in THF (20 mL), methylmagnesium bromide (1M THF solution)(1 mL, 1.0 mmol) was added, and the mixture was stirred at 5°C for 30 min. Water was added, and the mixture was extracted with ethyl acetate. The extract was dried (MgSO₄), and the solvent was evaporated under reduced pressure. The residue was purified by silica gel chromatography (ethyl acetate:hexane=6:4 - 10:0), and recrystallized from ethyl acetate-hexane to give the title compound (55 mg, yield 65%).
melting point 249-250°C
MS (ESI+) : 338 (M+H).
¹H-NMR (DMSO-d₆) δ: 1.32 (9H, s), 1.39 (3H, d, J = 6.4 Hz), 4.69-4.81 (1H, m), 5.33 (1H, d, J = 4.1 Hz), 7.25 (1H, d, J = 9.1 Hz), 7.41 (1H, d, J = 9.1 Hz), 7.52 (2H, d, J = 8.5 Hz), 8.01 (2H, d, J = 8.5 Hz), 8.25 (1H, s), 8.50 (1H, s), 11.05 (1H, s).
Elemental analysis: for C₂₀H₂₃N₃O₂
Calculated: C, 71.19; H, 6.87; N, 12.45.
Found: C, 71.11; H, 6.69; N, 12.44.

### Example 72

4-tert-butyl-N-[6-(methylaminomethyl)imidazo[1,2-a]pyridin-2-yl]benzamide

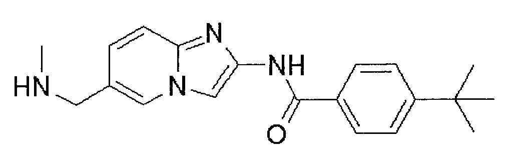

4-tert-Butyl-N-(6-formylimidazo[1,2-a]pyridin-2-yl)benzamide (168 mg, purity about 50%) obtained in Example 70, about 0.25 mmol), methylamine (solution for 40% methanol, 102 µL, 1.0 mmol) and acetic acid (500 µL) were dissolved in dichloromethane (2.5 mL)-N,N-dimethylformamide (2.5 mL), and the mixture was stirred at room temperature for 30 min, then sodium triacetoxyborohydride (212 mg, 1.0 mmol) was added and the mixture was stirred for 3 hr. Water was added, and the mixture was extracted with ethyl acetate. The extract was dried (MgSO₄) and the solvent was evaporated under reduced pressure. The residue was purified by silica gel chromatography (ethyl acetate:methanol=10:0 - 8:2), and recrystallized from ethyl acetate-hexane to give the title compound (60 mg, yield 71%).
melting point 204-205°C
MS (ESI+): 337 (M+H).
¹H-NMR (DMSO-d₆) δ: 1.32 (9H, s), 2.28 (3H, s), 3.62 (2H, s), 7.23 (1H, d, J = 9.0 Hz), 7.40 (1H, d, J = 9.0 Hz), 7.52 (2H, d, J = 8.6 Hz), 8.02 (2H, d, J = 8.6 Hz), 8.22 (1H, s), 8.46 (1H, s), 11.06 (1H, s), hidden (1H).
Elemental analysis: for C₂₀H₂₄N₄O
Calculated: C, 71.40; H, 7.19; N, 16.65.
Found: C, 71.12; H, 7.16; N, 16.52.

### Example 73

4-tert-butyl-N-[6-(1H-imidazol-2-yl)imidazo[1,2-a]pyridin-2-yl]benzamide

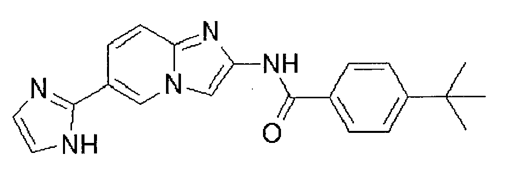

4-tert-Butyl-N-(6-formylimidazo[1,2-a]pyridin-2-yl)benzamide (337 mg, purity about 50%) about 0.5 mmol) obtained in Example 70 and glyoxal (1 mL) were dissolved in 2N ammonia gas-containing methanol (100 mL), and the mixture was stirred at room temperature overnight. Volatile components were evaporated under reduced pressure, and the residue was purified by silica gel chromatography (ethyl acetate:methanol=10:0 - 8:2) and recrystallized from ethyl acetate to give the title compound (75 mg, yield 42%).
melting point 300-301°C
MS (ESI+): 360 (M+H).
¹H-NMR (DMSO-d₆) δ: 1.32 (9H, s), 7.05 (1H, s), 7.29 (1H, s), 7.29-7.60 (3H, m), 7.79 (1H, d, J = 9.2 Hz), 8.03 (2H, d, J = 8.5 Hz), 8.34 (1H, s), 9.08 (1H, s), 11.17 (1H, s), 12.57 (1H, s) .

### Example 74

4-tert-butyl-N-[6-(1,3-oxazol-5-yl)imidazo[1,2-a]pyridazin-2-yl]benzamide

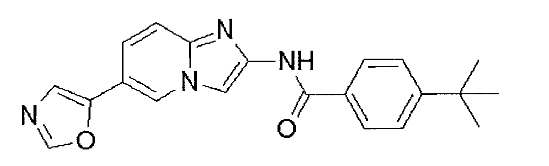

4-tert-Butyl-N-(6-formylimidazo[1,2-a]pyridin-2-yl)benzamide (168 mg, purity about 50%), about 0.25 mmol) obtained in Example 70 and (paratolylsulfonyl)methylisocyanide (73 mg, 0.38 mmol) were dissolved in methanol (25 mL), sodium methoxide (54 mg, 1.0 mmol) was added, and the mixture was stirred at 100°C for 3 hr. Volatile components were evaporated under reduced pressure, water was added, and the mixture was extracted with ethyl acetate. The extract was dried (MgSO₄), and the solvent was evaporated under reduced pressure. The residue was purified by silica gel chromatography (ethyl acetate:hexane=3:7 - 7:3), and recrystallized from dichloromethane-hexane to give the title compound (35 mg, yield 39%).
melting point 265-266°C
MS (ESI+): 361 (M+H).
¹H-NMR (DMSO-d₆) δ: 1.32 (9H, s), 7.54 (2H, d, J = 8.7 Hz), 7.56-7.65 (2H, m), 7.70 (1H, s), 8.03 (2H, d, J = 8.7 Hz), 8.40 (1H, s), 8.51 (1H, s), 9.04 (1H, s), 11.20 (1H, s). Elemental analysis: for C₂₁H₂₀N₄O₂
Calculated: C, 69.98; H, 5.59; N, 15.55.
Found: C, 69.83; H, 5.61; N, 15.50.

### Example 75

4-tert-butyl-N-[6-(1,3-oxazol-5-yl)imidazo[1,2-a]pyridazin-2-yl]benzamide dihydrochloride

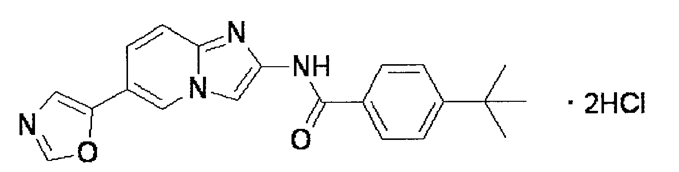

According to Example 52, 4-tert-butyl-N-[6-(1,3-oxazol-5-yl)imidazo[1,2-a]pyridazin-2-yl]benzamide obtained in Example 74, the title compound was synthesized.
MS (ESI+): 361 (M+H).
¹H-NMR (DMSO-d₆) δ: 1.33 (9H, s), 7.59 (2H, d, J = 8.4 Hz), 7.78-7.84 (2H, m), 7.93 (1H, d, J = 9.4 Hz), 8.05 (2H, d, J = 8.3 Hz), 8.41 (1H, s), 8.58 (1H, s), 9.21 (1H, s), 11.68 (1H, s).

### Example 76

4-tert-butyl-N-[6-(1H-imidazol-5-yl)imidazo[1,2-a]pyridin-2-yl]benzamide

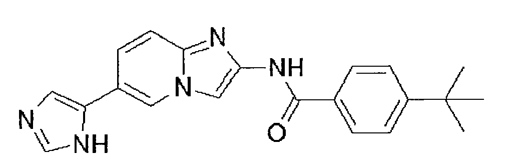

4-tert-Butyl-N-(6-formylimidazo[1,2-a]pyridin-2-yl)benzamide (500 mg, purity about 50%), about 0.75 mmol) obtained in Example 70 and (paratolylsulfonyl)methylisocyanide (195 mg, 1.0 mmol) were dissolved in methanol (200 mL), sodium cyanide (20 mg, 0.55 mmol) was added, and the mixture was stirred at room temperature for 4 hr. Volatile components were evaporated under reduced pressure, and the residue was dissolved in 2N ammonia gas-containing methanol (15 mL) and stirred using microwave at 110°C for 40 min. Volatile components were evaporated under reduced pressure, and the residue was purified by silica gel chromatography (ethyl acetate:methanol=10:0 - 8:2) to give the title compound (90 mg, yield 34%).
melting point 297-298°C
MS (ESI+): 360 (M+H).
¹H-NMR (DMSO-d₆) δ: 1.32 (9H, s), 7.45 (1H, d, J = 9.2 Hz), 7.53 (2H, d, J = 8.6 Hz), 7.61-7.70 (2H, m), 7.75 (1H, s), 8.03 (2H, d, J = 8.6 Hz), 8.30 (1H, s), 8.93 (1H, s), 11.09 (1H, s), 12.23 (1H, s).
Elemental analysis: for C₂₁H₂₁N₅O·0.2H₂O
Calculated: C, 69.48; H, 5.94; N, 19.29.
Found: C, 69.43; H, 6.03; N, 18.93.

### Example 77

4-tert-butyl-N-[6-(1H-imidazol-5-yl)imidazo[1,2-a]pyridin-2-yl]benzamide dihydrochloride

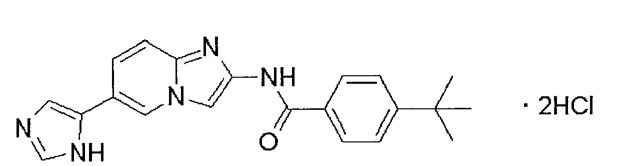

According to Example 52 and using 4-tert-butyl-N-[6-(1H-imidazol-5-yl)imidazo[1,2-a]pyridin-2-yl]benzamide obtained in Example 76, the title compound was synthesized.
MS (ESI+): 360 (M+H).
¹H-NMR (DMSO-d₆) δ: 1.33 (9H, s), 7.55 (2H, d, J = 8.5 Hz), 7.67-7.72 (2H, m), 8.04 (2H, d, J = 8.5 Hz), 8.13 (1H, s), 8.33 (1H, s), 9.11-9.23 (2H, m), 11.33 (1H, s), hidden (1H).

### Example 78

4-tert-butyl-N-[6-cyanoimidazo[1,2-a]pyridin-2-yl]benzamide

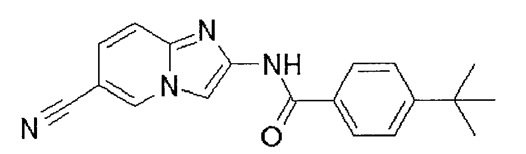

4-tert-Butyl-N-(6-iodoimidazo[1,2-a]pyridin-2-yl)benzamide (839 mg,2.0 mmol) synthesized in Example 49 was suspended in THF (50 mL) under a nitrogen atmosphere, isopropylmagnesium chloride (1M THF solution) (8 mL, 8.0 mmol) was added, and the mixture was stirred at -20°C for 2 hr. Then, paratoluenesulfonyl cyanide (725 mg, 4 mmol) was added at -70°C, and the mixture was stirred at -20°C for 4 hr. Water was added, and the mixture was extracted with ethyl acetate. The extract was dried (MgSO₄), and the solvent was evaporated under reduced pressure. The residue was purified by silica gel chromatography (ethyl acetate:hexane=3:7 - 7:3), and recrystallized from ethyl acetate-hexane to give the title compound (90 mg, yield 14%).
melting point 300-301°C
MS (ESI+) : 319 (M+H).
¹H-NMR (CDCl₃) δ: 1.35 (9H, s), 7.13-7.24 (2H, m), 7.51 (2H, d, J = 8.3 Hz), 7.89 (2H, d, J = 8.3 Hz), 8.38 (1H, s), 8.56 (1H, s), 9.63 (1H, s).

### Example 79

4-tert-butyl-N-[6-acetylimidazo[1,2-a]pyridin-2-yl]benzamide

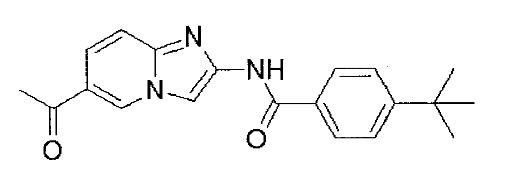

4-tert-Butyl-N-(6-iodoimidazo[1,2-a]pyridin-2-yl)benzamide (210 mg, 0.5 mmol) synthesized in Example 49, palladium acetate (11 mg, 0.05 mmol), lithium chloride (11 mg, 0.25 mmol), n-butylvinyl ether (150 mg,1.5 mmol) and potassium carbonate (173 mg,1.25 mmol) were stirred under a nitrogen atmosphere in DMF (10 mL) at 100°C overnight. The solvent was evaporated under reduced pressure and saturated brine was added to the residue. The mixture was extracted with dichloromethane. The extract was dried (MgSO₄), and the solvent was evaporated under reduced pressure. The residue was purified by silica gel chromatography (ethyl acetate:hexane=3:7 - 8:2). The resultant product was dissolved in dichloromethane (10 mL), 10% hydrochloric acid-containing methanol (5 mL) was added, and the mixture was stirred for 10 min. The solvent was evaporated under reduced pressure, aqueous sodium bicarbonate was added to the residue, and the mixture was extracted with dichloromethane. The extract was dried (MgSO₄), and the solvent was evaporated under reduced pressure. The residue was purified by silica gel chromatography (ethyl acetate:hexane=3:7 - 8:2), to give the title compound (18 mg, yield 11%).
MS (ESI+): 336 (M+H).
¹H-NMR (DMSO-d₆) δ: 1.32 (9H, s), 2.60 (3H, s), 7.49-7.56 (3H, m), 7.69 (1H, d, J = 9.4 Hz), 8.03 (2H, d, J = 8.7 Hz), 8.42 (1H, s), 9.49 (1H, s), 11.26 (1H, s).

### Example 80

4-tert-butyl-N-[6-(dimethylaminomethyl)imidazo[1,2-a]pyridin-2-yl]benzamide

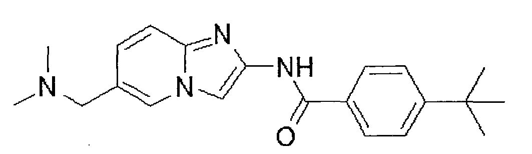

According to Example 72 and using dimethylamine, the title compound was synthesized.
melting point 236-237°C
MS (ESI+): 351 (M+H).
¹H-NMR (DMSO-d₆) δ: 1.32 (9H, s), 2.17 (6H, s), 3.37 (2H, s), 7.20 (1H, d, J = 9.0 Hz), 7.40 (1H, d, J = 9.0 Hz), 7.52 (2H, d, J = 8.6 Hz), 8.02 (2H, d, J = 8.6 Hz), 8.24 (1H, s), 8.47 (1H, s), 11. 07 (1H, s).

### Example 81

4-dimethylamino-N-[6-iodo[1,2-a]pyridin-2-yl]benzamide

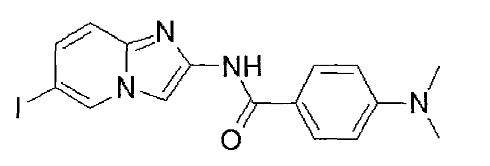

According to Example 49 and using 4-dimethylaminobenzoyl chloride, the title compound was synthesized.
melting point 237-238°C
MS (ESI+) : 406 (M+H).
¹H-NMR (CDCl₃) δ: 3.00 (9H, s), 6.73 (2H, d, J = 9.0 Hz), 7.29 (1H, d, J = 9.2 Hz), 7.39 (1H, d, J = 9.2 Hz), 7.97 (2H, d, J = 9.0 Hz), 8.23 (1H, s), 8.94 (1H, s), 10.81 (1H, s).

### Example 82

potassium 4-[6-(1H-imidazol-1-yl)imidazo[1,2-a]pyridazin-2-yl]aminocarbonylbenzoate

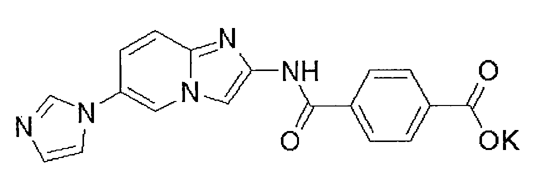

Methyl 4-{[6-(1H-imidazol-1-yl)imidazo[1,2-a]pyridin-2-yl]carbamoyl}benzoate (60 mg, 0.17 mmol) obtained in Example 99 was dissolved in THF (10 mL)-methanol (5 mL)-water (5 mL), potassium hydroxide (28 mg, 0.5 mmol) was added, and the mixture was stirred at 80°C for 3 hr. After evaporation of the solvent under reduced pressure, the residue was recrystallized from acetonitrile-water to give the title compound (33 mg, 45%).
melting point >400°C
MS (ESI+): 347 (M+H).
¹H-NMR (DMSO-d₆) δ: 7.15 (1H, s), 7.56 (1H, d, J = 9.5 Hz), 7.63 (1H, d, J = 9.5 Hz), 7.70 (1H, s), 7.88 (2H, d, J = 8.3 Hz), 7.94 (2H, d, J = 8.3 Hz), 8.19 (1H, s), 8.35 (1H, s), 9.07 (1H, s), 11.07 (1H, s).
Elemental analysis: for C₁₈H₁₂N₅O₃K+2.5H₂O
Calculated: C, 50.22; H, 3.98; N, 16.27.
Found: C, 50.45; H, 3.98; N, 16.38.

### Example 83

4-(1-hydroxy-1-methylethyl)-N-[6-(1H-imidazol-1-yl)imidazo[1,2-a]pyridin-2-yl]benzamide

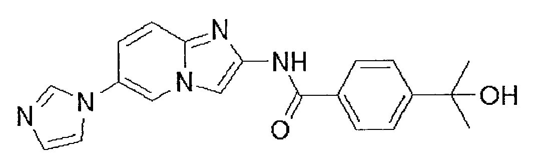

Methyl 4-{[6-(1H-imidazol-1-yl)imidazo[1,2-a]pyridin-2-yl]carbamoyl}benzoate (60 mg, 0.17 mmol) obtained in Example 99 was dissolved in THF (20 mL), 1M methylmagnesium bromide - THF solution (3.4 mL, 3.4 mmol) was added, and the mixture was stirred at 80°C for 3 hr. Saturated aqueous ammonium chloride solution was added, and the mixture was extracted with ethyl acetate. The extract was dried (MgSO₄), and the solvent was evaporated under reduced pressure. The residue was purified by silica gel chromatography (ethyl acetate:methanol=10:0 - 8:2) and recrystallized from ethyl acetate-methanol to give the title compound (28 mg, 41%).
melting point 281-282°C
MS (ESI+): 362 (M+H).
¹H-NMR (DMSO-d₆) δ: 1.46 (6H, s), 5.16 (1H, s), 7.15 (1H, s), 7.55-7.64 (4H, m), 7.70 (1H, s), 8.03 (2H, d, J = 8.5 Hz), 8.19 (1H, s), 8.34 (1H, s), 9.07 (1H, s), 11.22 (1H, s). Elemental analysis: for C₂₀H₁₉N₅O₂
Calculated: C, 66.47; H, 5.30; N, 19.38.
Found: C, 66.37; H, 5.30; N, 19.29.

### Example 84

4-(1-hydroxy-1-methylethyl)-N-[6-(1H-imidazol-1-yl)imidazo[1,2-a]pyridin-2-yl]benzamide dihydrochloride

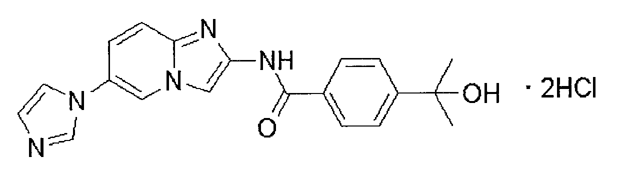

According to Example 52 and using 4-(1-hydroxy-1-methylethyl)-N-[6-(1H-imidazol-1-yl)imidazo[1,2-a]pyridin-2-yl]benzamide obtained in Example 83, the title compound was synthesized.
MS (ESI+): 362 (M+H).
¹H-NMR (DMSO-d₆) δ: 1.46 (6H, s), 5.06 (1H, s), 7.61 (2H, d, J = 8.5 Hz), 7.69 (1H, dd, J = 9.6, 2.1 Hz), 7.77 (1H, d, J = 9.6 Hz), 7.96 (1H, t, J = 1.7 Hz), 8.04 (2H, d, J = 8.5 Hz), 8.24 (1H, t, J = 1.7 Hz), 8.43 (1H, s), 9.28 (1H, t, J = 1.3 Hz), 9.68 (1H, s), 11.36 (1H, s).

### Example 85

4-dimethylamino-N-[6-(1H-imidazol-1-yl)imidazo[1,2-a]pyridin-2-yl]benzamide

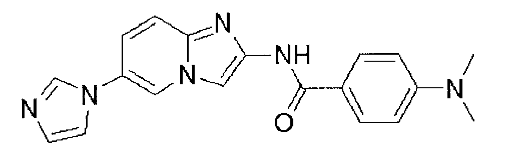

According to Example 51 and using 4-dimethylamino-N-[6-iodo[1,2-a]pyridin-2-yl]benzamide obtained in Example 81, the title compound was synthesized.
melting point 247-248°C
MS (ESI+): 347 (M+H).
¹H-NMR (DMSO-d₆) δ: 3.01 (6H, s), 6.74 (2H, d, J = 9.0 Hz),7.15 (1H, s), 7.50-7.62 (2H, m), 7.69 (1H, s), 7.99 (2H, d, J = 9.0 Hz), 8.18 (1H, s), 8.30 (1H,s), 9.04 (1H,s), 10.86 (1H,s). Elemental analysis: for C₁₉H₁₈N₆O
Calculated: C, 65.88; H, 5.24; N, 24.26.
Found: C, 65.64; H, 5.22; N, 24.12.

### Example 86

6-tert-butyl-N-[6-(1H-imidazol-1-yl)imidazo[1,2-a]pyridin-2-yl]nicotinamide

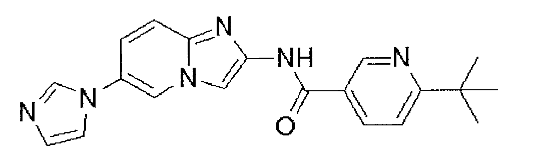

According to Example 49, 6-tert-butyl-N-(6-iodoimidazo[1,2-a]pyridin-2-yl)nicotinamide was synthesized using 6-tert-butylnicotinoyl chloride obtained from 6-iodoimidazo[1,2-a]pyridine-2-amine obtained in Reference Example 23 and 6-tert-butylnicotinic acid synthesized in Reference Example 45. According to Example 51 and using the compound, the title compound was synthesized.
melting point 242-245°C
MS (ESI+): 361 (M+H).
¹H-NMR (DMSO-d₆) δ: 1.36 (9H, s), 7.16 (s, 1H), 7.56-7.66 (3H, m), 7.71 (1H, s), 8.20 (1H, s), 8.32-8.38 (2H, m), 9.07-9.09 (1H, m), 9.14 (1H, d), 11.46 (1H, s).
Elemental analysis: for C₂₀H₂₀N₆O·0.6H₂O
Calculated: C, 64.71; H, 5.76; N, 22.64.
Found: C, 64.45; H, 5.69; N, 22.35.

### Example 87

6-tert-butyl-N-[6-(1H-imidazol-1-yl)imidazo[1,2-a]pyridin-2-yl]nicotinamide trihydrochloride

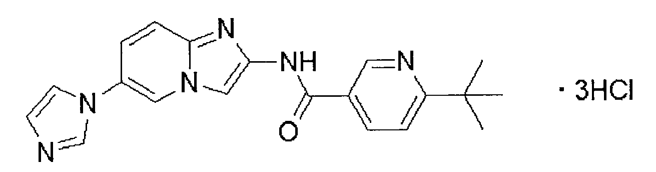

According to Example 52 and using 6-tert-butyl-N-[6-(1H-imidazol-1-yl)imidazo[1,2-a]pyridin-2-yl]nicotinamide obtained in Example 86, the title compound was synthesized.
MS (ESI+): 361 (M+H).
¹H-NMR (DMSO-d₆) δ: 1.39 (9H, s), 7.69-7.75 (2H, m), 7.80 (1H, d, J = 9.6 Hz), 7.98 (1H, t, J = 1.7 Hz), 8.26 (1H, t, J = 1.7 Hz), 8.44 (1H, s), 8.51 (1H, dd, J = 8.4, 2.2 Hz), 9.20 (1H, d, J = 1.9 Hz), 9.30-9.34 (1H, m), 9.73 (1H, t, J = 1.4 Hz), 11.74 (1H, s).
Elemental analysis: for C₂₀H₂₀N₆O·3HCl
Calculated: C, 51.13; H, 4.93; N, 17.89.
Found: C, 51.01; H, 5.00; N, 17.90.

### Example 88

4-(1H-imidazol-2-yl)-N-[6-(1H-imidazol-1-yl)imidazo[1,2-a]pyridin-2-yl]benzamide

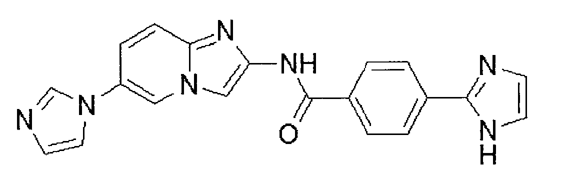

According to Example 49 and using 6-iodoimidazo[1,2-a]pyridine-2-amine obtained in Reference Example 23 and 4-bromobenzoyl chloride, 4-bromo-N-(6-iodoimidazo[1,2-a]pyridin-2-yl)benzamide was synthesized. According to Example 51, this compound (3.5 g, 7.9 mmol) was reacted to give 4-bromo-N-[6-(1H-imidazol-1-yl)imidazo[1,2-a]pyridin-2-yl]benzamide (350 mg, 12%) and the title compound (120 mg, 4%).
melting point 276-279°C (decomposed)
MS (ESI+): 370 (M+H).
¹H-NMR (DMSO-d₆) δ: 7.16 (2H, s), 7.56-7.67 (2H, m), 7.70 (1H, s), 7.86 (2H, d, J = 8.7 Hz), 7.91 (1H, s), 8.20 (1H, s), 8.25 (2H, d, J = 8.7 Hz), 8.37 (1H, s), 8.44 (1H, s), 9.08 (1H, d), 11.42 (1H, s).
Elemental analysis: for C₂₀H₁₅N₇O+H₂O
Calculated: C, 62.01; H, 4.42; N, 25.31.
Found: C, 61.92; H, 4.62; N, 25.22.

### Example 89

4-(3-furyl)-N-[6-(1H-imidazol-1-yl)imidazo[1,2-a]pyridin-2-yl]benzamide

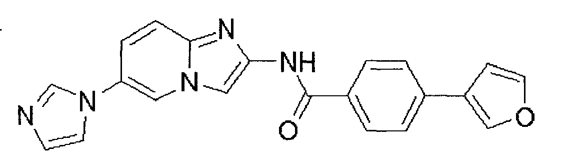

According to Example 57 and using 4-bromo-N-[6-(1H-imidazol-1-yl)imidazo[1,2-a]pyridin-2-yl]benzamide obtained in Example 88, the title compound was synthesized.
melting point 261-263°C
MS (ESI+): 370 (M+H).
¹H-NMR (DMSO-d₆) δ: 7.07-7.10 (1H, m), 7.14-7.17 (1H, m), 7.56-7.65 (2H, m), 7.70 (1H, t, J = 1.4 Hz), 7.76-7.81 (3H, m), 8.12 (2H, d, J = 8.5 Hz), 8.20 (1H, s), 8.36 (2H, br), 9.07-9.09 (1H, m), 11.31 (1H, s).
Elemental analysis: for C₂₁H₁₅N₅O₂
Calculated: C, 68.28; H, 4.09; N, 18.96.
Found: C, 68.00; H, 4.10; N, 18.86.

### Example 90

N-[6-chloroimidazo[1,2-a]pyridin-2-yl]-4-(2-hydroxy-1,1-dimethylethyl)benzamide

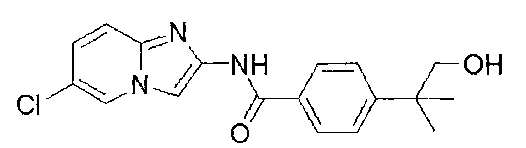

According to Example 49 and using 4-(2-{[tert-butyl(dimethyl)silyl]oxy}-1,1-dimethylethyl)benzoyl chloride obtained from 6-chloroimidazo[1,2-a]pyridine-2-amine and 4-(2-{[tert-butyl(dimethyl)silyl]oxy}-1,1-dimethylethyl)benzoic acid obtained in Reference Example 21, 4-(2-{[tert-butyl(dimethyl)silyl]oxy}-1,1-dimethylethyl)-N-(6-chloroimidazo[1,2-a]pyridin-2-yl)benzamide was synthesized. The obtained compound (330 mg, 0.72 mmol) was dissolved in THF (50 mL), 1M-tetra-N-butylammonium fluoride - THF solution (14.4 mL, 14.4 mmol) was added, and the mixture was stirred at 45°C for 4 hr. Under reduced pressure, the solvent was evaporated, saturated brine was added, and the mixture was extracted with dichloromethane. The extract was dried (MgSO₄) and the solvent was evaporated under reduced pressure. The residue was purified by silica gel chromatography (ethyl acetate:hexane=6:4 - 10:0), and recrystallized from ethyl acetate to give the title compound (110 mg, 44%).
melting point 240-242°C
MS (ESI+): 344 (M+H).
¹H-NMR (DMSO-d₆) δ: 1.26 (6H, s), 3.46 (2H, d, J = 5.5 Hz), 4.73 (1H, t, J = 5.5 Hz), 7.28 (1H, d, J = 9.5 Hz), 7.47-7.53 (3H, m), 8.00 (2H, d, J = 8.5 Hz), 8.33 (1H, s), 8.88 (1H,s), 11.19 (1H, s) .
Elemental analysis: for C₁₈H₁₉N₃O₂Cl
Calculated: C, 62.88; H, 5.28; N, 12.22.
Found: C, 62.79; H, 5.35; N, 12.18.

### Example 91

N-[6-chloroimidazo[1,2-a]pyridin-2-yl]-4-(2-hydroxy-1,1-dimethylethyl)benzamide hydrochloride

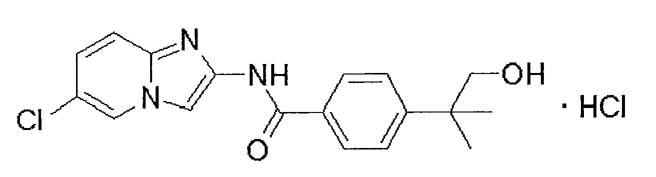

According to Example 52 and using N-[6-chloroimidazo[1,2-a]pyridin-2-yl]-4-(2-hydroxy-1,1-dimethylethyl)benzamide obtained in Example 90, the title compound was synthesized.
MS (ESI+): 344 (M+H).
¹H-NMR (DMSO-d₆) δ: 1.26 (6H, s), 3.47 (2H, s), 7.42-7.68 (4H, m), 8.01 (2H, d, J = 8.5 Hz), 8.32 (1H, s), 8.98 (1H,s), 11.47 (1H,s), hidden (1H).
Elemental analysis: for C₁₈H₁₉N₃O₂Cl·HCl
Calculated: C, 56.85; H, 5.04; N, 11.05.
Found: C, 56.75; H, 5.29; N, 11.32.

### Example 92

4-(1-amino-1-methylethyl)-N-(6-chloroimidazo[1,2-a]pyridin-2-yl)benzamide

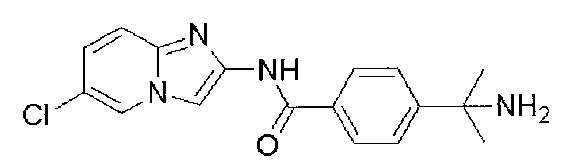

According to Example 49 and using 6-chloroimidazo[1,2-a]pyridine-2-amine obtained in Reference Example 21 and 4-cyanobenzoyl chloride, 4-cyano-N-[6-chloroimidazo[1,2-a]pyridin-2-yl]benzamide was synthesized. To a suspension (-60°C) of cerium chloride (60 mmol, 4.9 g) in THF (60 mL) was added methyl lithium (1.5M-THF solution)(60 mmol, 40 mL), and the mixture was stirred at -60°C for 1 hr. Then, 4-cyano-N-[6-chloroimidazo[1,2-a]pyridin-2-yl]benzamide (3.0 mmol, 890 mg) was added, and the mixture was stirred at room temperature for 4 hr. Under reduced pressure, the solvent was evaporated, saturated aqueous ammonium chloride solution was added, and the mixture was extracted with dichloromethane. The extract was dried (MgSO₄), and the solvent was evaporated under reduced pressure. The residue was purified by silica gel chromatography (ethyl acetate:hexane=8:2 - 10:0), and recrystallized from methanol to give the title compound (90 mg, 9%).
melting point 235-237°C
MS (ESI+): 329 (M+H).
¹H-NMR (DMSO-d₆) δ: 1.39 (6H, s), 1.98 (2H, s), 7.28 (1H, d, J = 9.5 Hz), 7.50 (1H, d, J = 9.5 Hz), 7.66 (2H, d, J = 8.5 Hz), 8.01 (2H, d, J = 8.5 Hz), 8.33 (1H, s), 8.88 (1H,s), 11.19 (1H,s) .
Elemental analysis: for C₁₇H₁₇N₄OCl
Calculated: C, 62.10; H, 5.21; N, 17.04.
Found: C, 61.93; H, 5.12; N, 16.98.

### Example 93

N-(6-chloroimidazo[1,2-a]pyridin-2-yl)-4-(1-cyano-1-methylethyl)benzamide

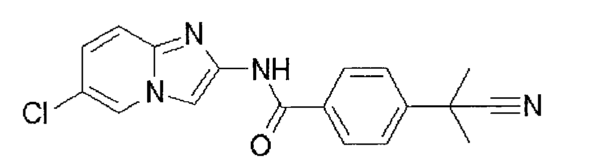

According to Example 49 and using 4-(1-cyano-1-methylethyl)benzoyl chloride from obtained 6-chloroimidazo[1,2-a]pyridine-2-amine obtained in Reference Example 21 and 4-(1-cyano-1-methylethyl)benzoic acid obtained in Reference Example 2, the title compound was synthesized. melting point 248-250°C
MS (ESI+): 339 (M+H).
¹H-NMR (DMSO-d₆) δ: 1.73 (6H, s), 7.29 (1H, d, J = 9.4 Hz), 7.51 (1H, d, J = 9.4 Hz), 7.66 (2H, d, J = 8.6 Hz), 8.13 (2H, d, J = 8.6 Hz), 8.34 (1H, s), 8.89 (1H,s), 11.35 (1H,s). Elemental analysis: for C₁₈H₁₅N₄OCl+0.7H₂O
Calculated: C, 61.52; H, 4.70; N, 15.94.
Found: C, 61.31; H, 4.40; N, 16.09.

### Example 94

4-(2-amino-1,1-dimethylethyl)-N-(6-chloroimidazo[1,2-a]pyridin-2-yl)benzamide

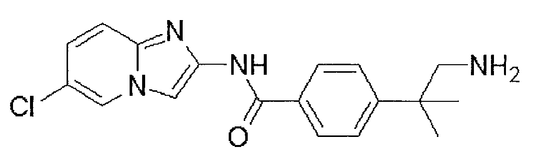

N-(6-Chloroimidazo[1,2-a]pyridin-2-yl)-4-(1-cyano-1-methylethyl)benzamide (0.3 mmol, 100 mg) obtained in Example 93 was dissolved in 3N-ammonia-containing methanol (50 mL), Raney-Co (1.0 g) was added, and the mixture was stirred under a hydrogen atmosphere at 50°C for 10 min. Raney-Co was removed by filtration and the solvent was evaporated under reduced pressure. The residue was purified by silica gel chromatography (ethyl acetate:methanol=10:0 - 9:1), and recrystallized from dichloromethane-hexane to give the title compound (60 mg, 58%).
melting point 245-247°C
MS (ESI+): 343 (M+H).
¹H-NMR (CDCl₃) δ: 1.34 (6H, s), 2.85 (2H, s), 7.12 (1H, d, J = 9.4Hz), 7.21-7.26 (1H, m), 7.47 (2H, d, J = 8.5 Hz), 7.90 (2H, d, J = 8.5 Hz), 8.17 (1H, s), 8.23 (1H,s), 9.15 (1H,s), hidden (2H).
Elemental analysis: for C₁₈H₁₉N₄OCl
Calculated: C, 63.06; H, 5.59; N, 16.34.
Found: C, 62.83; H, 5.60; N, 16.31.

### Example 95

N-(6-chloroimidazo[1,2-a]pyridin-2-yl)-4-(1H-pyrazol-1-yl)benzamide

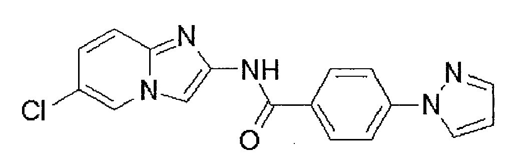

According to Example 49 and using 6-chloroimidazo[1,2-a]pyridine-2-amine obtained in Reference Example 21 and 4-(1H-pyrazol-1-yl)benzoyl chloride, the title compound was synthesized.
melting point 287-289°C
MS (ESI+): 338 (M+H).
¹H-NMR (DMSO-d₆) δ: 6.61 (1H, t, J = 2.1 Hz), 7.29 (1H, dd, J = 9.4, 2.1 Hz), 7.52 (1H, d, J = 9.4 Hz), 7.82 (1H, s), 8.00 (2H, d, J = 8.8 Hz), 8.22 (2H, d, J = 8.8 Hz), 8.35 (1H, s), 8.66 (1H,s), 8.89 (1H,s), 11.35 (1H, s).
Elemental analysis: for C₁₇H₁₂N₅OCl
Calculated: C, 60.45; H, 3.58; N, 20.73.
Found: C, 60.38; H, 3.57; N, 20.84.

### Example 96

5-tert-butyl-N-[6-chloroimidazo[1,2-a]pyridin-2-yl]pyridine-2-carboxamide

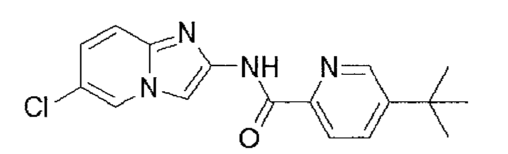

According to Example 49 and using 6-chloroimidazo[1,2-a]pyridine-2-amine obtained in Reference Example 21 and 5-tert-butylpyridine-2-carbonyl chloride obtained in Reference Example 46, the title compound was synthesized.
MS (ESI+): 329 (M+H).
¹H-NMR (CDCl₃) δ: 1.40 (9H, s), 7.15 (1H, d, J = 9.5 Hz), 7.43 (1H, d, J = 9.5 Hz), 7.88 (1H, dd, J = 8.2, 2.4 Hz), 8.16-8.24 (3H, m), 8.67 (1H, d, J = 2.4 Hz), 10.35 (1H, s).

### Example 97

N-[6-(1H-imidazol-1-yl)imidazo[1,2-a]pyridin-2-yl]-4-isopropylbenzamide

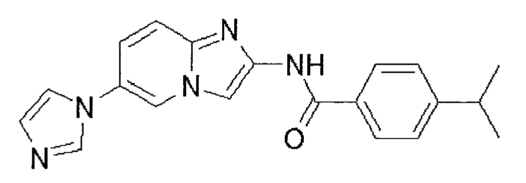

According to Example 49 and using 4-isopropylbenzoyl chloride, N-(6-iodoimidazo[1,2-a]pyridin-2-yl)-4-isopropylbenzamide was synthesized, then according to Example 51, converted to the title compound.
melting point 291-293°C
MS (ESI+): 346 (M+H).
¹H-NMR (DMSO-d₆) δ: 1.24 (6H, d, J = 7.0 Hz), 2.92-3.03 (1H, m), 7.15 (1H, s), 7.39 (2H, d, J = 8.3 Hz), 7.54-7.65 (2H, m), 7.70 (1H, t, J = 1.3 Hz), 8.02 (2H, d, J = 8.3 Hz), 8.19 (1H, s), 8.34 (1H, s), 9.05-9.08 (1H, m), 11.22 (1H, s).
Elemental analysis: for C₂₀H₁₉N₅O
Calculated: C, 69.55; H, 5.54; N, 20.28.
Found: C, 69.32; H, 5.48; N, 20.28.

### Example 98

N-[6-(1H-imidazol-1-yl)imidazo[1,2-a]pyridin-2-yl]-4-isopropylbenzamide dimethanesulfonate

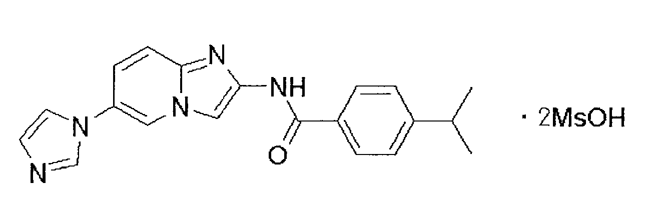

According to Example 53 and using N-[6-(1H-imidazol-1-yl)imidazo[1,2-a]pyridin-2-yl]-4-isopropylbenzamide obtained in Example 97, the title compound was synthesized.
MS (ESI+): 346 (M+H).
¹H-NMR (DMSO-d₆) δ: 1.24 (6H, d, J = 6.8 Hz), 2.34 (6H, s), 2.93-3.04 (1H, m), 7.41 (2H, d, J = 8.3 Hz), 7.69 (1H, dd, J = 9.5, 2.1 Hz), 7.78 (1H, d, J = 9.5 Hz), 7.96-7.98 (1H, m), 8.03 (2H, d, J = 8.3 Hz), 8.25 (1H, t, J = 1.7 Hz), 8.43 (1H, s), 9.27 (1H, d, J = 1.3 Hz), 9.67 (1H, s), 11.35 (1H, s). Elemental analysis: for C₂₀H₁₉N₅O·2MsOH·0.5H₂O
Calculated: C, 69.55; H, 5.54; N, 20.28.
Found: C, 69.32; H, 5.48; N, 20.28.

### Example 99

methyl 4-{[6-(1H-imidazol-1-yl)imidazo[1,2-a]pyridin-2-yl]carbamoyl}benzoate

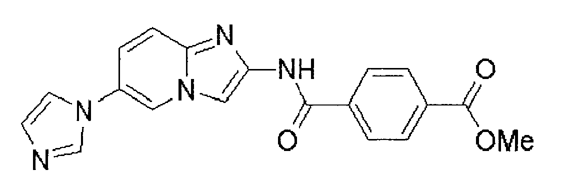

According to Example 49 and using methyl 4-(chlorocarbonyl)benzoate, methyl 4-[(6-iodoimidazo[1,2-a]pyridin-2-yl)carbamoyl]benzoate was synthesized, then according to Example 51, converted to the title compound.
MS (ESI+): 361 (M+H).
¹H-NMR (DMSO-d₆) δ: 3.90 (3H, s), 7.16 (1H, s), 7.59 (1H, d, J = 9.5 Hz), 7.65 (1H, d, J = 9.5 Hz), 7.70 (1H, t, J = 1.3 Hz), 8.08 (2H, d, J = 8.7 Hz), 8.17-8.22 (3H, m), 8.37 (1H, s), 9.07-9.10 (1H, m), 11.55 (1H, s).

### Example 100

6-tert-butyl-N-[6-(1,3-oxazol-5-yl)imidazo[1,2-a]pyridin-2-yl]nicotinamide

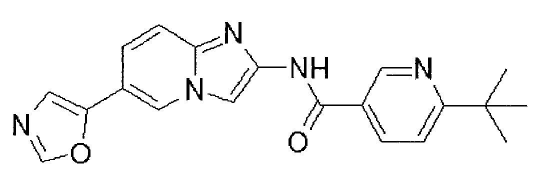

According to Example 74 and using 6-tert-butyl-N-(6-iodoimidazo[1,2-a]pyridin-2-yl)nicotinamide obtained in Example 86, the title compound was synthesized.
melting point 251-253°C MS (ESI+): 362 (M+H).
¹H-NMR (DMSO-d₆) δ: 1.35 (9H, s), 7.56-7.66 (3H, m), 7.70 (1H, s), 8.32-8.37 (1H, m), 8.41 (1H, s), 8.51 (1H, s), 9.06 (1H, s), 9.14 (1H, s), 11.44 (1H, s).

### Example 101

6-tert-butyl-N-[6-(1,3-oxazol-5-yl)imidazo[1,2-a]pyridin-2-yl]nicotinamide dihydrochloride

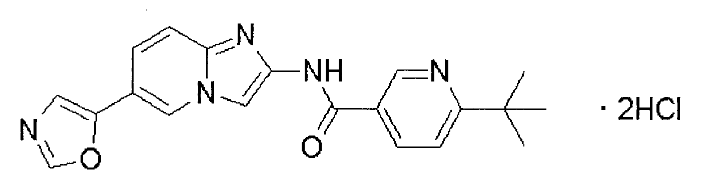

According to Example 52 and using 6-tert-butyl-N-[6-(1,3-oxazol-5-yl)imidazo[1,2-a]pyridin-2-yl]nicotinamide obtained in Example 100, the title compound was synthesized.
MS (ESI+): 362 (M+H).
¹H-NMR (DMSO-d₆) δ: 1.39 (9H, s), 7.70-7.79 (3H, m), 7.80-7.86 (1H, m), 8.44 (1H, s), 8.51 (1H, dd, J = 8.5, 2.3 Hz), 8.56 (1H, s), 9.15-9.22 (2H, m), 11.89 (1H, s).
Elemental analysis: for C₂₀H₁₉N₅O₂·2HCl·1.5H₂O
Calculated: C, 69.55; H, 5.54; N, 20.28.
Found: C, 69.32; H, 5.48; N, 20.28.

### Example 102

6-tert-butyl-N-(6-(pyridin-3-yl)imidazo[1,2-a]pyridin-2-yl)nicotinamide

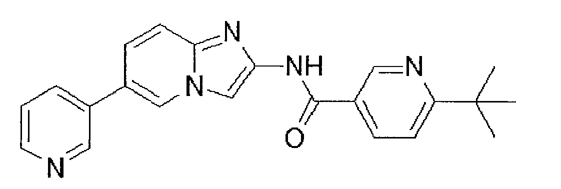

According to Example 57 and using 6-tert-butyl-N-[6-iodoimidazo[1,2-a]pyridin-2-yl]nicotinamide obtained in Example 86, the title compound was synthesized.
melting point 272-274°C MS (ESI+): 372 (M+H).
¹H-NMR (DMSO-d₆) δ: 1.36 (9H, s), 7.50-7.70 (4H, m), 8.12-8.16 (1H, m), 8.33-8.39 (2H, m), 8.61 (1H, dd, J = 4.8, 1.6 Hz), 8.96 (1H, d, J = 1.7 Hz), 9.08-9.10 (1H, m), 9.15 (1H, d, J = 1.7 Hz), 11.43 (1H, s).
Elemental analysis: for C₂₂H₂₁N₅O·0.2H₂O
Calculated: C, 70.46; H, 5.75; N, 18.67.
Found: C, 70.69; H, 5.60; N, 18.67.

### Example 103

4-(2-hydroxy-1,1-dimethylethyl)-N-[6-(1H-imidazol-1-yl)imidazo[1,2-a]pyridin-2-yl]benzamide

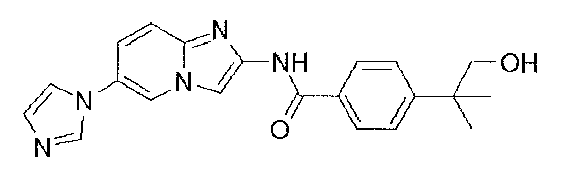

According to Example 49 and using 4-(2-{[tert-butyl(dimethyl)silyl]oxy}-1,1-dimethylethyl)benzoyl chloride obtained from 6-iodoimidazo[1,2-a]pyridine-2-amine (1.03 g, 4.0 mmol) obtained in Reference Example 23 and 4-(2-{[tert-butyl(dimethyl)silyl]oxy}-1,1-dimethylethyl)benzoic acid (1.23 g, 4.0 mmol) obtained in Reference Example 50, 4-(2-{[tert-butyl(dimethyl)silyl]oxy}-1,1-dimethylethyl)-N-(6-iodoimidazo[1,2-a]pyridin-2-yl)benzamide (770 mg, 1.40 mmol) was synthesized. The obtained compound was reacted according to Example 51 to give 4-(2-{[tert-butyl(dimethyl)silyl]oxy}-1,1-dimethylethyl)-N-[6-(1H-imidazol-1-yl)imidazo[1,2-a]pyridin-2-yl]benzamide (370 mg, 0.76 mmol). Then, 1M-tetra-N-butylammonium fluoride - THF solution (2.28 mL) was added, and the mixture was stirred at 45°C for 4 hr. Under reduced pressure, the solvent was evaporated, and the residue was purified by silica gel chromatography (ethyl acetate:methanol=10:0 - 8:2) and recrystallized from dichloromethane-hexane to give the title compound (210 mg, 74%).
melting point 278-280°C MS (ESI+) : 376 (M+H).
¹H-NMR (DMSO-d₆) δ: 1.26 (6H, s), 3.47 (2H, d, J = 5.3 Hz), 4.74 (1H, t, J = 5.3 Hz), 7.15 (1H, s), 7.51 (2H, d, J = 8.6 Hz), 7.55-7.64 (2H, m), 7.70 (1H, t, J = 1.2 Hz), 8.02 (2H, d, J = 8.6 Hz), 8.19 (1H, s), 8.34 (1H, s), 9.05-9.08 (1H, m), 11.22 (1H, s).
Elemental analysis: for C₂₁H₂₁N₅O₂·0.5H₂O
Calculated: C, 65.61; H, 5.77; N, 18.22.
Found: C, 65.30; H, 5.76; N, 18.03.

### Example 104

4-(2-hydroxy-1,1-dimethylethyl)-N-[6-(1H-imidazol-1-yl)imidazo[1,2-a]pyridin-2-yl]benzamide dihydrochloride

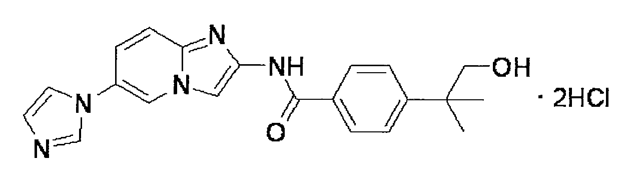

According to Example 52 and using 4-(2-hydroxy-1,1-dimethylethyl)-N-[6-(1H-imidazol-1-yl)imidazo[1,2-a]pyridin-2-yl]benzamide obtained in Example 103, the title compound was synthesized.
MS (ESI+): 376 (M+H).
¹H-NMR (DMSO-d₆) δ: 1.26 (6H, s), 3.47 (2H, s), 7.52 (2H, d, J = 8.5 Hz), 7.67 (1H, dd, J = 9.6, 2.1 Hz), 7.76 (1H, d, J = 9.6 Hz), 7.95 (1H, s), 8.02 (2H, d, J = 8.5 Hz), 8.23 (1H, s), 8.42 (1H, s), 9.24-9.27 (1H, m), 9.64 (1H, s), 11.22 (1H, s), hidden (1H).
Elemental analysis: for C₂₁H₂₁N₅O₂·2HCl·1.4H₂O
Calculated: C, 53.26; H, 5.49; N, 14.79.
Found: C, 53.58; H, 5.77; N, 14.69.

### Example 105

6-tert-butyl-N-[6-(1-methyl-1H-pyrazol-4-yl)imidazo[1,2-a]pyridin-2-yl]nicotinamide

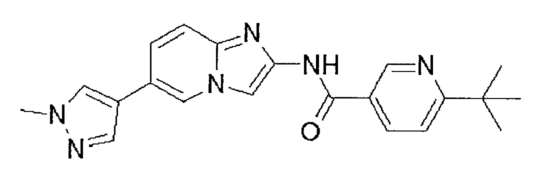

According to Example 57 and using 6-tert-butyl-N-[6-iodoimidazo[1,2-a]pyridin-2-yl]nicotinamide and 1-methyl-4-(4,4,5,5-tetramethyl-1,3,2-dioxaborolan-2-yl)-1H-pyrazole obtained in Example 86, the title compound was synthesized. melting point 228-230°C
MS (ESI+): 375 (M+H).
¹H-NMR (DMSO-d₆) δ: 1.35 (9H, s), 3.89 (3H, s), 7.49 (2H, br), 7.58 (1H, dd, J = 8.5, 0.8 Hz), 7.87 (1H, d, J = 0.8 Hz), 8.14 (1H, s), 8.24 (1H, s), 8.35 (1H, dd, J = 8.3, 2.4 Hz), 8.86 (1H, t, J = 1.3 Hz), 9.12-9.15 (1H, m), 11.36 (1H, s).
Elemental analysis: for C₂₁H₂₂N₆O·0.2H₂O
Calculated: C, 66.72; H, 5.97; N, 22.23.
Found: C, 66.89; H, 5.90; N, 22.22.

### Example 106

4-tert-butyl-N-[6-(6-methoxypyridin-3-yl)imidazo[1,2-a]pyridin-2-yl]benzamide

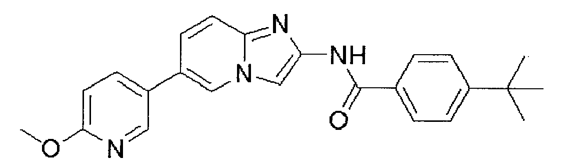

According to Example 57 and using 2-methoxy-5-(4,4,5,5-tetramethyl-1,3,2-dioxaborolan-2-yl)pyridine, the title compound was synthesized.
melting point 245-247°C
MS (ESI+): 401 (M+H).
¹H-NMR (CDCl₃) δ: 1.35 (9H, s), 4.00 (3H, s), 6.87 (1H, d, J = 8.7 Hz), 7.28-7.30 (2H, m), 7.50 (2H, d, J = 8.6 Hz), 7.75 (1H, dd, J = 8.7, 2.6 Hz), 7.91 (2H, d, J = 8.6 Hz), 8.23 (1H, t, J = 1.4 Hz), 8.30 (1H, s), 8.36 (1H, d, J = 2.1 Hz), 9.45 (1H, s). Elemental analysis: for C₂₄H₂₄N₄O₂
Calculated: C, 71.98; H, 6.04; N, 13.99.
Found: C, 71.22; H, 6.13; N, 13.81.

### Example 107

4-tert-butyl-N-[6-(2-thienyl)imidazo[1,2-a]pyridin-2-yl]benzamide

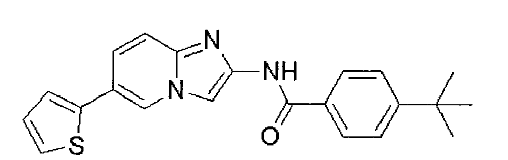

According to Example 57 and using 4,4,5,5-tetramethyl-2-(2-thienyl)-1,3,2-dioxaborolane, the title compound was synthesized.
melting point 273-275°C
MS (ESI+): 376 (M+H).
¹H-NMR (CDCl₃) δ: 1.35 (9H, s), 7.10-7.14 (1H, m), 7.27-7.34 (2H, m), 7.37 (1H, d, J = 9.3 Hz), 7.43 (1H, d, J = 9.3 Hz), 7.52 (2H, d, J = 8.6 Hz), 7.89 (2H, d, J = 8.6 Hz), 8.26 (1H, s), 8.34 (1H, s), 9.01 (1H, s).
Elemental analysis: for C₂₂H₂₁N₃OS
Calculated: C, 70.37; H, 5.64; N, 11.19.
Found: C, 70.07; H, 5.60; N, 11.16.

### Example 108

4-tert-butyl-N-[6-(3-thienyl)imidazo[1,2-a]pyridin-2-yl]benzamide

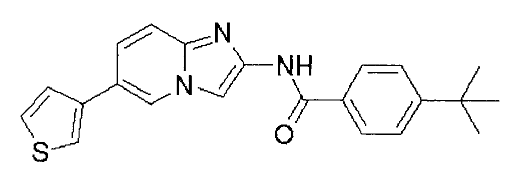

According to Example 57 and using 4,4,5,5-tetramethyl-2-(3-thienyl)-1,3,2-dioxaborolane, the title compound was synthesized.
melting point 261-263°C
MS (ESI+): 376 (M+H).
¹H-NMR (CDCl₃) δ: 1.36 (9H, s), 7.31-7.48 (5H, m), 7.51 (2H, d, J = 8.6 Hz), 7.90 (2H, d, J = 8.6 Hz), 8.26 (1H, s), 8.31-8.34 (1H, m), 9.12 (1H, s).
Elemental analysis: for C₂₂H₂₁N₃OS
Calculated: C, 70.37; H, 5.64; N, 11.19.
Found: C, 70.02; H, 5.54; N, 11.13.

### Example 109

N-[6-(benzylamino)imidazo[1,2-a]pyridin-2-yl]-4-tert-butylbenzamide

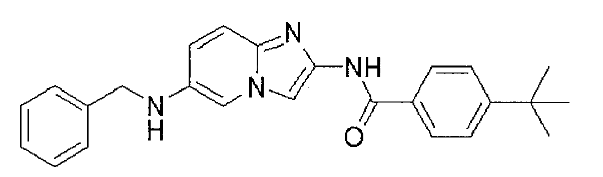

According to Example 51 and using benzylamine, the title compound was obtained.
MS (ESI+): 399 (M+H).
¹H-NMR (DMSO-d₆) δ: 1.31 (9H, s), 4.20 (2H, d, J = 6.0 Hz), 5.99 (1H, t, J = 6.0 Hz), 6.92 (1H, dd, J = 9.5, 2.2 Hz), 7.20-7.30 (2H, m), 7.32-7.39 (2H, m), 7.41-7.46 (2H, m), 7.50 (2H, d, J = 8.6 Hz), 7.66 (1H, d, J = 1.9 Hz), 7.99 (2H, d, J = 8.6 Hz), 8.02 (1H, s), 10.89 (1H, s).

### Example 110

N-(6-chloroimidazo[1,2-a]pyridin-2-yl)-4-[(methylsulfonyl)amino]benzamide

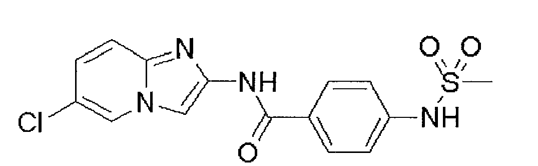

According to Example 49 and using 6-chloroimidazo[1,2-a]pyridine-2-amine obtained in Reference Example 21 and 4-[(methylsulfonyl)amino]benzoyl chloride, the title compound was synthesized.
melting point 293-295°C
MS (ESI+): 365 (M+H).
¹H-NMR (DMSO-d₆) δ: 3.09 (3H, s), 7.24-7.31 (3H, m), 7.50 (1H, d, J = 9.4 Hz), 8.05 (2H, d, J = 8.9 Hz), 8.31 (1H, s), 8.86-8.89 (1H, m), 10.22 (1H, s), 11.16 (1H, s).
Elemental analysis: for C₁₅H₁₃N₄O₃SCl
Calculated: C, 49.39; H, 3.59; N, 15.36.
Found: C, 49.36; H, 3.65; N, 15.39.

### Example 111

4-tert-butyl-N-[6-(1H-pyrazol-4-yl)imidazo[1,2-a]pyridin-2-yl]benzamide

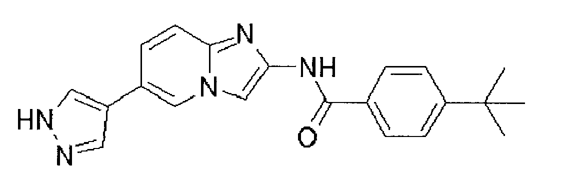

According to Example 57 and using 4-(4,4,5,5-tetramethyl-1,3,2-dioxaborolan-2-yl)-1H-pyrazole, the title compound was synthesized.
melting point 278-280°C
MS (ESI+): 360 (M+H).
¹H-NMR (DMSO-d₆) δ: 1.32 (9H, s), 7.44-7.57 (4H, m), 7.98-8.24 (5H, m), 8.88 (1H, s), 11.11 (1H, s), 13.01 (1H, s).
Elemental analysis: for C₂₁H₂₁N₅O
Calculated: C, 70.17; H, 5.89; N, 19.48.
Found: C, 70.04; H, 6.04; N, 19.20.

### Example 112

4-tert-butyl-N-[6-(1-phenyl-1H-pyrazol-4-yl)imidazo[1,2-a]pyridin-2-yl]benzamide

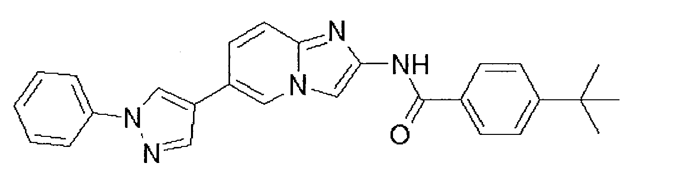

4-tert-Butyl-N-[6-(1H-pyrazol-4-yl)imidazo[1,2-a]pyridin-2-yl]benzamide (0.25 mmol, 90 mg) obtained in Example 111, copper acetate (0.25 mmol, 45 mg), pyridine (0.375 mmol, 30 mg), phenylboronic acid (0.5 mmol, 61 mg) were suspended in DMF (3 mL), and the mixture was stirred using microwave at 100°C for 20 min. Under reduced pressure, the solvent was evaporated, aqueous ammonia was added, and the mixture was extracted with ethyl acetate. The extract was dried (MgSO₄), and the solvent was evaporated under reduced pressure. The residue was purified by silica gel chromatography (ethyl acetate:methanol=10:0 - 9:1) to give the title compound (8 mg, 7%).
MS (ESI+): 436 (M+H).
¹H-NMR (DMSO-d₆) δ: 1.33 (9H, s), 7.35 (1H, t, J = 7.4 Hz), 7.51-7.70 (6H, m), 7.90 (2H, d, J = 8.6 Hz), 8.04 (2H, d, J = 8.6 Hz), 8.25 (2H, d, J = 6.8 Hz), 9.00 (1H, s), 9.04 (1H, s), 11.16 (1H, s).

### Example 113

4-tert-butyl-N-[6-(1-pyridin-3-yl-1H-pyrazol-4-yl)imidazo[1,2-a]pyridin-2-yl]benzamide

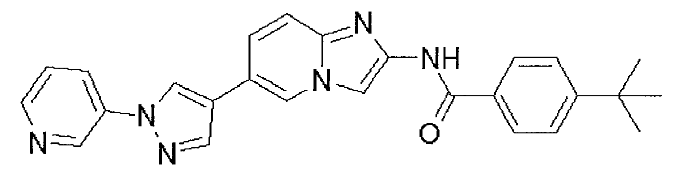

According to Example 112 and using 3-pyridineboronic acid, the title compound (8 mg, 7%) was synthesized.
MS (ESI+): 437 (M+H).
¹H-NMR (DMSO-d₆) δ: 1.33 (9H, s), 7.50-7.66 (5H, m), 8.05 (2H, d, J = 8.5 Hz), 8.26-8.33 (3H, m), 8.55-8.58 (1H, m), 9.02 (1H, s), 9.13 (1H, s), 9.17 (1H, d, J = 2.5 Hz), 11.17 (1H, s).

### Example 114

6-tert-butyl-N-[6-(1H-pyrazol-4-yl)imidazo[1,2-a]pyridin-2-yl]nicotinamide

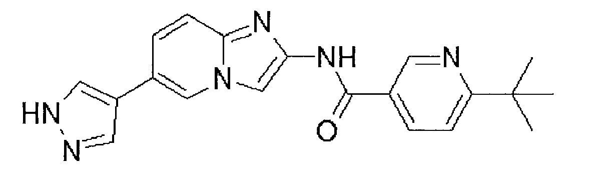

According to Example 57 and using 4-(4,4,5,5-tetramethyl-1,3,2-dioxaborolan-2-yl)-1H-pyrazole and 6-tert-butyl-N-(6-iodoimidazo[1,2-a]pyridin-2-yl)nicotinamide obtained in Example 86, the title compound was synthesized.
melting point 238-240°C
MS (ESI+): 361 (M+H).
¹H-NMR (DMSO-d₆) δ: 1.35 (9H, s), 7.48 (1H, d, J = 9.1 Hz), 7.52-7.60 (2H, m), 7.92-8.16 (2H, m), 8.23 (1H, s), 8.35 (1H, d, J = 8.3 Hz), 8.89 (1H, s), 9.13 (1H, d, J = 1.9 Hz), 11.36 (1H, s), 13.01 (1H, s).

### Example 115

4-tert-butyl-N-[6-(dimethylamino)imidazo[1,2-b]pyridazin-2-yl]benzamide hydrochloride

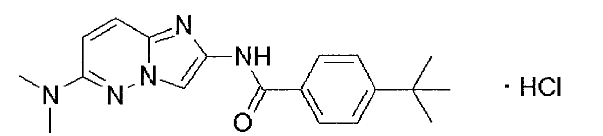

4-tert-Butyl-N-(6-chloroimidazo[1,2-b]pyridazin-2-yl)benzamide (0.73 mmol, 240 mg) obtained in Example 138, benzylamine hydrochloride (1.46 mmol, 209 mg), potassium carbonate (2.92 mmol, 403 mg) were suspended in DMF (4 mL), and the mixture was stirred using microwave at 130°C for 30 min. Under reduced pressure, the solvent was evaporated, saturated brine was added, and the mixture was extracted with ethyl acetate. The extract was dried (MgSO₄), and the solvent was evaporated under reduced pressure. The residue was purified by silica gel chromatography (ethyl acetate:hexane=8:2 - 10:0), and the title compound (60 mg, 22%) was obtained according to Example 52.
melting point 259-260°C
MS (ESI+): 338 (M+H).
¹H-NMR (CDCl₃) δ: 1.35 (9H, s), 3.20 (6H, s), 7.09 (1H, d, J = 10.0 Hz), 7.56 (2H, d, J = 8.6 Hz), 7.79 (1H, d, J = 10.0 Hz), 8.16 (2H, d, J = 8.6 Hz), 8.30-8.37 (1H, m), 11.68 (1H, s).
Elemental analysis: for C₁₉H₂₃N₅O·HCl·0.4H₂O
Calculated: C, 59.88; H, 6.56; N, 18.38.
Found: C, 59.69; H, 6.53; N, 18.28.

### Example 116

2-(4-{[(6-chloroimidazo[1,2-a]pyridin-2-yl)amino]carbonyl}phenyl)-2-methylpropanoic acid

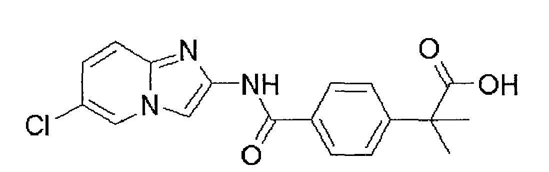

In the same manner as in Example 15 and using 4-(2-methoxy-1,1-dimethyl-2-oxoethyl)benzoic acid synthesized in Reference Example 1 and 6-chloroimidazo[1,2-a]pyridine-2-amine synthesized in Reference Example 21, methyl 2-methyl-2-{4-[(6-chloroimidazo[1,2-a]pyridin-2-yl)carbamoyl]phenyl}propanoate was obtained, which was treated in the same manner as in Example 116 to give the title compound.
melting point 311-313°C (decomp.)
MS (ESI+): 357 (M+).
¹H-NMR (DMSO-d₆) δ: 1.50 (6H, s), 7.28 (1H, dd, J = 9.5, 2.3 Hz), 7.44-7.53 (3H, m), 8.03 (2H, d, J = 8.3 Hz), 8.33 (1H, s), 8.86-8.89 (1H, m), 11.24 (1H, s), hidden (1H).
Elemental analysis: for C₁₈H₁₅N₃O₃Cl
Calculated: C, 60.42; H, 4.51; N, 11.74.
Found: C, 60.51; H, 4.81; N, 11.35.

### Example 117

3-(4-{[(6-chloroimidazo[1,2-a]pyridin-2-yl)amino]carbonyl}phenyl)-3-methylbutanoic acid

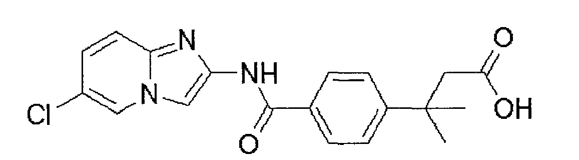

To a solution of ethyl 3-(4-{[(6-chloroimidazo[1,2-a]pyridin-2-yl)amino]carbonyl}phenyl)-3-methylbutanoate (0.15 mmol, 60 mg) obtained in Example 119 in THF (3 mL)-methanol (3 mL) was added 1N aqueous lithium hydroxide solution (1.5 mL) and the mixture was stirred at 80°C for 3 hr. Under reduced pressure, volatile solvent was evaporated, and hydrochloric acid was added to the residue to solidify the title compound. The solid was collected by filtration and recrystallized from DMSO-water to give the title compound (45 mg, 81%).
melting point 304-306°C (decomp.)
MS (ESI+): 371(M+).
¹H-NMR (DMSO-d₆) δ: 1.40 (6H, s), 2.63 (2H, s), 7.28 (1H, dd, J = 9.5, 2.3 Hz), 7.47-7.55 (3H, m), 8.00 (2H, d, J = 8.3 Hz), 8.33 (1H, s), 8.88 (1H, s), 11.20 (1H, s), hidden (1H). Elemental analysis: for C₁₉H₁₈N₃O₃Cl
Calculated: C, 61.38; H, 4.88; N, 11.30.
Found: C, 61.34; H, 4.60; N, 11.28.

### Example 118

4-(4-{[(6-chloroimidazo[1,2-a]pyridin-2-yl)amino]carbonyl}phenyl)-4-methylpentanoic acid

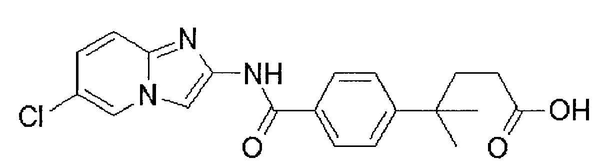

According to Example 117 and using ethyl 4-(4-{[(6-chloroimidazo[1,2-a]pyridin-2-yl)amino]carbonyl}phenyl)-4-methylpentanoate obtained in Example 120, the title compound was synthesized.
melting point 309-311°C (decomp.)
MS (ESI+): 385 (M+).
¹H-NMR (DMSO-d₆) δ: 1.30 (6H, s), 1.86-1.93 (4H, m), 7.28 (1H, dd, J = 9.4, 2.1 Hz), 7.43-7.54 (3H, m), 8.03 (2H, d, J = 8.5 Hz), 8.33 (1H, s), 8.88 (1H, s), 11.21 (1H, s), 11.99 (1H, s). Elemental analysis: for C₂₀H₂₀N₃O₃Cl
Calculated: C, 62.26; H, 5.22; N, 10.89.
Found: C, 62.14; H, 5.23; N, 10.80.

### Example 119

ethyl 3-(4-{[(6-chloroimidazo[1,2-a]pyridin-2-yl)amino]carbonyl}phenyl)-3-methylbutanoate

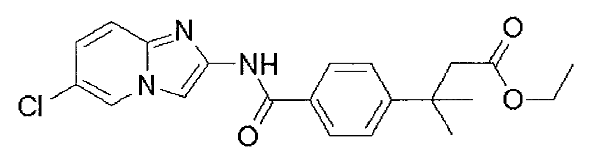

According to Example 49 and using 6-chloroimidazo[1,2-a]pyridin-2-amine obtained in Reference Example 21 and ethyl 3-[4-(chlorocarbonyl)phenyl]-3-methylbutanoate from obtained 4-(3-ethoxy-1,1-dimethyl-3-oxopropyl)benzoic acid obtained in Reference Example 5, the title compound was synthesized.
MS (ESI+): 365 (M+).
¹H-NMR (DMSO-d₆) δ: 1.02 (3H, t, J = 7.2 Hz), 1.46 (6H, s), 2.70 (2H, s), 3.90 (2H, q, J = 7.2 Hz), 7.28 (1H, dd, J = 9.5, 1.9 Hz), 7.48-7.53 (3H, m), 8.01 (2H, d, J = 8.7 Hz), 8.33 (1H, s), 8.88 (1H, s), 11.21 (1H, s).

### Example 120

ethyl 4-(4-{[(6-chloroimidazo[1,2-a]pyridin-2-yl)amino]carbonyl}phenyl)-4-methylpentanoate

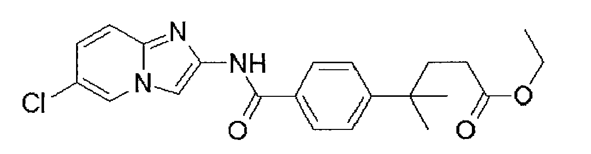

According to Example 49 and using 6-chloroimidazo[1,2-a]pyridin-2-amine obtained in Reference Example 21 and ethyl 4-[4-(chlorocarbonyl)phenyl]-4-methylpentanoate from obtained 4-(4-ethoxy-1,1-dimethyl-4-oxobutyl)benzoic acid obtained in Reference Example 6, the title compound was synthesized.
MS (ESI+): 399 (M+H).
¹H-NMR (CDCl₃) δ: 1.21 (3H, t, J = 7.2 Hz), 1.35 (6H, s), 1.97-2.13 (4H, m), 4.06 (2H, q, J = 7.2 Hz), 7.11 (1H, d, J = 9.5 Hz), 7.21 (1H, d, J= 9.5 Hz), 7.44 (2H, d, J = 8.2 Hz), 7.90 (2H, d, J = 8.2 Hz), 8.17 (1H, s), 8.24 (1H, s), 9.42 (1H, s).

### Example 121

N-(6-chloroimidazo[1,2-a]pyridin-2-yl)-4-[1-methyl-1-(5-oxo-4,5-dihydro-1,2,4-oxadiazol-3-yl)ethyl]benzamide

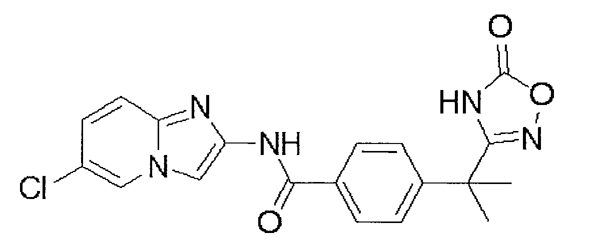

Hydroxylamine (2.0 mmol, 139 mg) and sodium hydrogen carbonate (2.4 mmol, 201 mg) was suspended in DMSO (3 mL), and the suspension was stirred at 40°C for 10 min. Then, N-(6-chloroimidazo[1,2-a]pyridin-2-yl)-4-(1-cyano-1-methylethyl)benzamide (0.2 mmol, 68 mg) obtained in Example 93 was added, and the mixture was stirred at 60°C overnight. Water was added, and the mixture was extracted with ethyl acetate. The extract was dried (MgSO₄), and the solvent was evaporated under reduced pressure. The residue (60 mg) were dissolved in THF (10 mL), N,N'-carbonyldiimidazole (0.3 mmol, 49 mg) and 1,8-diazabicyclo[5.4.0]undec-7-ene (0.3 mmol, 46 mg) were added, and the mixture was stirred for 3 hr. Water was added, and the mixture was extracted with ethyl acetate. The extract was dried (MgSO₄), and the solvent was evaporated under reduced pressure. The residue was recrystallized from DMSO-water to give the title compound (13 mg, 16%).
melting point 310-312°C (decomp.)
MS (ESI+): 397 (M).
¹H-NMR (DMSO-d₆) δ: 1.63 (6H, s), 7.26-7.32 (1H, m), 7.43-7.54 (3H, m), 8.07 (2H, d, J = 8.5 Hz), 8.33 (1H, s), 8.87-8.90 (1H, m), 11.31 (1H, s), 12.29 (1H, m).

### Example 122

4-[2-(acetylamino)-1,1-dimethylethyl]-N-(6-chloroimidazo[1,2-a]pyridin-2-yl)benzamide

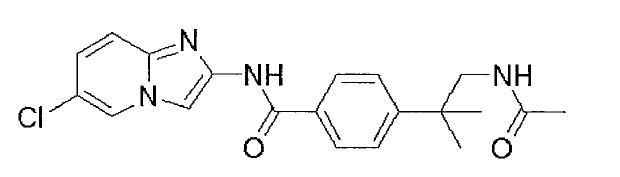

4-(2-Amino-1,1-dimethylethyl)-N-(6-chloroimidazo[1,2-a]pyridin-2-yl)benzamide (0.1 mmol, 34 mg) obtained in Example 94 and triethylamine (0.2 mmol, 20 mg) were dissolved in THF (20 mL), acetyl chloride (0.2 mmol, 16 mg) was added, and the mixture was stirred for 2 hr. Volatile components were evaporated under reduced pressure, and the residue was purified by silica gel chromatography (ethyl acetate:methanol=10:0 - 8:2), and recrystallized from methanol-ethyl acetate to give the title compound (24 mg, 63%). melting point 220-222°C
MS (ESI+): 384 (M).
¹H-NMR (DMSO-d₆) δ: 1.27 (6H, s), 1.78 (3H, s), 3.27-3.33 (2H, m), 7.29 (1H, d, J = 9.4, 2.1 Hz), 7.46-7.54 (3H, m), 7.62-7.69 (1H, m), 8.03 (2H, d, J = 8.5 Hz), 8.33 (1H, s), 8.88 (1H, d, J = 2.1 Hz), 11.22 (1H, s).

### Example 123

4-(aminosulfonyl)-N-(6-chloroimidazo[1,2-a]pyridin-2-yl)benzamide

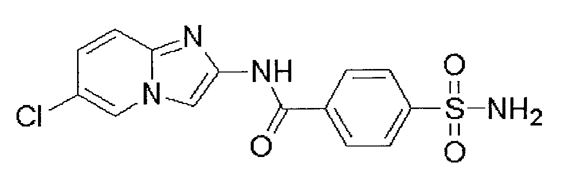

According to Example 49 and using 4-(aminosulfonyl)benzoyl chloride from obtained 6-chloroimidazo[1,2-a]pyridin-2-amine obtained in Reference Example 21 and 4-carboxybenzenesulfonamide, the title compound was synthesized.
melting point >320°C
¹H-NMR (DMSO-d₆) δ: 7.30 (1H, dd, J = 9.4, 2.1 Hz), 7.49-7.57 (3H, m), 7.94 (2H, d, J = 8.3 Hz), 8.20 (2H, d, J = 8.3 Hz), 8.30 (1H, s), 8.90 (1H, d, J = 2.1 Hz), 11.52 (1H, s).

### Example 124

4-[(acetylamino)sulfonyl]-N-(6-chloroimidazo[1,2-a]pyridin-2-yl)benzamide

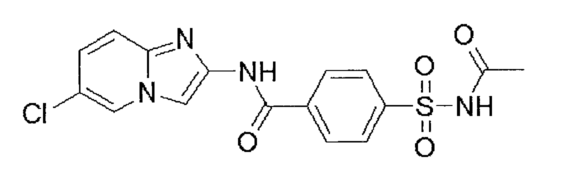

4-(Aminosulfonyl)-N-(6-chloroimidazo[1,2-a]pyridin-2-yl)benzamide (0.23 mmol, 80 mg) obtained in Example 123 was dissolved in pyridine (10 mL), acetic anhydride (0.5 mL) was added, and the mixture was stirred overnight at 80°C. Volatile components were evaporated under reduced pressure, hydrochloric acid was added, and the mixture was extracted with ethyl acetate. The extract was dried (MgSO₄), and the solvent was evaporated under reduced pressure. The residue was recrystallized from DMSO-water to give the title compound (10 mg, 11%).
MS (ESI+): 392 (M+).
¹H-NMR (DMSO-d₆) δ: 1.93 (3H, t, J = 7.8 Hz), 7.30 (1H, dd, J = 9.5, 2.1 Hz), 7.52 (1H, d, J = 9.5 Hz), 8.01 (2H, d, J = 8.5 Hz), 8.22 (2H, d, J = 8.5 Hz), 8.36 (1H, s), 8.90 (1H, d, J = 1.5 Hz), 11.56 (1H, s), 12.25 (1H, s).

### Example 125

N-[6-(1H-imidazol-1-yl)imidazo[1,2-a]pyridin-2-yl]-4-(methylsulfonyl)benzamide

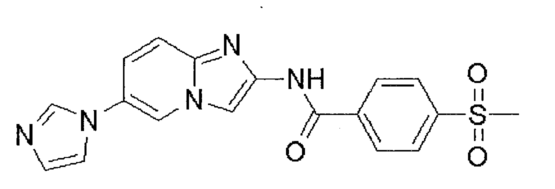

According to Example 49 and using 6-iodoimidazo[1,2-a]pyridin-2-amine (2.59 g, 10.0 mmol) obtained in Reference Example 23 and 4-(methylsulfonyl)benzoyl chloride (2.0 g, 10.0 mmol), N-(6-iodoimidazo[1,2-a]pyridin-2-yl)-4-(methylsulfonyl)benzamide (2.4 g, 54%) was synthesized. According to Example 51 and using the compound (1.0 mmol, 440 mg), the title compound (130 mg, 34%) was synthesized.
melting point 305-308°C (decomp.)
¹H-NMR (DMSO-d₆) δ: 3.31 (3H, s), 7.16 (1H, s), 7.57-7.68 (2H, m), 7.71 (1H, s), 8.07 (2H, d, J = 8.5 Hz), 8.20 (1H, s), 8.30 (2H, d, J = 8.5 Hz), 8.38 (1H, s), 9.09 (1H, s), 11.64 (1H, s).

### Example 126

N-(6-chloroimidazo[1,2-a]pyridin-2-yl)-4-[1,1-dimethyl-2-(propionylamino)ethyl]benzamide

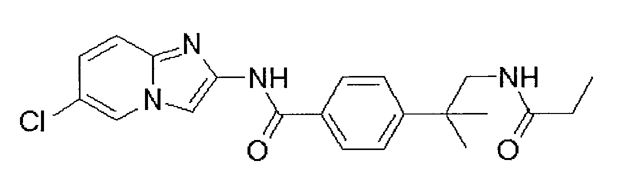

According to Example 122 and using propanoic acid anhydride, the title compound was synthesized.
melting point 225-227°C
MS (ESI+): 399 (M+H).
¹H-NMR (DMSO-d₆) δ: 0.93 (3H, t, J = 7.8 Hz), 1.26 (6H, s), 1.97-2.09 (2H, m), 3.30 (2H, d, J = 6.4 Hz), 7.29 (1H, dd, J = 9.5, 1.9 Hz), 7.47-7.53 (3H, m), 7.58 (1H, t, J = 6.3 Hz), 8.03 (2H, d, J = 8.7 Hz), 8.33 (1H, s), 8.88 (1H, d, J = 1.9 Hz), 11.22 (1H, s).

### Example 127

N-(6-chloroimidazo[1,2-a]pyridin-2-yl)-4-[2-(ethylamino)-1,1-dimethyl-2-oxoethyl]benzamide

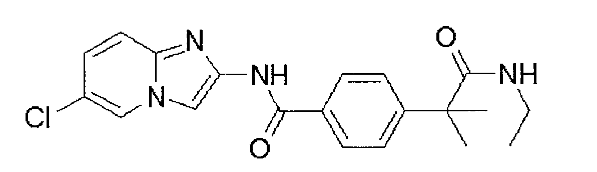

2-(4-{[(6-Chloroimidazo[1,2-a]pyridin-2-yl)amino]carbonyl}phenyl)-2-methylpropanoic acid (0.3 mmol, 100 mg) synthesized in Example 116 was suspended in dichloromethane (30 mL), oxalyl dichloride (1 mmol, 127 mg) and DMF (0.1 mL) were added, and the mixture was stirred for 1 hr. Volatile components were evaporated under reduced pressure, the residue was added to a solution of 2N ethylamine-containing THF solution (3 mmol, 1.5 mL) and triethylamine (1.2 mmol, 121 mg) in THF (30 mL), and the mixture was stirred overnight. Volatile components were evaporated under reduced pressure, and the residue was purified by silica gel chromatography (ethyl acetate:methanol=10:0 - 8:2), and recrystallized from methanol-ethyl acetate to give the title compound (70 mg, 61%).
melting point 228-230°C
MS (ESI+): 385 (M+H).
¹H-NMR (DMSO-d₆) δ: 0.97 (3H, t, J = 7.2 Hz), 1.47 (6H, s), 3.00-3.12 (2H, m), 7.29 (1H, dd, J = 9.5, 1.9 Hz), 7.39-7.53 (4H, m), 8.03 (2H, d, J = 8.3 Hz), 8.33 (1H, s), 8.88 (1H, d, J = 1.9 Hz), 11.23 (1H, s).

### Example 128

N-(6-chloroimidazo[1,2-a]pyridin-2-yl)-4-[1,1-dimethyl-3-(methylamino)-3-oxopropyl]benzamide

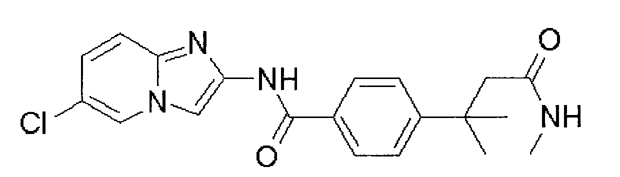

According to Example 127 and using 3-(4-{[(6-chloroimidazo[1,2-a]pyridin-2-yl)amino]carbonyl}phenyl)-3-methylbutanoic acid obtained in Example 117, the title compound was obtained.
melting point 228-230°C
MS (ESI+): 385 (M+H).
¹H-NMR (DMSO-d₆) δ: 1.38 (6H, s), 2.41 (2H, s), 2.47 (3H, s), 7.28 (1H, dd, J = 9.7, 2.1 Hz), 7.46-7.53 (3H, m), 7.61-7.69 (1H, m), 8.01 (2H, d, J = 8.3 Hz), 8.33 (1H, s), 8.88 (1H, s), 11.20 (1H, s).

### Example 129

N-(6-chloroimidazo[1,2-a]pyridin-2-yl)-4-[1-methyl-1-(propionylamino)ethyl]benzamide

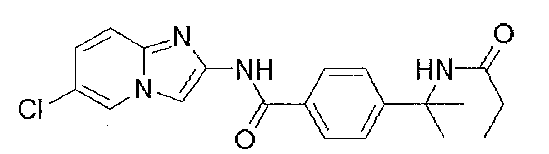

According to Example 126 and using 4-(1-amino-1-methylethyl)-N-(6-chloroimidazo[1,2-a]pyridin-2-yl)benzamide obtained in Example 92, the title compound was obtained.
melting point 249-252°C
MS (ESI+): 385 (M+H).
¹H-NMR (DMSO-d₆) δ: 0.96 (3H, t, J = 7.6 Hz), 1.55 (6H, s), 2.13 (2H, q, J = 7.6 Hz), 7.28 (1H, dd, J = 9.5, 2.3 Hz), 7.42 (2H, d, J = 8.5 Hz), 7.50 (1H, d, J = 9.5 Hz), 7.98 (2H, d, J = 8.5 Hz), 8.07 (1H, s), 8.33 (1H, s), 8.87 (1H, d, J = 1.5 Hz), 11.19 (1H, s).

### Example 130

4-(1-cyano-1-methylethyl)-N-[6-(1H-imidazol-1-yl)imidazo[1,2-a]pyridin-2-yl]benzamide

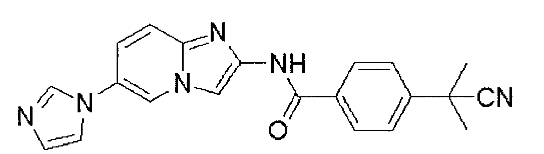

According to Examples 49 and 51 and using 4-(1-cyano-1-methylethyl)benzoyl chloride from obtained 4-(1-cyano-1-methylethyl)benzoic acid obtained in Reference Example 2, the title compound was synthesized.
melting point 241-244°C
MS (ESI+): 371 (M+H).
¹H-NMR (DMSO-d₆) δ: 1.74 (6H, s), 7.14-7.16 (1H, m), 7.56-7.63 (2H, m), 7.65-7.71 (3H, m), 8.14 (2H, d, J = 8.7 Hz), 8.19-8.20 (1H, m), 8.35 (1H, s), 9.06-9.09 (1H, m), 11.38 (1H, s).

### Example 131

N-(6-chloroimidazo[1,2-a]pyridin-2-yl)-4-[ethyl(methylsulfonyl)amino]benzamide

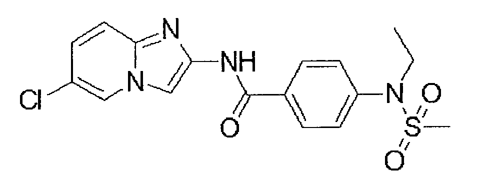

According to Example 49 and using 6-chloroimidazo[1,2-a]pyridin-2-amine obtained in Reference Example 21 and 4-[ethyl(methylsulfonyl)amino]benzoyl chloride obtained from 4-[ethyl(methylsulfonyl)amino]benzoic acid obtained in Reference Example 53, the title compound was synthesized.
melting point 245-247°C
MS (ESI+): 392 (M).
¹H-NMR (DMSO-d₆) δ: 1.01 (3H, t, J = 7.2 Hz), 3.03 (3H, s), 3.75 (2H, q, J = 7.2 Hz), 7.29 (1H, dd, J = 9.5, 2.3 Hz), 7.49-7.55 (3H, m), 8.12 (2H, d, J = 8.7 Hz), 8.32-8.36 (1H, m), 8.89 (1H, d, J = 1.5 Hz), 11.35 (1H, s).

### Example 132

4-(2-amino-1,1-dimethylethyl)-N-[6-(1H-imidazol-1-yl)imidazo[1,2-a]pyridin-2-yl]benzamide

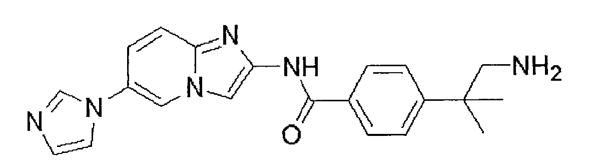

According to Example 94 and using 4-(1-cyano-1-methylethyl)-N-[6-(1H-imidazol-1-yl)imidazo[1,2-a]pyridin-2-yl]benzamide obtained in Example 130, the title compound was synthesized.
melting point 258-260°C
¹H-NMR (DMSO-d₆) δ: 1.26 (6H, s), 2.69 (2H, s), 7.14-7.17 (1H, m), 7.48 (2H, d, J = 8.5 Hz), 7.55-7.65 (2H, m), 7.70 (1H, t, J = 1.3 Hz), 8.04 (2H, d, J = 8.5 Hz), 8.18-8.20 (1H, m), 8.34 (1H, s), 9.05-9.09 (1H, m), 11.22 (1H, s), hidden (2H).

### Example 133

4-{1,1-dimethyl-2-[(methylsulfonyl)amino]ethyl}-N-[6-(1H-imidazol-1-yl)imidazo[1,2-a]pyridin-2-yl]benzamide

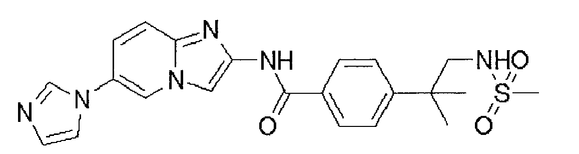

4-(2-Amino-1,1-dimethylethyl)-N-[6-(1H-imidazol-1-yl)imidazo[1,2-a]pyridin-2-yl]benzamide (0.2 mmol, 74 mg) obtained in Example 132 and triethylamine (0.6 mmol, 47 mg) were dissolved in THF (60 mL), methanesulfonyl chloride (0.3 mmol, 34 mg) was added, and the mixture was stirred overnight. Volatile components were evaporated under reduced pressure, and the residue was purified by silica gel chromatography (ethyl acetate:methanol=10:0 - 7:3), and recrystallized from hexane-ethyl acetate to give the title compound (34 mg, 38%).
MS (ESI+): 453 (M+H).
¹H-NMR (DMSO-d₆) δ: 1.31 (6H, s), 2.76 (3H, s), 3.14 (2H, d, J = 6.4 Hz), 6.84-6.90 (1H, m), 7.15 (1H, s), 7.50-7.65 (4H, m), 7.70 (1H, s), 8.06 (2H, d, J = 8.3 Hz), 8.19 (1H, s), 8.35 (1H, s), 9.07 (1H, s), 11.26 (1H, s).

### Example 134

4-[2-(benzoylamino)-1,1-dimethylethyl]-N-[6-(1H-imidazol-1-yl)imidazo[1,2-a]pyridin-2-yl]benzamide

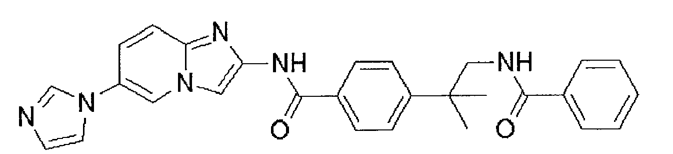

According to Example 133 and using benzoyl chloride, the title compound was synthesized.
MS (ESI+): 479 (M+H).
¹H-NMR (DMSO-d₆) δ: 1.35 (6H, s), 3.51 (2H, d, J = 6.4 Hz), 7.15 (1H, s), 7.40-7.79 (10H, m), 8.06 (2H, d, J = 8.3 Hz), 8.19 (1H, s), 8.28 (1H, t, J = 6.3 Hz), 8.34 (1H, s), 9.07 (1H, s), 11.25 (1H, s).

### Example 135

4-[2-(acetylamino)-1,1-dimethylethyl]-N-[6-(1H-imidazol-1-yl)imidazo[1,2-a]pyridin-2-yl]benzamide

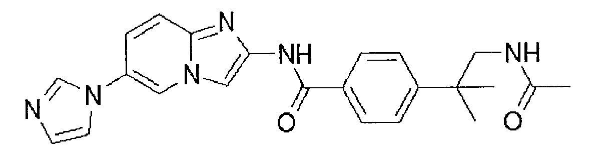

According to Example 133 and using acetyl chloride, the title compound was synthesized.
melting point 242-244°C
MS (ESI+): 417 (M+H).
¹H-NMR (DMSO-d₆) δ: 1.27 (6H, s), 1.78 (3H, s), 3.27-3.31 (2H, m), 7.15 (1H, s), 7.50 (2H, d, J = 8.3 Hz), 7.55-7.68 (3H, m), 7.70 (1H, s), 8.05 (2H, d, J = 8.3 Hz), 8.19 (1H, s), 8.35 (1H, s), 9.07 (1H, s), 11.24 (1H, s).

### Example 136

4-[1,1-dimethyl-2-(propylamino)ethyl]-N-[6-(1H-imidazol-1-yl)imidazo[1,2-a]pyridin-2-yl]benzamide

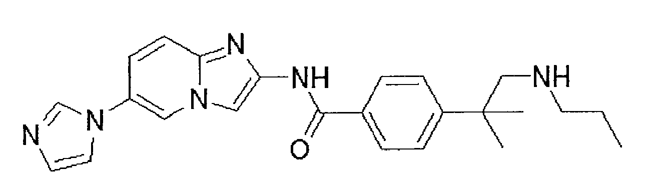

4-(2-Amino-1,1-dimethylethyl)-N-[6-(1H-imidazol-1-yl)imidazo[1,2-a]pyridin-2-yl]benzamide (0.2 mmol, 75 mg) obtained in Example 132, propionaldehyde (0.2 mmol, 12 mg) and acetic acid (0.03 mL) were dissolved in DMF (5 mL)-dichloromethane (5 mL), and the mixture was stirred for 30 min. Then, sodium triacetoxyborohydride (0.8 mmol, 170 mg) was added, and the mixture was stirred overnight. Volatile components were evaporated under reduced pressure, and the residue was purified by silica gel chromatography (ethyl acetate:methanol=10:0 - 8:2), recrystallized from hexane-dichloromethane to give the title compound (30 mg, 36%).
melting point 162-164°C
MS (ESI+): 417 (M+H).
¹H-NMR (DMSO-d₆) δ: 0.78 (3H, t, J = 7.4 Hz), 1.27-1.40 (8H, m), 2.41 (2H, t, J = 7.1 Hz), 2.68 (2H, s), 7.14-7.17 (1H, m), 7.50 (2H, d, J = 8.5 Hz), 7.55-7.65 (2H, m), 7.70 (1H, t, J = 1.3 Hz), 8.03 (2H, d, J = 8.5 Hz), 8.19 (1H, s), 8.34 (1H, s), 9.05-9.08 (1H, m), 11.22 (1H, s), hidden (1H).

### Example 137

4-tert-butyl-N-(2-tert-butyl-diimidazo[1,2-a:4',5'-e]pyridin-7-yl)benzamide

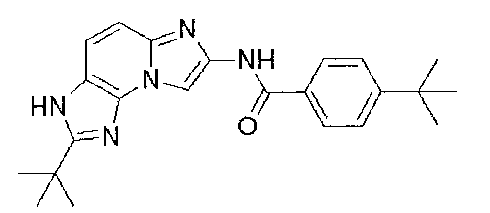

6-Chloro-3-nitropyridin-2-amine (9.8 g, 56.5 mmol) was dissolved in 2,2-dimethylpropanoic anhydride (60 ml), concentrated sulfuric acid (1 mL) was added, and the mixture was stirred at 90°C for 1hr. The mixture was allowed to cool, water (60 mL) was added, and the mixture was stirred for 30 min. The precipitated solid was filtered and washed with a mixed solvent of water-acetonitrile to give N-(6-chloro-3-nitropyridin-2-yl)-2,2-dimethylpropanamide (14 g, 94%). The compound (257 mg, 1 mmol), 4-methylbenzenesulfonamide (342 mg, 2 mmol), cesium carbonate (489 mg, 1.5 mmol), palladium acetate (11 mg, 0.05 mmol) and 9,9-dimethyl-4,5-bis(diphenylphosphino)xanthene (43 mg, 0.075 mmol) were suspended in dioxane (6 mL), and the mixture was stirred at 100°C for 1 hr under nitrogen. Water was added, and the mixture was extracted with dichloromethane. The extract was dried (MgSO₄), and the solvent was evaporated under reduced pressure. The residue was purified by silica gel chromatography (ethyl acetate:hexane=3:7 - 6:4). The obtained crude product (350 mg, a mixture of 2,2-dimethyl-N-(6-{[(4-methylphenyl)sulfonyl]amino}-3-nitropyridin-2-yl)propanamide and 4-methylbenzenesulfonamide) was dissolved in methanol, 10% palladium-carbon powder (150 mg) was added under a hydrogen atmosphere, and the mixture was stirred for 2 hr. 10% palladium-carbon powder was filtered off, and the filtrate was concentrated. Acetic acid (5 mL) was added to the residue, and the mixture was stirred at 60°C for 2 hr. The solvent was evaporated under reduced pressure. The residue was purified by silica gel chromatography (ethyl acetate:hexane=5:5 - 8:2) to give N-(2-tert-butyl-1H-imidazo[4,5-b]pyridin-5-yl)-4-methylbenzenesulfonamide (150 mg). Then, this compound was reacted according to Reference Examples 16 and 17 to give 2-tert-butyl-3H-diimidazo[1,2-a:4',5'-e]pyridin-7-amine, and the compound was subjected to acylation using 4-tert-butylbenzoyl chloride to give the title compound.
MS (ESI+): 390 (M+H).
¹H-NMR (DMSO-d₆) δ: 1.34 (9H, s), 1.42 (9H, s), 7.27 (1H, d, J = 9.4 Hz), 7.46 (1H, d, J = 9.4 Hz), 7.60 (2H, d, J = 8.5 Hz), 7.67 (1H, s), 8.03 (2H, d, J = 8.5 Hz), 10.89 (1H, s), 12.79 (1H, s).

### Example 138

4-tert-butyl-N-(6-chloroimidazo[1,2-b]pyridazin-2-yl)benzamide

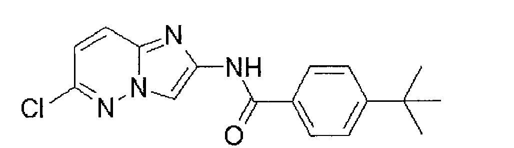

6-Chloroimidazo[1,2-b]pyridazin-2-amine (5.1 g, 30 mmol) obtained in Reference Example 34 was dissolved in N,N-dimethylacetamide (100 ml), and 4-tert-butylbenzoyl chloride (6.5 g, 33 mmol) was added. The mixture was stirred at room temperature, and after 30 min, the precipitation was generated. Then, the mixture was further stirred for 30 min and water (100 ml) was added to the reaction mixture. The precipitate was collected by filtration, and washed successively with water (100 ml), acetonitrile (30 ml, twice) and diethyl ether (50 ml) to give a green-white fluorescence title compound (yield 9.6 g, yield 96%).
¹H-NMR (DMSO-d₆) δ: 1.32 (9H, s), 7.37 (1H, d, J = 9.4 Hz), 7.55(2H, d, J = 8.5 Hz), 8.04 (2H, d, J = 8.5 Hz), 8.12 (1H, d, J = 9.4 Hz), 8.50 (1H, s), 11.40 (1H, s).

### Example 139

4-tert-butyl-N-(6-iodoimidazo[1,2-b]pyridazin-2-yl)benzamide

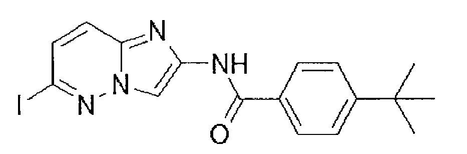

In the same manner as in Example 138 and using 6-iodoimidazo[1,2-b]pyridazin-2-amine (3.6 g, 14 mmol) obtained in Reference Example 36, the title compound (yield 5.4 g, yield 93%) was obtained.
¹H-NMR (DMSO-d₆) δ: 1.32 (9H, s), 7.53 (1H, d, J = 9.2 Hz), 7.54(2H, d, J = 8.5 Hz), 7.79 (1H, d, J = 9.2 Hz), 8.03 (2H, d, J = 8.5 Hz), 8.49 (1H, s), 11.36 (1H, s).

### Example 140

4-tert-butyl-N-[6-(1H-imidazol-1-yl)imidazo[1,2-b]pyridazin-2-yl]benzamide

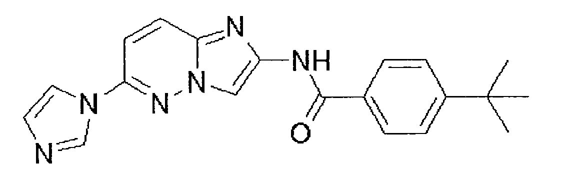

4-tert-Butyl-N-(6-chloroimidazo[1,2-b]pyridazin-2-yl)benzamide (400 mg, 1.2 mol) obtained in Example 138, imidazole (331 mg,4.9 mmol) and potassium carbonate (673 mg,4.9 mmol) were suspended in N,N-dimethylformamide (10 ml), and the mixture was stirred while applying microwave at 180°C for 20 min. After cooling the reaction mixture, water (50 ml) was added, and the precipitation was collected by filtration, and washed with acetonitrile. The precipitate was purified by basic silica gel column chromatography (1-5% methanol/ethyl acetate) to give the title compound (yield 280 mg, 64%).
¹H-NMR (DMSO-d₆) δ: 1.33 (9H, s), 7.21 (1H, s), 7.55 (2H, d, J = 8.5 Hz), 7.80 (1H, d, J = 9.6 Hz), 8.00 (1H, t, J = 1.3 Hz), 8.05 (2H, d, J = 8.5 Hz), 8.27 (1H, d, J = 9.6 Hz), 8.48 (1H, s), 8.59 (1H, s), 11.40 (1H, s).

### Example 141

4-tert-butyl-N-[6-(1H-imidazol-1-yl)imidazo[1,2-b]pyridazin-2-yl]benzamide dihydrochloride

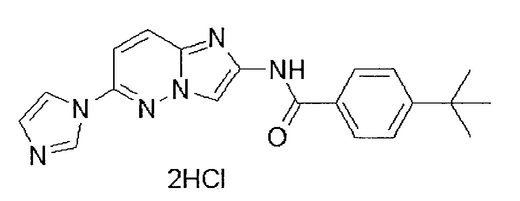

4-tert-Butyl-N-[6-(1H-imidazol-1-yl)imidazo[1,2-b]pyridazin-2-yl]benzamide (200 mg, 0.555 mmol) obtained in Example 140 was suspended in methanol (5 ml), and 10% hydrochloric acid gas-containing methanol solution (2 ml) was added to give a solution. The solution was decolored with activated carbon, and the filtrate was concentrated to dryness under reduced pressure. The residue was dissolved in 10% hydrochloric acid methanol solution (2 ml), and diethyl ether (4 ml) was added. The precipitated yellow white powder was collected by filtration, washed with diethyl ether and dried to give the title compound (yield 347 mg, yield 80%).
melting point 298-300°C
MS (ESI+): 361 (M+H).
¹H-NMR (DMSO-d₆) δ: 1.33 (9H, s), 7.56 (2H, d, J = 8.3 Hz), 7.98 (1H, d, J = 9.6 Hz),7.99 (1H, s), 8.05 (2H, d, J = 8.3 Hz), 8.46 (1H, d, J = 9.6 Hz), 8.50 (1H, t, J = 1.5 Hz), 8.58 (1H, s), 10.12 (1H, s), 11.53 (1H, s).
Elemental analysis: for C₂₀H₂₀N₆O·2HCl·0.8H₂O
Calculated: C, 53.65; H, 5.31; N, 18.77; Cl, 15.84.
Found: C, 53.61; H, 5.31; N, 18.75; Cl, 15.63.

### Example 142

4-tert-butyl-N-{6-[4-(cyanomethyl)-1H-imidazol-1-yl]imidazo[1,2-b]pyridazin-2-yl}benzamide

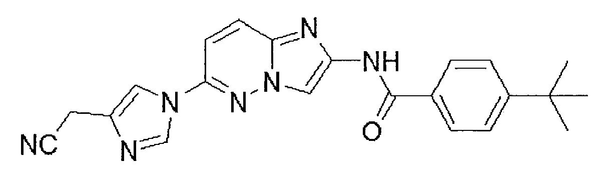

In the same manner as in Example 140 and using 4-tert-butyl-N-(6-chloroimidazo[1,2-b]pyridazin-2-yl)benzamide (500 mg, 1.5 mmol), 1H-imidazol-4-ylacetonitrile (552 mg, 5.2 mmol) and potassium carbonate (841 mg, 6.1 mmol), the title compound (yield 340 mg, yield 62%) was obtained.
melting point 314-316°C (decomp.)
MS (ESI+): 400 (M+H).
¹H-NMR (DMSO-d₆) δ: 1.33 (9H, s), 4.00 (2H, s), 7.55 (2H, d, J = 8.7 Hz), 7.81 (1H, d, J = 9.6 Hz), 7.94 (1H, d, J = 1.3 Hz), 8.05 (2H, d, J = 8.7 Hz), 8.27 (1H, d, J = 9.6 Hz), 8.49 (1H, s), 8.61 (1H, d, J = 1.3 Hz), 11.4 (1H, s).
Elemental analysis: for C₂₂H₂₁N₇O
Calculated: C, 66.15; H, 5.30; N, 24.55.
Found: C, 65.62; H, 5.32; N, 24.27.

### Example 143

4-tert-butyl-N-[6-(2,4-dimethyl-1H-imidazol-1-yl)imidazo[1,2-b]pyridazin-2-yl]benzamide

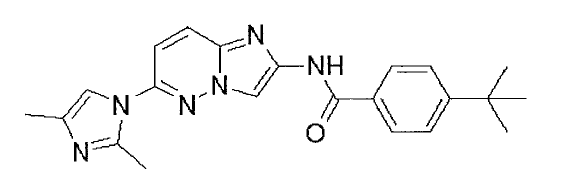

In the same manner as in Example 140, 4-tert-butyl-N-(6-chloroimidazo[1,2-b]pyridazin-2-yl)benzamide (500 mg, 1.5 mmol), 2,4-dimethyl-1H-imidazole (731 mg, 7.6 mmol) and potassium carbonate (841 mg, 6.1 mmol) were stirred at 200°C for 40 min to give the title compound (yield 210 mg, 36%).
melting point 254-255°C (acetonitrile)
MS (ESI+): 389 (M+H).
¹H-NMR (DMSO-d₆) δ: 1.33 (9H, s), 2.13 (3H, d, J = 1.1 Hz), 2.49 (3H, s), 7.32 (1H, d, J = 1.1 Hz), 7.48 (1H, d, J = 9.4 Hz), 7.55 (2H, d, J = 8.7 Hz), 8.05 (2H, d, J = 8.7 Hz), 8.21 (1H, d, J = 9.4 Hz), 8.50 (1H, s), 11.40 (1H, s).
Elemental analysis: for C₂₂H₂₄N₆O
Calculated: C, 68.02; H, 6.23; N, 21.63.
Found: C, 67.77; H, 6.19; N, 21.67.

### Example 144

4-tert-butyl-N-[6-(2-ethyl-4-methyl-1H-imidazol-1-yl)imidazo[1,2-b]pyridazin-2-yl]benzamide

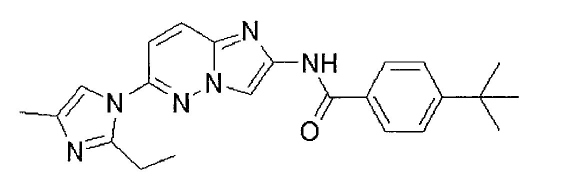

In the same manner as in Example 140, 4-tert-butyl-N-(6-chloroimidazo[1,2-b]pyridazin-2-yl)benzamide (500 mg, 1.5 mmol), 2-ethyl-4-methyl-1H-imidazole (670 mg,6.1 mmol) and potassium carbonate (631 mg, 4.6 mmol) were stirred at 200°C for 60 min to give the title compound (yield 242 mg, 40%).
melting point 220-221°C (acetonitrile)
MS (ESI+): 403 (M+H).
¹H-NMR (DMSO-d₆) δ: 1.21 (3H, t, J = 7.4 Hz), 1.33 (9H, s), 2.15 (3H, s), 2.86 (2H, q, J = 7.4 Hz), 7.29 (1H, d, J = 0.9 Hz), 7.46 (1H, d, J = 9.4 Hz), 7.55 (2H, d, J = 8.5 Hz), 8.05 (2H, d, J = 8.5 Hz), 8.21 (1H, d, J = 9.4 Hz), 8.50 (1H, s), 11.41 (1H, s).
Elemental analysis: for C₂₃H₂₅N₆O
Calculated: C, 68.63; H, 6.51; N, 20.88.
Found: C, 68.64; H, 6.50; N, 20.93.

### Example 145

4-tert-butyl-N-{6-[4-(hydroxymethyl)-1H-imidazol-1-yl]imidazo[1,2-b]pyridazin-2-yl}benzamide

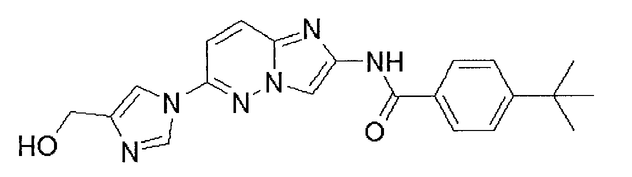

4-tert-Butyl-N-(6-chloroimidazo[1,2-b]pyridazin-2-yl)benzamide (500 mg, 1.5 mmol), 1H-imidazol-4-ylmethanol (614 mg,4.6 mmol), sodium hydride (60%/ mineral oil,243 mg,6.1 mmol) and tetrabutylammonium iodide (281 mg, 0.76 mmol) were suspended in N-methylpyrrolidinone (10 ml), and the suspension was stirred at 110°C for 12 hr. After cooling, water (20 ml) was added, and the mixture was extracted with a mixed solvent (50 ml) of 50% ethyl acetate/tetrahydrofuran. The organic layer was washed with saturated brine, dried over anhydrous magnesium sulfate, filtered and concentrated under reduced pressure. Diethyl ether was added to the obtained crude product, and the precipitated orange solid was collected by filtration, and recrystallized from methanol to give a pale-yellow title compound (yield 88 mg, 15%).
melting point 298-301°C (methanol)
MS (ESI+): 391 (M+H).
¹H-NMR (DMSO-d₆) δ: 1.33 (9H, s), 4.45 (2H, d, J = 5.7 Hz), 5.10 (1H, t, J = 5.7 Hz), 7.55 (2H, d, J = 8.5 Hz), 7.79 (1H, d, J = 9.6 Hz), 7.81 (1H, s), 8.04 (2H, d, J = 8.5 Hz), 8.25 (1H, d, J = 9.6 Hz), 8.52 (1H, d, J = 1.1 Hz), 11.38 (1H, s). Elemental analysis: for C₂₁H₂₂N₆O₂
Calculated: C, 64.60; H, 5.68; N, 21.52.
Found: C, 63.58; H, 5.69; N, 21.24.

### Example 146

4-tert-butyl-N-[6-(4,5-dichloro-1H-imidazol--1-yl)imidazo[1,2-b]pyridazin-2-yl]benzamide

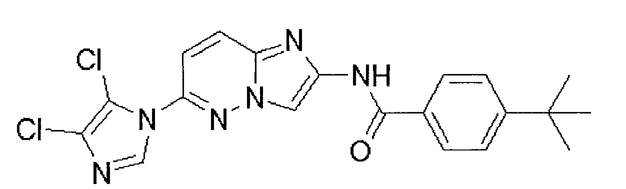

In the same manner as in Example 143, 4-tert-butyl-N-(6-chloroimidazo[1,2-b]pyridazin-2-yl)benzamide (500 mg, 1.5 mmol), 4,5-dichloro-1H-imidazole (625 mg, 4.6 mmol) and potassium carbonate (631 mg, 4.6 mmol) are reacted, purified by basic silica gel chromatography (50% methylene chloride/ethyl acetate), and recrystallized from acetonitrile to give the title compound (yield 133 mg, 20%).
melting point 281-282°C (acetonitrile)
MS (ESI+): 429 (M+H).
¹H-NMR (DMSO-d₆) δ: 1.33 (9H, s), 7.56 (2H, d, J = 8.5 Hz), 7.62 (2H, d, J = 9.4 Hz), 8.05 (2H, d, J = 8.5 Hz), 8.36 (1H, dd, J = 9.4, 0.6 Hz), 8.37 (1H, s), 8.59 (1H, d, J = 0.6 Hz), 11.50 (1H, s).
Elemental analysis: for C₂₀H₁₈Cl₂N₆O
Calculated: C, 55.95; H, 4.23; N, 19.58.
Found: C, 56.09; H, 4.26; N, 19.66.

### Example 147

N-[6-(4-bromo-1H-imidazol-1-yl)imidazo[1,2-b]pyridazin-2-yl]-4-tert-butylbenzamide

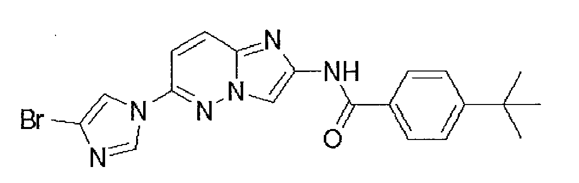

In the same manner as in Example 140, 4-tert-butyl-N-(6-chloroimidazo[1,2-b]pyridazin-2-yl)benzamide (500 mg, 1.5 mmol), 4-bromo-1H-imidazole (670 mg,4.6 mmol) and potassium carbonate (840 mg, 6.1 mmol) were reacted, purified by basic silica gel chromatography (100% THF) and recrystallized from tetrahydrofuran to give a white title compound (yield 180 mg, 27%).
melting point 306-307°C (tetrahydrofuran)
MS (ESI+): 441 (M+H).
¹H-NMR (DMSO-d₆) δ: 1.33 (9H, s), 7.55 (2H, d, J = 8.5 Hz), 7.78 (1H, d, J = 9.6 Hz), 8.04 (2H, d, J = 8.5 Hz), 8.18 (1H, d, J = 1.3 Hz), 8.30 (1H, d, J = 9.6 Hz), 8.48 (1H, s), 8.59 (1H, d, J = 1.3 Hz), 11.42 (1H, s).
Elemental analysis: for C₂₀H₁₉N₆OBr
Calculated: C, 54.68; H, 4.36; N, 19.13; Br, 18.19.
Found: C, 54.58; H, 4.30; N, 19.18; Br, 18.06.

### Example 148

N-(6-aminoimidazo[1,2-b]pyridazin-2-yl)-4-tert-butylbenzamide

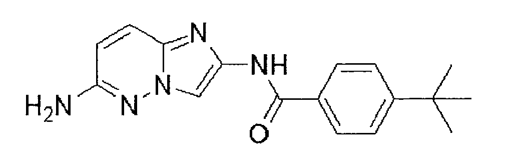

4-tert-Butyl-N-(6-chloroimidazo[1,2-b]pyridazin-2-yl)benzamide (1.0 g, 3.0 mmol) obtained in Example 138 and sodium azide (590 mg,9.1 mmol) was suspended in N,N-dimethylformamide (30 ml), and the mixture was stirred at 110°C for 48 hr. The solvent was evaporated under reduced pressure. The obtained brown crude product was dissolved in a mixed solvent (100 ml) of 50% tetrahydrofuran/ethyl acetate, washed with water and saturated brine, dried over anhydrous magnesium sulfate, and filtered. The solvent was evaporated under reduced pressure, and the obtained brown solid was purified by basic silica gel chromatography (100% tetrahydrofuran) to give N-(6-azidoimidazo[1,2-b]pyridazin-2-yl)-4-tert-butylbenzamide as a mixture (830 mg) with the reaction starting material.
The mixture (480 mg) was dissolved in tetrahydrofuran (20 ml) 10% activated palladium carbon (400 mg) was added, and the and the mixture was stirred at room temperature for 30 min under a hydrogen atmosphere. The reaction mixture was filtered, and the filtrate was concentrated under reduced pressure. The obtained yellow solid was purified by basic silica gel chromatography (80% ethyl acetate/hexane-100% ethyl acetate) to give a white title compound (yield 270 mg, 49%).
melting point 267-268°C (acetonitrile)
MS (ESI+): 310 (M+H).
¹H-NMR (DMSO-d₆) δ: 1.32 (9H, s), 6.29 (2H, s), 6.62 (1H, d, J = 9.6 Hz), 7.52 (2H, d, J = 8.5 Hz), 7.61 (1H, d, J = 9.6 Hz), 7.99 (1H, s), 8.00 (2H, d, J = 8.5 Hz), 11.01 (1H, s). Elemental analysis: for C₁₇H₁₉N₅O
Calculated: C, 66.00; H, 6.19; N, 22.64.
Found: C, 65.95; H, 6.16; N, 22.69.

### Example 149

N-(6-aminoimidazo[1,2-b]pyridazin-2-yl)-4-tert-butylbenzamide hydrochloride

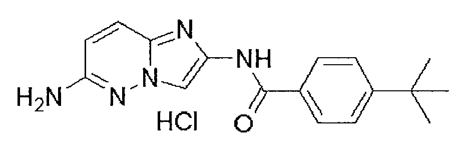

To N-(6-aminoimidazo[1,2-b]pyridazin-2-yl)-4-tert-butylbenzamide (110 mg, 0.36 mmol) obtained in Example 148 was added tetrahydrofuran (5 ml), and then 1.5N hydrochloric acid methanol solution (10 ml) was added to dissolve the compound completely. The solvent was evaporated under reduced pressure to give a white solid. The solid was azeotroped with methanol several times. The white solid was recrystallized by dissolving the solid in ethanol, and adding hexane to give the title compound (yield 41 mg, 33%).
melting point 312-313°C (ethanol/hexane)
MS (ESI+): 310 (M+H).
¹H-NMR (DMSO-d₆) δ: 1.33 (9H, s), 3.65-5.24 (3H, brs), 6.94 (1H, d, J = 9.6 Hz), 7.58(2H, d, J = 8.5 Hz), 7.86 (1H, d, J = 9.6 Hz), 7.97 (1H, s), 8.04 (2H, d, J = 8.5 Hz), 11.69 (1H, s).
Elemental analysis: for C₁₇H₁₉N₅O·HCl
Calculated: C, 59.04; H, 5.83; N, 20.25.
Found: C, 58.78; H, 5.85; N, 20.09.

### Example 150

4-tert-butyl-N-(6-pyrrolidin-1-ylimidazo[1,2-b]pyridazin-2-yl)benzamide hydrochloride·monohydrate

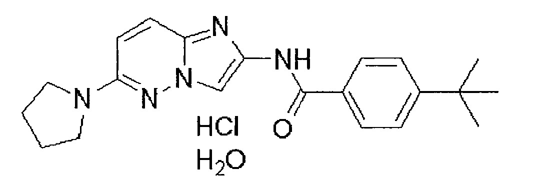

In the same manner as in Example 140, 4-tert-butyl-N-(6-chloroimidazo[1,2-b]pyridazin-2-yl)benzamide (400 mg, 1.2 mmol), pyrrolidine (865 mg, 12.2 mol) and potassium carbonate (504 mg, 3.7 mmol) were reacted. Water (60 ml) was added, and the precipitated yellow solid was collected by filtration, and washed with 30%acetonitrile/diethyl ether. The solid was suspended in methanol (10 ml), 10% hydrochloric acid methanol solution (4 ml) was added to give a complete solution. Then, methanol was evaporated under reduced pressure, and the residue was azeotroped with a mixed solvent of methanol/toluene to give a white solid. The solid was recrystallization from acetonitrile to give the title compound (yield 210 mg, 43%) as a white needle.
melting point 257-259°C (acetonitrile)
MS (ESI+): 364 (M+H).
¹H-NMR (DMSO-d₆) δ: 1.33 (9H, s), 1.92-2.03 (4H, m), 3.41-3.51 (4H, m), 7.18 (1H, d, J = 10.0 Hz), 7.59 (2H, d, J = 8.5 Hz), 7.99 (1H, d, J = 10.0 Hz), 8.07 (1H, s), 8.09 (2H, d, J = 8.5 Hz), 12.03 (1H, s).
Elemental analysis: for C₂₁H₂₆N₅O·H₂O·HCl
Calculated: C, 60.35; H, 6.75; N, 16.76; Cl, 8.48.
Found: C, 60.38; H, 6.72; N, 16.87; Cl, 8.40.

### Example 151

N-(6-acetylimidazo[1,2-b]pyridazin-2-yl)-4-tert-butylbenzamide

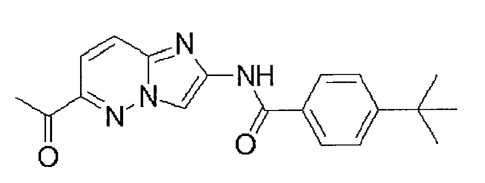

4-tert-Butyl-N-(6-chloroimidazo[1,2-b]pyridazin-2-yl)benzamide (13 g, 39.5 mmol) obtained in Example 138, tributyl(1-ethoxyvinyl)tin (15.7 g, 43.5 mmol) and bis(triphenylphosphine)palladium(II) chloride (1.4 g, 2.0 mmol) were dissolved in N,N-dimethylformamide (130 ml), and the mixture was stirred at 100°C for 18 hr. After the reaction, potassium fluoride (5 g, 86.3 mmol) was dissolved in water (150 ml), and the obtained solution was added. The resulting solid was collected by filtration, was washed successively with acetonitrile and diethyl ether to give a green solid. The solid was dissolved in a mixed solvent of methanol/methylene chloride (100 ml/200 ml), 3N hydrochloric acid (100 ml) was added, and the mixture was stirred at room temperature for 5 hr. The solvent was evaporated under reduced pressure, and the residue was weakly alkalified with saturated aqueous sodium hydrogen carbonate solution, and extracted with methylene chloride. The organic layer was washed with saturated brine, dried over anhydrous magnesium sulfate, and filtered, and the solvent was evaporated under reduced pressure to give a green solid. The solid was purified by silica gel chromatography (50% ethyl acetate/methylene chloride), and the obtained green solid was washed with diethyl ether to give the title compound (yield 9.7 g, 72%) as a pale-yellow powder.
melting point 237-238°C
MS (ESI+): 337 (M+H).
¹H-NMR (DMSO-d₆) δ: 1.33 (9H, s), 2.69 (3H, s), 7.55 (2H, d, J = 8.5 Hz), 7.70 (1H, d, J = 9.4 Hz), 8.06 (2H, d, J = 8.5 Hz), 8.14 (1H, d, J = 9.4 Hz), 8.58 (1H, s), 11.49 (1H, s). Elemental analysis: for C₁₉H₂₀N₄O₂
Calculated: C, 67.48; H, 5.99; N, 16.66.
Found: C, 67.74; H, 5.98; N, 16.64.

### Example 152

4-tert-butyl-N-(6-glycoloylimidazo[1,2-b]pyridazin-2-yl)benzamide

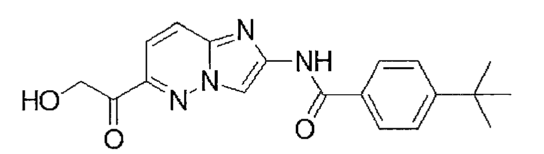

N-(6-Acetylimidazo[1,2-b]pyridazin-2-yl)-4-tert-butylbenzamide (3.2 g, 9.6 mmol) obtained in Example 151 was dissolved in 50% methanol/methylene chloride (100 ml), sodium methoxide (28% methanol solution,1.3 g, 24 mmol) and iodosobenzene (3.2 g, 14 mmol) were successively added, and the mixture was stirred at room temperature for 10 min. Saturated aqueous sodium hydrogen carbonate solution (50 ml) was added to the reaction solution, and the solvent was evaporated under reduced pressure. The obtained residue was washed with water, acetonitrile and diethyl ether to give a yellow white solid (2.9 g). The solid (2.9 g) was added to acetone (150 ml), and then p-toluenesulfonic acid monohydrate (2.8 g, 15 mmol) and water (45 ml) was added, and the mixture was heated at 80°C for 6 hr. After cooling the reaction solution, acetone was evaporated under reduced pressure. Saturated aqueous sodium hydrogen carbonate solution (50 ml) was added to the residue, and the mixture was extracted with 50% tetrahydrofuran/ethyl acetate. The organic layer was washed with saturated brine, dried over anhydrous magnesium sulfate, and filtered, and the solvent was evaporated under reduced pressure. The obtained orange crude product was washed with ethyl acetate to give the title compound (yield 1.9 g, 56%) as a yellow powder.
MS (ESI+): 353 (M+H).
¹H-NMR (DMSO-d₆) δ: 1.33 (9H, s), 4.99 (2H, d, J = 6.0 Hz), 5.26 (1H, t, J = 6.0 Hz), 7.55 (2H, d, J = 8.5 Hz), 7.69 (1H, d, J = 9.4 Hz), 8.01 (2H, d, J = 8.5 Hz), 8.16 (1H, d, J = 9.4 Hz), 8.56 (1H, s), 11.50 (1H, s).

### Example 153

4-tert-butyl-N-[6-(1H-imidazol-5-yl)imidazo[1,2-b]pyridazin-2-yl]benzamide

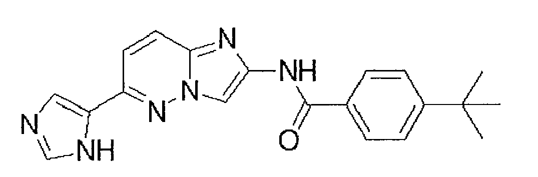

4-tert-Butyl-N-(6-glycoloylimidazo[1,2-b]pyridazin-2-yl)benzamide (160 mg, 0.45 mmol) obtained in Example 152, copper(II) diacetate (820 mg,4.5 mmol) and formaldehyde (37% aqueous solution,74 mg, 0.91 mmol) was added to a mixed solvent of N,N-dimethylformamide/28% aqueous ammonia solution (8 ml/24 ml), and the mixture was stirred at 100°C for 30 min. After the reaction solvent was cooled, water (10 ml) was added, and the precipitated yellow brown solid was collected by filtration, and washed successively with acetonitrile and diethyl ether. The yellow brown solid was added to ethanol (50 ml), and hydrogen sulfide was blown for 10 min while heating at 80°C. The precipitated copper sulfide was filtered off on hot, and the filtrate was concentrated under reduced pressure to give a yellow crude solid. The solid was purified by basic silica gel chromatography (50% ethyl acetate/methylene chloride -10% methanol/methylene chloride) to give a yellow title compound (yield 90 mg, 66%).
MS (ESI+): 361 (M+H).
¹H-NMR (DMSO-d₆) δ:1.33 (9H, s), 7.54 (2H, d, J = 8.5 Hz), 7.80 (1H, d, J = 9.4 Hz), 7.85(1H s), 7.88 (1H, s), 8.00 (1H, d, J = 9.4 Hz), 8.05 (2H, d, J = 8.5 Hz), 8.39 (1H, s), 11.29 (1H, s).

### Example 154

4-tert-butyl-N-[6-(1H-imidazol-5-yl)imidazo[1,2-b]pyridazin-2-yl]benzamide hydrochloride

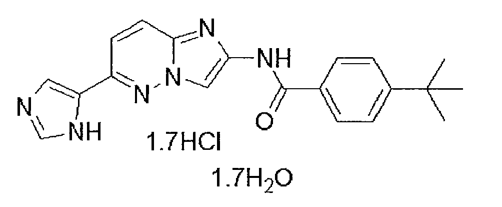

In the same manner as in Example 150, the hydrochloride was prepared from 4-tert-butyl-N-[6-(1H-imidazol-5-yl)imidazo[1,2-b]pyridazin-2-yl]benzamide (90 mg, 0.25 mmol) obtained in Example 153 and recrystallized from chloroform.
MS (ESI+): 361 (M+H).
¹H-NMR (DMSO-d₆) δ: 1.33 (9H, s), 4.20-4.80 (4H, brs), 7.56 (2H, d, J = 8.5 Hz), 7.86 (1H, d, J = 9.4 Hz), 8.05 (2H, d, J = 8.5 Hz), 8.26 (1H, d, J = 9.4 Hz), 8.53 (1H, s), 8.57 (1H, d, J = 0.8 Hz), 9.33 (1H, d, J = 0.8 Hz), 11.47 (1H, s).
Elemental analysis: for C₂₁H₂₆N₅O·1.7H₂O·1.7HCl
Calculated: C, 53.02; H, 5.58; N, 18.55; Cl, 13.30.
Found: C, 52.50; H, 5.23; N, 18.30; Cl, 13.31.

### Example 155

4-tert-butyl-N-[6-(2-methyl-1H-imidazol-5-yl)imidazo[1,2-b]pyridazin-2-yl]benzamide

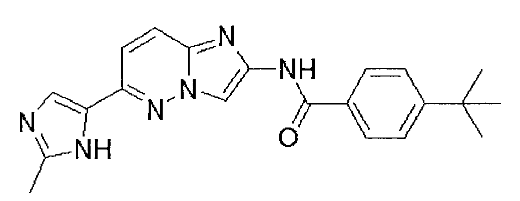

In the same manner as in Example 153 and using 4-tert-butyl-N-(6-glycoloylimidazo[1,2-b]pyridazin-2-yl)benzamide (200 mg, 0.57 mmol), copper(II) diacetate (1000 mg, 5.7 mmol) and acetaldehyde (90% aqueous solution, 56 mg, 1.1 mmol), the title compound (yield 89 mg, 53%) was obtained.
MS (ESI+): 361 (M+H).
¹H-NMR (DMSO-d₆) δ: 1.33 (9H, s), 7.54 (2H, d, J = 8.5 Hz), 7.80 (1H, d, J = 9.4 Hz), 7.85(1H, s), 7.88 (1H, s), 8.00 (1H, d, J = 9.4 Hz), 8.05 (2H, d, J = 8.5 Hz), 8.39 (1H, s), 11.29 (1H, s).

### Example 156

4-tert-butyl-N-[6-(2-methyl-1H-imidazol-5-yl)imidazo[1,2-b]pyridazin-2-yl]benzamide hydrochloride

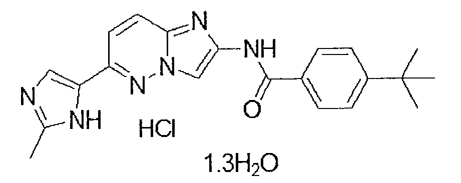

In the same manner as in Example 150, the hydrochloride was prepared from 4-tert-butyl-N-[6-(2-methyl-1H-imidazol-5-yl)imidazo[1,2-b]pyridazin-2-yl]benzamide (87 mg, 0.23 mmol) obtained in Example 155 and recrystallized from methanol/acetonitrile to give the title compound (yield 39 mg, 41%).
melting point 295-297°C (decomp.)
MS (ESI+): 375 (M+H).
¹H-NMR (DMSO-d₆) δ: 1.33 (9H, s), 2.66 (3H, s), 7.55 (2H, d, J = 8.5 Hz), 7.80 (1H, d, J = 9.4 Hz), 8.05 (2H, d, J = 8.5 Hz), 8.24 (1H, d, J = 9.4 Hz), 8.43 (1H, s), 8.52 (1H, s,), 9.33 (1H, d, J = 0.8 Hz), 11.45 (1H, s), 14.52-15.17 (2H, brs).
Elemental analysis: for C₂₁H₂₂N₅O·1.3H₂O·HCl
Calculated: C, 58.07; H, 5.94; N, 19.35; Cl, 8.16.
Found: C, 57.44; H, 5.34; N, 19.10; Cl, 8.57.

### Example 157

4-tert-butyl-N-[6-(2-isopropyl-1H-imidazol-5-yl)imidazo[1,2-b]pyridazin-2-yl]benzamide

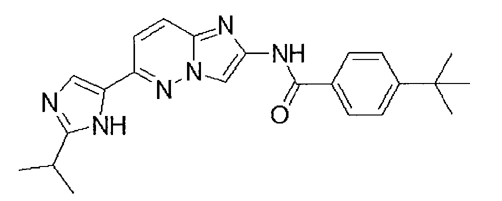

In the same manner as in Example 153 and using 4-tert-butyl-N-(6-glycoloylimidazo[1,2-b]pyridazin-2-yl)benzamide (200 mg, 0.57 mmol), copper diacetate (II) (1000 mg, 5.7 mmol) and 2-methylpropanal (82 mg, 1.1 mmol), the title compound (yield 100 mg, 56%) was obtained.
MS (ESI+): 403 (M+H).
¹H-NMR (DMSO-d₆) δ: 1.25-1.37 (15H, m), 3.04 (1H, sept, J = 7.0 Hz), 7.54 (2H, d, J = 8.5 Hz), 7.72-7.80 (2H, m), 7.96(1H, d, J = 9.5 Hz),8.05 (2H, d, J = 8.5 Hz), 8.38 (0.7H, s), 8.41 (0.3H, s), 11.28 (0.7H, s), 11.32 (0.3H, s),12.20 (0.7H, s), 12.50 (0.3H, s).

### Example 158

4-tert-butyl-N-[6-(2-isopropyl-1H-imidazol-5-yl)imidazo[1,2-b]pyridazin-2-yl]benzamide hydrochloride

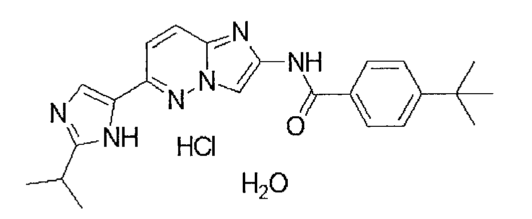

In the same manner as in Example 156, hydrochloride was prepared from 4-tert-butyl-N-[6-(2-isopropyl-1H-imidazol-5-yl)imidazo[1,2-b]pyridazin-2-yl]benzamide (100 mg, 0.27 mmol) obtained in Example 157 to give the title compound (yield 40 mg, 36%).
melting point 304-305°C (decom.)
MS (ESI+): 361 (M+H).
¹H-NMR (DMSO-d₆) δ: 1.33 (9H, s), 1.42 (6H, d, J = 7.0 Hz), 3.42 (1H, m, J = 7.0 Hz), 7.55 (2H, d, J = 8.5 Hz), 7.84 (1H, d, J = 9.4 Hz), 8.05 (2H, d, J = 8.5 Hz), 8.23 (1H, d, J = 9.4 Hz), 8.46 (1H, s), 8.53 (1H, s), 11.45 (1H, s), 14.83(1.5H, brs).
Elemental analysis: for C₂₃H₂₆N₆O·H₂O·HCl
Calculated: C, 60.45; H, 6.40; N, 18.39; Cl, 7.76.
Found: C, 60.33; H, 6.23; N, 18.42; Cl, 7.42.

### Example 159

4-tert-butyl-N-{6-[(pyridin-4-ylmethyl)amino]imidazo[1,2-b]pyridazin-2-yl}benzamide

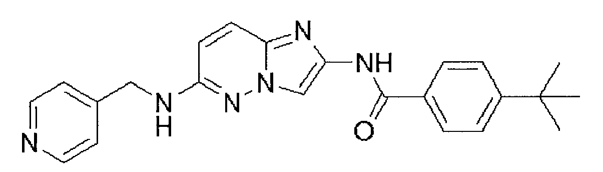

4-tert-Butyl-N-(6-iodoimidazo[1,2-b]pyridazin-2-yl)benzamide (110 mg, 0.26 mmol) obtained in Example 139, copper iodide(I) (25.4 mg, 0.13 mmol), proline (15.4 mg, 0.13 mmol), 1-pyridin-4-ylmethanamine (86.7 mg, 0.79 mmol) and potassium carbonate (148 mg, 1.0 mmol) were added to dimethylsulfoxide (2.0 ml), and the mixture was stirred at 80°C for 2.5 hr under a nitrogen atmosphere. Then, the reaction mixture was diluted with 50% tetrahydrofuran/ethyl acetate (50 ml), washed three times with 28% aqueous ammonia solution (5 ml) and washed with saturated brine. The organic layer was dried over anhydrous magnesium sulfate. After filtration, the solvent was evaporated under reduced pressure to give a brown crude product. The product was purified by basic silica gel chromatography (90-100% ethyl acetate/hexane) and then silica gel chromatography (0-5% methanol/ethyl acetate) to give a pale-yellow title compound (yield 44 mg, 42%).
melting point 222-223°C (acetonitrile)
MS (ESI+): 401 (M+H).
¹H-NMR (DMSO-d₆) δ: 1.31 (9H, s), 4.49 (2H, d, J = 6.0 Hz), 6.74 (1H, d, J = 9.6 Hz), 7.39 (2H, d, J = 5.8 Hz), 7.51 (2H, d, J = 8.5 Hz), 7.55 (1H, d, J = 6.0 Hz), 7.65 (1H, d, J = 9.6 Hz), 7.97 (1H, s), 7.99 (2H, d, J = 8.5 Hz), 8.52 (2H, d, J = 5.8 Hz), 10.99 (1H, s).
Elemental analysis: for C₂₃H₂₄N₆O
Calculated: C, 68.98; H, 6.04; N, 20.99.
Found: C, 68.56; H, 5.88; N, 20.79.

### Example 160

4-tert-butyl-N-{6-[(pyridin-3-ylmethyl)amino]imidazo[1,2-b]pyridazin-2-yl}benzamide

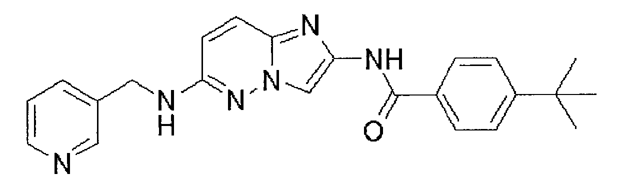

4-tert-Butyl-N-(6-iodoimidazo[1,2-b]pyridazin-2-yl)benzamide (200 mg, 0.48 mmol) obtained in Example 139, copper iodide(I) (46.2 mg, 0.24 mmol), proline (28.0 mg, 0.24 mmol), 1-pyridin-3-ylmethanamine (157.5 mg, 1.43 mmol) and potassium carbonate (268 mg, 1.9 mmol) were added to dimethylsulfoxide (5.0 ml), and the mixture was stirred at 80°C for 3.0 hr under a nitrogen atmosphere. Then, the reaction mixture was diluted with 50% tetrahydrofuran/ethyl acetate (100 ml), washed three times with 28% aqueous ammonia solution (10 ml) and washed with saturated brine. The organic layer was dried over anhydrous magnesium sulfate. After filtration, the solvent was evaporated under reduced pressure to give brown crude product. The product was purified by basic silica gel chromatography (90-100% ethyl acetate/hexane) and then silica gel chromatography (0-5% methanol/ethyl acetate) to give a pale-yellow title compound (yield 100 mg, 53%).
melting point 232-233°C (acetonitrile)
MS (ESI+): 401 (M+H).
¹H-NMR (DMSO-d₆) δ: 1.31 (9H, s), 4.47 (2H, d, J = 5.7 Hz), 6.70 (1H, d, J = 9.6 Hz), 7.38 (1H, dd, J = 7.7, 4.7 Hz), 7.46 (1H, t, J = 5.7 Hz), 7.51 (2H, d, J = 8.5 Hz), 7.63 (1H, d, J = 9.6 Hz), 7.83 (1H, ddd, J = 7.7, 7.4, 1.7 Hz), 8.00 (2H, d, J = 8.5 Hz), 8.02 (1H, s), 8.48 (1H, dd, J = 4.7, 1.3 Hz), 8.64 (1H, d, J = 1.7 Hz), 10.99 (1H, s).
Elemental analysis: for C₂₃H₂₄N₆O
Calculated: C, 68.98; H, 6.04; N, 20.99.
Found: C, 68.56; H, 5.88; N, 20.79.

### Example 161

4-tert-butyl-N-{6-[(pyridin-2-ylmethyl)amino]imidazo[1,2-b]pyridazin-2-yl}benzamide

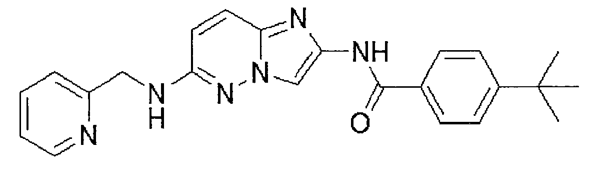

In the same manner as in Example 160 and using 1-pyridin-2-ylmethanamine (157.5 mg, 1.43 mmol), the title compound (yield 123 mg, 65%) was obtained.
melting point 196-197°C (acetonitrile)
MS (ESI+): 401 (M+H).
¹H-NMR (DMSO-d₆) δ: 1.31 (9H, s), 4.55 (2H, d, J = 5.7 Hz), 6.81 (1H, d, J = 9.6 Hz), 7.28 (1H, dd, J = 7.5, 4.9 Hz), 7.44 (1H, d, J = 7.9 Hz), 7.47-7.54 (1H, m), 7.51 (2H, d, J = 8.5 Hz), 7.63 (1H, d, J = 9.6 Hz), 7.77 (1H, td, J = 7.7, 1.7 Hz), 7.97 (1H, s), 7.99 (2H, d, J = 8.5 Hz), 8.55 (1H, m), 10.89 (1H, s).
Elemental analysis: for C₂₃H₂₄N₆O
Calculated: C, 68.98; H, 6.04; N, 20.99.
Found: C, 68.84; H, 5.99; N, 20.97.

### Example 162

4-tert-butyl-N-{6-[(2-pyridin-4-ylethyl)amino]imidazo[1,2-b]pyridazin-2-yl}benzamide

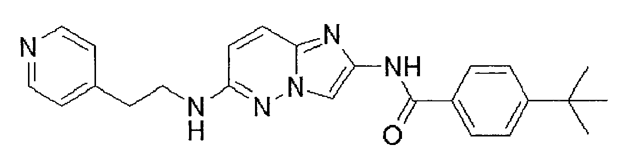

In the same manner as in Example 160 and using 2-pyridin-4-ylethaneamine (177.9 mg, 1.43 mmol), the title compound (yield 135 mg, 68%) was obtained.
melting point 279°C (acetonitrile)
MS (ESI+): 415 (M+H).
¹H-NMR (DMSO-d₆) δ: 1.32 (9H, s), 2.94 (2H, t, J = 7.0 Hz), 3.30 (2H, td, J = 7.0, 5.5 Hz), 6.62 (1H, d, J = 9.6 Hz), 7.01 (1H, t, J = 5.5 Hz), 7.32 (2H, d, J = 5.8 Hz), 7.52 (2H, d, J = 8.5 Hz), 7.59 (1H, d, J = 9.6 Hz), 8.00 (2H, d, J = 8.5 Hz), 8.05 (1H, s), 8.48 (2H, d, J = 5.8 Hz), 10.99 (1H, s). Elemental analysis: for C₂₄H₂₆N₆O
Calculated: C, 69.54; H, 6.32; N, 20.27.
Found: C, 69.46; H, 6.26; N, 20.24.

### Example 163

4-tert-butyl-N-{6-[(2-pyridin-3-ylethyl)amino]imidazo[1,2-b]pyridazin-2-yl}benzamide

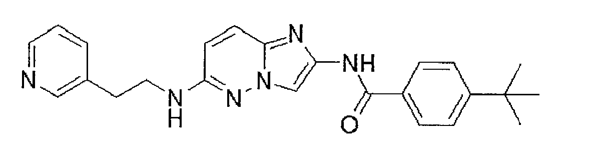

In the same manner as in Example 160 and using 2-pyridin-3-ylethaneamine (177.9 mg, 1.43 mmol), the title compound (yield 138 mg, 70%) was obtained.
melting point 231°C (acetonitrile)
MS (ESI+): 415 (M+H).
¹H-NMR (DMSO-d₆) δ: 1.32 (9H, s), 2.93 (2H, t, J = 7.0 Hz), 3.49 (2H, td, J = 7.0, 5.5 Hz), 6.63 (1H, d, J = 9.6 Hz), 7.01 (1H, t, J = 5.5 Hz), 7.34 (1H, dd, J = 7.7, 4.7 Hz), 7.51 (2H, d, J = 8.5 Hz), 7.59 (1H, d, J = 9.6 Hz), 7.72 (1H, ddd, J = 7.7, 2.1, 1.5 Hz), 8.00 (2H, d, J = 8.5 Hz), 8.04 (1H, s), 8.43 (1H, dd, J = 4.7, 1.5 Hz), 8.51 (1H, d, J = 2.1 Hz), 10.99 (1H, s).
Elemental analysis: for C₂₄H₂₆N₆O
Calculated: C, 69.54; H, 6.32; N, 20.27.
Found: C, 69.45; H, 6.28; N, 20.37.

### Example 164

4-tert-butyl-N-[6-(cyclohexylamino)imidazo[1,2-b]pyridazin-2-yl]benzamide

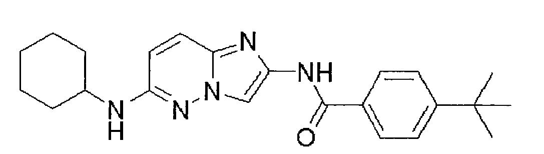

In the same manner as in Example 160, the reaction was performed using cyclohexylamine (144.5 mg, 1.43 mmol), the reaction mixture was poured into 28% aqueous ammonia solution (25 ml), was extracted three times with 50% tetrahydrofuran/ethyl acetate (40 ml). The organic layers were combined, was washed with saturated brine, and dried over anhydrous magnesium sulfate. After filtration, the solvent was evaporated under reduced pressure to give crude product. The product was purified by basic silica gel chromatography (50-75% ethyl acetate/hexane) to give the title compound (yield 110 mg, 59%).
melting point 296-297°C (acetonitrile)
MS (ESI+): 392 (M+H).
¹H-NMR (DMSO-d₆) δ: 1.14-1.46 (5H, m), 1.32 (9H, s), 1.54-1.67 (1H, m), 1.67-1.81 (2H, m), 1.93-2.05 (2H, m), 3.53-3.73 (1H, m), 6.63 (1H, d, J = 9.6 Hz), 6.71 (1H, d, J = 7.5 Hz), 7.51 (2H, d, J = 8.5 Hz), 7.56 (1H, d, J = 9.6 Hz), 7.98 (1H, s), 7.99 (2H, d, J = 8.5 Hz), 10.95 (1H, s).
Elemental analysis: for C₂₃H₂₉N₅O
Calculated: C, 70.56; H, 7.47; N, 17.89.
Found: C, 70.49; H, 7.44; N, 18.01.

### Example 165

4-tert-butyl-N-[6-(cyclopentylamino)imidazo[1,2-b]pyridazin-2-yl]benzamide

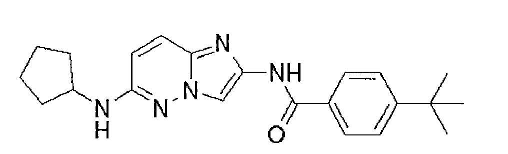

In the same manner as in Example 164 and using cyclopentylamine (124 mg, 1.43 mmol), the reaction was performed to give a crude product. This was purified by basic silica gel chromatography (50% ethyl acetate/hexane-100% ethyl acetate) to give the title compound (yield 120 mg, 66.8%). melting point 272-273°C (acetonitrile)
MS (ESI+): 392 (M+H).
¹H-NMR (DMSO-d₆) δ: 1.32 (9H, s), 1.40-1.78 (6H, m), 1.90-2.06 (2H, m), 4.03 (1H, m, J = 6.4 Hz), 6.61 (1H, d, J = 9.6 Hz), 6.85 (1H, d, J = 6.4 Hz), 7.51 (2H, d, J = 8.5 Hz), 7.56 (1H, d, J = 9.6 Hz), 8.00 (1H, s), 8.00 (2H, d, J= 8.5 Hz), 10.95 (1H, s).
Elemental analysis: for C₂₂H₂₇N₅O
Calculated: C, 70.00; H, 7.21; N, 18.55.
Found: C, 70.03; H, 7.19; N, 18.65.

### Example 166

4-tert-butyl-N-[6-(cyclopropylamino)imidazo[1,2-b]pyridazin-2-yl]benzamide

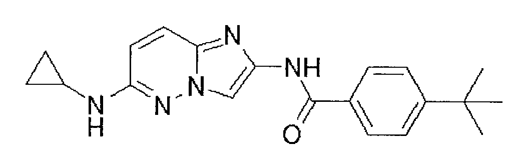

In the same manner as in Example 164 and using cyclopropylamine (138.6 mg, 2.38 mmol), the title compound (yield 124 mg, 74.6%) was obtained.
melting point 274-275°C (acetonitrile)
MS (ESI+): 350 (M+H).
¹H-NMR (DMSO-d₆) δ: 0.43-0.51 (2H, m), 0.74 (2H, td, J = 6.8, 4.7 Hz), 1.32 (9H, s), 2.57-2.67 (1H, m), 6.63 (1H, d, J = 9.6 Hz), 7.15 (1H, d, J = 2.8 Hz), 7.52 (2H, d, J = 8.5 Hz), 7.61 (1H, d, J = 9.6 Hz), 8.00 (2H, d, J = 8.5 Hz), 8.05 (1H, s), 10.99 (1H, s).
Elemental analysis: for C₂₀H₂₃N₅O
Calculated: C, 68.74; H, 6.63; N, 20.04.
Found: C, 68.59; H, 6.62; N, 20.18.

### Example 167

4-tert-butyl-N-[6-(propylamino)imidazo[1,2-b]pyridazin-2-yl]benzamide

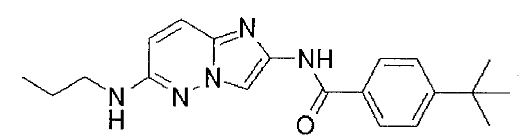

In the same manner as in Example 164 and using propylamine (143.5 mg, 2.38 mmol), the title compound (yield 120 mg, 71.8%) was obtained.
melting point 243-244°C (acetonitrile)
MS (ESI+): 352 (M+H).
¹H-NMR (DMSO-d₆) δ: 0.96 (3H, t, J = 7.4 Hz), 1.32 (9H, s), 1. 61 (2H, qt, J = 7. 4, 6. 8 Hz), 3.18 (2H, td, J = 6. 8, 5. 3 Hz), 6.64 (1H, d, J = 9.6 Hz), 6.85 (1H, t, J = 5.3 Hz), 7.51 (2H, d, J = 8.5 Hz), 7.57 (1H, d, J = 9.6 Hz), 8.00 (1H, s), 8.00 (2H, d, J = 8.5 Hz), 10.96 (1H, s).
Elemental analysis: for C₂₀H₂₅N₅O
Calculated: C, 68.35; H, 7.17; N, 19.93.
Found: C, 68.39; H, 7.14; N, 20.10.

### Example 168

4-tert-butyl-N-{6-[(1-methylpiperidin-4-yl)amino]imidazo[1,2-b]pyridazin-2-yl}benzamide

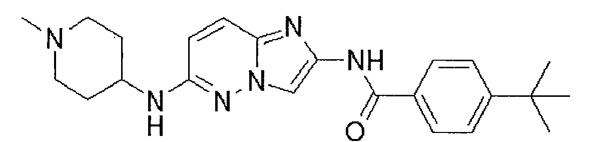

In the same manner as in Example 164 and using 1-methylpiperidin-4-ylamine (271.3 mg, 1.43 mmol), the title compound (yield 118 mg, 61.0%) was obtained.

melting point 275-277°C (acetonitrile)
MS (ESI+): 407 (M+H).
¹H-NMR (DMSO-d₆) δ: 1.32 (9H, s), 1.37-1.55 (2H, m), 1.89-2.11 (4H, m), 2.18 (3H, s), 2.67-2.79 (2H, m),3.50-3.66 (1H, m), 6.62 (1H, d, J = 9.6 Hz), 6.76 (1H, d, J = 7.4 Hz), 7.51 (2H, d, J = 8.5 Hz), 7.57 (1H, d, J = 9.6 Hz), 7.99 (1H, s), 7.99 (2H, d, J = 8.5 Hz), 10.96 (1H, s).
Elemental analysis: for C₂₃H₃₀N₆O
Calculated: C, 67.95; H, 7.44; N, 20.67.
Found: C, 67.25; H, 7.46; N, 20.50.

### Example 169

4-tert-butyl-N-{6-[(2-hydroxyethyl)amino]imidazo[1,2-b]pyridazin-2-yl}benzamide

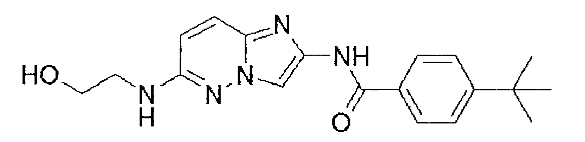

In the same manner as in Example 164 and using 2-aminoethanol (89.0 mg, 1.43 mmol), the crude product was obtained. This was purified by basic silica gel chromatography (80% ethyl acetate/hexane-3% methanol/ethyl acetate) to give the title compound (yield 146 mg, 86.8%).
melting point 231-233°C (acetonitrile)
MS (ESI+): 354 (M+H).
¹H-NMR (DMSO-d₆) δ: 1.32 (9H, s), 3.31 (2H, q, J = 5.7 Hz), 3.61 (2H, q, J = 5.7 Hz), 4.76 (1H, t, J = 5.5 Hz), 6.69 (1H, d, J = 9.6 Hz), 6.90 (1H, t, J = 5.5 Hz), 7.51 (2H, d, J = 8.5 Hz), 7.57 (1H, d, J = 9.6 Hz), 8.00 (2H, d, J = 8.5 Hz), 8.00 (1H, s), 10.97 (1H, s).
Elemental analysis: for C₁₉H₂₃N₅O₂
Calculated: C, 64.57; H, 6.56; N, 19.82.
Found: C, 64.53; H, 6.56; N, 19.96.

### Example 170

4-tert-butyl-N-{6-[(pyrrolidin-3-yl)amino]imidazo[1,2-b]pyridazin-2-yl}benzamide

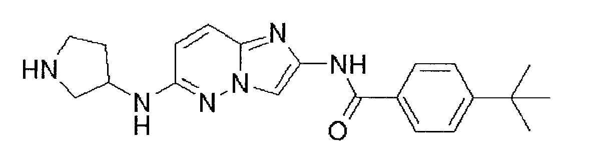

In the same manner as in Example 164 and using tert-butyl 3-aminopyrrolidine-1-carboxylate (271.3 mg, 1.43 mmol), the crude product was obtained. This was purified by basic silica gel chromatography (30-50% ethyl acetate/hexane) to give tert-butyl 3-({2-[(4-tert-butylbenzoyl)aminolimidazo[1,2-b]pyridazin-6-yl}amino)pyrrolidine-1-carboxylate (yield 154 mg). This was stirred for one day in 20% trifluoroacetic acid/dichloromethane (10 ml), and the solvent was evaporated under reduced pressure. The residue was diluted with 10% methanol/ethyl acetate (50 ml), washed with 1N aqueous sodium hydroxide solution (5 ml), washed with saturated brine, dried over anhydrous magnesium sulfate, and filtered. The solvent was evaporated under reduced pressure to give crude product. This was purified by basic silica gel chromatography (100% ethyl acetate-20% methanol/ethyl acetate) to give the title compound (yield 78 mg, 43.3%).
melting point 264-265°C (acetonitrile)
MS (ESI+): 379 (M+H).
¹H-NMR (DMSO-d₆) δ: 1.32 (9H, s), 1.49-1.76 (1H, m), 1.96-2.12 (1H, m), 2.63 (1H, dd, J = 11.3, 4.3 Hz), 2.72-2.95 (2H, m), 3.07 (1H, dd, J = 11.3, 6.4 Hz), 6.62 (1H, d, J = 9.6 Hz), 6.95 (1H, d, J = 6.4 Hz), 7.51 (2H, d, J = 8.5 Hz), 7.57 (1H, d, J = 9.6 Hz), 8.00 (2H, d, J = 8.5 Hz), 8.01 (1H, s), 10.97 (1H, s).
Elemental analysis: for C₂₁H₂₆N₆O·0.5H₂O
Calculated: C, 65.09; H, 7.02; N, 21.69.
Found: C, 65.03; H, 6.95; N, 21.56.

### Example 171

4-tert-butyl-N-[6-(piperidin-4-ylamino)imidazo[1,2-b]pyridazin-2-yl]benzamide

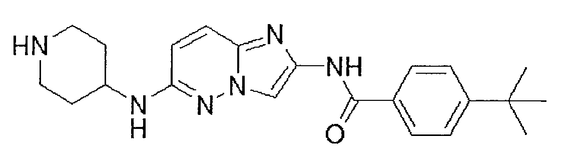

In the same manner as in Example 164 and using tert-butyl 4-aminopiperidine-1-carboxylate (291.8 mg, 1.43 mmol), the reaction was performed to give a crude product. The product was purified by basic silica gel chromatography (30-80% ethyl acetate/hexane) to give tert-butyl 4-({2-[(4-tert-butylbenzoyl)amino]imidazo[1,2-b]pyridazin-6-yl}amino)piperidine-1-carboxylate (yield 210 mg). This was dissolved in 10% concentrated hydrochloric acid/ethanol (6 ml), and the solution was stirred at 70°C for 30 min. After cooling, the reaction mixture was poured into saturated aqueous sodium hydrogen carbonate solution (30 ml), and the mixture was extracted three times with 10% methanol/dichloromethane (40 ml). The organic layers were combined, washed with saturated brine, and dried over anhydrous magnesium sulfate. After filtration, the solvent was evaporated under reduced pressure, and the obtained crude product was purified by basic silica gel chromatography (5-15% methanol/ethyl acetate) to give the title compound (yield 133 mg, 41.3%).
melting point 242-243°C (acetonitrile)
MS (ESI+): 393 (M+H).
¹H-NMR (DMSO-d₆) δ: 1.19-1.37 (2H, m), 1.32 (9H, s), 1.86-1.98 (2H, m), 1.96-2.12 (1H, brs), 2.51-2.62 (2H, m), 2.89-3.01 (2H, m),3.57-3.74 (1H, m), 6.62 (1H, d, J = 9.6 Hz), 6.76 (1H, d, J = 7.4 Hz), 7.51 (2H, d, J = 8.5 Hz), 7.57 (1H, d, J = 9.6 Hz), 7.98 (1H, s), 7.99 (2H, d, J = 8.5 Hz), 10.96 (1H, s). Elemental analysis: for C₂₂H₂₈N₆O
Calculated: C, 67.32; H, 7.19; N, 21.41.
Found: C, 67.38; H, 7.21; N, 21.56.

### Example 172

4-tert-butyl-N-(6-{[2-(methylamino)ethyl]amino}imidazo[1,2-b]pyridazin-2-yl)benzamide

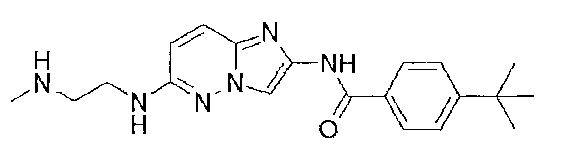

In the same manner as in Example 164 and using tert-butyl (2-aminoethyl)methylcarbamate (253.8 mg, 1.43 mmol), the reaction was performed to give a crude product. The product was purified by basic silica gel chromatography (30-80% ethyl acetate/hexane) to give tert-butyl [2-({2-[(4-tert-butylbenzoyl)amino]imidazo[1,2-b]pyridazin-6-yl}amino)ethyl]methylcarbamate (yield 192 mg). The compound was subjected to deprotection in the same manner as in Example 171, and purified by basic silica gel chromatography (5-15% methanol/ethyl acetate) to give the title compound (yield 108 mg, 59.2%).
melting point 200-201°C (toluene)
MS (ESI+): 367 (M+H).
¹H-NMR (DMSO-d₆) δ: 1.32 (9H, s), 1.81 (1H, brs), 2.32 (3H, s), 2.70 (2H, t, J = 6.2 Hz), 3.29 (2H, td, J = 6.2, 5.3 Hz), 6.67 (1H, d, J = 9.6 Hz), 6.81 (1H, t, J = 5.3 Hz), 7.51 (2H, d, J = 8.5 Hz), 7.57 (1H, d, J = 9.6 Hz), 8.00 (1H, s), 8.00 (2H, d, J = 8.5 Hz), 10.97 (1H, s).
Elemental analysis: for C₂₀H₂₆N₆O·0.2H₂O
Calculated: C, 64.91; H, 7.19; N, 22.71.
Found: C, 64.72; H, 7.14; N, 22.64.

### Example 173

4-tert-butyl-N-[6-(1-methyl-1H-pyrazol-4-yl)imidazo[1,2-b]pyridazin-2-yl]benzamide

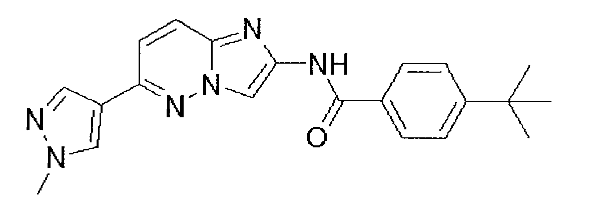

4-tert-Butyl-N-(6-iodoimidazo[1,2-b]pyridazin-2-yl)benzamide (200 mg, 0.48 mmol) obtained in Example 139 was added to a mixed solvent (7.5 ml) of N,N-dimethylformamide/1,2-dimethoxyethane (1/2), and 1-methyl-4-(4,4,5,5-tetramethyl-1,3,2-dioxaborolan-2-yl)-1H-pyrazole (197 mg, 0.93 mmol), dipalladium acetate (II) (9.0 mg, 0.05 mmol), 2M-aqueous sodium carbonate solution (0.46 ml,0.93 mmol) and triphenylphosphine (37.3 mg, 0.14 mmol) were added. The mixture was stirred at 80°C for 1.5 hr under a nitrogen atmosphere. After cooling, the reaction solution was poured into water (10 ml), and the mixture was extracted three times with 50% tetrahydrofuran/ethyl acetate (40 ml). The combined organic layer were washed with saturated brine, and dried over anhydrous magnesium sulfate. After filtration, the solvent was evaporated under reduced pressure to give crude product. This was purified by basic silica gel chromatography (50-70% ethyl acetate/hexane) to give a pale-yellow title compound (yield 168 mg, yield 96.5%).
melting point 245-246°C (acetonitrile)
MS (ESI+): 375 (M+H).
¹H-NMR (DMSO-d₆) δ: 1.33 (9H, s), 3.93 (3H, s), 7.54 (2H, d, J = 8.5 Hz), 7.57 (1H, d, J = 9.4 Hz), 8.01 (1H, d, J = 9.4 Hz), 8.04 (2H, d, J = 8.5 Hz), 8.09 (1H, s), 8.39 (1H, s), 8.44 (1H, s), 11.29 (1H, s).
Elemental analysis: for C₂₁H₂₂N₆O
Calculated: C, 67.36; H, 5.92; N, 22.44.
Found: C, 67.14; H, 5.93; N, 22.43.

### Example 174

4-tert-butyl-N-[6-(1H-pyrazol-4-yl)imidazo[1,2-b]pyridazin-2-yl]benzamide

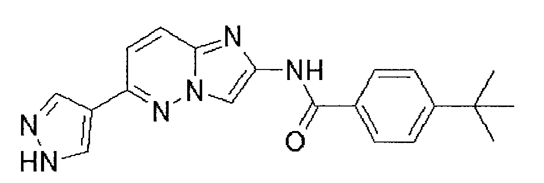

In the same manner as in Example 173 and using 4-(4,4,5,5-tetramethyl-1,3,2-dioxaborolan-2-yl)-1H-pyrazole (184 mg, 0.93 mmol), the reaction was performed to give a crude product. The product was washed with tetrahydrofuran to give a pale-yellow title compound (yield 64 mg, 38%).
melting point 358-362°C (decomposition) (tetrahydrofuran)
MS (ESI+): 361 (M+H).
¹H-NMR (DMSO-d₆) δ: 1.33 (9H, s), 7.54 (2H, d, J = 8.5 Hz), 7.63 (1H, d, J = 9.4 Hz), 8.01 (1H, d, J = 9.4 Hz), 8.04 (2H, d, J = 8.5 Hz), 8.15 (1H, brs), 8.40 (1H, s), 8.48 (1H, brs), 11.78 (1H, s), 13.28 (1H, s).
Elemental analysis: for C₂₀H₂₀N₆O·0.3H₂O
Calculated: C, 65.67; H, 5.68; N, 22.97.
Found: C, 66.00; H, 5.66; N, 22.59.

### Example 175

6-tert-butyl-N-(6-iodoimidazo[1,2-b]pyridazin-2-yl)nicotinamide

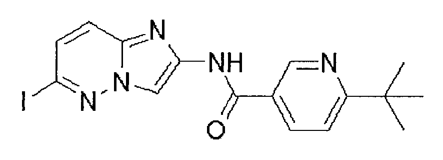

6-tert-Butylnicotinic acid (179 mg, 1.0 mmol) obtained in Reference Example 37 and 6-iodoimidazo[1,2-b]pyridazin-2-amine (200 mg, 0.77 mmol) obtained in Reference Example 36 were dissolved in N,N-dimethylformamide (4 mL), diethyl cyanophosphate (90% solution, 266 mg, 1.15 mmol) and triethylamine (238 mg,2.31 mmol) were added successively thereto at room temperature, and the mixture was stirred overnight. After the reaction, The solvent was evaporated under reduced pressure, and the obtained residue was purified by basic silica gel chromatography (50% ethyl acetate/hexane) to give a white title compound (yield 88 mg, 27%).
¹H-NMR (DMSO-d₆) δ: 1.35 (9H, s), 7.55 (1H, d, J = 9.3 Hz), 7.59 (1H, d, J = 8.3 Hz), 7.81 (1H, d, J = 9.3 Hz), 8.36 (1H, dd, J = 8.3, 2.4 Hz), 8.49 (1H, s), 9.14 (1H, d, J = 2.4 Hz), 11.61 (1H, s).

### Example 176

6-tert-butyl-N-[6-(1-methyl-1H-pyrazol-4-yl)imidazo[1,2-b]pyridazin-2-yl]nicotinamide

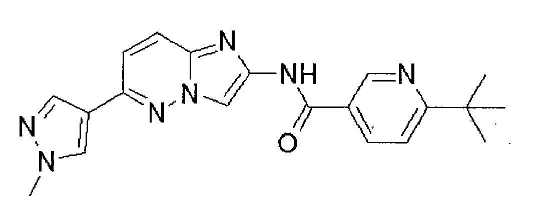

Using 6-tert-butyl-N-(6-iodoimidazo[1,2-b]pyridazin-2-yl)nicotinamide (82 mg, 0.19 mmol) obtained in Example 175 and in the same manner as in Example 173, the title compound (yield 68 mg, 93.3%) was obtained.
melting point 251-252°C (acetonitrile)
MS (ESI+): 376 (M+H).
¹H-NMR (DMSO-d₆) δ: 1.36 (9H, s), 3.93 (3H, s), 7.59 (1H, d, J = 9.6 Hz), 7.59 (1H, d, J = 8.5 Hz), 8.03 (1H, d, J = 9.6 Hz), 8.09 (1H, s), 8.36 (1H, dd, J = 8.5, 2.5 Hz), 8.39 (1H, s), 8.44 (1H, s), 9.15 (1H, d, J = 2.5 Hz), 11.51 (1H, s). Elemental analysis: for C₂₀H₂₁N₇O·H₂O
Calculated: C, 61.05; H, 5.89; N, 24.92.
Found: C, 60.75; H, 5.83; N, 24.84.

### Example 177

4-tert-butyl-N-(6-{1-[2-(tetrahydro-2H-pyran-2-yloxy)ethyl]-1H-pyrazol-4-yl}imidazo[1,2-b]pyridazin-2-yl)benzamide

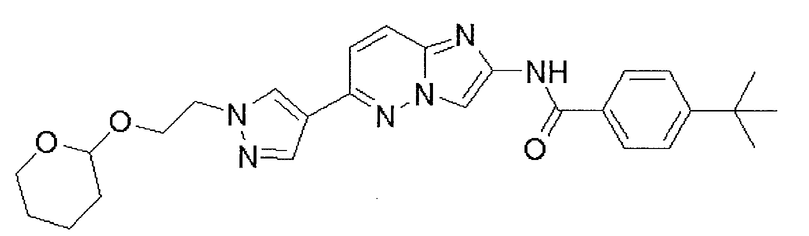

Using 1-[2-(tetrahydro-2H-pyran-2-yloxy)ethyl]-4-(4,4,5,5-tetramethyl-1,3,2-dioxaborolan-2-yl)-1H-pyrazole (125 mg, 0.93 mmol) obtained in Reference Example 38 and in the same manner as in Example 173, the reaction was performed. The obtained crude product was purified by basic silica gel chromatography (50-80% ethyl acetate/hexane) to give a yellow solid. This was washed with diethyl ether to give a pale-yellow title compound (yield 90 mg, 77.4%).
¹H-NMR (DMSO-d₆) δ: 1.33 (9H, s), 1.35-1.50 (4H, m), 1.51-1.75 (2H, m), 3.34-3.44 (1H, m), 3.51-3.64 (1H, m), 3.74-3.86 (1H, m), 3.92-4.06 (1H, m), 4.31-4.45 (2H, m), 4.54-4.61 (1H, m), 7.54 (2H, d, J = 8.3 Hz), 7.59 (1H, d, J = 9.4 Hz), 8.01 (1H, d, J = 9.4 Hz), 8.04 (2H, d, J = 8.3 Hz), 8.13 (1H, s), 8.39 (1H, s), 8.48 (1H, s), 11.28 (1H, s).

### Example 178

4-tert-butyl-N-{6-[1-(2-hydroxyethyl)-1H-pyrazol-4-yl]imidazo[1,2-b]pyridazin-2-yl}benzamide

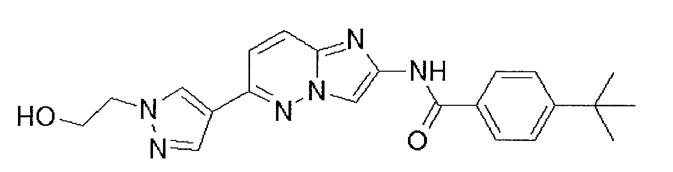

4-tert-Butyl-N-{6-[1-(2-hydroxyethyl)-1H-pyrazol-4-yl]imidazo[1,2-b]pyridazin-2-yl}benzamide (85 mg, 0.17 mmol) obtained in Example 177 was dissolved in 10% concentrated hydrochloric acid/ethanol (3 ml), and the solution was stirred at 70°C for 15 min. After cooling, the reaction mixture was poured into saturated aqueous sodium hydrogen carbonate solution (30 ml), and the mixture was extracted three times with ethyl acetate (30 ml). The organic layers were combined, washed with saturated brine, and dried over anhydrous magnesium sulfate. After filtration, the solvent was evaporated under reduced pressure. The obtained crude product was purified by basic silica gel chromatography (0-5% methanol/ethyl acetate) to give the title compound (yield 63 mg, 89.5%).
melting point 226-229°C (acetonitrile)
MS (ESI+): 405 (M+H).
¹H-NMR (DMSO-d₆) δ: 1.33 (9H, s), 3.79 (2H, td, J = 5.5, 4.7Hz), 4.22 (2H, t, J = 5.5 Hz), 4.98 (1H, t, J = 4.7 Hz), 7.54 (2H, d, J = 8.5 Hz), 7.60 (1H, d, J = 9.4 Hz), 8.01 (1H, d, J = 9.4 Hz), 8.04 (2H, d, J = 8.5 Hz), 8.11 (1H, s), 8.39 (1H, s), 8.44 (1H, s), 11.28 (1H, s).
Elemental analysis: for C₂₂H₂₄N₆O₂
Calculated: C, 65.33; H, 5.98; N, 20.78.
Found: C, 65.26; H, 6.01; N, 20.87.

### Example 179

methyl 2-methyl-2-{4-[(5-methylimidazo[1,2-a]pyridin-2-yl)carbamoyl]phenyl}propanoate

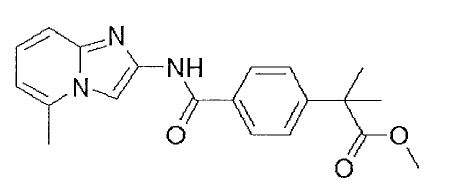

4-(2-Methoxy-1,1-dimethyl-2-oxoethyl)benzoic acid (200 mg, 0.88 mmol) obtained in Reference Example 1 was dissolved in dichloromethane (8 ml), oxalyl dichloride (171 mg, 1.35 mmol) and N,N-dimethylformamide (13 mg, 0.18 mmol) were added, and the mixture was stirred at room temperature for 30 min. Then, the solvent was evaporated under reduced pressure, and the residue was azeotroped with a toluene-mixed solvent to give a colorless acid chloride. On the other hand, 5-methylimidazo[1,2-a]pyridin-2-amine (125 mg, 0.85 mmol) obtained in Reference Example 20 and triethylamine (263 mg, 2.60 mmol) was dissolved in tetrahydrofuran (10 ml), a solution of the obtained acid chloride in tetrahydrofuran (5 ml) was added thereto at room temperature. After stirring for 30 min, saturated aqueous sodium hydrogen carbonate solution (15 ml) was added to the reaction solution, and the mixture was extracted three times with ethyl acetate (50 ml). The organic layers were combined, washed with saturated brine, and dried over anhydrous magnesium sulfate. After filtration, the solvent was evaporated under reduced pressure to give a yellow white crude solid. This was purified by basic silica gel chromatography (50% ethyl acetate/hexane) to give the title compound (yield 273 mg, 91.6%) as a white solid.
¹H-NMR (CDCl₃) δ: 1.58 (6H, s), 2.63 (3H, s), 3.66 (3H, s), 6.62(1H, d, J = 7.0 Hz), 6.85 (1H, d, J = 9.0 Hz), 7.02 (1H, dd, J = 9.0, 7.0 Hz), 7.38 (2H, d, J = 8.5 Hz), 7.95 (2H, d, J = 8.5 Hz), 8.18 (1H, s), 10.60 (1H, s).

### Example 180

ethyl 4-methyl-4-14-[(5-methylimidazo[1,2-a]pyridin-2-yl)carbamoyl]phenyl}pentanoate

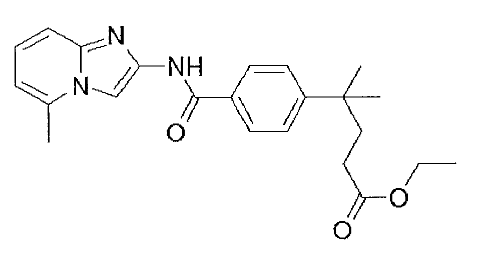

Using 4-(4-ethoxy-1,1-dimethyl-4-oxobutyl)benzoic acid (400 mg, 1.48 mmol) obtained in Reference Example 6 and in the same manner as in Example 179, the reaction was performed to give the title compound (yield 551 mg, 95.9%).
¹H-NMR (DMSO-d₆) δ: 1.13 (3H, t, J = 7.2 Hz), 1.31 (6H, s), 1.86-2.10 (4H, m), 2.62 (3H, s), 3.97 (2H, q, J = 7.2 Hz), 6.80 (1H, d, J = 7.0 Hz), 7.23 (1H, dd, J = 9.0, 7.0 Hz), 7.38 (1H, d, J = 9.0 Hz), 7.49 (2H, d, J = 8.5 Hz), 8.06 (2H, d, J = 8.5 Hz), 8.07 (1H, s), 11.18 (1H, s).

### Example 181

methyl 1-{4-[(5-methylimidazo[1,2-a]pyridin-2-yl)carbamoyl]phenyl}cyclobutanecarboxylate

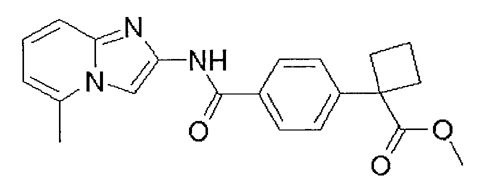

Using 4-[1-(methoxycarbonyl)cyclobutyl]benzoic acid (191 mg, 0.80 mmol) obtained in Reference Example 12 and in the same manner as in Example 179, the reaction was performed to give the title compound (yield 264 mg, 90.8%).
¹H-NMR (CDCl₃) δ: 1.80-1.95 (1H, m), 2.00-2.16 (1H, m), 2.40-2.55 (2H, m), 2.63 (3H, s), 2.78-2.91 (2H, m), 3.65 (3H, s), 6.61(1H, d, J = 7.0 Hz), 6.75 (1H, d, J = 9.0 Hz), 6.99 (1H, dd, J = 9.0, 7.0 Hz), 7.32 (2H, d, J = 8.3 Hz), 7.97 (2H, d, J = 8.3 Hz), 8.19 (1H, s), 11.08 (1H, s).

### Example 182

methyl 1-{4-[(6-chloroimidazo[1,2-a]pyridin-2-yl)carbamoyl]phenyl}cyclopropanecarboxylate

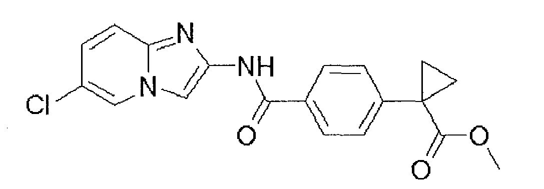

Using 4-[1-(methoxycarbonyl)cyclopropyl]benzoic acid (250 mg, 1.11 mmol) obtained in Reference Example 11 and 6-chloroimidazo[1,2-a]pyridin-2-amine (150 mg, 0.89 mmol) obtained in Reference Example 21 and in the same manner as in Example 179, the reaction was performed to give the title compound (yield 202 mg, 61.0%).
¹H-NMR (CDCl₃) δ: 1.21 (2H, dd, J = 7.2, 4.2 Hz), 1.67(2H, dd, J = 7.2, 4.2 Hz), 3.65 (3H, s), 7.03-7.11 (2H, m), 7.44 (2H, d, J = 8.3Hz), 7.90 (2H, d, J = 8.3 Hz), 8.16 (1H, t, J = 1.5 Hz), 8.25 (1H, s), 9.74 (1H, s).

### Example 183

methyl 1-{4-[(6-chloroimidazo[1,2-a]pyridin-2-yl)carbamoyl]phenyl}cyclobutanecarboxylate

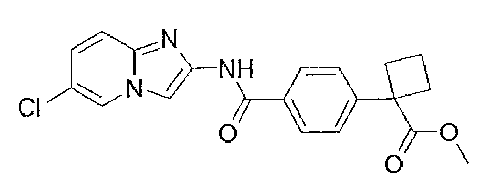

Using 4-[1-(methoxycarbonyl)cyclobutyl]benzoic acid (270 mg, 1.13 mmol) obtained in Reference Example 12 and in the same manner as in Example 182, the reaction was performed to give the title compound (yield 232 mg, 67.5%).
¹H-NMR (CDCl₃) δ: 1.82-1.98 (1H, m), 2.01-2.19 (1H, m), 2.43-2.58 (2H, m), 2.81-2.95 (2H, m), 3.66 (3H, s), 6.93 (1H, d, J = 9.5 Hz), 7.04 (1H, dd, J = 9.5, 1.9 Hz), 7.38 (2H, d, J = 8.3 Hz), 7.92 (2H, d, J = 8.3 Hz), 8.16 (1H, d, J = 1.9 Hz), 8.26 (1H, s), 10.12 (1H, s).

### Example 184

methyl 1-{4-[(6-chloroimidazo[1,2-a]pyridin-2-yl)carbamoyl]phenyl}cyclopentanecarboxylate

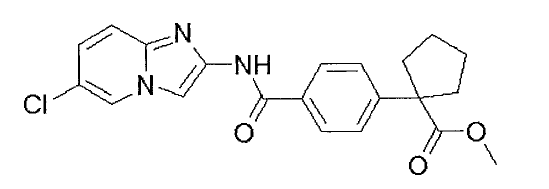

Using 4-[1-(methoxycarbonyl)cyclopentyl]benzoic acid (200 mg, 0.79 mmol) obtained in Reference Example 13 and in the same manner as in Example 182, the reaction was performed to give the title compound (yield 200 mg, 70.2%).
¹H-NMR (CDCl₃) δ: 1.71-1.82 (4H, m), 1.84-1.99 (2H, m), 2.60-2.72 (2H, m), 3.63 (3H, s), 6.90 (1H, d, J = 9.5 Hz), 7.02 (1H, dd, J = 9.5, 1.9 Hz), 7.43 (2H, d, J = 8.3 Hz), 7.90 (2H, d, J = 8.3 Hz), 8.16 (1H, dd, J = 1.9, 0.8 Hz), 8.26 (1H, s), 10.18 (1H, s).

### Example 185

methyl 1-(4-{[(6-chloroimidazo[1,2-a]pyridin-2-yl)amino]carbonyl}phenyl)cyclohexanecarboxylate

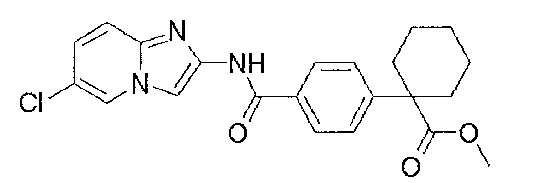

Using 4-[1-(methoxycarbonyl)cyclohexyl]benzoic acid (250 mg, 0.99 mmol) obtained in Reference Example 14 and in the same manner as in Example 182, the reaction was performed to give the title compound (yield 140 mg, 70.1%).
MS (ESI+): 412 (M+H).
¹H-NMR (CDCl₃) δ: 1.21-1.39 (1H, m), 1.40-1.58 (2H, m), 1.59-1.82 (5H, m), 2.41-2.54 (2H, m), 3.65 (3H, s), 6.95 (1H, d, J = 9.5 Hz), 7.04 (1H, dd, J = 9.5, 1.9 Hz), 7.47 (2H, d, J = 8.3 Hz), 7.91 (2H, d, J = 8.3 Hz), 8.16 (1H, dd, J = 1.9, 0.8 Hz), 8.25 (1H, s), 10.03 (1H, s).
Elemental analysis: for C₂₂H₂₂N₃O₃Cl
Calculated: C, 64.15; H, 5.38; N, 10.20.
Found: C, 64.09; H, 5.40; N, 10.23.

### Example 186

2-methyl-2-{4-[(5-methylimidazo[1,2-a]pyridin-2-yl)carbamoyl]phenyl}propanoic acid

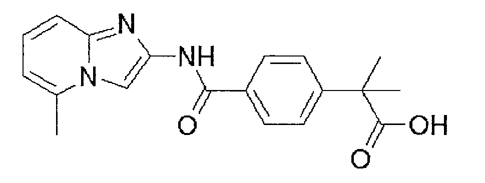

Methyl 2-methyl-2-{4-[(5-methylimidazo[1,2-a]pyridin-2-yl)carbamoyl]phenyl}propanoate (220 mg, 0.61 mmol) obtained in Example 179 was added to 50% tetrahydrofuran/methanol (6.0 ml), and then 2N aqueous potassium hydroxide solution (3.0 ml, 6.0 mmol) was added, and the mixture was stirred at 45-50°C for 3 hr. Then, the solvent was evaporated under reduced pressure. Water (5 ml) was added to the obtained white crude solid, and the mixture was weakly acidified to pH 4-5 with 1N hydrochloric acid to give white precipitate. The precipitate was collected by filtration, washed successively with water, acetonitrile and diethyl ether, and dried to give a white title compound (yield 98 mg, 47%).
melting point 282-283°C (acetonitrile)
MS (ESI+): 338 (M+H).
¹H-NMR (DMSO-d₆) δ: 1.52 (6H, s), 2.62 (3H, m), 6.80 (1H, d, J = 6.8Hz), 7.23 (1H, dd, J = 8.9, 6.8 Hz), 7.38 (1H, d, J = 8.9 Hz), 7.48 (2H, d, J = 8.5 Hz), 8.06 (2H, d, J = 8.5 Hz), 8. 07 (1H, s), 11.23 (1H, s), 12.50 (1H, s).
Elemental analysis: for C₁₉H₁₉N₃O₃
Calculated: C, 67.64; H, 5.68; N, 12.46.
Found: C, 67.52; H, 5.54; N, 12.43.

### Example 187

4-methyl-4-(4-{[(5-methylimidazo[1,2-a]pyridin-2-yl)amino]carbonyl}phenyl)pentanoic acid

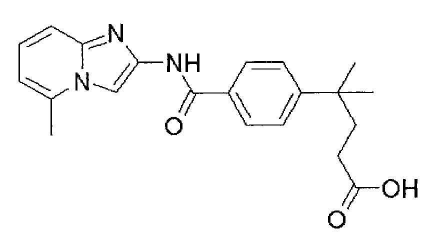

Ethyl 4-methyl-4-{4-[(5-methylimidazo[1,2-a]pyridin-2-yl)carbamoyl]phenyl}pentanoate (220 mg, 0.56 mmol) obtained in Example 180 was added to 50% tetrahydrofuran/ethanol (4.0 ml), and then 5N aqueous potassium hydroxide solution (1.0 ml,5 mmol) was added, and the mixture was stirred for one day at room temperature. Then, the solvent was evaporated under reduced pressure. Water (5 ml) was added to the obtained white crude solid, and the mixture was weakly acidified to pH 4-5 with 1N hydrochloric acid to give white precipitate. The precipitate was collected by filtration, washed successively with water, acetonitrile and diethyl ether, and dried to give a white title compound (yield 148 mg, 72%).
melting point 260°C (acetonitrile)
MS (ESI+): 366 (M+H).
¹H-NMR (DMSO-d₆) δ: 1.31 (6H, s), 1.91 (4H, s), 2.62 (3H, m), 6.80 (1H, d, J = 6.8 Hz), 7.23 (1H, dd, J = 8.9, 6.8 Hz), 7.38 (1H, d, J = 8.9 Hz), 7.49 (2H, d, J = 8.5 Hz), 8.06 (2H, d, J = 8.5 Hz), 8. 07 (1H, s), 11.17 (1H, s), 12.04 (1H, s).
Elemental analysis: for C₂₁H₂₃N₃O₃
Calculated: C, 69.02; H, 6.34; N, 11.50.
Found: C, 69.02; H, 6.33; N, 11.51.

### Example 188

1-{4-[(5-methylimidazo[1,2-a]pyridin-2-yl)carbamoyl]phenyl}cyclobutanecarboxylic acid

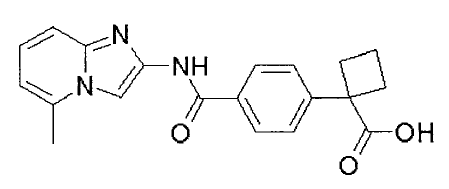

Methyl 1-{4-[(5-methylimidazo[1,2-a]pyridin-2-yl)carbamoyl]phenyl}cyclobutanecarboxylate (252 mg, 0.69 mmol) obtained in Example 181 was added to 50% tetrahydrofuran/ethanol (6.0 ml), and then 5N aqueous potassium hydroxide solution (1.0 ml) was added, and the mixture was stirred at 50°C for 1.5 hr. Then, a white title compound (yield 169 mg, 70%) was obtained in the same manner as in Example 186.
melting point 272°C (acetonitrile)
MS (ESI+): 350 (M+H).
¹H-NMR (DMSO-d₆) δ: 1.73-1.89 (1H, m), 1.89-2.07 (1H, m), 2.38-2.49 (2H, m), 2.62(3H, s), 2.68-2.82 (2H, m), 6.80(1H, d, J = 6.8 Hz), 7.23 (1H, dd, J = 9.1, 6.8 Hz), 7.38 (1H, d, J = 9.1 Hz),7.40 (2H, d, J = 8.3 Hz), 8.07 (2H, d, J = 8.3 Hz), 8.07 (1H, s), 11.23 (1H, s), 12.52 (1H, s).
Elemental analysis: for C₂₁H₂₃N₃O₃
Calculated: C, 68.75; H, 5.48; N, 12.03.
Found: C, 68.82; H, 5.44; N, 12.00.

### Example 189

1-{4-[(6-chloroimidazo[1,2-a]pyridin-2-yl)carbamoyl]phenyl}cyclopropanecarboxylic acid

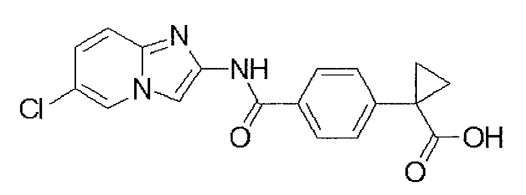

Methyl 1-{4-[(6-chloroimidazo[1,2-a]pyridin-2-yl)carbamoyl]phenyl}cyclopropanecarboxylate (198 mg, 0.54 mmol) obtained in Example 182 was added to 50% tetrahydrofuran/ethanol (6.0 ml), and then 5N aqueous potassium hydroxide solution (1.0 ml) was added, and the mixture was stirred at 40-50°C for 3 hr. Then, a white title compound (yield 152 mg, 79.8%) was obtained in the same manner as in Example 186.
melting point 330-331°C (dimethyl sulfoxide/ethanol)
MS (ESI+): 356 (M+H).
¹H-NMR (DMSO-d₆) δ: 1.15-1.25 (2H, m), 1.45-1.53 (2H, m), 7.29 (1H, dd, J = 9.5, 1.9 Hz), 7.46 (2H, d, J = 8.3 Hz), 7.50 (1H, d, J = 9.5 Hz), 8.00 (2H, d, J = 8.3 Hz), 8.34 (1H, s), 8.88 (1H, d, J = 1.9 Hz), 11.28 (1H, s), 12.48 (1H, s).
Elemental analysis: for C₁₈H₁₄N₃O₃Cl
Calculated: C, 60.77; H, 3.97; N, 11.81.
Found: C, 60.36; H, 4.12; N, 11.57.

### Example 190

1-{4-[(6-chloroimidazo[1,2-a]pyridin-2-yl)carbamoyl]phenyl}cyclobutanecarboxylic acid

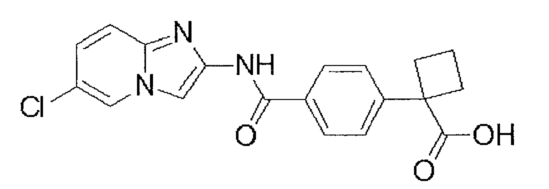

Using methyl 1-{4-[(6-chloroimidazo[1,2-a]pyridin-2-yl)carbamoyl]phenyl}cyclobutanecarboxylate (218 mg, 0.57 mmol) obtained in Example 183 and in the same manner as in Example 189, a white title compound (yield 163 mg, 77.6%) was obtained.
melting point 317.7°C (decomposition) (dimethyl
sulfoxide/ethanol)
MS (ESI+): 370 (M+H). ¹H-NMR (DMSO-d₆) δ: 1.71-2.08 (2H, m), 2.36-2.52 (2H, m), 2.68-2.82 (2H, m), 7.29 (1H, dd, J = 9.5, 1.9 Hz), 7.40 (2H, d, J = 8.3 Hz), 7.51 (1H, d, J = 9.5 Hz), 8.05 (2H, d, J = 8.3 Hz), 8.34 (1H, s), 8.89 (1H, d, J = 1.9 Hz), 11.28 (1H, s), 12.54 (1H, s).
Elemental analysis: for C₁₉H₁₆N₃O₃Cl
Calculated: C, 61.71; H, 4.36; N, 11.36.
Found: C, 61.55; H, 4.35; N, 11.31.

### Example 191

1-{4-[(6-chloroimidazo[1,2-a]pyridin-2-yl)carbamoyl]phenyl}cyclopentanecarboxylic acid

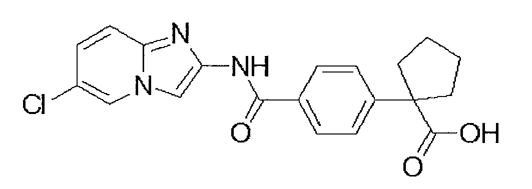

Using methyl 1-{4-[(6-chloroimidazo[1,2-a]pyridin-2-yl)carbamoyl]phenyl}cyclopentanecarboxylate (200 mg, 0.50 mmol) obtained in Example 184 and in the same manner as in Example 189, a white title compound (yield 153 mg, 79.3%) was obtained.
melting point 313°C (decomposition) (dimethyl
sulfoxide/ethanol)
MS (ESI+): 384 (M+H).
¹H-NMR (DMSO-d₆) δ: 1.55-1.77 (4H, m), 1.77-1.95 (2H, m), 2.50-2.65 (2H, m), 7.29 (1H, dd, J = 9.5, 2.3 Hz), 7.47 (2H, d, J = 8.3 Hz), 7.51 (1H, d, J = 9.5 Hz), 8.02 (2H, d, J = 8.3 Hz), 8.33 (1H, s), 8.88 (1H, d, J = 2.3 Hz), 11.26 (1H, s), 12.45 (1H, s).
Elemental analysis: for C₂₀H₁₈N₃O₃Cl
Calculated: C, 62.58; H, 4.73; N, 10.95; Cl, 9.24.
Found: C, 62.20; H, 4.69; N, 10.81; Cl, 9.19.

### Example 192

1-(4-{[(6-chloroimidazo[1,2-a]pyridin-2-yl)amino]carbonyl}phenyl)cyclohexanecarboxylic acid

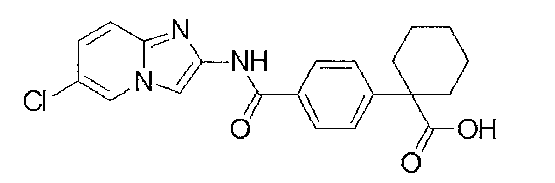

Using methyl 1-(4-{[(6-chloroimidazo[1,2-a]pyridin-2-yl)amino]carbonyl}phenyl)cyclohexanecarboxylate (145 mg, 0.36 mmol) obtained in Example 185 and in the same manner as in Example 189, a white title compound (yield 98 mg, 70.1%) was obtained.
melting point 288°C (dimethyl sulfoxide/water)
MS (ESI+): 398 (M+H).
¹H-NMR (DMSO-d₆) δ: 1.16-1.81 (8H, m), 2.25-2.34 (2H, m), 7.29 (1H, dd, J = 9.4, 2.1 Hz), 7.51 (1H, d, J = 9.4 Hz), 7.52 (2H, d, J = 8.5 Hz), 8.04 (2H, d, J = 8.5 Hz), 8.33 (1H, s), 8.88 (1H, d, J = 2.1 Hz), 11.26 (1H, s), 12.52 (1H, s).
Elemental analysis: for C₂₁H₂₀N₃O₃Cl
Calculated: C, 63.40; H, 5.07; N, 10.56.
Found: C, 63.16; H, 5.03; N, 10.37.

### Example 193

N-[3-bromo-5-(hydroxymethyl)imidazo[1,2-a]pyridin-2-yl]-4-tert-butylbenzamide

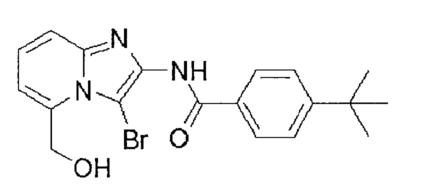

N-[3-Bromo-5-(bromomethyl)imidazo[1,2-a]pyridin-2-yl]-4-tert-butyl-N-(4-tert-butylbenzoyl)benzamide (1.00 g, 1.57 mmol) obtained in Reference Example 40 and potassium acetate (1.57 g, 15.7 mmol) were added to a 25% chloroform/ethanol mixed solvent (20 ml), and the mixture was stirred under reflux for 60 min. After cooling, the solvent was evaporated under reduced pressure, and the obtained crude product was dissolved in methylene chloride (200 ml). The mixture was successively washed with saturated aqueous sodium hydrogen carbonate solution and saturated brine and dried over anhydrous magnesium sulfate. After filtration, the solvent was evaporated under reduced pressure to give a yellow crude solid. The crude product was dissolved in methanol (20 ml), sodium methoxide (28% methanol solution, 1.51 g, 7.84 mmol) was added, and the mixture was stirred at room temperature for 60 min. Then, water (50 ml) was added to the reaction solution, and the precipitated yellow white solid was collected by filtration and washed with water, acetonitrile and diethyl ether to give a yellow white title compound (yield 506 mg, 80%).
MS (ESI+): 402 (M+H).
¹H-NMR (DMSO-d₆) δ: 1.33 (9H, s), 5.23 (2H, d, J = 5.5 Hz), 5.71 (1H, t, J = 5.5 Hz), 7.08 (1H, dd, J = 7.0, 0.8 Hz), 7.33 (1H, dd, J = 8.9, 7.0 Hz), 7.52 (1H, d, J = 8.9, 0.8 Hz), 7.55 (2H, d, J = 8.5 Hz), 7.95 (2H, d, J = 8.5 Hz), 10.33 (1H, s).
Elemental analysis: for C₁₉H₂₀BrN₃O₂·0.3H₂O
Calculated: C, 55.97; H, 5.09; N, 10.31.
Found: C, 55.95; H, 5.08; N, 10.39.

### Example 194

4-tert-butyl-N-[5-(hydroxymethyl)imidazo[1,2-a]pyridin-2-yl]benzamide

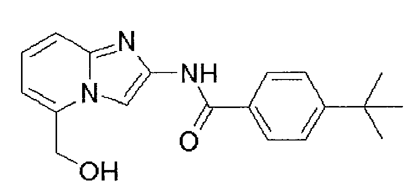

N-[3-Bromo-5-(hydroxymethyl)imidazo[1,2-a]pyridin-2-yl]-4-tert-butylbenzamide (200 mg, 0.49 mmol) obtained in Example 193 was suspended in tetrahydrofuran (15 ml), and triturated in an ultrasonication washing machine. Then, the suspension was cooled to -78°C, n-butyllithium (1.6M hexane solution, 1.37 ml, 2.19 mmol) was added, and the mixture was stirred for 30 min, which turned the suspension into an orange solution. Thereafter, methanol (1 ml) was added to the reaction solution, and the mixture was poured into water (40 ml). The mixture was extracted 3 times with methylene chloride (50 ml). The organic layers were combined, and washed successively with 10% aqueous sodium thiosulfate solution (10 ml) and saturated brine, and dried over anhydrous magnesium sulfate. After filtration, the solvent was evaporated under reduced pressure to give a yellow crude solid. The crude product was washed with a mixed solvent (12 ml) of toluene/diethyl ether (1/2) to give the title compound (yield 147 mg, 93.6%) as a white solid.
melting point 229-230°C (acetonitrile)
MS (ESI+): 324 (M+H).
¹H-NMR (DMSO-d₆) δ: 1.32 (9H, s), 4.77 (2H, d, J = 5.5 Hz), 5.73 (1H, t, J = 5.5 Hz), 6.93 (1H, d, J = 8.0 Hz), 7.28 (1H, dd, J = 8.9, 8.0 Hz), 7.43 (1H, d, J = 8.9 Hz), 7.54 (2H, d, J = 8.5 Hz), 8.03 (2H, d, J = 8.5 Hz), 8.20 (1H, s), 11.15 (1H, s).

### Example 195

4-tert-butyl-N-(6-chloroimidazo[1,2-b]pyridazin-2-yl)benzamide

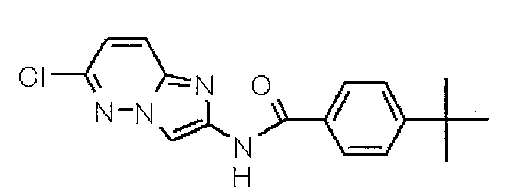

A mixture of 6-chloroimidazo[1,2-b]pyridazine-2-amine (3.37 g, 20.0 mmol), 4-tert-butylbenzoyl chloride (4.00 g, 20.3 mmol) and N,N-dimethylacetamide (50 mL) was stirred at room temperature for 1 hr. The reaction mixture was poured into water. The precipitated solid was collected by filtration, washed with water then with isopropyl ether to give the title compound (5.88 g, 89%) as pale-yellow crystals. ¹H-NMR (CDCl₃) δ: 1.36 (9H, s), 6.99 (1H, d, J = 9.2 Hz), 7.43 (1H, d, J = 9.2 Hz), 7.48-7.53 (2H, m), 7.90-7.96 (2H, m), 8.62 (1H, s), 9.80 (1H, s).
LC-MS 329(M+H), 351(M+Na).

### Example 196

4-tert-butyl-N-[6-(1H-imidazol-1-yl)imidazo[1,2-b]pyridazin-2-yl]benzamide

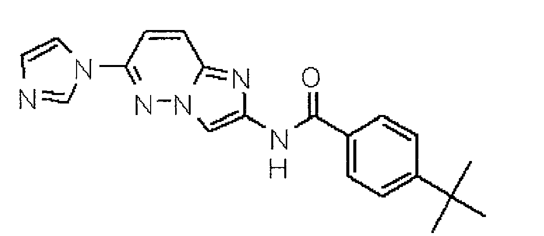

A mixture of 4-tert-butyl-N-(6-chloroimidazo[1,2-b]pyridazin-2-yl)benzamide (86 mg, 0.26 mmol), 1H-imidazole (340 mg, 5.0 mmol), potassium carbonate (690 mg, 5.0 mmol) and N,N-dimethylformamide (2 mL) was stirred with heating at 120°C for 5 hr. The reaction mixture was poured into water. The precipitated solid was collected by filtration, washed with water then with isopropyl ether to give the title compound (55 mg, 59%) as pale-yellow crystals.
¹H NMR (CDCl₃) δ: 1.37 (9H, s), 7.20 (1H, d, J = 9.6 Hz), 7.54 (2H, d, J = 8.3 Hz), 7.66 (1H, s), 7.76 (1H, d, J = 9.6 Hz), 7.91 (2H, d, J = 8.3 Hz), 8.26 (1H, s), 8.64 (1H, s), 9.11 (1H, s).
LC-MS 361(M+H), 383(M+Na).

### Example 197

4-tert-butyl-N-[6-(1H-pyrazol-1-yl)imidazo[1,2-b]pyridazin-2-yl]benzamide

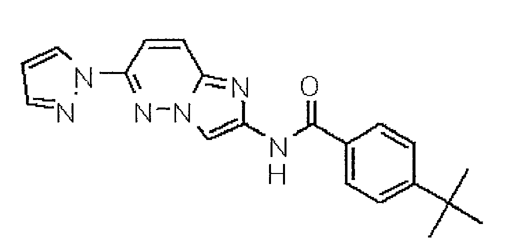

In the same manner as in Example 196, the title compound (yield 61%) was synthesized from 4-tert-butyl-N-(6-chloroimidazo[1,2-b]pyridazin-2-yl)benzamide.
¹H-NMR (CDCl₃) δ: 1.36 (9H, s), 6.55 (1H, dd, J = 2.6, 1.7 Hz), 7.49-7.55 (2H, m), 7.65 (1H, d, J = 9.6 Hz), 7.79 (1H, d, J = 1.1 Hz), 7.85-7.95 (3H, m), 8.50 (1H, d, J = 2.1 Hz), 8.58 (1H, s), 9.28 (1H, s).
LC-MS 361(M+H), 383(M+Na).

### Example 198

4-tert-butyl-N-[6-(1H-1,2,4-triazol-1-yl)imidazo[1,2-b]pyridazin-2-yl]benzamide

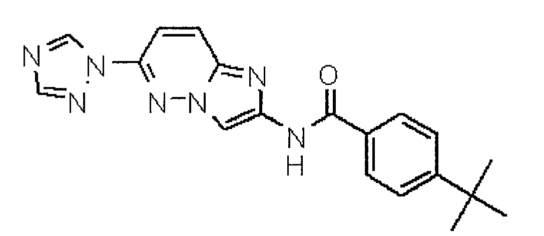

In the same manner as in Example 196, the title compound (yield 39%) was synthesized from 4-tert-butyl-N-(6-chloroimidazo[1,2-b]pyridazin-2-yl)benzamide.
¹H-NMR (CDCl₃) δ: 1.36 (9H, s), 7.49-7.57 (2H, m), 7.66-7.79 (2H, m), 7.87-7.97 (2H, m), 8.16 (1H, s), 8.66 (1H, s), 9.13 (1H, s), 9.40 (1H, s).
LC-MS 362(M+H), 384(M+Na).

### Example 199

4-tert-butyl-N-[6-(2-methyl-1H-imidazol-1-yl)imidazo[1,2-b]pyridazin-2-yl]benzamide

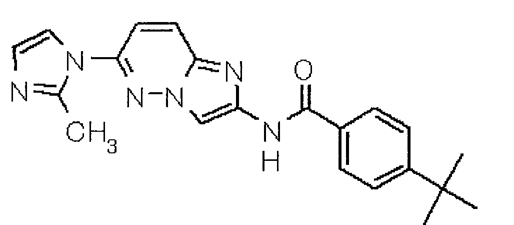

In the same manner as in Example 196, the title compound (yield 25%) was synthesized from 4-tert-butyl-N-(6-chloroimidazo[1,2-b]pyridazin-2-yl)benzamide.
LC-MS 375(M+H).

### Example 200

4-tert-butyl-N-(6-phenylimidazo[1,2-b]pyridazin-2-yl)benzamide

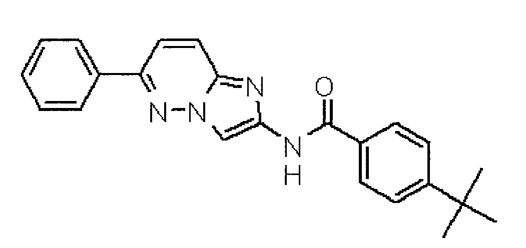

A mixture of 4-tert-butyl-N-(6-chloroimidazo[1,2-b]pyridazin-2-yl)benzamide (71 mg, 0.22 mmol), dihydroxyphenylborane (40 mg, 0.33 mmol), potassium carbonate (30 mg, 0.22 mmol), PS-PPh₃-Pd (50 mg, 0.005 mmol of Pd), 1,2-dimethoxyethane (10 mL), ethanol (5 mL) and water (5 mL) was stirred at 100°C for 14 hr. The resin reagent was removed by filtration, and the filtrate was concentrated. The residue was purified by preparative HPLC to give the title compound (22 mg, 27%) as pale-yellow crystals.
¹H-NMR (CDCl₃) δ: 1.36 (9H, s), 7.44-7.59 (6H, m), 7.68 (1H, d, J = 9.4 Hz), 7.88-8.00 (4H, m), 8.67 (1H, s), 9.28 (1H, s).
LC-MS 371(M+H), 393(M+Na).

### Example 201

4-tert-butyl-N-imidazo[1,2-b]pyridazin-2-ylbenzamide

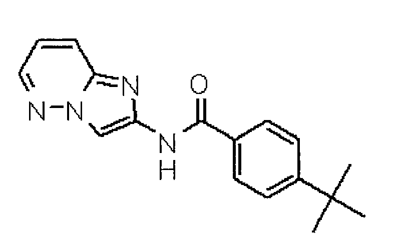

A mixture of 4-tert-butyl-N-(6-chloroimidazo[1,2-b]pyridazin-2-yl)benzamide (50 mg, 0.152 mmol), palladium carbon (20 mg), ethanol (5 mL) and tetrahydrofuran (5 mL) was stirred at room temperature for 14 hr under ambient hydrogen atmosphere. The insoluble material was removed by filtration, and the filtrate was concentrated. The residue was purified by preparative HPLC to give the title compound (22 mg, 27%) as yellow crystals.
¹H-NMR (CDCl₃) δ: 1.36 (9H, s), 6.99 (1H, dd, J = 9.1, 4.6 Hz), 7.47-7.60 (3H, m), 7.86-7.95 (2H, m), 8.30 (1H, dd, J = 4.6, 1.6 Hz), 8.64 (1H, s), 9.27 (1H, s).
LC-MS 295(M+H), 317(M+Na).

### Example 202

4-tert-butyl-N-(6-pyridin-3-ylimidazo[1,2-b]pyridazin-2-yl)benzamide

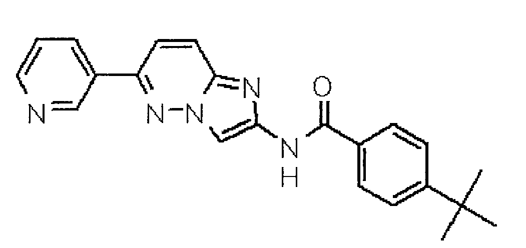

A mixture of 4-tert-butyl-N-(6-chloroimidazo[1,2-b]pyridazin-2-yl)benzamide (330 mg, 1.0 mmol), pyridin-3-ylboronic acid (190 mg, 1.5 mmol), 1N aqueous potassium carbonate solution (1 mL, 1.0 mmol), PS-PPh₃-Pd (100 mg, 0.01 mmol of Pd) and 1,2-dimethoxyethane (2 mL) was stirred in a sealed tube at 160°C for 20 min in a microwave reaction apparatus. The resin reagent was removed by filtration, and the filtrate was diluted with ethyl acetate, washed with water and concentrated. The residue was purified by preparative HPLC to give the title compound (55 mg, 17%) as pale-yellow crystals.
¹H-NMR (CDCl₃) δ: 1.37 (9H, s), 7.41-7.59 (4H, m), 7.75 (1H, d, J = 9.4 Hz), 7.92 (2H, d, J = 8.5 Hz), 8.29-8.37 (1H, m), 8.70 (1H, s), 8.74 (1H, dd, J = 4.7, 1.5 Hz), 9.03-9.12 (1H, m), 9.18 (1H, d, J = 1.7 Hz).
LC-MS 372(M+H), 394(M+Na).

### Example 203

4-tert-butyl-N-(6-methylimidazo[1,2-b]pyridazin-2-yl)benzamide

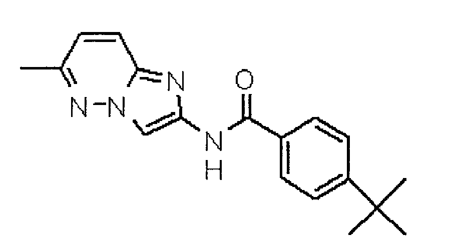

A mixture of 4-tert-butyl-N-(6-chloroimidazo[1,2-b]pyridazin-2-yl)benzamide (33 mg, 0.1 mmol), methylboronic acid (30 mg, 1.0 mmol), potassium carbonate (27 mg, 0.2 mmol), PS-PPh₃-Pd (50 mg, 0.005 mmol of Pd) and 1,4-dioxane (3 mL) was stirred at 100°C for 12 hr. The resin reagent was removed by filtration, and the filtrate was diluted with ethyl acetate, washed with water and concentrated. The residue was purified by preparative HPLC to give the title compound (5.0 mg, 16%) as a pale-yellow oil.
LC-MS 309(M+H), 331(M+Na).

### Example 204

4-tert-butyl-N-{6-[3-(trifluoromethyl)phenyl]imidazo[1,2-b]pyridazin-2-yl}benzamide trifluoroacetate

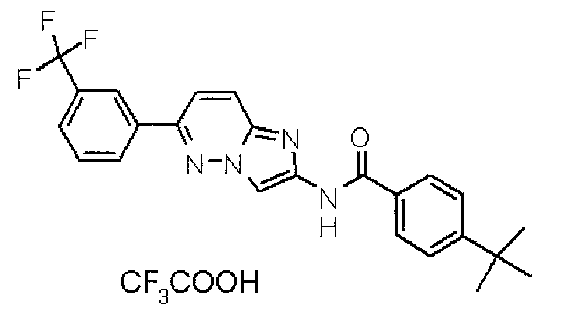

A mixture of 4-tert-butyl-N-(6-chloroimidazo[1,2-b]pyridazin-2-yl)benzamide (25 mg, 0.076 mmol), [3-(trifluoromethyl)phenyl]boronic acid (57 mg, 0.3 mmol), potassium carbonate (138 mg, 1.0 mmol), PS-PPh₃-Pd (50 mg, 0.005 mmol of Pd), 1,2-dimethoxyethane (2 mL), ethanol (1 mL) and water (1 mL) was stirred at 100°C for 14 hr. The resin reagent was removed by filtration, and the filtrate was concentrated. The residue was purified by preparative HPLC to give the title compound (7.0 mg, 17%).
LC-MS 439(M+H), 461(M+Na).

In the same manner as in Example 204, the compounds in Examples 205 - 213 were synthesized.

### Example 205

methyl 4-{2-[(4-tert-butylbenzoyl)amino]imidazo[1,2-b]pyridazin-6-yl}benzoate trifluoroacetate

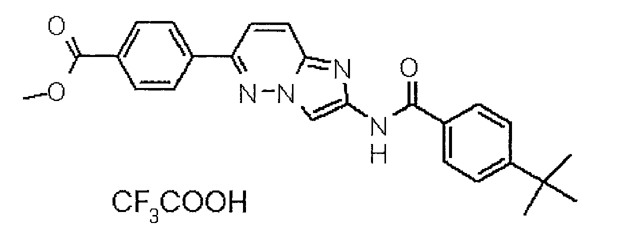

In the same manner as in Example 204, 4-tert-butyl-N-(6-chloroimidazo[1,2-b]pyridazin-2-yl)benzamide and [4-(methoxycarbonyl)phenyl]boronic acid were reacted to synthesize the title compound (yield 11%).
LC-MS 429(M+H), 451(M+Na).

### Example 206

ethyl 4-{2-[(4-tert-butylbenzoyl)amino]imidazo[1,2-b]pyridazin-6-yl}benzoate trifluoroacetate

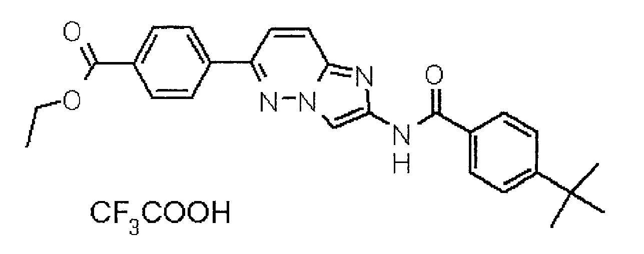

The title compound (yield 6%) was obtained as a byproduct of the reaction in Example 205 (transesterification reaction by ethanol solvent).
LC-MS 443(M+H), 465(M+Na).

### Example 207

N-{6-[3-(acetylamino)phenyl]imidazo[1,2-b]pyridazin-2-yl}-4-tert-butylbenzamide trifluoroacetate

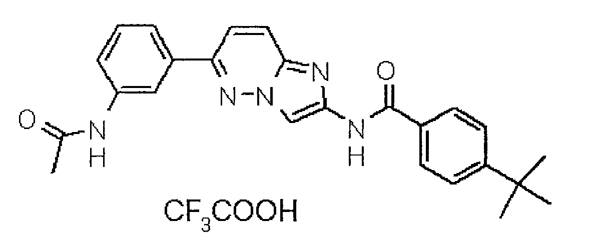

In the same manner as in Example 204, 4-tert-butyl-N-(6-chloroimidazo[1,2-b]pyridazin-2-yl)benzamide and [3-(acetylamino)phenyl]boronic acid were reacted to synthesize the title compound (yield 24%).
LC-MS 428(M+H).

### Example 208

4-tert-butyl-N-{6-[3-(trifluoromethoxy)phenyl]imidazo[1,2-b]pyridazin-2-yl}benzamide trifluoroacetate

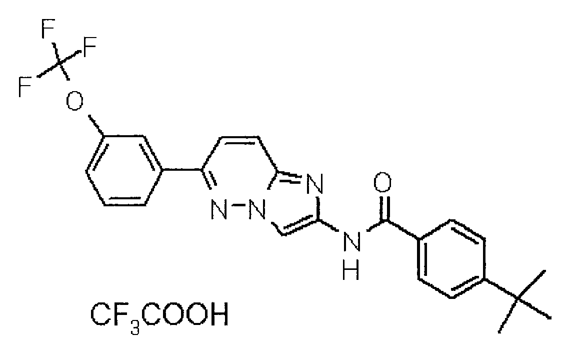

In the same manner as in Example 204, 4-tert-butyl-N-(6-chloroimidazo[1,2-b]pyridazin-2-yl)benzamide and [3-(trifluoromethoxy)phenyl]boronic acid were reacted to synthesize the title compound (yield 2.3%).
LC-MS 455(M+H), 477 (M+Na) .

### Example 209

4-tert-butyl-N-{6-[4-(dimethylamino)phenyl]imidazo[1,2-b]pyridazin-2-yl}benzamide ditrifluoroacetate

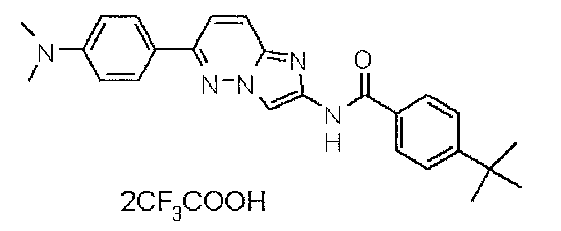

In the same manner as in Example 204, 4-tert-butyl-N-(6-chloroimidazo[1,2-b]pyridazin-2-yl)benzamide and [4-(dimethylamino)phenyl]boronic acid were reacted to synthesize the title compound (yield 2.5%).
LC-MS 414(M+H), 436(M+Na).

### Example 210

4-tert-butyl-N-[6-(4-methoxyphenyl)imidazo[1,2-b]pyridazin-2-yl]benzamide trifluoroacetate

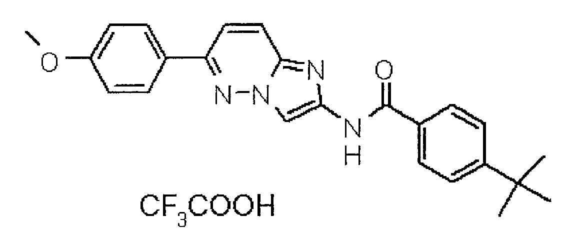

In the same manner as in Example 204, 4-tert-butyl-N-(6-chloroimidazo[1,2-b]pyridazin-2-yl)benzamide and (4-methoxyphenyl)boronic acid were reacted to synthesize the title compound (yield 25%).
¹H-NMR (CDCl₃) δ: 1.37 (9H, s), 3.91 (3H, s), 7.05-7.10 (2H, m), 7.57 (2H, d, J = 8.7 Hz), 7.78 (1H, d, J = 9.6 Hz), 7.94-7.99 (2H, m), 8.06 (1H, d, J = 9.4 Hz), 8.11 (2H, d, J = 8.5 Hz), 8.75 (1H, s), 11.87 (1H, s).
LC-MS 401(M+H).

### Example 211

4-tert-butyl-N-[6-(4-cyanophenyl)imidazo[1,2-b]pyridazin-2-yl]benzamide trifluoroacetate

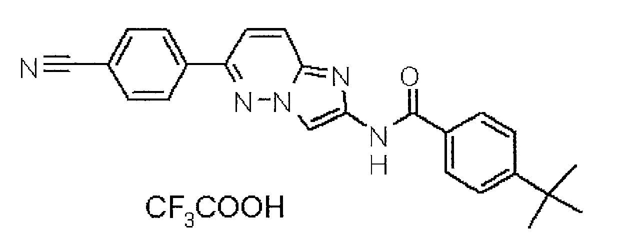

In the same manner as in Example 204, 4-tert-butyl-N-(6-chloroimidazo[1,2-b]pyridazin-2-yl)benzamide and (4-cyanophenyl)boronic acid were reacted to synthesize the title compound (yield 10%).
LC-MS 396 (M+H), 418(M+Na).

### Example 212

4-tert-butyl-N-{6-[4-(methylsulfonyl)phenyl]imidazo[1,2-b]pyridazin-2-yl}benzamide ditrifluoroacetate

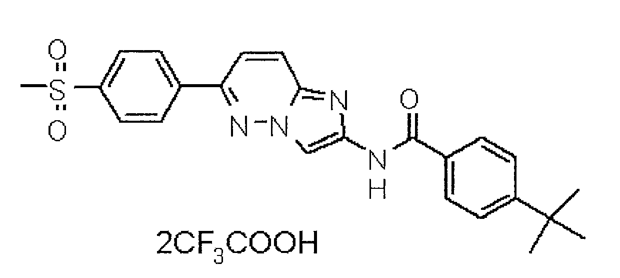

In the same manner as in Example 204, 4-tert-butyl-N-(6-chloroimidazo[1,2-b]pyridazin-2-yl)benzamide and [4-(methylsulfonyl)phenyl]boronic acid were reacted to synthesize the title compound (yield 22%).
LC-MS 449(M+H), 471(M+Na).

### Example 213

4-tert-butyl-N-(6-pyrimidin-5-ylimidazo[1,2-b]pyridazin-2-yl)benzamide ditrifluoroacetate

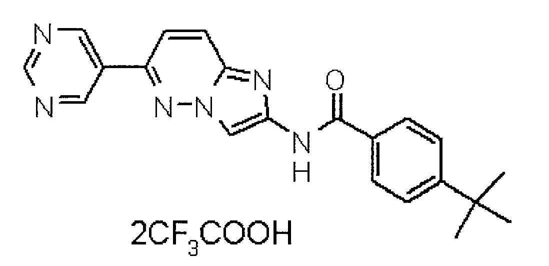

In the same manner as in Example 204, 4-tert-butyl-N-(6-chloroimidazo[1,2-b]pyridazin-2-yl)benzamide and (pyrimidin-5-yl)boronic acid were reacted to synthesize the title compound (yield 3.5%).
LC-MS 373(M+H), 395 (M+Na) .

### Example 214

4-tert-butyl-N-[6-(1H-pyrrol-2-yl)imidazo[1,2-b]pyridazin-2-yl]benzamide formate

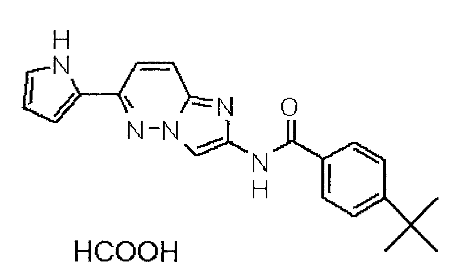

In the same manner as in Example 204, 4-tert-butyl-N-(6-chloroimidazo[1,2-b]pyridazin-2-yl)benzamide and [1-(tert-butoxycarbonyl)-1H-pyrrol-2-yl]boronic acid were reacted. The reaction mixture was purified by preparative HPLC (gradient cycle: B) to synthesize the title compound (yield 3.4%).
LC-MS 360 (M+H), 382 (M+Na).

### Example 215

4-tert-butyl-N-[6-(3-furyl)imidazo[1,2-b]pyridazin-2-yl]benzamide formate

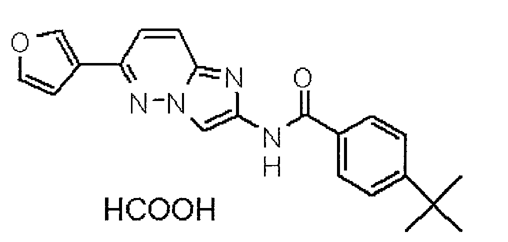

In the same manner as in Example 214, 4-tert-butyl-N-(6-chloroimidazo[1,2-b]pyridazin-2-yl)benzamide and 3-furylboronic acid were reacted to synthesize the title compound (yield 2.5%).
LC-MS 361 (M+H), 383 (M+Na).

### Example 216

N-[6-(1,3-benzodioxol-5-yl)imidazo[1,2-b]pyridazin-2-yl]-4-tert-butylbenzamide formate

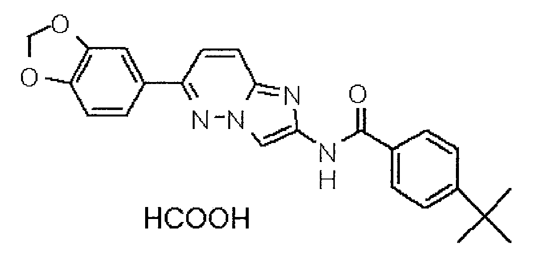

In the same manner as in Example 214, 4-tert-butyl-N-(6-chloroimidazo[1,2-b]pyridazin-2-yl)benzamide and (1,3-benzodioxol-5-yl)boronic acid were reacted to synthesize the title compound (yield 4.3%).
LC-MS 415 (M+H).

### Example 217

4-tert-butyl-N-(6-morpholin-4-ylimidazo[1,2-b]pyridazin-2-yl)benzamide diformate

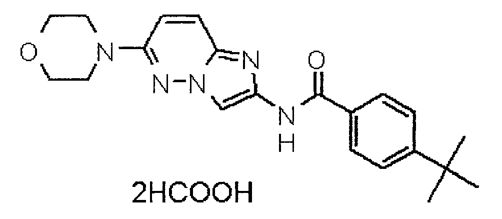

A mixture of 4-tert-butyl-N-(6-chloroimidazo[1,2-b]pyridazin-2-yl)benzamide (33 mg, 0.10 mmol), morpholine (0.2 mL) and N,N-dimethylformamide (3 mL) was stirred in a sealed tube at 180°C for 20 min in a microwave reaction apparatus. The reaction mixture was purified by preparative HPLC to give the title compound (10.1 mg, yield 21%) as a yellow oil.
LC-MS 380(M+H), 402(M+Na).

### Example 218

4-tert-butyl-N-{6-[methyl(pyridin-2-ylmethyl)amino]imidazo[1,2-b]pyridazin-2-yl}benzamide tritrifluoroacetate

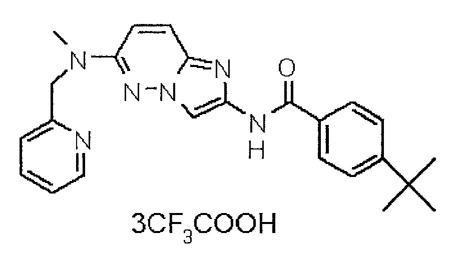

A mixture of 4-tert-butyl-N-(6-chloroimidazo[1,2-b]pyridazin-2-yl)benzamide (33 mg, 0.10 mmol), methyl(pyridin-2-ylmethyl)amine (41 mg) and N,N-dimethylformamide (2 mL) was stirred in a sealed tube at 220°C for 10 min in a microwave reaction apparatus. The reaction mixture was purified by preparative HPLC to give the title compound (7.7 mg, 10%) as a yellow oil.
LC-MS 415(M+H).

### Example 219

4-tert-butyl-N-{6-[methyl(2-pyridin-2-ylethyl)amino]imidazo[1,2-b]pyridazin-2-yl}benzamide tritrifluoroacetate

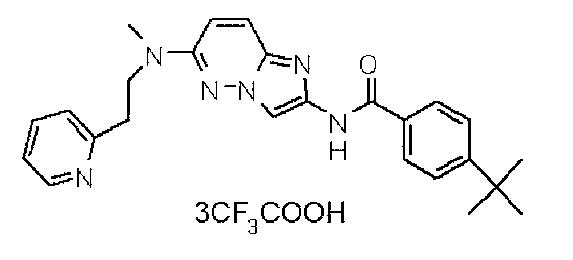

In the same manner as in Example 218, the title compound (yield 10%) was synthesized from 4-tert-butyl-N-(6-chloroimidazo[1,2-b]pyridazin-2-yl)benzamide and methyl (2-pyridin-2-ylethyl)amine.
LC-MS 429(M+H), 451(M+Na).

### Example 220

4-tert-butyl-N-[6-(2-pyridin-2-ylpyrrolidin-1-yl)imidazo[1,2-b]pyridazin-2-yl]benzamide tritrifluoroacetate

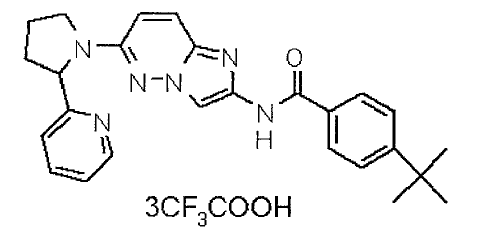

In the same manner as in Example 218, the title compound (yield 13%) was synthesized from 4-tert-butyl-N-(6-chloroimidazo[1,2-b]pyridazin-2-yl)benzamide and 2-(pyrrolidin-2-yl)pyridine.
LC-MS 441(M+H), 463(M+Na).

### Example 221

4-tert-butyl-N-[6-(2-pyridin-3-ylpyrrolidin-1-yl)imidazo[1,2-b]pyridazin-2-yl]benzamide tritrifluoroacetate

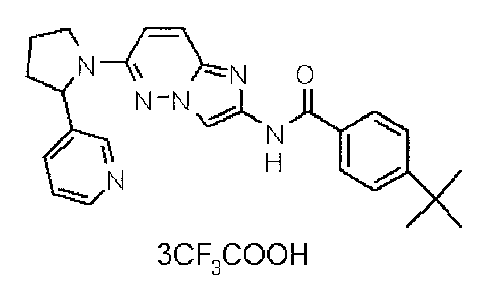

In the same manner as in Example 218, the title compound (yield 1.4%) was synthesized from 4-tert-butyl-N-(6-chloroimidazo[1,2-b]pyridazin-2-yl)benzamide and 3-(pyrrolidin-2-yl)pyridine.
LC-MS 441(M+H), 463(M+Na).

### Example 222

4-tert-butyl-N-[6-(2-pyridin-4-ylpyrrolidin-1-yl)imidazo[1,2-b]pyridazin-2-yl]benzamide tritrifluoroacetate

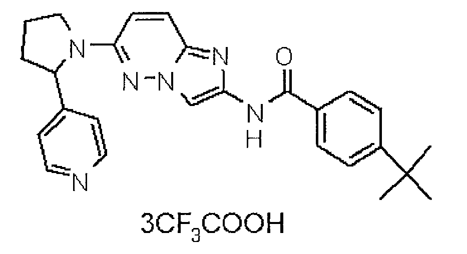

In the same manner as in Example 218, the title compound (yield 0.6%) was synthesized from 4-tert-butyl-N-(6-chloroimidazo[1,2-b]pyridazin-2-yl)benzamide and 4-(pyrrolidin-2-yl)pyridine.
LC-MS 441(M+H), 463(M+Na).

### Example 223

4-tert-butyl-N-{6-[2-(pyridin-3-ylmethyl)pyrrolidin-1-yl]imidazo[1,2-b]pyridazin-2-yl}benzamide tritrifluoroacetate

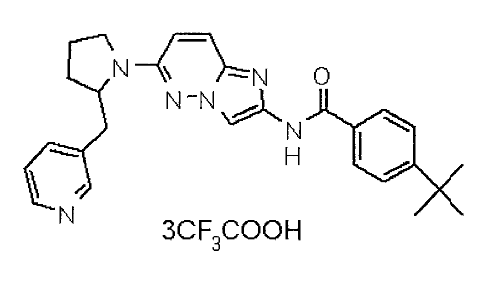

In the same manner as in Example 218, the title compound (yield 5.8%) was synthesized from 4-tert-butyl-N-(6-chloroimidazo[1,2-b]pyridazin-2-yl)benzamide and 3-(pyrrolidin-2-ylmethyl)pyridine.
LC-MS 455(M+H), 477(M+Na).

### Example 224

4-tert-butyl-N-{6-[4-(pyridin-2-ylmethyl)piperazin-1-yl]imidazo[1,2-b]pyridazin-2-yl}benzamide tetratrifluoroacetate

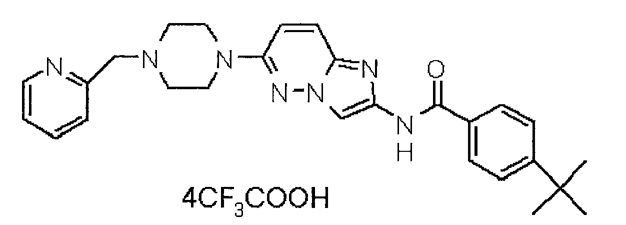

In the same manner as in Example 218, the title compound (yield 14%) was synthesized from 4-tert-butyl-N-(6-chloroimidazo[1,2-b]pyridazin-2-yl)benzamide and 1-(pyridin-2-ylmethyl)piperazine.
LC-MS 470(M+H), 492 (M+Na) .

### Example 225

4-tert-butyl-N-{6-[4-(pyridin-3-ylmethyl)piperazin-1-yl]imidazo[1,2-b]pyridazin-2-yl}benzamide tetratrifluoroacetate

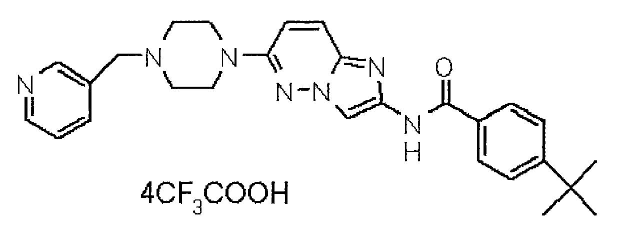

In the same manner as in Example 218, the title compound (yield 24%) was synthesized from 4-tert-butyl-N-(6-chloroimidazo[1,2-b]pyridazin-2-yl)benzamide and 1-(pyridin-3-ylmethyl)piperazine.
¹HNMR (CDCl₃) δ: 1.36 (9H, s), 3.20-3.27 (4H, m), 3.85-3.95 (4H, m), 4.32 (2H, s), 7.14 (1H, d, J = 10.0 Hz), 7.55 (2H, d, J = 8.7 Hz), 7.88 (1H, dd, J = 7.9, 5.3 Hz), 7.99 (1H, d, J = 9.6 Hz), 8.07 (2H, d, J = 8.5 Hz), 8.47 (1H, s), 8.49 (1H, s), 8.81 (1H, dd, J = 5.6, 1.2 Hz), 9.11 (1H, d, J = 1.7 Hz), 12.00 (1H, s).
LC-MS 470(M+H), 492(M+Na).

### Example 226

4-tert-butyl-N-{6-[4-(pyridin-4-ylmethyl)piperazin-1-yl]imidazo[1,2-b]pyridazin-2-yl}benzamide tetratrifluoroacetate

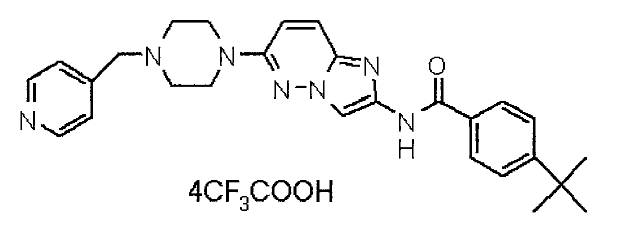

In the same manner as in Example 218, the title compound (yield 17%) was synthesized from 4-tert-butyl-N-(6-chloroimidazo[1,2-b]pyridazin-2-yl)benzamide and 1-(pyridin-4-ylmethyl)piperazine.
LC-MS 470(M+H), 492(M+Na).

### Example 227

In the same manner as in Example 218, 4-tert-butyl-N-{6-[4-(pyridin-2-ylmethyl)piperidin-1-yl]imidazo[1,2-b]pyridazin-2-yl}benzamide tritrifluoroacetate

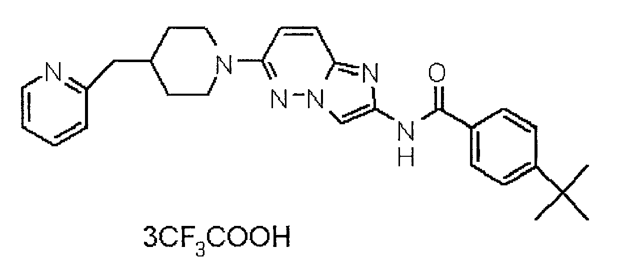

In the same manner as in Example 218, the title compound (yield 7.2%) was synthesized from 4-tert-butyl-N-(6-chloroimidazo[1,2-b]pyridazin-2-yl)benzamide and 4-(pyridin-2-ylmethyl)piperidine.
LC-MS 469(M+H), 491(M+Na).

### Example 228

4-tert-butyl-N-{6-[4-(pyridin-3-ylmethyl)piperidin-1-yl]imidazo[1,2-b]pyridazin-2-yl}benzamide tritrifluoroacetate

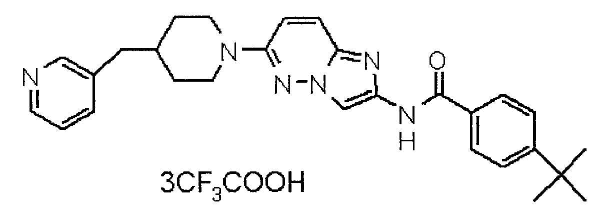

In the same manner as in Example 218, the title compound (yield 5.2%) was synthesized from 4-tert-butyl-N-(6-chloroimidazo[1,2-b]pyridazin-2-yl)benzamide and 4-(pyridin-3-ylmethyl)piperidine. LC-MS 469(M+H), 491(M+Na).

### Example 229

4-tert-butyl-N-{6-[4-(pyridin-4-ylmethyl)piperidin-1-yl]imidazo[1,2-b]pyridazin-2-yl}benzamide tritrifluoroacetate

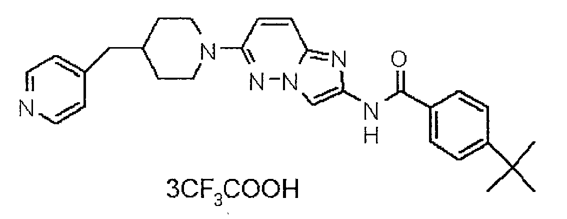

In the same manner as in Example 218, the title compound (yield 3.5%) was synthesized from 4-tert-butyl-N-(6-chloroimidazo[1,2-b]pyridazin-2-yl)benzamide and 4-(pyridin-4-ylmethyl)piperidine. LC-MS 469(M+H), 491(M+Na).

### Example 230

4-tert-butyl-N-{6-[4-(phenylsulfonyl)piperidin-1-yl]imidazo[1,2-b]pyridazin-2-yl}benzamide ditrifluoroacetate

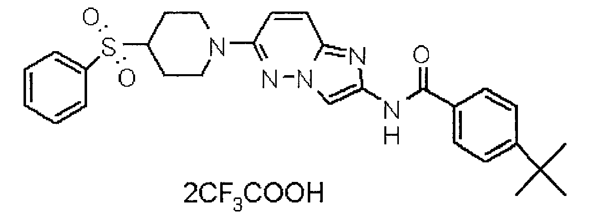

In the same manner as in Example 218, the title compound (yield 2.0%) was synthesized from 4-tert-butyl-N-(6-chloroimidazo[1,2-b]pyridazin-2-yl)benzamide and 4-(phenylsulfonyl)piperidine.
LC-MS 518(M+H).

### Example 231

4-tert-butyl-N-(6-thiomorpholin-4-ylimidazo[1,2-b]pyridazin-2-yl)benzamide

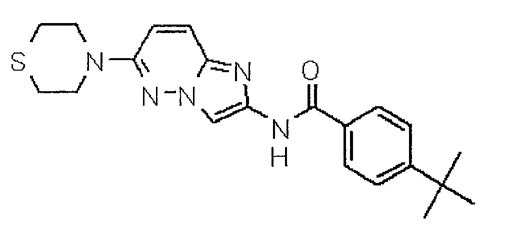

A mixture of 4-tert-butyl-N-(6-chloroimidazo[1,2-b]pyridazin-2-yl)benzamide (165 mg, 0.5 mmol), thiomorpholine (31 mg, 0.3 mmol) and 1,3-dimethyl-2-imidazolidinone (2 mL) was stirred with heating at 220°C for 10 min in a microwave reaction apparatus. The reaction mixture was poured into water, and the mixture was extracted with ethyl acetate. The ethyl acetate layer was concentrated, and the residue was purified by silica gel column chromatography (ethyl acetate:hexane=1:9 - 4:1, volume ratio) to give the title compound (90 mg, 46%). pale-yellow crystal.
¹H-NMR (CDCl₃) δ: 1.35 (9H, s), 2.69-2.76 (4H, m), 3.85-3.91 (4H, m), 6.65 (1H, d, J = 10.0 Hz), 7.20 (1H, d, J = 9.6 Hz), 7.48 (2H, d, J = 8.5 Hz), 7.89 (2H, d, J = 8.5 Hz), 8.32 (1H, s), 9.63 (1H, s).

### Example 232

4-tert-butyl-N-(6-pyrrolidin-1-ylimidazo[1,2-b]pyridazin-2-yl)benzamide ditrifluoroacetate

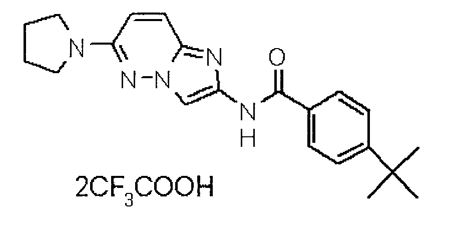

A mixture of 4-tert-butyl-N-(6-chloroimidazo[1,2-b]pyridazin-2-yl)benzamide (33 mg, 0.1 mmol), pyrrolidine (10.5 mg, 0.15 mmol), potassium carbonate (28.0 mg, 0.2 mmol) and N,N-dimethylformamide (2 mL) was stirred with heating at 220°C for 500 sec. The reaction mixture was filtered, and the filtrate was purified by preparative HPLC to give the title compound (11.7 mg, 20%). pale-yellow crystal.
LC-MS 364(M+H).

### Example 233

4-tert-butyl-N-[6-(3-hydroxypyrrolidin-1-yl)imidazo[1,2-b]pyridazin-2-yl]benzamide ditrifluoroacetate

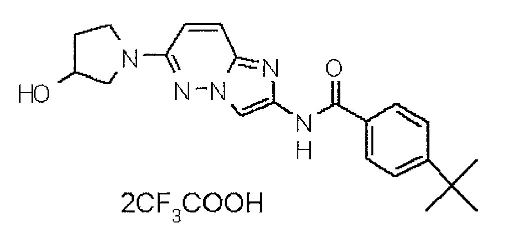

In the same manner as in Example 232, the title compound (yield 15%) was synthesized from 4-tert-butyl-N-(6-chloroimidazo[1,2-b]pyridazin-2-yl)benzamide and 3-hydroxypyrrolidine. pale-yellow crystal.
LC-MS 380(M+H).

### Example 234

4-tert-butyl-N-[6-(pyrrolidin-3-yloxy)imidazo[1,2-b]pyridazin-2-yl]benzamide ditrifluoroacetate

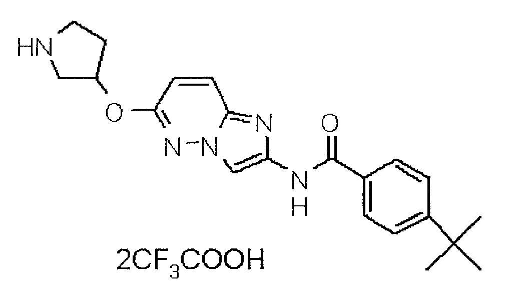

The title compound (yield 10%) was synthesized as a byproduct of the reaction in Example 233. pale-yellow crystal.
LC-MS 380(M+H).

### Example 235

4-tert-butyl-N-[6-(4-hydroxypiperidin-1-yl)imidazo[1,2-b]pyridazin-2-yl]benzamide ditrifluoroacetate

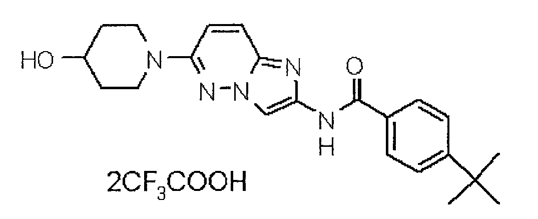

In the same manner as in Example 232, the title compound (yield 8.7%) was synthesized from 4-tert-butyl-N-(6-chloroimidazo[1,2-b]pyridazin-2-yl)benzamide and 4-hydroxypiperidine. pale-yellow crystal.
LC-MS 394(M+H).

### Example 236

4-tert-butyl-N-[6-(piperidin-4-yloxy)imidazo[1,2-b]pyridazin-2-yl]benzamide ditrifluoroacetate

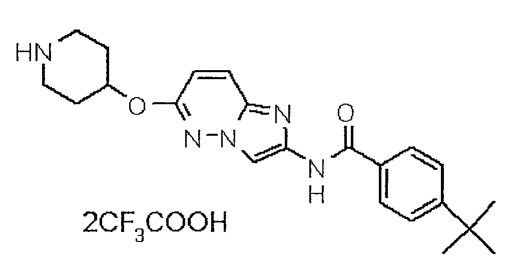

The title compound (yield 6.4%) was synthesized as a byproduct of the reaction in Example 235. pale-yellow crystal. LC-MS 394(M+H).

### Example 237

4-tert-butyl-N-[6-(3-hydroxypiperidin-1-yl)imidazo[1,2-b]pyridazin-2-yl]benzamide ditrifluoroacetate

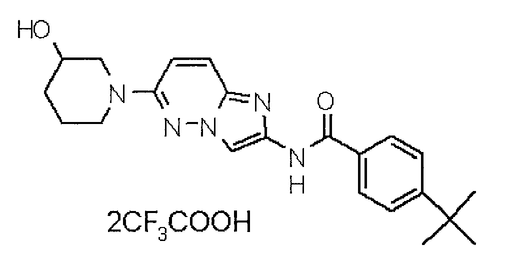

In the same manner as in Example 232, the title compound (yield 9.5%) was synthesized from 4-tert-butyl-N-(6-chloroimidazo[1,2-b]pyridazin-2-yl)benzamide and 3-hydroxypiperidine. pale-yellow crystal.
LC-MS 394(M+H).

### Example 238

4-tert-butyl-N-[6-(piperidin-3-yloxy)imidazo[1,2-b]pyridazin-2-yl]benzamide ditrifluoroacetate

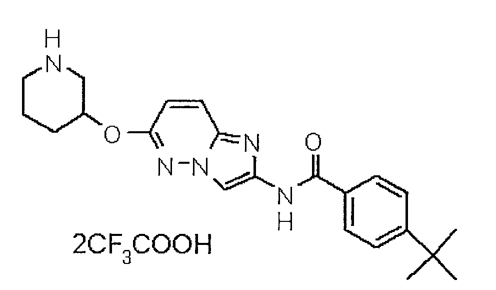

The title compound (yield 5.8%) was synthesized as a byproduct of the reaction in Example 237. pale-yellow crystal. LC-MS 394(M+H).

### Example 239

4-tert-butyl-N-{6-[(2S)-2-(methoxymethyl)pyrrolidin-1-yl]imidazo[1,2-b]pyridazin-2-yl}benzamide ditrifluoroacetate

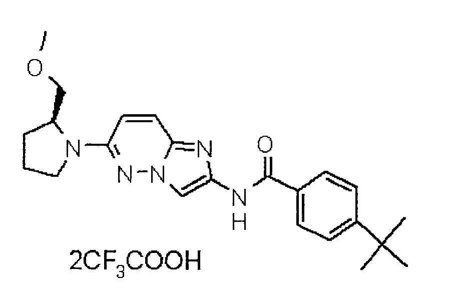

In the same manner as in Example 232, the title compound (yield 2.0%) was synthesized from 4-tert-butyl-N-(6-chloroimidazo[1,2-b]pyridazin-2-yl)benzamide and (2S)-2-(methoxymethyl)pyrrolidine. pale-yellow crystal.
LC-MS 408(M+H).

### Example 240

4-tert-butyl-N-[6-(1,3-dihydro-2H-isoindol-2-yl)imidazo[1,2-b]pyridazin-2-yl]benzamide ditrifluoroacetate

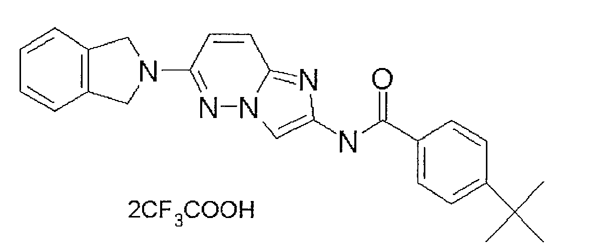

In the same manner as in Example 232, the title compound (yield 2.7%) was synthesized from 4-tert-butyl-N-(6-chloroimidazo[1,2-b]pyridazin-2-yl)benzamide and 1,3-dihydro-2H-isoindole. pale-yellow crystal.
LC-MS 412(M+H).

### Example 241

1-{2-[(4-tert-butylbenzoyl)amino]imidazo[1,2-b]pyridazin-6-yl}piperidine-4-carboxamide ditrifluoroacetate

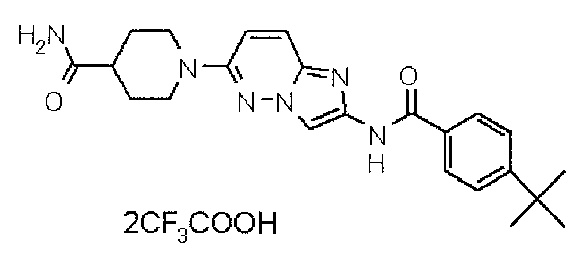

In the same manner as in Example 232, the title compound (yield 5.7%) was synthesized from 4-tert-butyl-N-(6-chloroimidazo[1,2-b]pyridazin-2-yl)benzamide and piperidine-4-carboxamide. pale-yellow crystal.
LC-MS 421(M+H).

### Example 242

1-{2-[(4-tert-butylbenzoyl)amino]imidazo[1,2-b]pyridazin-6-yl}piperidine-3-carboxamide ditrifluoroacetate

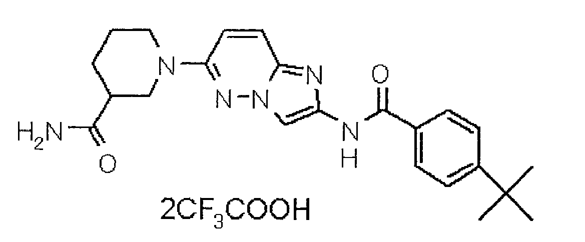

In the same manner as in Example 232, the title compound (yield 4.9%) was synthesized from 4-tert-butyl-N-(6-chloroimidazo[1,2-b]pyridazin-2-yl)benzamide and piperidine-3-carboxamide. pale-yellow crystal.
LC-MS 421(M+H).

### Example 243

N-{6-[3-(acetylamino)pyrrolidin-1-yl]imidazo[1,2-b]pyridazin-2-yl}-4-tert-butylbenzamide ditrifluoroacetate

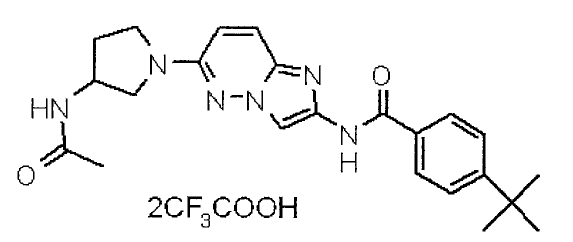

In the same manner as in Example 232, the title compound (yield 11%) was synthesized from 4-tert-butyl-N-(6-chloroimidazo[1,2-b]pyridazin-2-yl)benzamide and 3-(acetylamino)pyrrolidine. pale-yellow crystal.
LC-MS 421(M+H).

### Example 244

4-tert-butyl-N-{6-[2-(2-hydroxyethyl)piperidin-1-yl]imidazo[1,2-b]pyridazin-2-yl}benzamide ditrifluoroacetate

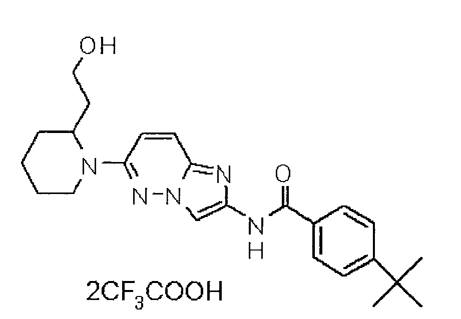

In the same manner as in Example 232, the title compound (yield 26%) was synthesized from 4-tert-butyl-N-(6-chloroimidazo[1,2-b]pyridazin-2-yl)benzamide and 2-(2-hydroxyethyl)piperidine. pale-yellow crystal.
LC-MS 422(M+H).

### Example 245

1-{2-[(4-tert-butylbenzoyl)amino]imidazo[1,2-b]pyridazin-6-yl}piperidine-3-carboxylic acid ditrifluoroacetate

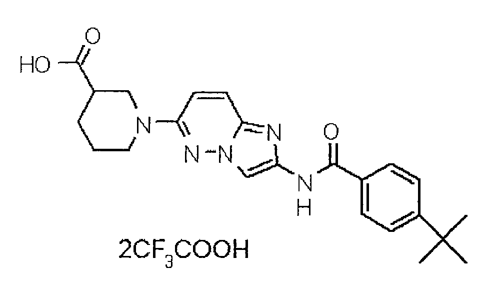

A mixture of 4-tert-butyl-N-(6-chloroimidazo[1,2-b]pyridazin-2-yl)benzamide (33 mg, 0.1 mmol), ethyl piperidine-3-carboxylate (23.0 mg, 0.15 mmol), potassium carbonate (28.0 mg, 0.2 mmol) and N,N-dimethylformamide (2 mL) was stirred with heating at 220°C for 500 sec in a microwave reaction apparatus. The reaction mixture was filtered, and the filtrate was purified by preparative HPLC to give the title compound (6.9 mg, 11%). pale-yellow crystals.
LC-MS 422(M+H).

### Example 246

4-tert-butyl-N-[6-(5-fluoro-1H-indol-1-yl)imidazo[1,2-b]pyridazin-2-yl]benzamide trifluoroacetate

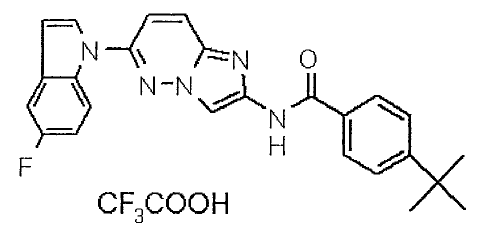

A mixture of 4-tert-butyl-N-(6-chloroimidazo[1,2-b]pyridazin-2-yl)benzamide (33 mg, 0.1 mmol), 5-fluoroindoline (20.5 mg, 0.15 mmol), potassium carbonate (28.0 mg, 0.2 mmol) and N,N-dimethylformamide (2 mL) was stirred with heating at 220°C for 500 sec in a microwave reaction apparatus. The reaction mixture was filtered, and the filtrate was purified by preparative HPLC to give the title compound (2.8 mg, 5.2%). pale-yellow crystal.
LC-MS 428(M+H).

### Example 247

[3-({2-[(4-tert-butylbenzoyl)amino]imidazo[1,2-b]pyridazin-6-yl}oxy)phenoxy]acetic acid trifluoroacetate

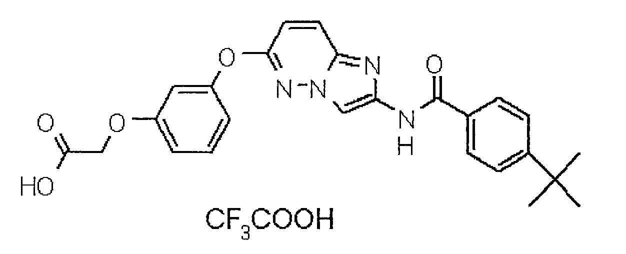

A mixture of 4-tert-butyl-N-(6-chloroimidazo[1,2-b]pyridazin-2-yl)benzamide (33 mg, 0.1 mmol), ethyl (3-hydroxyphenoxy)acetate (29.0 mg, 0.15 mmol), potassium carbonate (28.0 mg, 0.2 mmol) and N,N-dimethylformamide (2 mL) was stirred with heating at 220°C for 500 sec in a microwave reaction apparatus. The reaction mixture was filtered, and the filtrate was purified by preparative HPLC to give the title compound (12.8 mg, 22%). pale-yellow crystal.
LC-MS 461(M+H).

### Examples 248 - 271

In the same manner as in Example 247, various phenol, amine and mercaptane were reacted with 4-tert-butyl-N-(6-chloroimidazo[1,2-b]pyridazin-2-yl)benzamide to give the objective resultant products at a purity of not less than 80% (LC/MS). The structural formulas of the compounds obtained in Examples 248 - 271 and mass spectrum data are shown in Table 3
- Table 6.

**Table 3**

| Ex. No. | Chemical formula | MS(m/Z) |
|---|---|---|
| 248 | 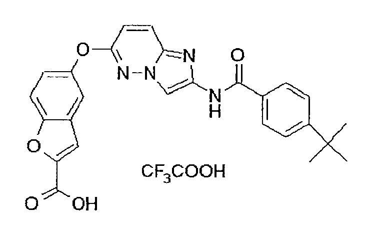 | 471 |
| 249 | 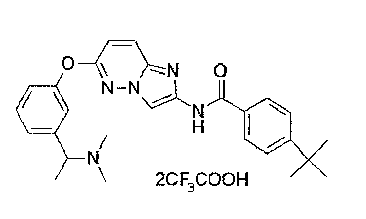 | 458 |
| 250 | 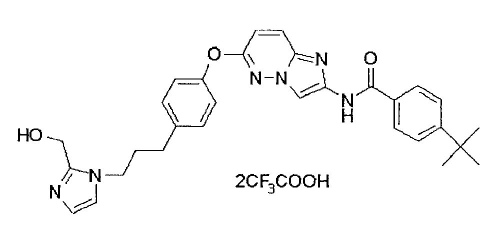 | 525 |
| 251 | 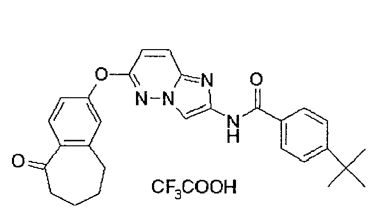 | 469 |
| 252 | 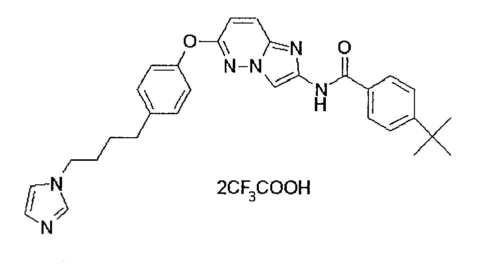 | 509 |
| 253 | 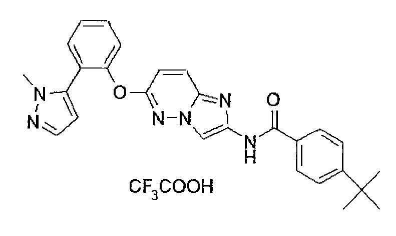 | 467 |

**Table 4**

| Ex. No. | Chemical formula | MS (m/Z) |
|---|---|---|
| 254 | 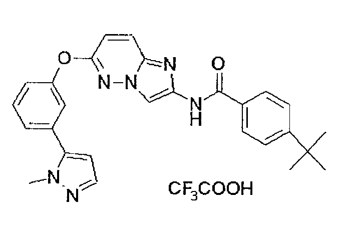 | 467 |
| 255 | 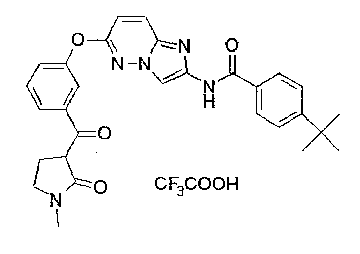 | 512 |
| 256 | 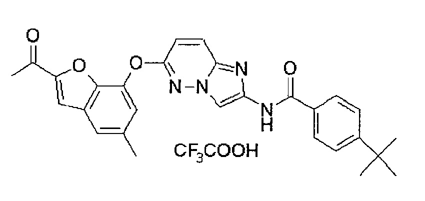 | 483 |
| 257 | 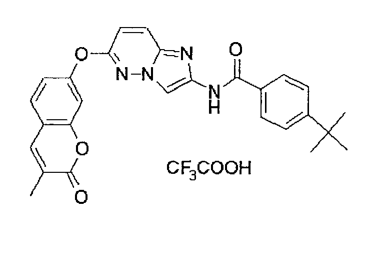 | 469 |
| 258 | 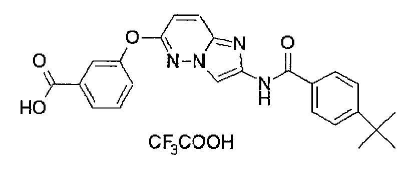 | 431 |
| 259 | 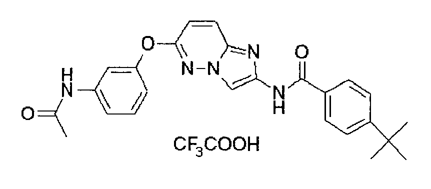 | 444 |

**Table 5**

| Ex. No. | Chemical formula | MS (m/Z) |
|---|---|---|
| 260 | 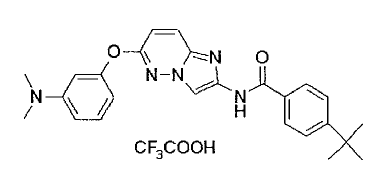 | 430 |
| 261 | 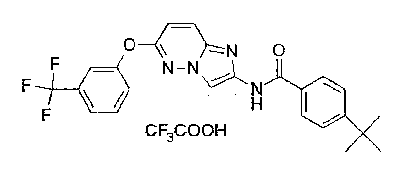 | 455 |
| 262 | 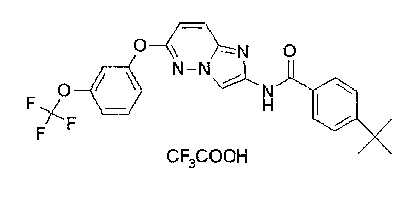 | 471 |
| 263 | 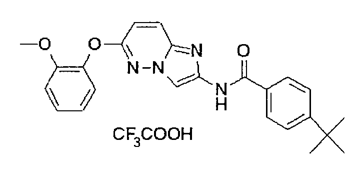 | 417 |
| 264 | 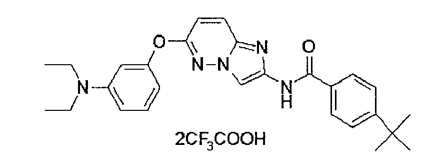 | 458 |
| 265 | 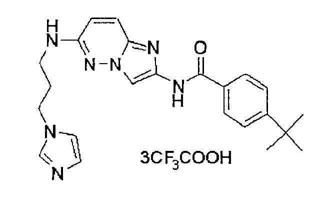 | 418 |

**Table 6**

| Ex. No. | Chemical formula | MS (m/Z) |
|---|---|---|
| 266 | 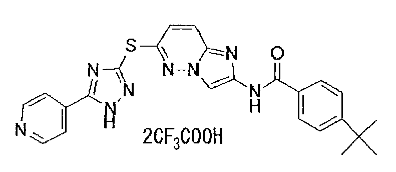 | 471 |
| 267 | 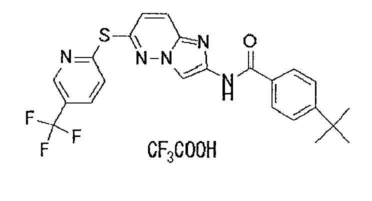 | 472 |
| 268 | 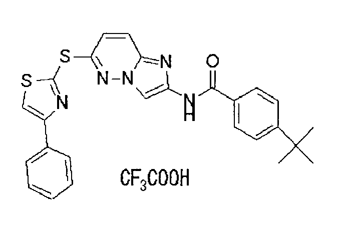 | 486 |
| 269 | 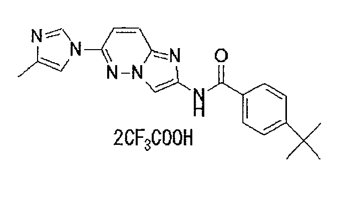 | 375 |
| 270 | 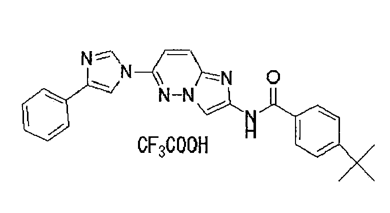 | 437 |
| 271 | 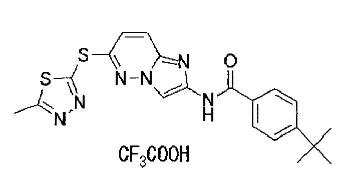 | 425 |

### Example 272

4-tert-butyl-N-[6-(4-tert-butyl-1H-imidazol-1-yl)imidazo[1,2-b]pyridazin-2-yl]benzamide

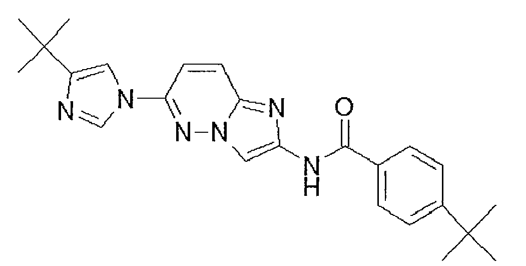

A mixture of 4-tert-butyl-N-(6-chloroimidazo[1,2-b]pyridazin-2-yl)benzamide (164 mg), 4-tert-butylimidazole (124 mg, 1.0 mmol), potassium carbonate (138 mg, 1.0 mmol) and N,N-dimethylformamide (5 mL) was stirred with heating at 180°C for 500 sec in a microwave reaction apparatus. The reaction mixture was poured into water, and the mixture was extracted with ethyl acetate. The organic layer was dried over anhydrous magnesium sulfate and concentrated. The residue was purified by silica gel column chromatography and developed with hexane-ethyl acetate (volume ratio, gradient from 4:1 to 0:1) to give the objective product (70 mg, yield 34%) as colorless crystals. ¹H-NMR (CDCl₃) δ 1.36 (18H, s), 7.17 (1H, d, J = 9.4 Hz), 7.35 (1H, d, J = 1.1 Hz), 7.53 (2H, d, J = 8.5 Hz), 7.68 (1H, d, J = 9.4 Hz), 7.91 (2H, d, J = 8.3 Hz), 8.15 (1H, s), 8.62 (1H, s), 9.23 (1H, s).

### Examples 273 - 278

In the same manner as in Example 195, 6-chloroimidazo[1,2-b]pyridazine-2-amine and various acid chlorides were reacted to synthesize the compounds of Examples 273 - 278. The synthesized compounds are shown in Table 7.

**Table 7**

| Ex. No. | Chemical formula | MS (m/Z) |
|---|---|---|
| 273 | 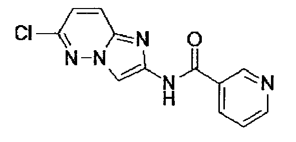 | 274 |
| 274 | 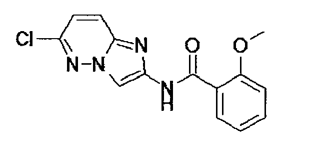 | 303 |
| 275 | 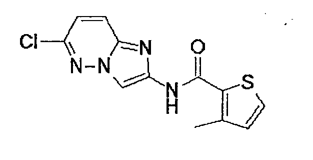 | 293 |
| 276 | 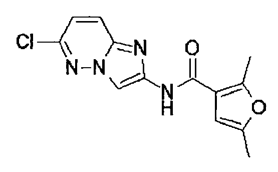 | 291 |
| 277 | 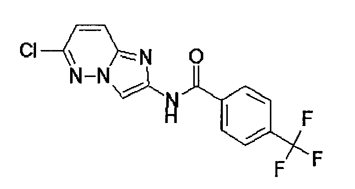 | 341 |
| 278 | 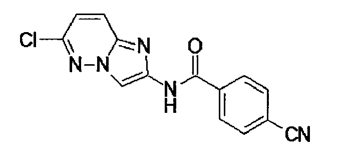 | 298 |

### Example 279

N-(6-chloroimidazo[1,2-b]pyridazin-2-yl)-4-[(dimethylamino)sulfonyl]benzamide

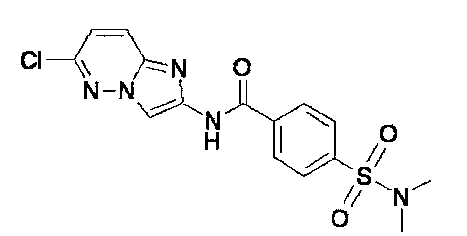

A mixture of 6-chloroimidazo[1,2-b]pyridazine-2-amine (168 mg, 1.0 mmol), 4-[(dimethylamino)sulfonyl]benzoic acid (230 mg, 1.0 mmol), diethyl cyanophosphate (180 mg, 1.1 mmol), triethylamine (110 mg, 1.1 mmol) and N,N-dimethylformamide (3 mL) was stirred at room temperature for 3 hr. The reaction mixture was poured into water, and the mixture was extracted with ethyl acetate. The organic layer was dried over anhydrous magnesium sulfate and concentrated. The residue was purified by silica gel column chromatography and developed with hexane-ethyl acetate (volume ratio, gradient from 4:1 to 0:1) to give the objective product (21 mg, yield 5.5%) as pale-yellow crystals.
¹H-NMR (DMSO-d₆) δ 2.66 (6H, s), 7.40 (1H, d, J = 9.4 Hz), 7.90 (2H, d, J = 8.5 Hz), 8.15 (1H, d, J = 9.4 Hz), 8.30 (2H, d, J = 8.5 Hz), 8.52 (1H, s), 11.77 (1H, s).

### Example 280

diethyl (4-{[(6-chloroimidazo[1,2-b]pyridazin-2-yl)amino]carbonyl}benzyl)phosphate

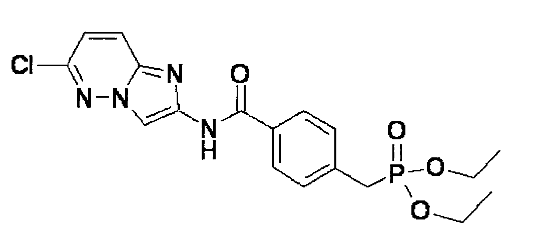

In the same manner as in Example 279, the title compound (yield 12%) was synthesized from 6-chloroimidazo[1,2-b]pyridazine-2-amine and 4-[(diethylphosphonoethyl)methyl]benzoic acid.
LC-MS 423(M+H).

### Example 281

2,5-dimethyl-N-{6-[(1-methyl-1H-imidazol-2-yl)thio]imidazo[1,2-b]pyridazin-2-yl}-3-furamide

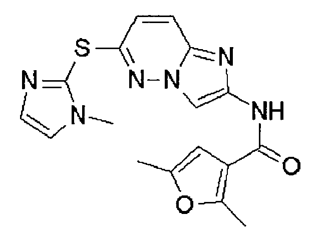

A mixture of N-(6-chloroimidazo[1,2-b]pyridazin-2-yl)-2,5-dimethyl-3-furamide (29 mg, 0.1 mmol), 2-mercapto-1-methylimidazole (23 mg, 0.2 mmol), potassium carbonate (27 mg, 0.2 mmol) and N,N-dimethylformamide (2 mL) was stirred with heating at 180°C for 500 sec in a microwave reaction apparatus. The reaction mixture was filtered, and the filtrate was purified by preparative HPLC to give the title compound (23.3 mg, 63%).
LC-MS 369(M+H).

### Examples 282 - 313

In the same manner as in Example 281, the compounds obtained in Examples 282-313 were reacted with various phenols, amine and mercaptane to give the objective resultant products at a purity of not less than 80% (LC/MS). The structural formulas of the compounds obtained in Examples 282 - 313 and mass spectrum data are shown in Table 8 - Table 11.

**Table 8**

| Ex. No. | Chemical formula | MS (m/Z) |
|---|---|---|
| 282 | 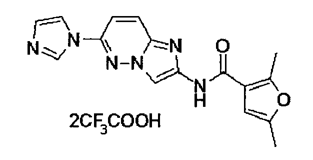 | 323 |
| 283 | 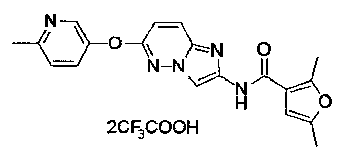 | 364 |
| 284 | 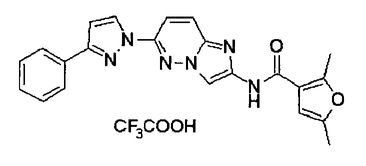 | 399 |
| 285 | 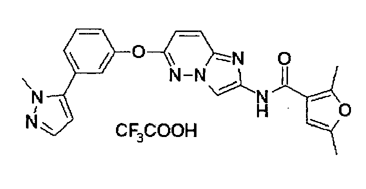 | 429 |
| 286 | 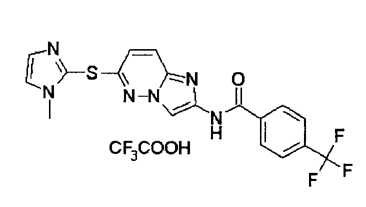 | 419 |
| 287 | 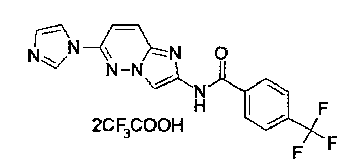 | 373 |
| 288 | 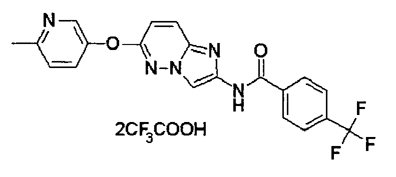 | 414 |
| 289 | 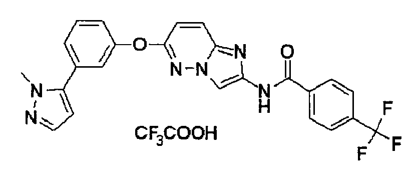 | 479 |

**Table 9**

| Ex. No. | Chemical formula | MS(m/Z) |
|---|---|---|
| 290 | 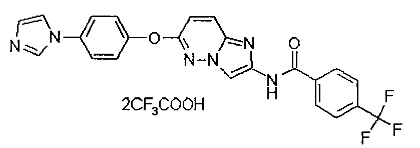 | 465 |
| 291 | 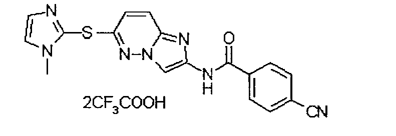 | 376 |
| 292 | 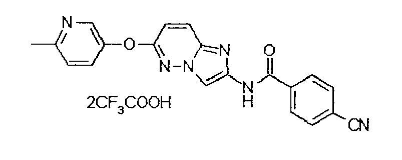 | 371 |
| 293 | 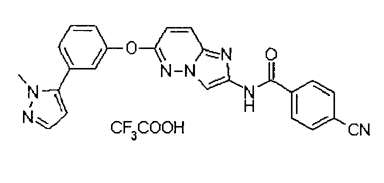 | 436 |
| 294 | 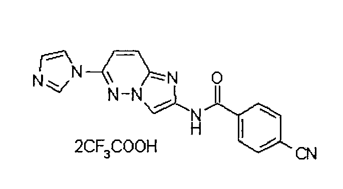 | 330 |
| 295 | 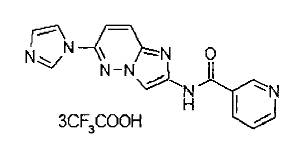 | 306 |
| 296 | 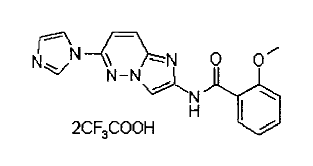 | 335 |
| 297 | 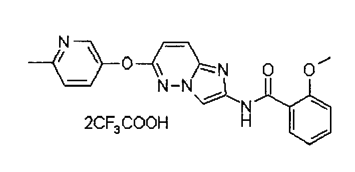 | 376 |

**Table 10**

| Ex. No. | Chemical formula | MS(m/Z) |
|---|---|---|
| 298 | 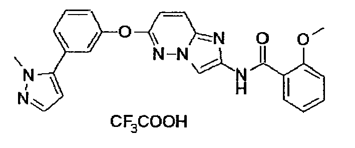 | 441 |
| 299 | 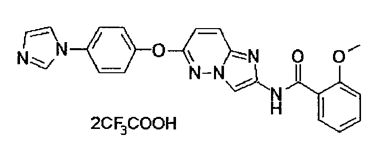 | 427 |
| 300 | 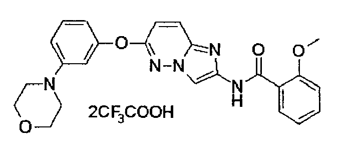 | 446 |
| 301 | 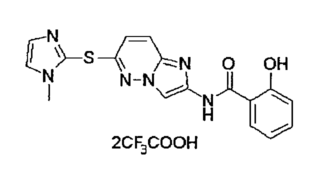 | 367 |
| 302 | 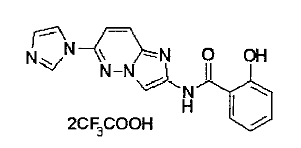 | 321 |
| 303 | 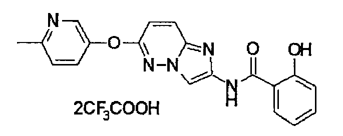 | 362 |
| 304 | 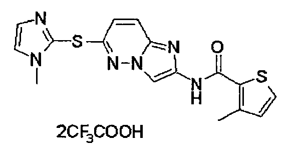 | 371 |
| 305 | 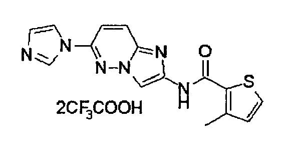 | 325 |

**Table 11**

| Ex. No. | Chemical formula | MS (m/Z) |
|---|---|---|
| 306 | 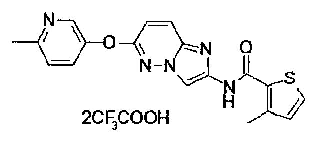 | 366 |
| 307 | 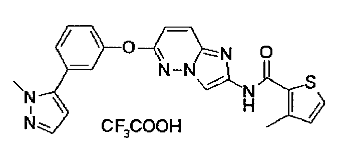 | 431 |
| 308 | 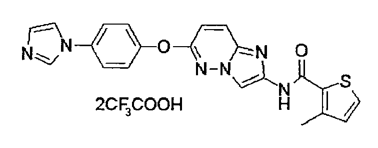 | 417 |
| 309 | 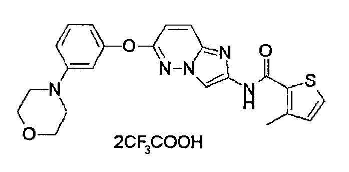 | 436 |
| 310 | 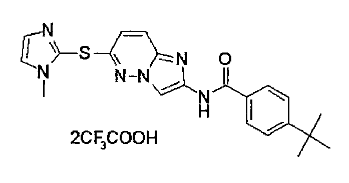 | 407 |
| 311 | 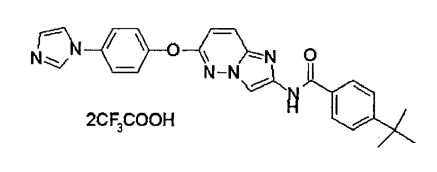 | 453 |
| 312 | 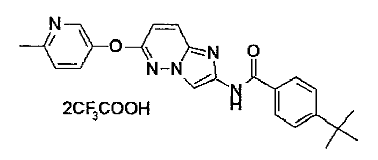 | 402 |
| 313 | 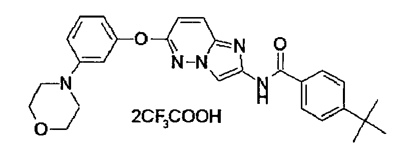 | 472 |

### Example 314

N-(6-iodoimidazo[1,2-b]pyridazin-2-yl)-4-propylbenzamide

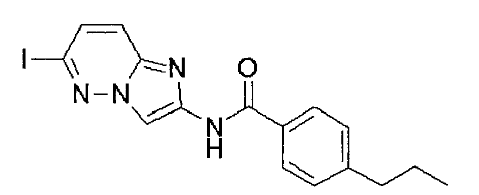

A mixture of 6-iodoimidazo[1,2-b]pyridazine-2-amine (520 mg, 2.0 mmol), 4-propylbenzoyl chloride (390 mg, 2.2 mmol) and N,N-dimethylacetamide (2.5 mL) was stirred at room temperature for 1 hr. The reaction mixture was poured into water, and the precipitated solid was collected by filtration, washed with water then with isopropyl ether to give the title compound (0.72 g, 89%) as pale-yellow crystals.
¹H-NMR (400 MHz, CDCl₃) δ 0.96 (3H, t, J = 7.3 Hz), 1.63-1.73 (2H, m), 2.66 (2H, t, J = 7.7 Hz), 7.17-7.32 (4H, m), 7.87 (2H, d, J = 8.3 Hz), 8.63 (1H, s), 9.38 (1H, s).

In the same manner as in Example 314, the compounds of Examples 315 - 317 were synthesized.

### Example 315

3-trifluoromethyl-N-(6-iodoimidazo[1,2-b]pyridazin-2-yl)benzamide

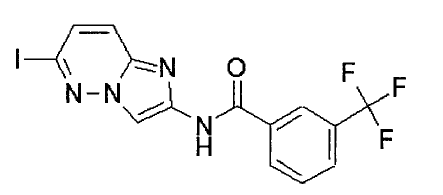

yield 91%
¹H-NMR (CDCl₃) δ 7.12 (1H, d, J = 9.3 Hz), 7.24-7.31 (1H, m), 7.62 (1H, t, J = 7.8 Hz), 7.82 (1H, d, J = 7.8 Hz), 8.16 (1H, d, J = 7.8 Hz), 8.24 (1H, s), 8.66 (1H, s), 10.05 (1H, s).

### Example 316

N-(6-iodoimidazo[1,2-b]pyridazin-2-yl)nicotinamide

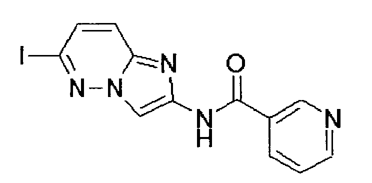

yield 78%
¹H-NMR (DMSO-d₆) δ 7.49-7.60 (2H, m), 7.80 (1H, d, J = 9.3 Hz), 8.40 (1H, d, J = 8.1 Hz), 8.49 (1H, s), 8.76 (1H, d, J = 3.7 Hz), 9.20 (1H, d, J = 1.7 Hz).

### Example 317

2-chloro-N-(6-iodoimidazo[1,2-b]pyridazin-2-yl)benzamide

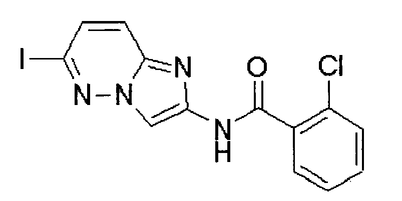

yield87%
¹H-NMR (CDCl₃) δ 7.18 (1H, d, J = 9.3 Hz), 7.29 (1H, d, J = 9.3 Hz), 7.36-7.48 (3H, m), 7.83 (1H, d, J = 7.8 Hz), 8.62 (1H, s), 9. 61 (1H, s).

### Example 318

N-(6-morpholin-4-ylimidazo[1,2-b]pyridazin-2-yl)-4-propylbenzamide ditrifluoroacetate

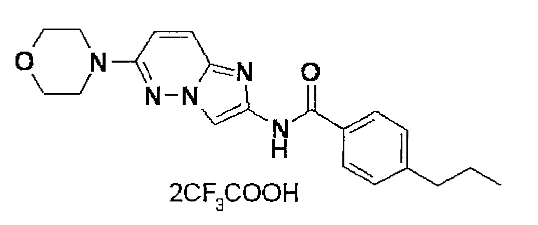

A mixture of N-(6-iodoimidazo[1,2-b]pyridazin-2-yl)-4-propylbenzamide (24 mg, 0.06 mmol), morpholine (43 mg, 0.5 mmol) and 1-methyl-2-pyrrolidone (0.5 mL) was stirred with heating at 230°C for 720 sec in a microwave reaction apparatus. The reaction mixture was purified by preparative HPLC to give the title compound (23.3 mg, 33%).
LC-MS 366(M+H).

### Examples 319 - 398

In the same manner as in Example 318, the reaction was performed to give the object resultant products at a purity of not less than 80% (LC/MS). The structural formulas of the compounds obtained in Examples 319 - 398 and mass spectrum data are shown in Table 12 - Table 21.

**Table 12**

| Ex. No. | Chemical formula | MS (m/Z) |
|---|---|---|
| 319 | 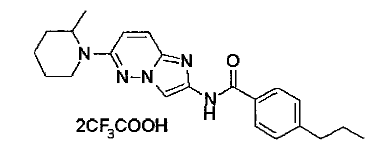 | 378 |
| 320 | 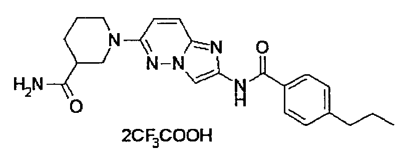 | 407 |
| 321 | 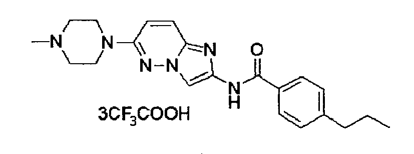 | 379 |
| 322 | 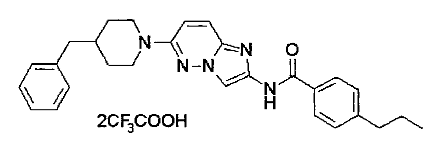 | 454 |
| 323 | 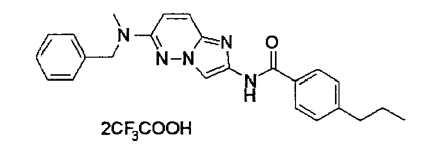 | 400 |
| 324 | 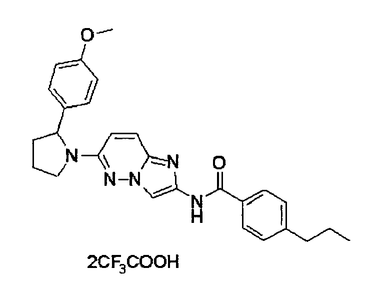 | 456 |
| 325 | 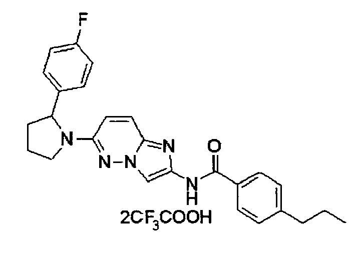 | 444 |

**Table 13**

| Ex. No. | Chemical formula | MS (m/Z) |
|---|---|---|
| 326 | 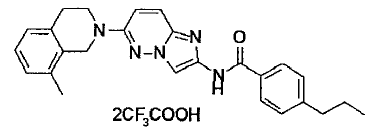 | 426 |
| 327 | 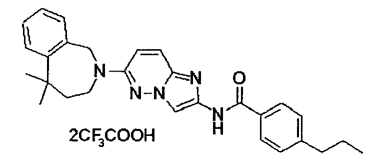 | 454 |
| 328 | 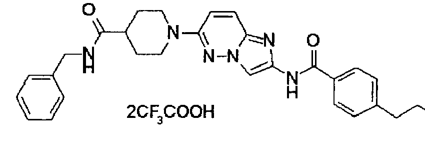 | 497 |
| 329 | 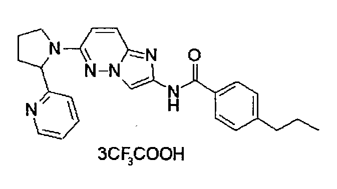 | 427 |
| 330 | 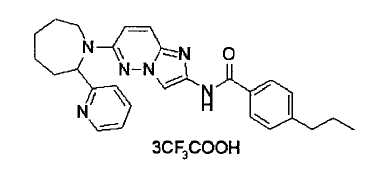 | 455 |
| 331 | 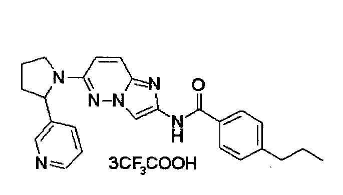 | 427 |
| 332 | 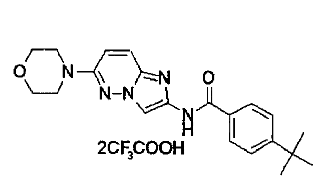 | 380 |
| 333 | 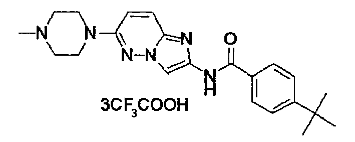 | 393 |

**Table 14**

| Ex. No. | Chemical formula | MS (m/Z) |
|---|---|---|
| 334 | 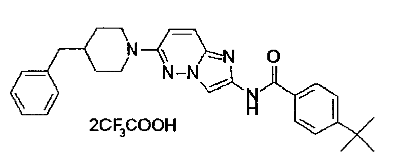 | 468 |
| 335 | 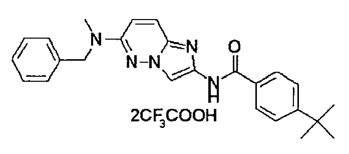 | 414 |
| 336 | 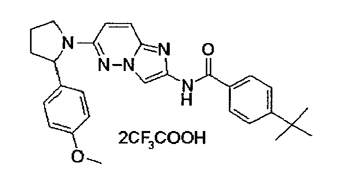 | 470 |
| 337 | 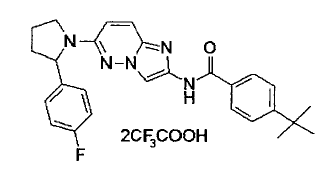 | 458 |
| 338 | 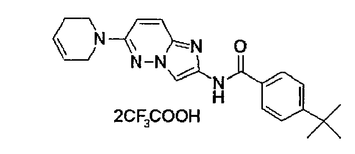 | 376 |
| 339 | 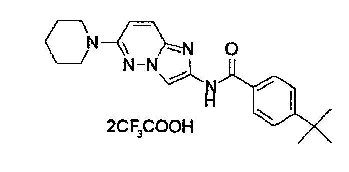 | 378 |
| 340 | 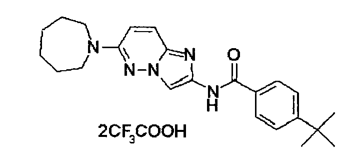 | 392 |
| 341 | 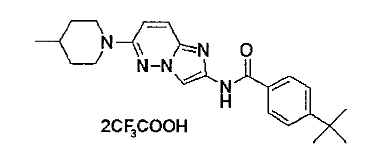 | 392 |

**Table 15**

| Ex. No. | Chemical formula | MS (m/Z) |
|---|---|---|
| 342 | 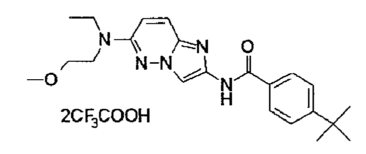 | 396 |
| 343 | 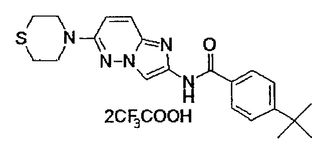 | 396 |
| 344 | 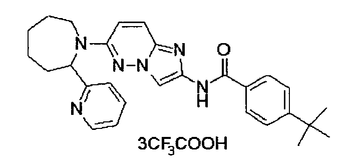 | 469 |
| 345 | 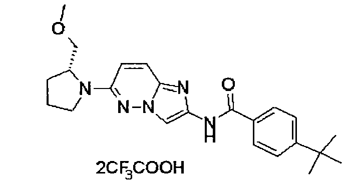 | 408 |
| 346 | 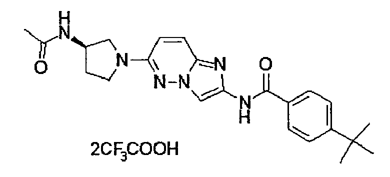 | 421 |
| 347 | 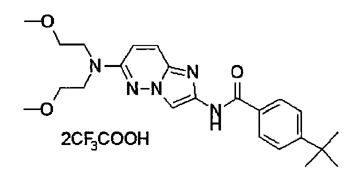 | 426 |
| 348 | 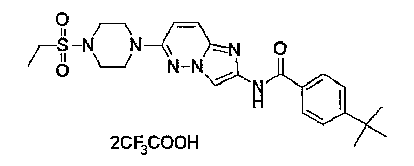 | 471 |
| 349 | 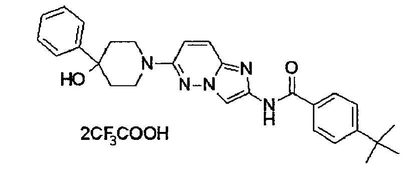 | 470 |

**Table 16**

| Ex. No. | Chemical formula | MS (m/Z) |
|---|---|---|
| 350 | 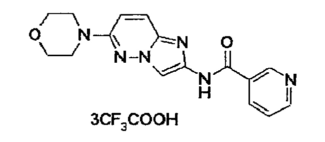 | 325 |
| 351 | 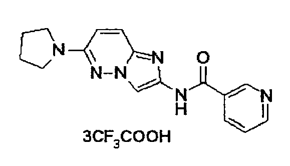 | 309 |
| 352 | 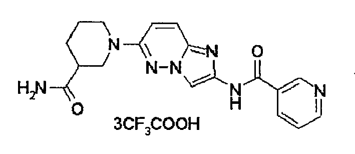 | 366 |
| 353 | 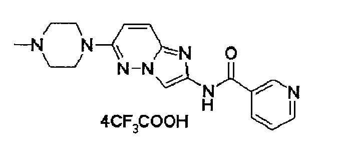 | 338 |
| 354 | 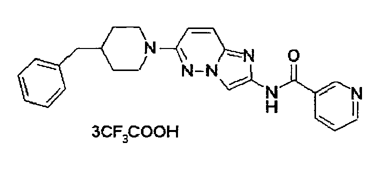 | 413 |
| 355 | 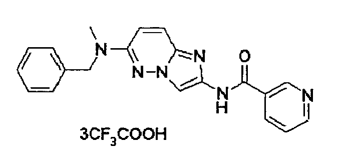 | 359 |
| 356 | 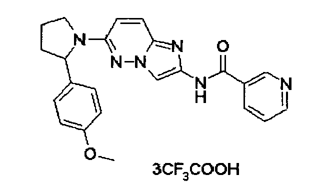 | 415 |
| 357 | 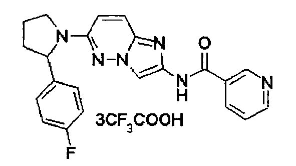 | 403 |

**Table 17**

| Ex. No. | Chemical formula | MS (m/Z) |
|---|---|---|
| 358 | 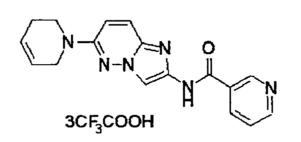 | 321 |
| 359 | 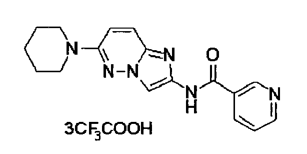 | 323 |
| 360 | 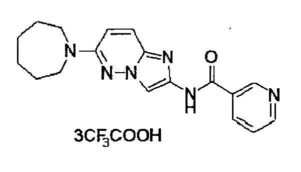 | 337 |
| 361 | 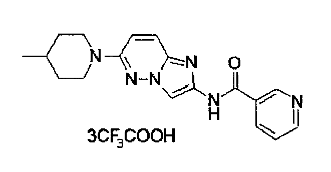 | 337 |
| 362 | 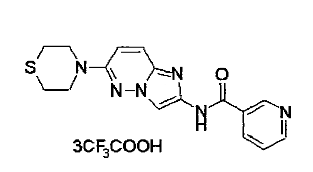 | 341 |
| 363 | 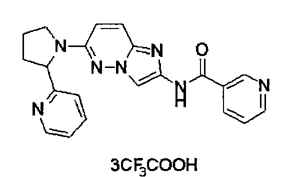 | 386 |
| 364 | 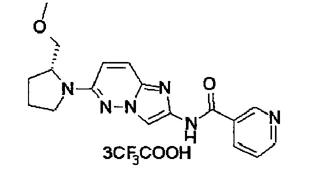 | 353 |
| 365 | 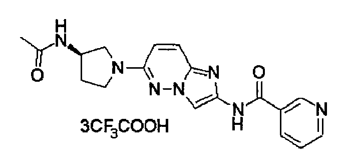 | 366 |

**Table 18**

| Ex. No. | Chemical formula | MS (m/Z) |
|---|---|---|
| 366 | 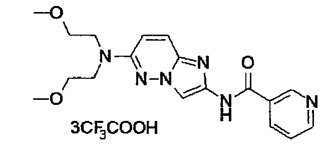 | 371 |
| 367 | 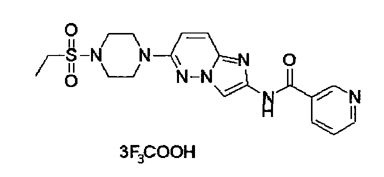 | 416 |
| 368 | 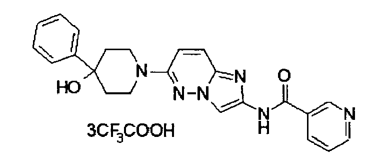 | 415 |
| 369 | 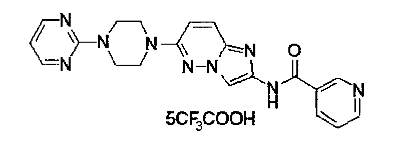 | 402 |
| 370 | 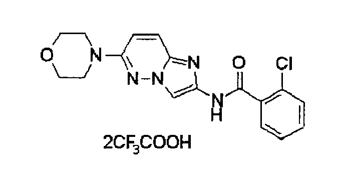 | 358 |
| 371 | 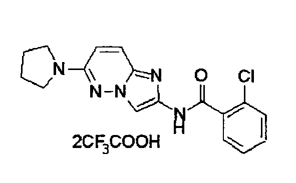 | 342 |
| 372 | 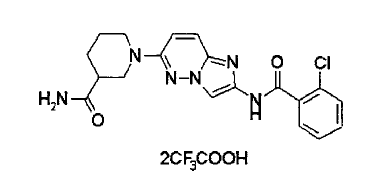 | 399 |
| 373 | 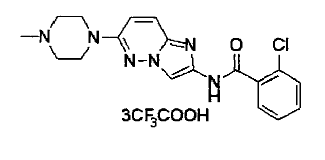 | 371 |

**Table 19**

| Ex. No. | Chemical formula | MS (m/Z) |
|---|---|---|
| 374 | 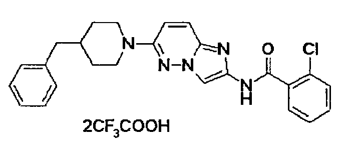 | 446 |
| 375 | 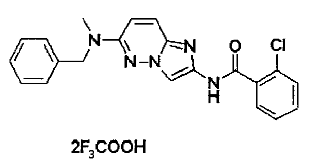 | 392 |
| 376 | 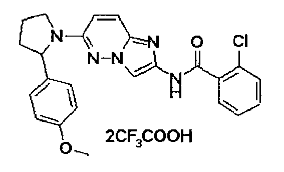 | 448 |
| 377 | 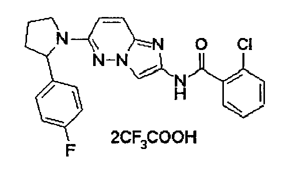 | 436 |
| 378 | 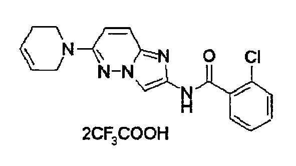 | 354 |
| 379 | 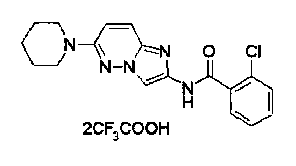 | 356 |
| 380 | 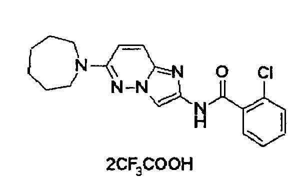 | 370 |
| 381 | 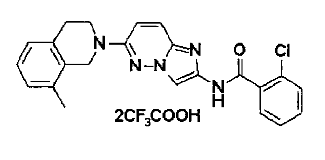 | 418 |

**Table 20**

| Ex. No. | Chemical formula | MS (m/Z) |
|---|---|---|
| 382 | 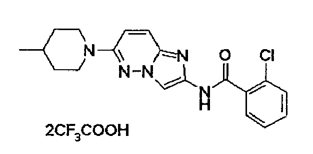 | 370 |
| 383 | 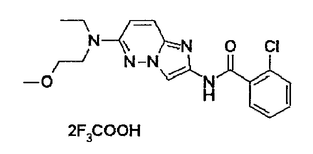 | 374 |
| 384 | 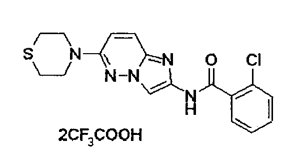 | 374 |
| 385 | 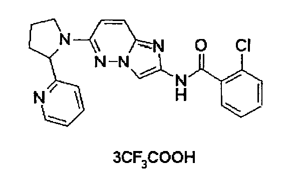 | 419 |
| 386 | 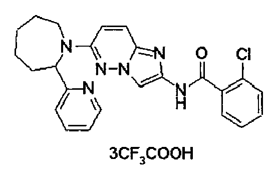 | 447 |
| 387 | 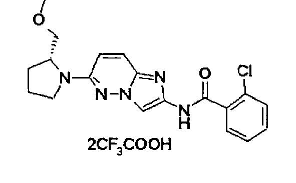 | 386 |
| 388 | 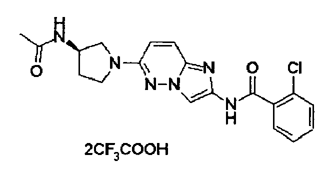 | 399 |
| 389 | 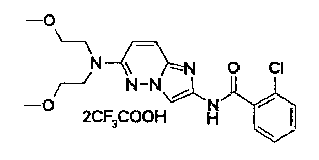 | 404 |

**Table 21**

| Ex. No. | Chemical formula | MS (m/Z) |
|---|---|---|
| 390 | 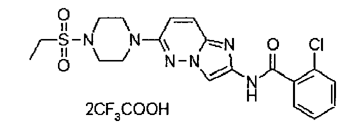 | 449 |
| 391 | 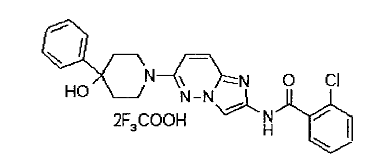 | 448 |
| 392 | 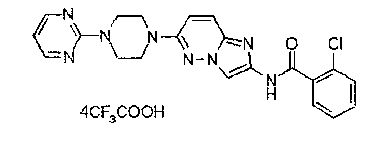 | 435 |
| 393 | 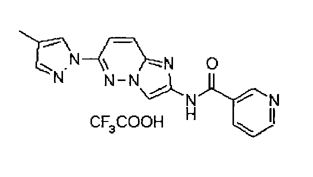 | 320 |
| 394 | 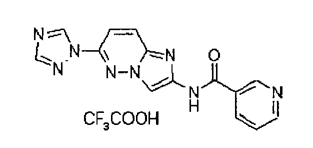 | 307 |
| 395 | 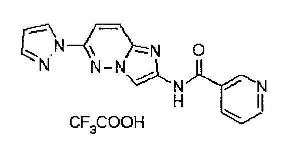 | 306 |
| 397 | 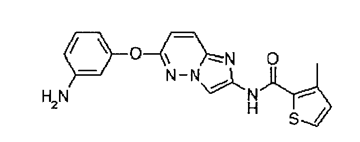 | 366 |
| 398 | 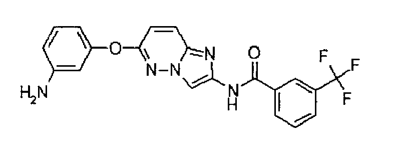 | 414 |

### Example 399

N-(6-{3-[(cyclopropylcarbonyl)amino]phenoxy}imidazo[1,2-b]pyridazin-2-yl)-3-methylthiophene-2-carboxamide

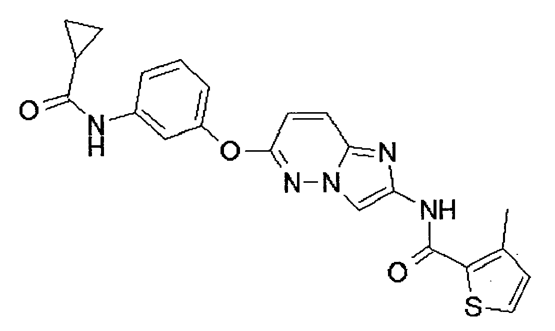

A mixture of N-[6-(3-aminophenoxy)imidazo[1,2-b]pyridazin-2-yl]-3-methylthiophene-2-carboxamide (22 mg, 0.06 mmol), cyclopropanecarbonyl chloride (95 mg, 0.09 mmol) and N,N-dimethylacetamide (0.5 mL) was stirred at room temperature for 14 hr. The reaction mixture was purified by HPLC to give the title compound (11.8 mg, yield 45%).
LC-MS 434(M+H).

### Examples 403-436

In the same manner as in Example 399, various amine forms and acid chloride were reacted to give the objective resultant products at a purity of not less than 80% (LC/MS). The structural formulas of the compounds obtained in Examples 403 - 436 and mass spectrum data are shown in Table 22 - Table 25.

**Table 22**

| Ex. No. | Chemical formula | MS (m/Z) |
|---|---|---|
| 403 | 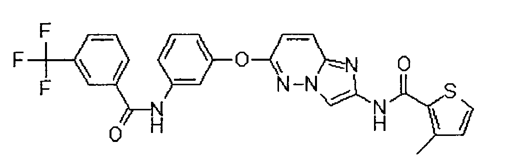 | 538 |
| 404 | 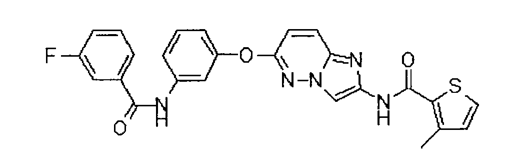 | 488 |
| 405 | 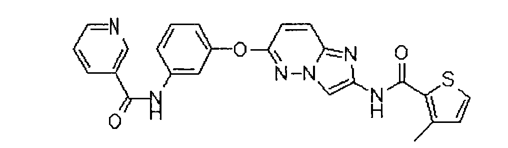 | 471 |
| 406 | 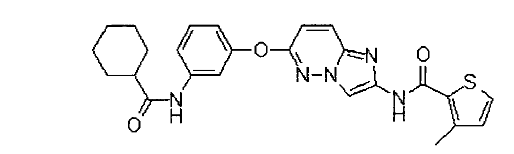 | 476 |
| 407 | 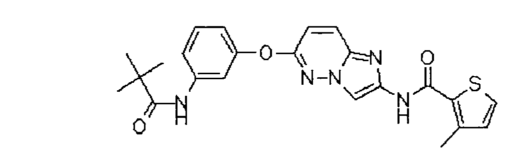 | 450 |
| 408 | 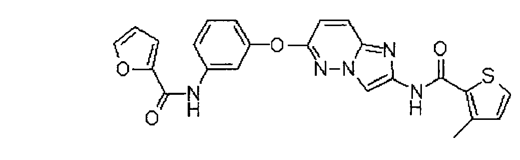 | 460 |

**Table 23**

| Ex. No. | Chemical formula | MS (m/Z) |
|---|---|---|
| 409 | 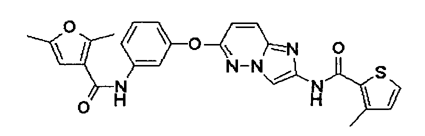 | 488 |
| 410 | 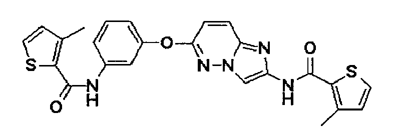 | 490 |
| 411 | 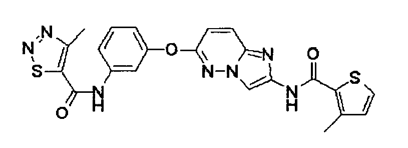 | 492 |
| 412 | 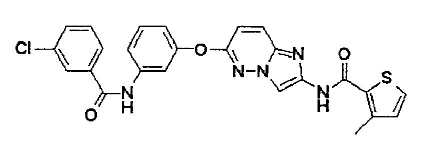 | 504 |
| 413 | 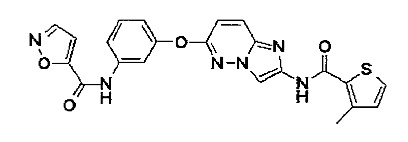 | 461 |
| 414 | 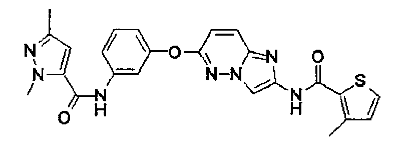 | 488 |
| 415 | 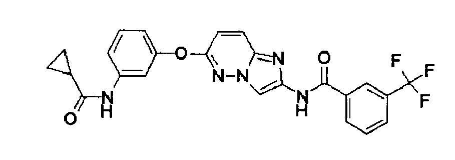 | 482 |
| 416 | 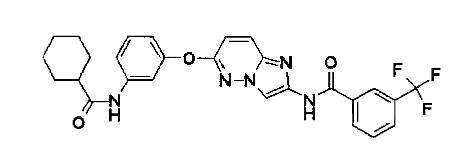 | 524 |
| 417 | 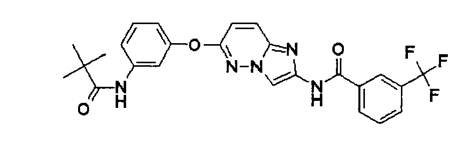 | 498 |

**Table 24**

| Ex. No. | Chemical formula | MS (m/Z) |
|---|---|---|
| 418 | 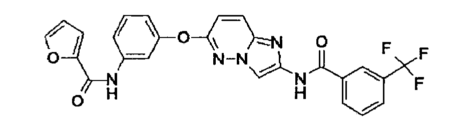 | 508 |
| 419 | 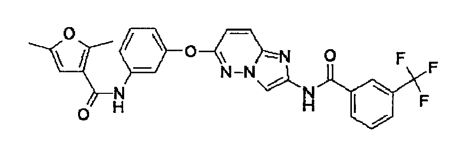 | 536 |
| 420 | 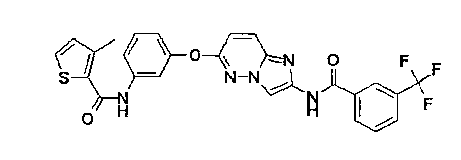 | 538 |
| 421 | 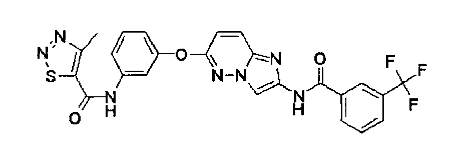 | 540 |
| 422 | 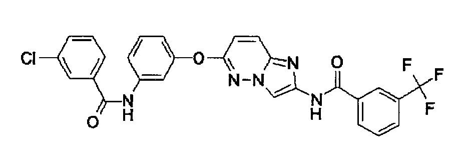 | 552 |
| 423 | 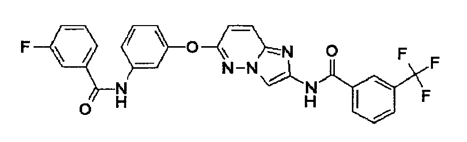 | 536 |
| 424 | 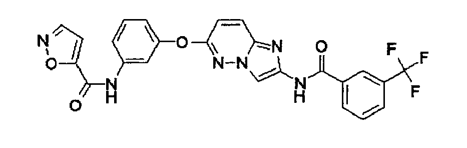 | 509 |
| 425 | 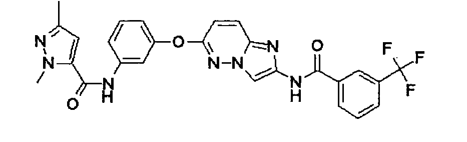 | 536 |
| 426 | 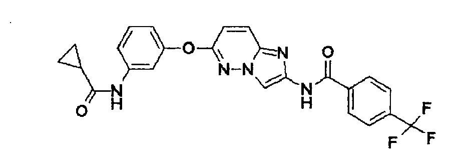 | 482 |

**Table 25**

| Ex. No. | Chemical formula | MS (m/Z) |
|---|---|---|
| 427 | 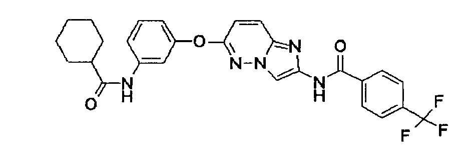 | 524 |
| 428 | 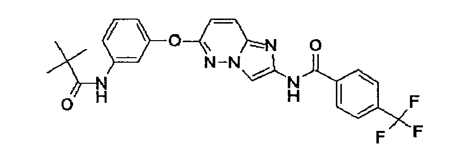 | 498 |
| 429 | 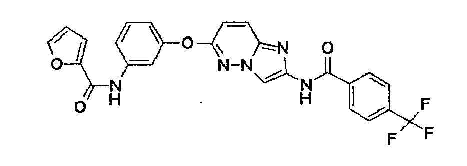 | 508 |
| 430 | 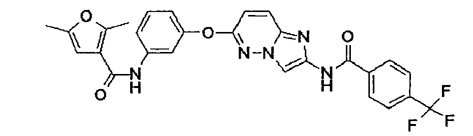 | 536 |
| 431 | 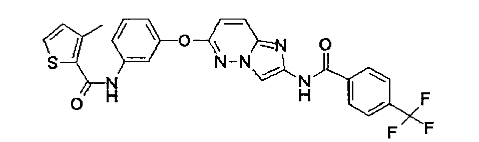 | 538 |
| 432 | 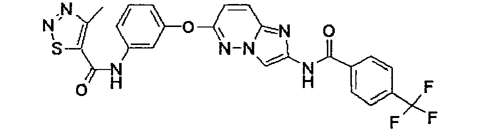 | 540 |
| 433 | 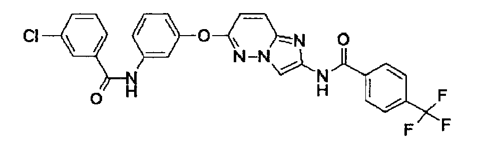 | 552 |
| 434 | 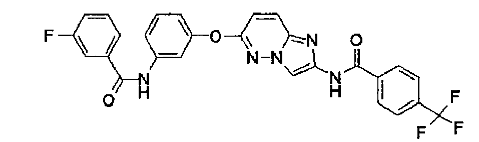 | 536 |
| 435 | 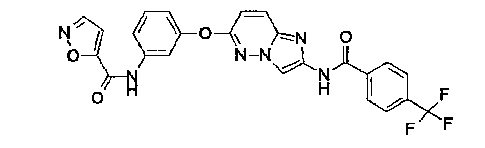 | 509 |
| 436 | 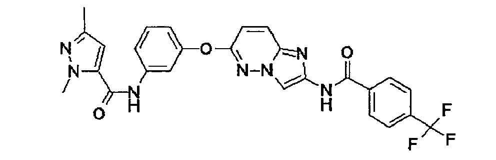 | 536 |

### Example 437

4-[1,1-dimethyl-3-(5-oxo-4,5-dihydro-1,2,4-oxadiazol-3-yl)propyl]-N-[6-(1H-imidazol-1-yl)imidazo[1,2-a]pyridin-2-yl]benzamide

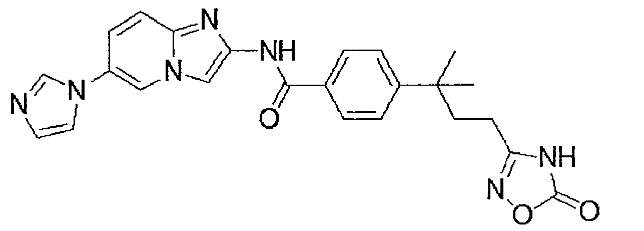

In the same manner as in Example 37 and using 4-(3-cyano-1,1-dimethylpropyl)-N-[6-(1H-imidazol-1-yl)imidazo[1,2-a]pyridin-2-yl]benzamide (0.20 g, 0.50 mmol) synthesized in Example 45, the title compound (yield 64 mg, 28%) was obtained.
melting point 283-284°C
MS (ESI+): 458 (M+H).
¹H-NMR (DMSO-d₆) δ: 1.35 (6H, s), 1.95-2.01 (2H, m), 2.18-2.24 (2H, m), 7.14-7.15 (1H, m), 7.51 (2H, d, J = 8.5 Hz), 7.57 (1H, dd, J = 9.5, 2.0 Hz), 7.62 (1H, d, J = 9.5 Hz), 7.69 (1H, t, J = 1.3 Hz), 8.06 (2H, d, J = 8.5 Hz), 8.18-8.19 (1H, m), 8.34 (1H, s), 9.06 (1H, dd, J = 2.0, 1.0 Hz), 11.25 (1H, s), 12.11 (1H, br).
Elemental analysis: for C₂₄H₂₃N₇O₃
Calculated: C, 63.01; H, 5.07; N, 21.43.
Found: C, 62.98; H, 5.10; N, 21.64.

### Example 438

4-(3-cyano-1,1-dimethylpropyl)-N-(6-methylimidazo[1,2-a]pyridin-2-yl)benzamide

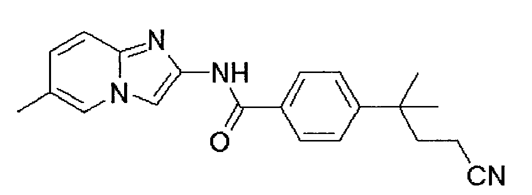

In the same manner as in Example 15 and using 4-(3-cyano-1,1-dimethylpropyl)benzoic acid (1.5 g, 7.0 mmol) synthesized in Reference Example 3 and 6-methylimidazo[1,2-a]pyridine-2-amine (1.0 g, 3.0 mmol) synthesized in Reference Example 26, the title compound (yield 0.57 g, 24%) was obtained.
melting point 194-195°C
MS (ESI+): 347 (M+H).
¹H-NMR (DMSO-d₆) δ: 1.32 (6H, s), 2.00 (2H, t, J = 8.0 Hz), 2.19 (2H, t, J = 8.0 Hz), 2.28 (3H, s), 7.10 (1H, d, J = 9.1 Hz), 7.36 (1H, d, J = 9.1 Hz), 7.58 (2H, d, J = 8.3 Hz), 8.05 (2H, d, J = 8.3 Hz), 8.19 (1H, s), 8.38 (1H, s), 11.09 (1H, s). Elemental analysis: for C₂₁H₂₂N₄O
Calculated: C, 72.81; H, 6.40; N, 16.17.
Found: C, 72.78; H, 6.36; N, 16.26.

### Example 439

4-[1,1-dimethyl-3-(1H-tetrazol-5-yl)propyl]-N-(6-methylimidazo[1,2-a]pyridin-2-yl)benzamide

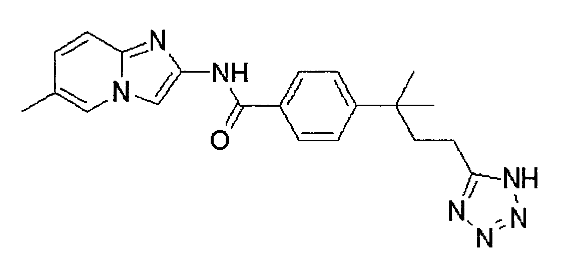

In the same manner as in Example 36 and using 4-(3-cyano-1,1-dimethylpropyl)-N-(6-methylimidazo[1,2-a]pyridin-2-yl)benzamide (0.20 g, 0.58 mmol) synthesized in Example 438, the title compound (yield 56 mg, 25%) was obtained.
melting point 245-246°C
MS (ESI+): 390 (M+H).
¹H-NMR (DMSO-d₆) δ: 1.37 (6H, s), 2.05-2.11 (2H, m), 2.28 (3H, s), 2.55-2.62 (2H, m), 7.10 (1H, dd, J = 9.1, 1.7 Hz), 7.36 (1H, d, J = 9.1 Hz), 7.54 (2H, d, J = 8.3 Hz), 8.19 (1H, s), 8.38 (1H, s), 11.09 (1H, s), 15.91 (1H, br).
Elemental analysis: for C₂₁H₂₃N₇O
Calculated: C, 64.79; H, 5.95; N, 25.18.
Found: C, 64.62; H, 5.95; N, 25.12.

### Example 440

4-[1,1-dimethyl-3-(5-oxo-4,5-dihydro-1,2,4-oxadiazol-3-yl)propyl]-N-(6-methylimidazo[1,2-a]pyridin-2-yl)benzamide

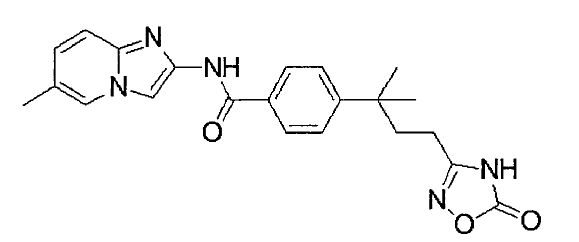

In the same manner as in Example 37 and using 4-(3-cyano-1,1-dimethylpropyl)-N-(6-methylimidazo[1,2-a]pyridin-2-yl)benzamide (0.17 g, 0.50 mmol) synthesized in Example 438, the title compound (yield 35 mg, 17%) was obtained.
melting point 267-268°C
MS (ESI+): 406 (M+H).
¹H-NMR (DMSO-d₆) δ: 1.34 (6H, s), 1.95-2.01 (2H, m), 2.18-2.24 (2H, m), 2.28 (3H, s), 7.10 (1H, dd, J = 9.2, 1.6 Hz), 7.36 (1H, d, J = 9.2 Hz), 7.51 (2H, d, J = 8.5 Hz), 8.05 (2H, d, J = 8.5 Hz), 8.19 (1H, s), 8.38 (1H, s), 11.08 (1H, s), 12.11
(1H, br).
Elemental analysis: for C₂₄H₂₃N₅O₃
Calculated: C, 65.17; H, 5.72; N, 17.27.
Found: C, 65.15; H, 5.57; N, 17.46.

### Example 441

4-(3-cyano-1,1-dimethylpropyl)-N-(1,2-dihydrofuro[2,3-e]imidazo[1,2-a]pyridin-7-yl)benzamide

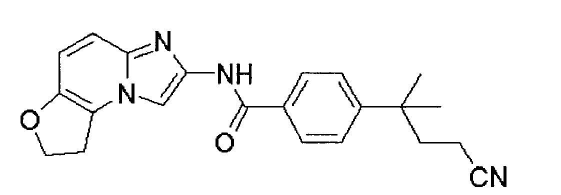

In the same manner as in Example 15 and using 4-(3-cyano-1,1-dimethylpropyl)benzoic acid (0.46 g, 2.1 mmol) synthesized in Reference Example 3 and 1,2-dihydrofuro[2,3-e]imidazo[1,2-a]pyridine-7-amine (0.35 g, 2.0 mmol) synthesized in Reference Example 17, the title compound (yield 0.29 g, 37%) was obtained.
melting point 227-228°C
MS (ESI+): 375 (M+H).
¹H-NMR (DMSO-d₆) δ: 1.32 (6H, s), 1.97-2.04 (2H, m), 2.16-2.21 (2H, m), 3.53 (2H, t, J = 9.1 Hz), 4.76 (2H, t, J = 9.1 Hz), 7.06 (1H, d, J = 9.5 Hz), 7.31 (1H, d, J = 9.5 Hz), 7.51 (2H, d, J = 8.3 Hz), 8.01 (1H, s), 8.06 (2H, d, J = 8.3 Hz), 11.16
(1H, s).
Elemental analysis: for C₂₂H₂₂N₄O₂
Calculated: C, 70.57; H, 5.92; N, 14.96.
Found: C, 70.43; H, 5.89; N, 15.19.

### Example 442

N-(1,2-dihydrofuro[2,3-e]imidazo[1,2-a]pyridin-7-yl)-4-[1,1-dimethyl-3-(5-oxo-4,5-dihydro-1,2,4-oxadiazol-3-yl)propyl]benzamide

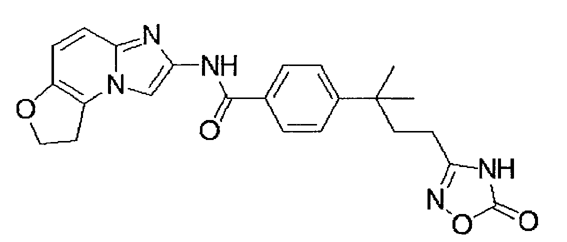

In the same manner as in Example 37 and using 4-(3-cyano-1,1-dimethylpropyl)-N-(1,2-dihydrofuro[2,3-e]imidazo[1,2-a]pyridin-7-yl)benzamide (0.19 g, 0.50 mmol) synthesized in Example 441, the title compound (yield 25 mg, 12%) was obtained.
MS (ESI+): 434 (M+H).
¹H-NMR (DMSO-d₆) δ: 1.34 (6H, s), 1.94-2.01 (2H, m), 2.17-2.24 (2H, m), 3.53 (2H, t, J = 9.1 Hz), 4.76 (2H, t, J = 9.1 Hz), 7.06 (1H, d, J = 9.5 Hz), 7.31 (1H, d, J = 9.5 Hz), 7.51 (2H, d, J = 8.7 Hz), 8.01 (1H, s), 8.07 (2H, d, J = 8.7 Hz), 11.14 (1H, s), 12.09 (1H, br).

### Example 443

4-(3-cyano-1,1-dimethylpropyl)-N-[6-(3-thienyl)imidazo[1,2-a]pyridin-2-yl]benzamide

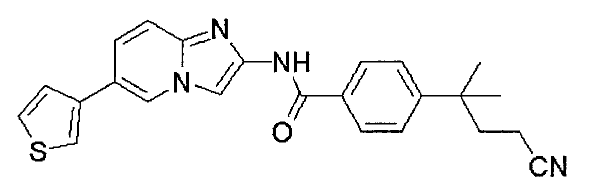

In the same manner as in Example 24 and using N-(6-iodoimidazo[1,2-a]pyridin-2-yl)-4-(3-cyano-1,1-dimethylpropyl)benzamide (0.60 g, 1.3 mmol) synthesized in Example 44 and 3-thienylboronic acid (0.50 g, 3.9 mmol), the title compound (yield 0.44 g, 82%) was obtained.
MS (ESI+): 415 (M+H). ¹H-NMR (DMSO-d₆) δ: 1.33 (6H, s), 1.97-2.04 (2H, m), 2.16-2.22 (2H, m), 7.49-7.53 (3H, m), 7.59 (1H, dd, J = 5.1, 1.9 Hz), 7.67 (1H, dd, J = 9.5, 1.9 Hz), 7.71 (1H, dd, J = 5.1, 3.0 Hz), 7.91 (1H, dd, J = 3.0, 1.1 Hz), 8.07 (2H, d, J = 8.3 Hz), 8.29 (1H, s), 9.03 (1H, s), 11.18 (1H, s).
Elemental analysis: for C₂₄H₂₂N₄OS+0.1H₂O
Calculated: C, 69.24; H, 5.37; N, 13.46.
Found: C, 69.47; H, 5.66; N, 13.08.

### Example 444

4-[1,1-dimethyl-3-(5-oxo-4,5-dihydro-1,2,4-oxadiazol-3-yl)propyl]-N-[6-(3-thienyl)imidazo[1,2-a]pyridin-2-yl]benzamide

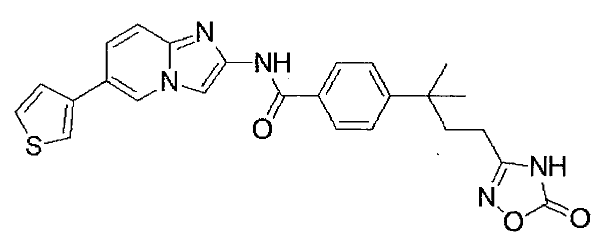

In the same manner as in Example 37 and using 4-(3-cyano-1,1-dimethylpropyl)-N-[6-(3-thienyl)imidazo[1,2-a]pyridin-2-yl]benzamide (0.21 g, 0.50 mmol) synthesized in Example 443, the title compound (yield 90 mg, 38%) was obtained.
MS (ESI+): 474 (M+H).
¹H-NMR (DMSO-d₆) δ: 1.34 (6H, s), 1.95-2.01 (2H, m), 2.18-2.24 (2H, m), 7.49-7.53 (3H, m), 7.59 (1H, dd, J = 5.1, 1.3 Hz), 7.66 (1H, dd, J = 9.3, 1.7 Hz), 7.71 (1H, dd, J = 5.1, 2.8 Hz), 7.91 (1H, dd, J = 2.8, 1.3 Hz), 8.07 (2H, d, J = 8.7 Hz), 8.29 (1H, s), 9.03 (1H, s), 11.16 (1H, s), 12.10 (1H, br).

### Example 445

4-(3-cyano-1,1-dimethylpropyl)-N-[6-(trifluoromethyl)imidazo[1,2-a]pyridin-2-yl]benzamide

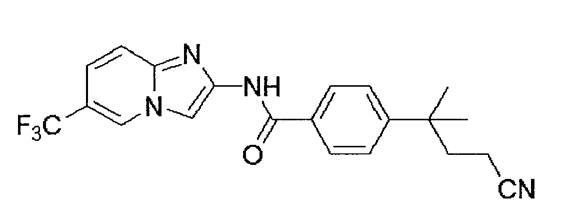

In the same manner as in Example 15 and using 4-(3-cyano-1,1-dimethylpropyl)benzoic acid (1.27 g, 5.9 mmol) synthesized in Reference Example 3 and 6-(trifluoromethyl)imidazo[1,2-a]pyridine-2-amine (1.1 g, 5.3 mmol) synthesized in Reference Example 47, the title compound (yield 0.85 g, 40%) was obtained.
melting point 233-234°C
MS (ESI+): 401 (M+H).
¹H-NMR (DMSO-d₆) δ: 1.32 (6H, s), 1.98-2.03 (2H, m), 2.16-2.21 (2H, m), 7.47 (1H, dd, J = 9.4, 1.9 Hz), 7.51 (2H, d, J = 8.5 Hz), 7.65 (1H, dd, J = 9.4, 0.8 Hz), 8.06 (2H, d, J = 8.3 Hz), 8.47 (1H, s), 9.26-9.28 (1H, m), 11.31 (1H, s).
Elemental analysis: for C₂₁H₁₉N₄OF₃
Calculated: C, 62.99; H, 4.78; N, 13.99.
Found: C, 62.91; H, 4.81; N, 14.09.

### Example 446

4-[1,1-dimethyl-3-(1H-tetrazol-5-yl)propyl]-N-[6-(trifluoromethyl)imidazo[1,2-a]pyridin-2-yl]benzamide

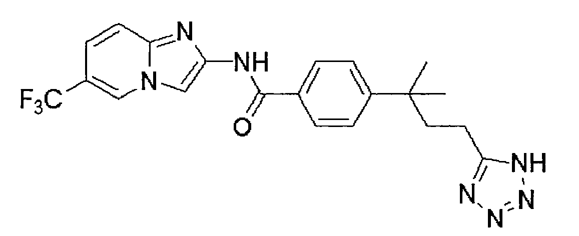

In the same manner as in Example 36 and using 4-(3-cyano-1,1-dimethylpropyl)-N-[6-(trifluoromethyl)imidazo[1,2-a]pyridin-2-yl]benzamide (0.30 g, 0.75 mmol) synthesized in Example 445, the title compound (yield 0.17 g, 50%) was obtained.
melting point 250-251°C
MS (ESI+): 444 (M+H).
¹H-NMR (DMSO-d₆) δ: 1.38 (6H, s), 2.05-2.12 (2H, m), 2.55-2.63 (2H, m), 7.47 (1H, dd, J = 9.5, 1.9 Hz), 7.55 (2H, d, J = 8.3 Hz), 7.66 (1H, d, J = 9.5 Hz), 8.08 (2H, d, J = 8.3 Hz), 8.48 (1H, s), 9.28(1H, s), 11.31 (1H, s).
Elemental analysis: for C₂₁H₂₀N₇OF₃
Calculated: C, 56.88; H, 4.55; N, 22.11.
Found: C, 56.48; H, 4.75; N, 22.48.

### Example 447

4-[1,1-dimethyl-3-(5-oxo-4,5-dihydro-1,2,4-oxadiazol-3-yl)propyl]-N-[6-(trifluoromethyl)imidazo[1,2-a]pyridin-2-yl]benzamide

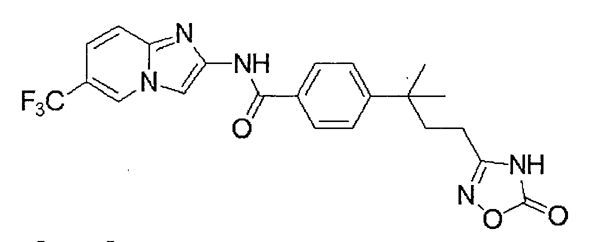

In the same manner as in Example 37 and using 4-(3-cyano-1,1-dimethylpropyl)-N-[6-(trifluoromethyl)imidazo[1,2-a]pyridin-2-yl]benzamide (0.30 g, 0.75 mmol) synthesized in Example 445, the title compound (yield 0.16 g, 47%) was obtained.
melting point 268-270°C
MS (ESI+): 460 (M+H).
¹H-NMR (DMSO-d₆) δ: 1.35 (6H, s), 1.95-2.02 (2H, m), 2.18-2.25 (2H, m), 7.48 (1H, d, J = 9.5 Hz), 7.53 (2H, d, J = 8.3 Hz), 7.66 (1H, d, J = 9.5 Hz), 8.07 (2H, d, J = 8.3 Hz), 8.48 (1H, s), 9.29 (1H, s), 11.30 (1H, s), 12.09 (1H, br).
Elemental analysis: for C₂₂H₂₀N₅O₃F₃
Calculated: C, 57.51; H, 4.39; N, 15.24.
Found: C, 57.44; H, 4.36; N, 15.31.

### Example 448

4-{2-[(2,3-dihydroxypropyl)amino]-1,1-dimethyl-2-oxoethyl}-N-[6-(1H-imidazol-1-yl)imidazo[1,2-a]pyridin-2-yl]benzamide

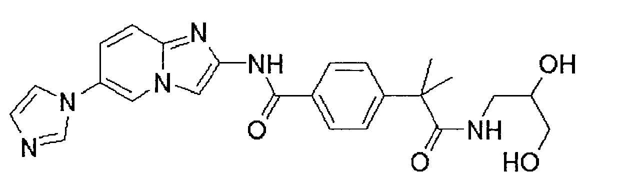

Using 2-(4-{[6-(1H-imidazol-1-yl)imidazo[1,2-a]pyridin-2-yl]carbamoyl}phenyl)-2-methylpropanoic acid (84 mg, 0.21 mmol) synthesized in Example 43, 3-aminopropane-1,2-diol (39.3 mg, 0.43 mmol), WSC (82.7 mg, 0.43 mmol) and HOBt (49.6 mg, 0.32 mmol) and in the same manner as in Example 34, the obtained crude product was purified by basic silica gel chromatography (0-20% methanol/ethyl acetate) to give the title compound (yield 34 mg, 35%).
MS (ESI+): 463 (M+H).
¹H-NMR (DMSO-d₆) δ: 1.50 (6H, s), 2.96-3.09 (1H, m), 3.12-3.28 (3H, m), 3.42-3.57 (1H, m), 4.48 (1H, t, J = 5.7 Hz), 4.70 (1H, d, J = 4.9 Hz), 7.15 (1H, s), 7.28 (1H, t, J = 5.5 Hz), 7.45 (2H, d, J = 8.3 Hz), 7.57 (1H, dd, J = 9.8, 1.9 Hz), 7.63 (1H, d, J = 7.6 Hz), 7.68-7.73 (1H, m), 8.05 (2H, d, J = 8.3 Hz), 8.19 (1H, s), 8.35 (1H, s), 9.04-9.10 (1H, m), 11.27 (1H, s).

### Example 449

4-[2-(glyceroylamino)-1,1-dimethylethyl]-N-[6-(1H-imidazol-1-yl)imidazo[1,2-a]pyridin-2-yl]benzamide

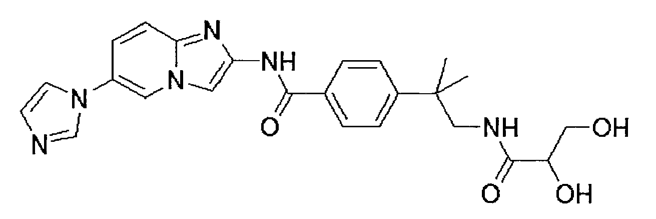

Using 4-(2-amino-1,1-dimethylethyl)-N-[6-(1H-imidazol-1-yl)imidazo[1,2-a]pyridin-2-yl]benzamide (90 mg, 0.24 mmol) obtained in Example 132, glyceric acid (40% aqueous solution 125 mg, 0.48 mmol), WSC (92.2 mg, 0.48 mmol) and HOBt (55.2 mg, 0.36 mmol) and in the same manner as in Example 448, the title compound (yield 42 mg, 39%) was obtained.
MS (ESI+): 463 (M+H).
¹H-NMR (DMSO-d₆) δ: 1.28 (6H, s), 3.36 (2H, d, J = 6.4 Hz), 3.48-3.58 (1H, m), 3.80-3.90 (1H, m), 4.68 (1H, t, J = 5.7 Hz), 5.52 (1H, d, J = 5.3 Hz), 7.15 (1H, s), 7.17 (1H, t, J = 6.4 Hz), 7.53 (2H, d, J = 8.7 Hz), 7.57 (1H, dd, J = 9.5, 1.9 Hz), 7.63 (1H, d, J = 9.5 Hz), 7.70 (1H, t, J = 1.3 Hz), 8.06 (2H, d, J = 8.7 Hz), 8.19 (1H, s), 8.35 (1H, s), 9.07 (1H, s), 11.26 (1H, s).

### Example 450

N-(6-chloroimidazo[1,2-a]pyridin-2-yl)-4-[2-(glyceroylamino)-1,1-dimethylethyl]benzamide

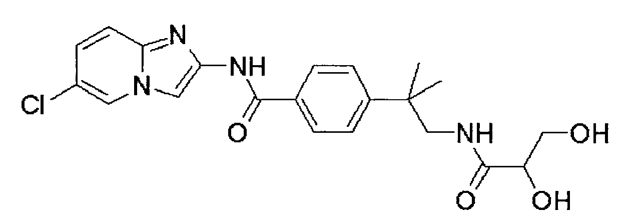

Using 4-(2-amino-1,1-dimethylethyl)-N-(6-chloroimidazo[1,2-a]pyridin-2-yl)benzamide (65.1 mg, 0.19 mmol) obtained in Example 94, glyceric acid (40% aqueous solution 126 mg, 0.48 mmol), WSC (82.2 mg, 0.43 mmol) and HOBt (45 mg, 0.29 mmol) and in the same manner as in Example 448, the title compound (yield 31 mg, 38%) was obtained.
melting point 228-229°C
MS (ESI+): 431 (M+H).
¹H-NMR (DMSO-d₆) δ: 1.28 (6H, s), 3.36 (2H, d, J = 6.4 Hz), 3.41 (1H, t, J = 6.1 Hz), 3.48-3.59 (1H, m), 3.79-3.91 (1H, m), 4.66 (1H, dd, J = 6.1 Hz), 5.50 (1H, d, J = 5.3 Hz), 7.16 (1H, t, J = 6.4 Hz), 7.28 (1H, dd, J = 9.5, 1.9 Hz), 7.50 (1H, d, J = 9.5 Hz), 7.52 (2H, d, J = 8.3 Hz), 8.05 (2H, d, J = 8.3 Hz), 8.34 (1H, s), 8.88 (1H, d, J = 1.9 Hz), 11.23 (1H, s).

### Example 451

N-(6-chloroimidazo[1,2-a]pyridin-2-yl)-4-{2-[(1H-imidazol-5-ylacetyl)amino]-1,1-dimethylethyl}benzamide

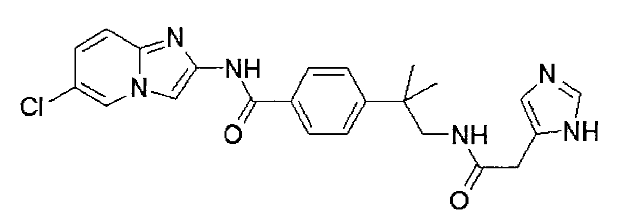

Using 4-(2-amino-1,1-dimethylethyl)-N-(6-chloroimidazo[1,2-a]pyridin-2-yl)benzamide (65.1 mg, 0.19 mmol) synthesized in Example 94, 1H-imidazol-5-ylacetic acid hydrochloride (154 mg, 0.38 mmol), WSC (82.2 mg, 0.43 mmol), HOBt (45 mg, 0.29 mmol) and triethylamine (59 mg, 0.58 mmol) and in the same manner as in Example 448, the title compound (yield 82 mg, 96%) was obtained.
melting point 270°C (decomp.)
MS (ESI+): 451 (M+H).
¹H-NMR (DMSO-d₆) δ: 1.25 (6H, s), 3.16-3.55 (4H, m), 6.60 (0.3H, s), 6.87 (0.7H, brs), 7.28 (1H, dd, J = 9.5, 1.89 Hz), 7.47 (2H, d, J = 8.3 Hz), 7.50 (1H, d, J = 9.5 Hz), 7.54 (1H, brs), 7.69 (1H, t, J = 6.1Hz), 8.02 (2H, d, J= 8.3 Hz), 8.34 (1H, s), 8.88 (1H, d, J = 1.9 Hz), 11.21 (1H, brs), 11. 86 (1H, brs).

### Example 452

4-tert-butyl-N-[6-(trifluoromethyl)imidazo[1,2-a]pyridin-2-yl]benzamide

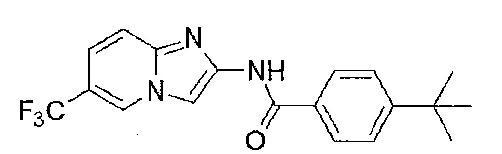

Using 6-(trifluoromethyl)imidazo[1,2-a]pyridine-2-amine (70 mg, 0.35 mmol) synthesized in Reference Example 47, 4-tert-butylbenzoyl chloride (83.8 mg, 0.42 mmol) and triethylamine (53.9 mg, 0.53 mmol) and in the same manner as in Example 14, the obtained crude product was purified by silica gel chromatography (25-30% ethyl acetate/hexane) to give the title compound (yield 80 mg, 64%) as a white solid. melting point 234-235°C
MS (ESI+): 362 (M+H).
¹H-NMR (DMSO-d₆) δ: 1.32, (9H, s), 7.47 (1H, dd, J = 9.5, 1.5 Hz), 7.54 (2H, d, J = 8.3 Hz), 7.66 (1H, d, J = 9.5 Hz), 8.03 (2H, d, J = 8.3 Hz), 8.47 (1H, s), 9.29 (1H, s), 11.29 (1H, s).

### Example 453

N-(6-chloroimidazo[1,2-a]pyridin-2-yl)-4-(2-{[(2,2-dimethyl-1,3-dioxolan-4-yl)methyl]amino}-1,1-dimethyl-2-oxoethyl)benzamide

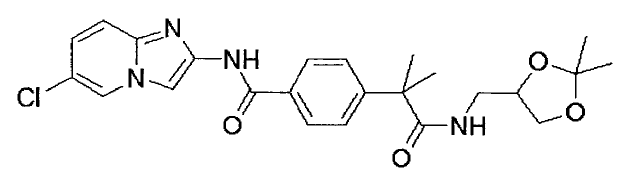

Using 2-{4-[(6-chloroimidazo[1,2-a]pyridin-2-yl)carbamoyl]phenyl}-2-methylpropanoic acid (80 mg, 0.22 mmol) synthesized in Example 116, 1-(2,2-dimethyl-1,3-dioxolan-4-yl)methanamine (74.8 mg, 0.57 mmol), WSC (96.2 mg, 0.50 mmol) and HOBt (52.4 mg, 0.34 mmol) and in the same manner as in Example 448, the obtained crude product was purified by silica gel chromatography (80-100% ethyl acetate/hexane) to give the title compound (yield 83 mg, 79%).
¹H-NMR (DMSO-d₆) δ: 1.23 (3H, s), 1.27 (3H, s), 1.48 (6H, s), 3.04-3.16 (1H, m), 3.19-3.31 (1H, m), 3.54 (1H, dd, J = 8.3, 6.1 Hz), 3.85 (1H, dd, J = 8.3, 6.1 Hz), 3.99-4.11 (3H, m), 7.28 (2H, dd, J = 9.5, 1.9 Hz), 7.43 (5H, d, J = 8.3 Hz), 7.49 (1H, t, J = 5.9 Hz), 7.50 (1H, d, J = 9.5 Hz), 8.04 (2H, d, J = 8.3 Hz),8.88 (1H, d, J = 1.9 Hz), 11.24 (1H, s).

### Example 454

N-(6-chloroimidazo[1,2-a]pyridin-2-yl)-4-{2-[(2,3-dihydroxypropyl)amino]-1,1-dimethyl-2-oxoethyl}benzamide

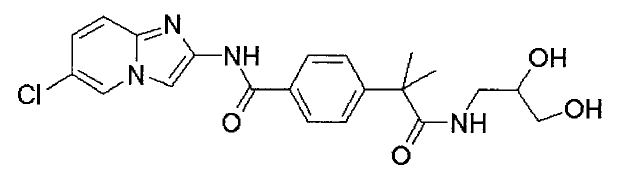

N-(6-Chloroimidazo[1,2-a]pyridin-2-yl)-4-(2-{[(2,2-dimethyl-1,3-dioxolan-4-yl)methyl]amino}-1,1-dimethyl-2-oxoethyl)benzamide (78 mg, 0.16 mmol) synthesized in Example 453 was dissolved in methanol (6 mL), p-toluenesulfonic acid-monohydrate (15.8 mg, 0,08 mmol) was added thereto, and the mixture was stirred at room temperature overnight (18 hr). Then, the solvent was evaporated under reduced pressure, water (10 mL) and saturated aqueous sodium hydrogen carbonate solution (5 mL) were added to the obtained white solid, and the mixture was extracted with a mixed solvent of 10% methanol/ethyl acetate. The organic layer was washed with saturated brine and dried over magnesium sulfate. The solvent was evaporated under reduced pressure, and the obtained crude product was recrystallized from acetonitrile to give the title compound (yield 64 mg, 92%) as white needle crystals.
melting point 198-199°C
MS (ESI+): 431 (M+H).
¹H-NMR (DMSO-d₆) δ: 1.49 (6H, s), 2.96-3.08 (1H, m), 3.11-3.20 (1H, m), 3.23 (2H, t, J = 5.5 Hz), 3.42-3.55 (1H, m), 4.47 (1H, t, J = 5.7 Hz), 4.69 (1H, d, J = 4.9 Hz), 7.27 (1H, t, J = 5.7 Hz), 7.28 (1H, dd, J = 9.5, 1.9 Hz), 7.44 (2H, d, J = 8.3 Hz), 7.50 (1H, d, J = 9.5 Hz), 8.04 (2H, d, J = 8.3 Hz), 8.33 (1H, s), 8.82-8.95 (1H, m), 11.24 (1H, s).

### Example 455

4-(1-cyano-1-methylethyl)-N-[6-(trifluoromethyl)imidazo[1,2-a]pyridin-2-yl]benzamide

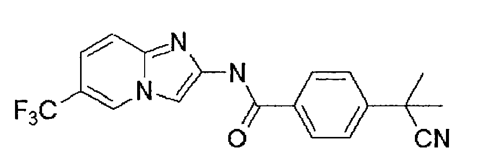

In the same manner as in Example 15 and using 4-(1-cyano-1-methylethyl)benzoic acid (1.0 g, 5.2 mmol) obtained in Reference Example 2 and 6-(trifluoromethyl)imidazo[1,2-a]pyridine-2-amine (0.96 g, 4.7 mmol) obtained in Reference Example 47, the reaction was performed. The obtained crude product was purified by silica gel chromatography (30-40% ethyl acetate/hexane) to give the title compound (yield 1.4 g, 63%).
melting point 230-231°C
MS (ESI+): 373 (M+H).
¹H-NMR (DMSO-d₆) δ: 1.74 (6H, s), 7.48 (1H, dd, J = 9.4, 1.7 Hz), 7.66 (1H, d, J = 9.4 Hz), 7.68 (2H, d, J = 8.7 Hz), 8.15 (2H, d, J =8.7Hz), 8.48 (1H, s), 9.27-9.32 (1H, m), 11.44 (1H, s).

### Example 456

N-[2-methyl-2-(4-{[6-(trifluoromethyl)imidazo[1,2-a]pyridin-2-yl]carbamoyl}phenyl)propyl]-1H-imidazole-4(5)-carboxamide

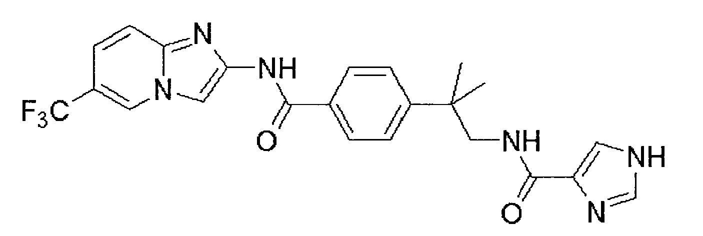

Raney cobalt (300 mg) was washed with water and methanol, and stirred in 2N ammonia/methanol solution together with 4-(1-cyano-1-methylethyl)-N-[6-(trifluoromethyl)imidazo[1,2-a]pyridin-2-yl]benzamide (150 mg, 0.40 mmol) synthesized in Example 455 for 8 hr under a hydrogen atmosphere. The reaction mixture was filtered, and the filtrate was concentrated under reduced pressure to give 4-(2-amino-1,1-dimethylethyl)-N-[6-(trifluoromethyl)imidazo[1,2-a]pyridin-2-yl]benzamide as a white solid. To a solution of the solid was added to a solution of 1H-imidazole-4(5)-carboxylic acid (68.4 mg, 0.61 mmol), WSC (117 mg, 0.61 mmol) and HOBt (74.7 mg, 0.49 mmol) in DMF (5 mL) prepared in advance, and the mixture was stirred at room temperature overnight. Then, water (10 mL) was added to the reaction mixture, and the mixture was extracted with a mixed solvent of 50% THF/ethyl acetate. The organic layer was washed with saturated brine, dried over anhydrous magnesium sulfate, and the solvent was evaporated under reduced pressure to give a brown crude product. This was purified by silica gel chromatography (0-10% methanol/ethyl acetate) to give the title compound (yield 173 mg, yield 92%).
melting point 174-176°C (acetonitrile)
MS (ESI+): 471 (M+H).
¹H-NMR (DMSO-d₆) δ: 1.32 (6H, s), 3.54 (2H, d, J = 6.4 Hz), 7.26 (1H, t, J = 6.4 Hz), 7.47 (1H, dd, J = 9.4, 1.7 Hz,) 7.53-7.62 (3H, m), 7.63-7.71 (2H, m), 8.07 (2H, d, J = 8.5 Hz), 8.48 (1H, s), 9.29 (1H, s), 11.33 (1H, s), 12.46 (1H, brs).

### Example 457

N-[2-methyl-2-(4-{[6-(trifluoromethyl)imidazo[1,2-a]pyridin-2-yl]carbamoyl}phenyl)propyl]-1H-imidazole-2-carboxamide

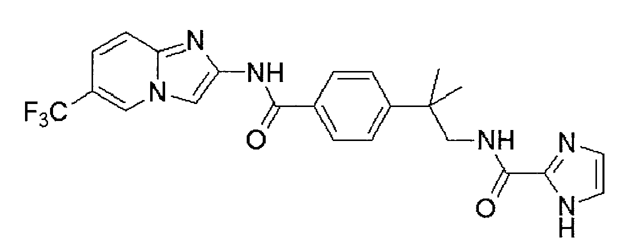

Using 1H-imidazole-2-carboxylic acid (68.4 mg, 0.61 mmol) and in the same manner as in Example 456, the title compound (yield 123 mg, 66%) was obtained.
melting point 292-293°C
MS (ESI+): 471 (M+H).
¹H-NMR (DMSO-d₆) δ: 1.34 (6H, s), 3.54 (2H, d, J = 6.6 Hz), 7.11 (2H, brs) 7.47 (1H, dd, J = 9.4, 1.9 Hz), 7.58 (2H, d, J = 8.5 Hz), 7.66 (1H, d, J = 9.4 Hz), 7.76 (1H, t, J = 6.6 Hz), 8.07 (2H, d, J = 8.5 Hz), 8.48 (1H, s), 9.25-9.33 (1H, m), 11.33 (1H, brs), 13.01 (1H, brs).

### Example 458

4-{2-[(1H-imidazol-5-ylacetyl)amino]-1,1-dimethylethyl}-N-[6-(trifluoromethyl)imidazo[1,2-a]pyridin-2-yl]benzamide

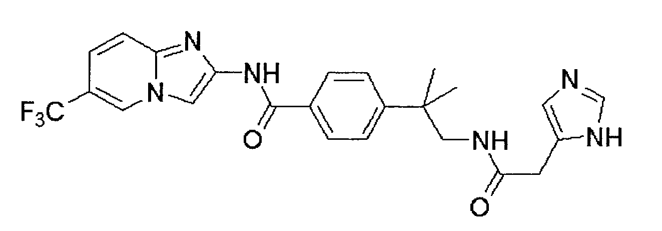

Using 1H-imidazol-4-ylacetic acid hydrochloride (362 mg, 0.89 mmol) and triethylamine (138 mg, 1.37 mmol) and in the same manner as in Example 456, a crude product was obtained and purified by basic silica gel chromatography (0-10% methanol/ethyl acetate) to give the title compound (yield 67 mg, 31%).
melting point 261-263°C
MS (ESI+): 485 (M+H).
¹H-NMR (DMSO-d₆) δ: 1.26 (6H, s), 3.28-3.37 (4H, m), 6.80 (1H, s), 7.44-7.56 (4H, m), 7.66 (1H, d, J = 9.2 Hz), 7.69 (1H, t, J = 6.1 Hz), 8.03 (2H, d, J = 8.5 Hz), 8.48 (1H, s), 9.29 (1H, s), 11.31 (1H, s), 11.87 (1H, brs).

### Example 459

4-(3-hydroxy-1,1-dimethylpropyl)-N-[6-(trifluoromethyl)imidazo[1,2-a]pyridin-2-yl]benzamide

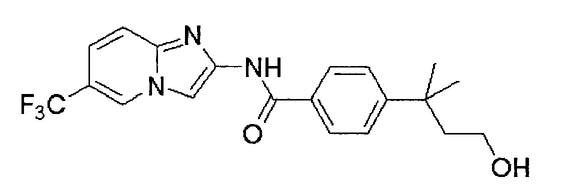

6-(Trifluoromethyl)imidazo[1,2-a]pyridine-2-amine(1.0 g, 3.9 mmol) synthesized in Reference Example 47 was acylated in the same manner as in Example 21, and purified by silica gel chromatography (40-50%ethyl acetate/hexane) to give ethyl 4-methyl-4-(4-{[6-(trifluoromethyl)imidazo[1,2-a]pyridin-2-yl]carbamoyl}phenyl)butanoate (yield 1.39 g, 82%). The compound (1.1 g, 2.5 mmol) was dissolved in tetrahydrofuran (30 mL), and diisobutylaluminum hydride (1M hexane solution, 11 mL, 11 mmol) was added dropwise to the mixture over 3 min under ice-cooling. The mixture was further stirred for 15 min under ice-cooling. Then, 10% aqueous potassium sodium tartrate solution was added, and the mixture was stirred at room temperature for 30 min and extracted with ethyl acetate. The organic layer was washed with saturated brine, and dried over anhydrous magnesium sulfate. The solvent was evaporated under reduced pressure and the obtained crude product was purified by silica gel chromatography (60-80% ethyl acetate/hexane) to give the title compound (yield 930 mg, 79%) as a white solid.
melting point 152-153°C
MS (ESI+): 392 (M+H).
¹H-NMR (DMSO-d₆) δ: 1.31 (6H, s), 1.75-1.94 (2H, m), 3.12-3.28 (2H, m), 4.27 (1H, t, J = 5.1 Hz), 7.47 (1H, dd, J = 9.2, 1.9 Hz), 7.49 (2H, d, J = 8.5 Hz), 7.66 (1H, d, J = 9.2 Hz), 8.04 (2H, d, J = 8.5 Hz), 8.47 (1H, s), 9.25-9.33 (1H, m), 11.29 (1H, s).

### Example 460

4-(4-hydroxy-1,1-dimethylbutyl)-N-[6-(trifluoromethyl)imidazo[1,2-a]pyridin-2-yl]benzamide

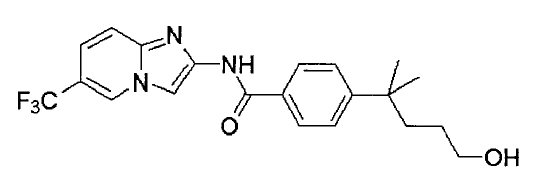

6-(Trifluoromethyl)imidazo[1,2-a]pyridine-2-amine(1.14 g, 5.6 mmol) synthesized in Reference Example 47 was acylated in the same manner as in Example 180, and purified by silica gel chromatography (40-50% ethyl acetate/hexane) to give ethyl 4-methyl-4-(4-{[6-(trifluoromethyl)imidazo[1,2-a]pyridin-2-yl]carbamoyl}phenyl)pentanoate (yield 1.94 g, 78%). The compound (1.93 g, 4.23 mmol) was reduced in the same manner as in Example 459, and the obtained crude product was purified by silica gel chromatography (60-80% ethyl acetate/hexane) to give the title compound (yield 1.5 g, 87%).
MS (ESI+): 406 (M+H).
¹H-NMR (DMSO-d₆) δ: 1.07-1.24 (2H, m), 1.30 (6H, s), 1.56-1.69 (2H, m), 3.30 (2H, td, J = 6.4, 5.3 Hz), 4.33 (1H, t, J = 5.3 Hz), 7.47 (1H, dd, J = 9.4, 1.9 Hz), 7.48 (2H, d, J = 8.5 Hz), 7.66 (1H, d, J = 9.4 Hz), 8.04 (2H, d, J = 8.5 Hz), 8.48 (1H, s), 9.28 (1H, s), 11.29 (1H, s).

### Example 461

4-(2-hydroxy-1,1-dimethylethyl)-N-[6-(trifluoromethyl)imidazo[1,2-a]pyridin-2-yl]benzamide

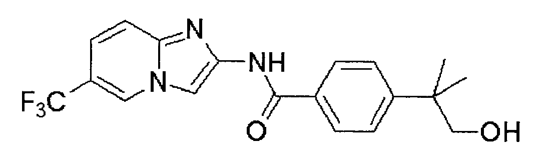

6-(trifluoromethyl)imidazo[1,2-a]pyridine-2-amine (929 mg, 4.5 mmol) synthesized in Reference Example 47 was acylated in the same manner as in Example 90, and purified to give 4-(2-{[tert-butyl(dimethyl)silyl]oxy}-1,1-dimethylethyl)-N-[6-(trifluoromethyl)imidazo[1,2-a]pyridin-2-yl]benzamide (yield 1.7 g, yield 88%). The compound was dissolved in THF (30 mL), tetrabutylammonium fluoride (1M THF solution, 6.9 mL, 6.9 mmol) was gradually added at room temperature, and the mixture was stirred overnight. Then, saturated aqueous ammonium chloride solution was poured into the reaction mixture, and the mixture was extracted with ethyl acetate. The organic layer was washed with saturated brine, dried over anhydrous magnesium sulfate, and the solvent was evaporated under reduced pressure to give a crude orange oil product. The product was purified by silica gel chromatography (60-80% ethyl acetate/hexane) to give a white title compound (yield 1.2 g, 95%).
melting point 227-229°C
MS (ESI+): 378 (M+H).
¹H-NMR (DMSO-d₆) δ: 1.26 (6H, s), 3.47 (2H, d, J = 5.3 Hz), 4.74 (1H, t, J = 5.3 Hz), 7.51 (2H, d, J = 8.5 Hz), 7.47 (1H, dd, J = 9.4, 1.9 Hz), 7.65 (1H, d, J = 9.4 Hz), 8.03 (2H, d, J = 8.5 Hz), 8.48 (1H, s), 9.27-9.30 (1H, m), 11.29 (1H, s).

### Example 462

4-(2-hydroxy-1,1-dimethylethyl)-N-[6-(1-methyl-1H-pyrazol-4-yl)imidazo[1,2-a]pyridin-2-yl]benzamide

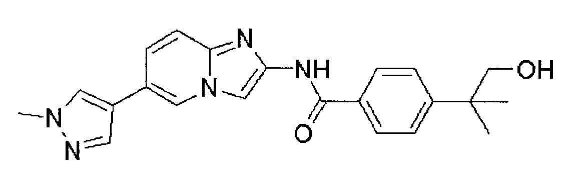

According to Example 103, 4-(2-{[tert-butyl(dimethyl)silyl]oxy}-1,1-dimethylethyl)-N-(6-iodoimidazo[1,2-a]pyridin-2-yl)benzamide was synthesized. According to Example 57 and Example 103 and using 1-methyl-4-(4,4,5,5-tetramethyl-1,3,2-dioxaborolan-2-yl)-1H-pyrazole, the title compound was synthesized.
melting point 275-277°C
MS (ESI+): 390 (M+H).
¹H-NMR (DMSO-d₆) δ: 1.26 (6H, s), 3.47 (2H, d, J = 5.5 Hz), 3.89 (3H, s), 4.73 (1H, t, J = 5.5 Hz), 7.45-7.53 (4H, m), 7. 87 (1H, s), 8.01 (2H, d, J = 8.3 Hz), 8.14 (1H, s), 8.23 (1H, s), 8.84 (1H, s), 11.11 (1H, s).

### Example 463

4-(2-hydroxy-1,1-dimethylethyl)-N-[6-(1-methyl-1H-pyrazol-4-yl)imidazo[1,2-a]pyridin-2-yl]benzamide hydrochloride

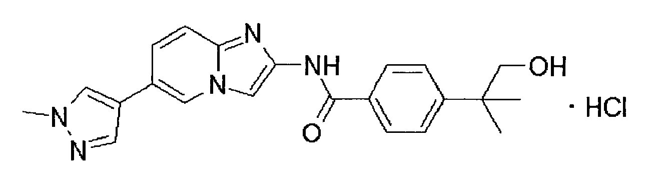

According to Example 52 and using 4-(2-hydroxy-1,1-dimethylethyl)-N-[6-(1-methyl-1H-pyrazol-4-yl)imidazo[1,2-a]pyridin-2-yl]benzamide obtained in Example 462, the title compound was synthesized.
MS (ESI+): 390 (M+H).
¹H-NMR (DMSO-d₆) δ: 1.27 (6H, s), 3.48 (2H, s), 3.91 (3H, s), 7.58 (2H, d, J = 8.7 Hz), 7.83 (1H, d, J = 9.1 Hz), 7.93-7.98 (2H, m), 8.03 (2H, d, J = 8.3 Hz), 8.22-8.25 (2H, m), 9.11 (1H, s), 11.82 (1H, s), hidden (1H).

### Example 464

4-(2-hydroxy-1,1-dimethylethyl)-N-[6-(1H-pyrazol-4-yl)imidazo[1,2-a]pyridin-2-yl]benzamide

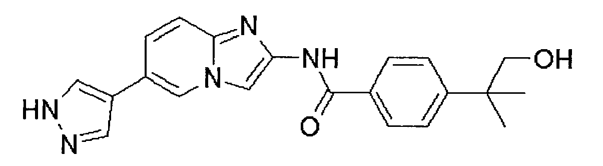

According to Example 103, 4-(2-{[tert-butyl(dimethyl)silyl]oxy}-1,1-dimethylethyl)-N-(6-iodoimidazo[1,2-a]pyridin-2-yl)benzamide was synthesized, then according to Examples 57 and 103 and using 4-(4,4,5,5-tetramethyl-1,3,2-dioxaborolan-2-yl)-1H-pyrazole, the title compound was synthesized.
melting point 293-296°C
MS (ESI+): 376 (M+H).
¹H-NMR (DMSO-d₆) δ: 1.26 (6H, s), 3.47 (2H, d, J = 5.3 Hz), 4.73 (1H, t, J = 5.3 Hz), 7.44-7.57 (4H, m), 8.01 (2H, d, J = 8.3 Hz), 8.22 (1H, s), 8.88 (1H, s), 11.11 (1H, s), 13.01 (1H, s), hidden (2H).

### Example 465

N-[6-(3-furyl)imidazo[1,2-a]pyridin-2-yl]-4-(2-hydroxy-1,1-dimethylethyl)benzamide

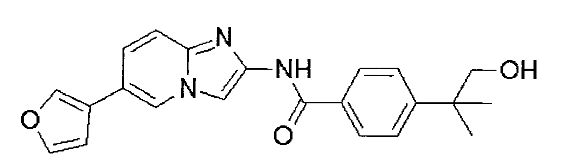

According to Example 103, 4-(2-{[tert-butyl(dimethyl)silyl]oxy}-1,1-dimethylethyl)-N-(6-iodoimidazo[1,2-a]pyridin-2-yl)benzamide was synthesized, then according to Examples 57 and 103 and using 3-furylboronic acid, the title compound was synthesized.
melting point 246-248°C
MS (ESI+): 376 (M+H).
¹H-NMR (DMSO-d₆) δ: 1.26 (6H, s), 3.47 (2H, d, J = 5.1 Hz), 4.73 (1H, t, J = 5.1 Hz), 6.98 (1H, d, J = 1.5 Hz), 7.46-7.56 (4H, m), 7.79 (1H, s), 8.01 (2H, d, J = 8.3 Hz), 8.22 (1H, s), 8.25 (1H, s), 8.90 (1H, s), 11.14 (1H, s).
Elemental analysis: for C₂₂H₂₁N₃O₃
Calculated: C, 70.38; H, 5.64; N, 11.19.
Found: C, 70.27; H, 5.52; N, 11.31.

### Example 466

4-(2-hydroxy-1,1-dimethylethyl)-N-[6-(3-thienyl)imidazo[1,2-a]pyridin-2-yl]benzamide

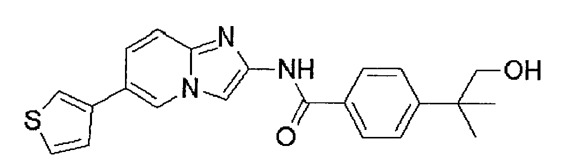

According to Example 103, 4-(2-{[tert-butyl(dimethyl)silyl]oxy}-1,1-dimethylethyl)-N-(6-iodoimidazo[1,2-a]pyridin-2-yl)benzamide was synthesized, then according to Examples 57 and 103 and using 3-thienylboronic acid, the title compound was synthesized.
melting point 250-252°C
MS (ESI+): 392 (M+H).
¹H-NMR (DMSO-d₆) δ: 1.26 (6H, s), 3.47 (2H, d, J = 5.4 Hz), 4.73 (1H, t, J = 5.4 Hz), 7.47-7.53 (3H, m), 7.59 (1H, d, J = 4.9 Hz), 7.63-7.74 (2H, m), 7.89-7.92 (1H, m), 8.02 (2H, d, J = 8.3 Hz), 8.28 (1H, s), 9.03 (1H, s), 11.14 (1H, s). Elemental analysis: for C₂₂H₂₁N₃O₂S
Calculated: C, 67.50; H, 5.41; N, 10.73.
Found: C, 67.44; H, 5.33; N, 10.87.

### Example 467

N-(6-chloroimidazo[1,2-a]pyridin-2-yl)-4-(3-hydroxy-1,1-dimethylpropyl)benzamide

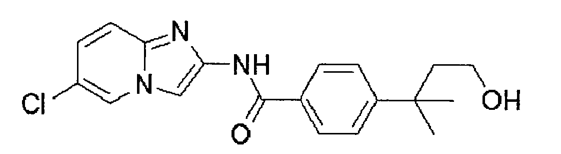

According to Example 90 and using 4-(3-{[tert-butyl(dimethyl)silyl]oxy}-1,1-dimethylpropyl)benzoic acid obtained in Reference Example 52, the title compound was synthesized.
melting point 182-184°C
MS (ESI+): 358 (M+H).
¹H-NMR (DMSO-d₆) δ: 1.31 (6H, s), 1.80-1.87 (2H, m), 3.15-3.24 (2H, m), 4.27 (1H, t, J = 5.1 Hz), 7.28 (1H, dd, J = 9.6, 2.1 Hz), 7.45-7.53 (3H, m), 8.02 (2H, d, J = 8.5 Hz), 8.33 (1H, s), 8.86-8.89 (1H, m), 11.19 (1H, s).

### Example 468

4-(3-hydroxy-1,1-dimethylpropyl)-N-[6-(1H-imidazol-1-yl)imidazo[1,2-a]pyridin-2-yl]benzamide

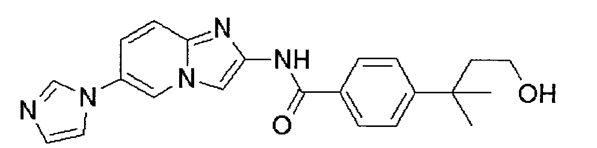

According to Example 103 and using 4-(3-{[tert-butyl(dimethyl)silyl]oxy}-1,1-dimethylpropyl)benzoic acid obtained in Reference Example 52, the title compound was synthesized.
melting point 225-227°C
MS (ESI+): 390 (M+H).
¹H-NMR (DMSO-d₆) δ: 1.31 (6H, s), 1.81-1.88 (2H, m), 3.17-3.25 (2H, m), 4.26 (1H, t, J = 4.9 Hz), 7.49 (2H, d, J = 8.7 Hz), 7.57 (1H, dd, J = 9.5, 1.9 Hz), 7.62 (1H, d, J = 9.5 Hz), 7.69-7.70 (1H, m), 8.04 (2H, d, J = 8.3 Hz), 8.19 (2H, s), 8.34 (1H, s), 9.06 (1H, s), 11.21 (1H, s).
Elemental analysis: for C₂₂H₂₃N₅O₂
Calculated: C, 67.85; H, 5.95; N, 17.98.
Found: C, 67.77; H, 5.90; N, 18.03.

### Example 469

4-tert-butyl-N-[6-(1H-1,2,4-triazol-1-yl)imidazo[1,2-a]pyridin-2-yl]benzamide

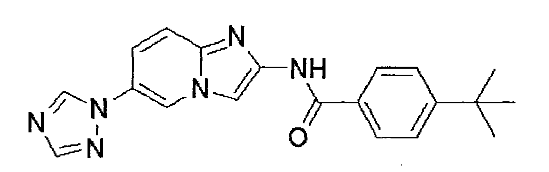

According to Example 51 and using 1,2,4-triazole, the title compound was synthesized.
melting point 274-276°C
MS (ESI+): 361 (M+H).
¹H-NMR (DMSO-d₆) δ: 1.33 (9H, s), 7.54 (2H, d, J = 8.3 Hz), 7.66 (1H, d, J = 9.5 Hz), 7.73-7.78 (1H, m), 8.03 (2H, d, J = 8.3 Hz), 8.29 (1H, s), 8.43 (1H, s), 9.21-9.25 (2H, m), 11.21 (1H, s).

### Example 470

N-[6-(3-aminophenoxy)imidazo[1,2-b]pyridazin-2-yl]-4-(trifluoromethyl)benzamide

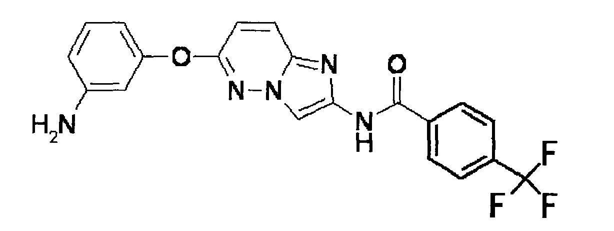

The title compound was synthesized according to Example 138.
MS (m/Z) 414

### Example 471

N-{3-[(2-{[(1,3-dimethyl-1H-pyrazol-5-yl)carbonyl]amino}imidazo[1,2-b]pyridazin-6-yl)oxy]phenyl}-1,3-dimethyl-1H-pyrazole-5-carboxamide

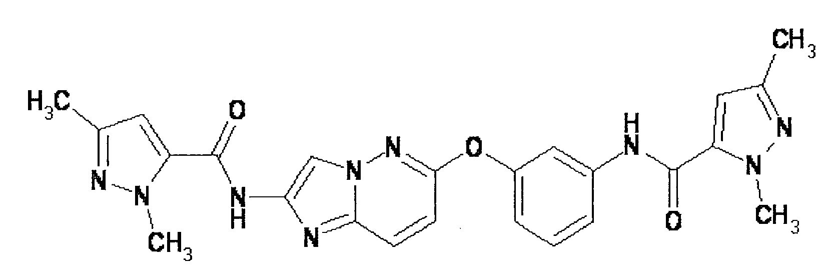

To a solution of 6-(3-aminophenoxy)imidazo[1,2-b]pyridazine-2-amine (100 mg, 0.41 mmol) in N-methylpyrrolidone (2 mL) was added 1,3-dimethyl-1H-pyrazole-5-carbonyl chloride (163 mg, 1.03 mmol), and the mixture was stirred at room temperature for 4 hr. Ethyl acetate was added to the reaction mixture, and the precipitate was collected by filtration and recrystallized from ethanol to give the title compound (131 mg, 65%) as a white solid.
¹H-NMR (DMSO-d₆, 300 MHz) δ 2.18 (3H, s), 2.19 (3H, s), 3.98 (3H, s), 4.03 (3H, s), 6.85 (1H, s), 7.00-7.07 (1H, m), 7.03 (1H, s), 7.13 (1H, d, J = 9.6 Hz), 7.44 (1H, t, J = 8.2 Hz), 7.60-7.66 (1H, m), 7.71 (1H, t, J = 2.2 Hz), 8.11 (1H, dd, J = 9.6, 0.6 Hz), 8.17 (1H, s), 10.28 (1H, s), 11.28 (1H, s).

### Example 472

N-[6-(3-{[3-(trifluoromethyl)benzoyl]amino}phenoxy)imidazo[1,2-b]pyridazin-2-yl]pyrrolidine-3-carboxamide

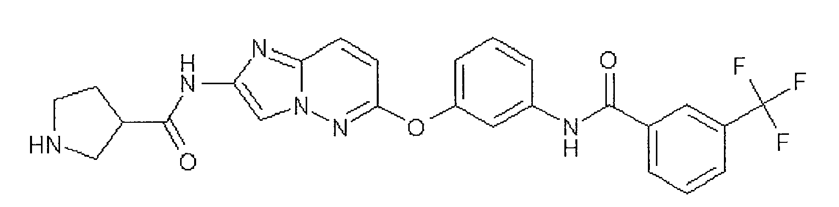

To a solution of N-{3-[(2-aminoimidazo[1,2-b]pyridazin-6-yl)oxy]phenyl}-3-(trifluoromethyl)benzamide (150 mg, 0.363 mmol) in N,N-dimethylformamide (5 mL) were added 1-(tert-butoxycarbonyl)pyrrolidine-3-carboxylic acid (117 mg, 0.544 mmol), N-[3-(dimethylamino)propyl]-N'-ethylcarbodiimide hydrochloride (104 mg, 0.544 mmol) and 1-hydroxybenzotriazole (74 mg, 0.544 mmol), and the mixture was stirred at room temperature for 15 hr. Saturated aqueous ammonium chloride was added to the reaction mixture, and the mixture was extracted with ethyl acetate. The extract was washed with saturated brine, dried over anhydrous magnesium sulfate and filtered. The solvent was evaporated under reduced pressure, and the residue was purified by silica gel column chromatography (ethyl acetate/hexane=30/70→100/0) to give a pale-yellow oil. A mixture of the oil and trifluoroacetic acid (5 mL) was stirred at room temperature for 1.5 hr. Saturated aqueous sodium hydrogen carbonate was added to the reaction mixture, and the mixture was extracted with ethyl acetate. The extract was washed with saturated brine, dried over anhydrous magnesium sulfate and filtered. The residue was washed with ethanol to give the title compound (134 mg, 72%) as a white powder.
¹H-NMR (DMSO-d₆, 300 MHz) δ 1.95-2.10 (1H, m), 2.16-2.31 (1H, m), 3.13-3.26 (2H, m), 3.34-3.43 (3H, m), 7.01-7.07 (1H, m), 7.12 (1H, d, J = 9.6 Hz), 7.47 (1H, t, J = 8.3 Hz), 7.66 (1H, d, J = 8.3 Hz), 7.74-7.83 (2H, m), 7.94-8.13 (3H, m), 8.22-8.31 (2H, m), 8.84 (1H, brs), 10.62 (1H, s), 11.15 (1H, s).

### Example 473

Production of 1-methyl-N-[6-(3-{[3-(trifluoromethyl)benzoyl]amino}phenoxy)imidazo[1,2-b]pyridazin-2-yl]pyrrolidine-3-carboxamide

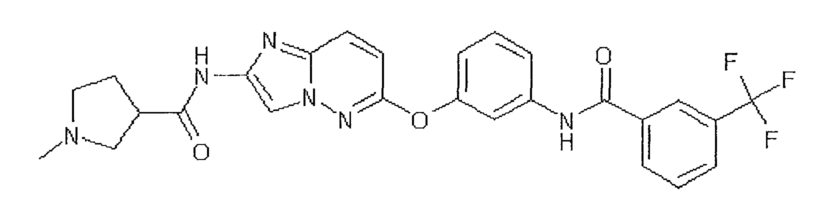

To a mixture of N-[6-(3-{[3-(trifluoromethyl)benzoyl]amino}phenoxy)imidazo[1,2-b]pyridazin-2-yl]pyrrolidine-3-carboxamide (139 mg, 0.273 mmol), formaldehyde (228 mg, 2.73 mmol), acetic acid (1 mL) and methanol (5 mL) was added sodium tricyanoborohydride (103 mg, 1.63 mmol), and the mixture was stirred at room temperature for 20 hr. Saturated aqueous sodium hydrogen carbonate was added to the reaction mixture, and the mixture was extracted with ethyl acetate. The extract was washed with saturated brine, dried over anhydrous magnesium sulfate and filtered. The solvent was evaporated under reduced pressure, and the residue was purified by silica gel column chromatography (ethyl acetate/methanol=100/0→90/10) to give a pale-yellow powder (46 mg, 32%).
¹H-NMR (DMSO-d₆, 300 MHz) δ 1.92-2.03 (2H, m), 2.23 (3H, s), 2.32-2.47 (2H, m), 2.54-2.62 (1H, m), 2.74-2.82 (1H, m), 3.08-3.23 (1H, m), 7.00-7.11 (2H, m), 7.46 (1H, t, J = 8.2 Hz), 7.63-7.83 (3H, m), 7.94-8.08 (3H, m), 8.21-8.31 (2H, m), 10.59 (1H, s), 10.78 (1H, s).

### Example 474

tert-butyl 3-({[6-(3-{[3-(1-cyanocyclopropyl)benzoyl]amino}phenoxy)imidazo[1,2-b]pyridazin-2-yl)amino}carbonyl)pyrrolidine-1-carboxylate

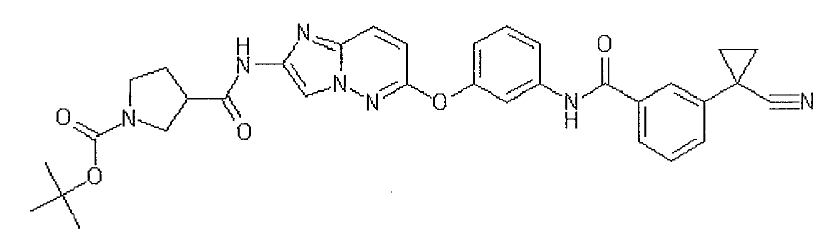

To a solution of N-{3-[(2-aminoimidazo[1,2-b]pyridazin-6-yl)oxy]phenyl}-3-(1-cyanocyclopropyl)benzamide (300 mg, 0.731 mmol) in N,N-dimethylformamide (10 mL) were added 1-(tert-butoxycarbonyl)pyrrolidine-3-carboxylic acid (188 mg, 0.877 mmol), N-[3-(dimethylamino)propyl]-N'-ethylcarbodiimide hydrochloride (210 mg, 1.09 mmol) and 1-hydroxybenzotriazole (148 mg, 1.09 mmol), and the mixture was stirred at room temperature for 16 hr. Saturated aqueous ammonium chloride solution was added to the reaction mixture, and the mixture was extracted with ethyl acetate. The extract was washed with saturated brine, dried over anhydrous magnesium sulfate and filtered. The solvent was evaporated under reduced pressure, and the residue was purified by silica gel column chromatography (ethyl acetate/hexane=0/100→100/0) to give the title compound (345 mg, 77%) as a green powder.
¹H-NMR (DMSO-d₆, 300 MHz) δ 1.40 (9H, s), 1.57-1.65 (2H, m), 1.77-1.85 (2H, m), 1.93-2.17 (2H, m), 3.16-3.28 (2H, m), 3.33-3.43 (2H, m), 3.46-3.57 (1H, m), 7.02 (1H, dd, J = 8.2, 2.0 Hz), 7.09 (1H, d, J = 9.6 Hz), 7.45 (1H, t, J = 8.2 Hz), 7.51-7.61 (2H, m), 7.65 (1H, d, J = 8.2 Hz), 7.72 (1H, t, J = 2.0 Hz), 7.82 (1H, s), 7.84-7.91 (1H, m), 8.01-8.11 (2H, m), 10.42 (1H, s), 10.97 (1H, brs).

### Example 475

N-[6-(3-{[3-(1-cyanocyclopropyl)benzoyl]amino}phenoxy)imidazo[1,2-b]pyridazin-2-yl]pyrrolidine-3-carboxamide

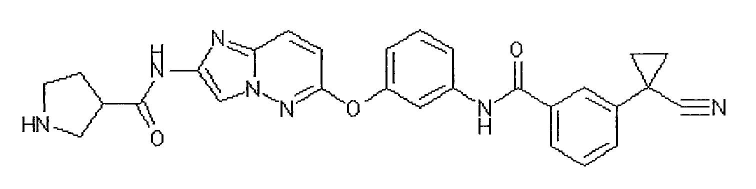

A mixture of tert-butyl 3-({[6-(3-{[3-(1-cyanocyclopropyl)benzoyl]amino}phenoxy)imidazo[1,2-b]pyridazin-2-yl]amino}carbonyl)pyrrolidine-1-carboxylate (290 mg, 0.477 mmol) and trifluoroacetic acid (5 mL) was stirred at room temperature for 4 hr. Saturated aqueous sodium hydrogen carbonate was added to the reaction mixture, and the mixture was extracted with ethyl acetate. The extract was washed with saturated brine, dried over anhydrous magnesium sulfate and filtered. The residue was washed with ethanol to give the title compound (133 mg, 55%) as a white powder.
¹H-NMR (DMSO-d₆, 300 MHz) δ 1.57-1.64 (2H, m), 1.77-1.85 (2H, m), 1.96-2.14 (1H, m), 2.17-2.36 (1H, m), 3.15-3.27 (2H, m), 3.38 (3H, s), 7.02 (1H, dd, J = 8.0, 1.8 Hz), 7.11 (1H, d, J = 9.5 Hz), 7.45 (1H, t, J = 8.0 Hz), 7.53-7.59 (2H, m), 7.63 (1H, d, J = 8.0 Hz), 7.76 (1H, t, J = 1.8 Hz), 7.82 (1H, s), 7.84-7.89 (1H, m), 8.03 (1H, s), 8.09 (1H, d, J = 9.5 Hz), 8.78 (1H, brs), 10.44 (1H, s), 11.16 (1H, s).

### Example 476

N-[6-(3-{[3-(1-cyanocyclopropyl)benzoyl]amino}phenoxy)imidazo[1,2-b]pyridazin-2-yl]-1-methylpyrrolidine-3-carboxamide

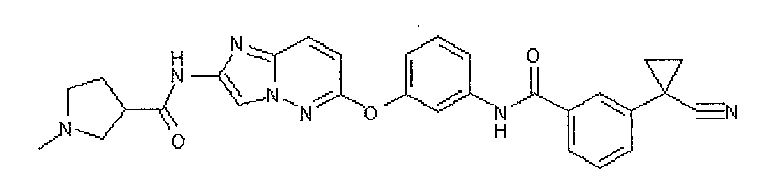

Using N-[6-(3-{[3-(1-cyanocyclopropyl)benzoyl]amino}phenoxy)imidazo[1,2-b]pyridazin-2-yl]pyrrolidine-3-carboxamide (140 mg, 2.75 mmol), formaldehyde (230 mg, 2.75 mmol), sodium tricyanoborohydride (104 mg, 1.65 mmol), acetic acid (1.5 mL) and methanol (3.5 mL) as starting materials and in the same manner as in Example 473, the title compound (16 mg, 12%) was obtained as a pale-orange powder.
¹H-NMR (CDCl₃, 300 MHz) δ 1.45-1.52 (2H, m), 1.75-1.83 (2H, m), 2.05-2.17 (1H, m), 2.22-2.43 (2H, m), 2.50 (3H, s), 2.96-3.15 (2H, m), 3.42-3.50 (1H, m), 3.59-3.70 (1H, m), 6.86 (1H, d, J = 9.6 Hz), 6.97-7.05 (1H, m), 7.37-7.63 (4H, m), 7.69 (1H, t, J = 1.9 Hz), 7.71-7.79 (3H, m), 7.95-8.02 (1H, m), 8.15 (1H, s), 9.87 (1H, brs).

### Example 477

N-(3-{[2-(benzoylamino)imidazo[1,2-b]pyridazin-6-yl]oxy}phenyl)-3-(1-cyanocyclopropyl)benzamide

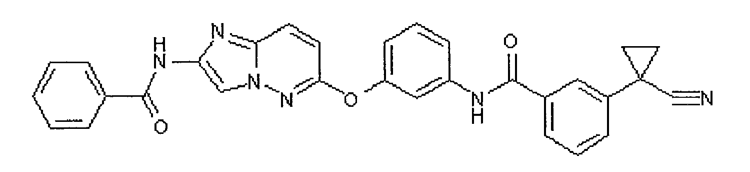

To a solution of N-{3-[(2-aminoimidazo[1,2-b]pyridazin-6-yl)oxy]phenyl}-3-(1-cyanocyclopropyl)benzamide (150 mg, 0.365 mmol) in N,N-dimethylacetamide (3 mL) was added benzoyl chloride (51 µL, 0.438 mmol), and the mixture was stirred at room temperature for 3 hr. Water was added to the reaction mixture, and the precipitated solid was filtered and washed with water to give the title compound (154 mg, 82%) as a pale-green powder.
¹H-NMR (DMSO-d₆, 300 MHz) δ 1.56-1.65 (2H, m), 1.77-1.85 (2H, m), 7.05 (1H, dd, J = 8.0, 1.8 Hz), 7.12 (1H, d, J = 9.6 Hz), 7.41-7.63 (6H, m), 7.67 (1H, d, J = 9.0 Hz), 7.75 (1H, t, J = 1.8 Hz), 7.83 (1H, s), 7.85-7.91 (1H, m), 8.03-8.15 (3H, m), 8.24 (1H, s), 10.44 (1H, s), 11.33 (1H, s).

### Example 478

N-[6-(3-{[3-(1-cyanocyclopropyl)benzoyl]amino}phenoxy)imidazo[1,2-b]pyridazin-2-yl]-1,3-oxazole-4-carboxamide

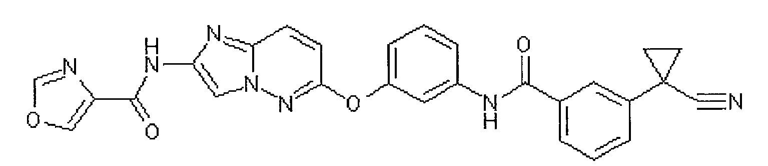

To a solution of 1,3-oxazole-4-carboxylic acid (44 mg, 0.394 mmol) in tetrahydrofuran (3 mL) were added N,N-dimethylformamide (1 drop) and oxalyl chloride (57 µL, 0.657 mmol), and the mixture was stirred at room temperature for 1.5 hr. The solvent was evaporated under reduced pressure, the residue and N-{3-[(2-aminoimidazo[1,2-b]pyridazin-6-yl)oxy]phenyl}-3-(1-cyanocyclopropyl)benzamide (135 mg, 0.328 mmol) were dissolved in N-methylpyrrolidone (3 mL), and the mixture was stirred at room temperature for 18 hr. Saturated aqueous sodium hydrogen carbonate was added to the reaction mixture, and the mixture was extracted with ethyl acetate. The extract was washed with saturated brine, dried over anhydrous magnesium sulfate and filtered. The solvent was evaporated under reduced pressure, and the residue was purified by silica gel column chromatography (ethyl acetate/hexane=30/70→100/0) to give the title compound (115 mg, 69%) as a pale-brown powder.
¹H-NMR (DMSO-d₆, 300 MHz) δ 1.58-1.65 (2H, m), 1.77-1.84 (2H, m), 7.05 (1H, dd, J = 8.2, 2.0 Hz), 7.14 (1H, d, J = 9.6 Hz), 7.46 (1H, t, J = 8.2 Hz), 7.51-7.60 (2H, m), 7.64-7.70 (1H, m), 7.74 (1H, t, J = 2.0 Hz), 7.81-7.84 (1H, m), 7.85-7.90 (1H, m), 8.09-8.18 (2H, m), 8.59 (1H, d, J = 0.8 Hz), 8.89 (1H, d, J = 0.8 Hz), 10.44 (1H, s), 10.75 (1H, s).

### Example 479

N-[6-(3-{[3-(1-cyanocyclopropyl)benzoyl]amino}phenoxy)imidazo[1,2-b]pyridazin-2-yl]-3-furamide

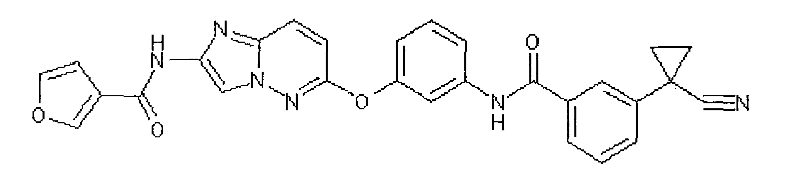

Using N-{3-[(2-aminoimidazo[1,2-b]pyridazin-6-yl)oxy]phenyl}-3-(1-cyanocyclopropyl)benzamide (135 mg, 0.328 mmol), 3-furoic acid (44 mg, 0.394 mmol), oxalyl chloride (57 µL, 0.657 mmol), N,N-dimethylformamide (1 drop), tetrahydrofuran (3 mL) and N-methylpyrrolidone (3 mL) as starting materials and in the same manner as in Example 478, the title compound (85 mg, 51%) was obtained as a pale-brown powder.
¹H-NMR (DMSO-d₆, 300 MHz) δ 1.58-1.65 (2H, m), 1.78-1.84 (2H, m), 7.01-7.15 (3H, m), 7.46 (1H, t, J = 8.2 Hz), 7.52-7.61 (2H, m), 7.64-7.70 (1H, m), 7.73 (1H, t, J = 2.1 Hz), 7.78 (1H, t, J = 1.7 Hz), 7.81-7.84 (1H, m), 7.85-7.90 (1H, m), 8.09 (1H, d, J = 9.6 Hz), 8.15 (1H, s), 8.50 (1H, s), 10.43 (1H, s), 11.13 (1H, s).

### Example 480

N-[6-(3-{[3-(1-cyanocyclopropyl)benzoyl]amino}phenoxy)imidazo[1,2-b]pyridazin-2-yl]thiophene-3-carboxamide

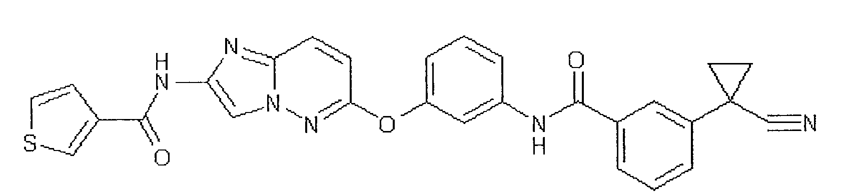

Using N-{3-[(2-aminoimidazo[1,2-b]pyridazin-6-yl)oxy]phenyl}-3-(1-cyanocyclopropyl)benzamide (170 mg, 0.381 mmol), thiophene-3-carboxylic acid (58 mg, 0.457 mmol), oxalyl chloride (66 µL, 0.762 mmol), N,N-dimethylformamide (1 drop), tetrahydrofuran (3 mL) and N,N-dimethylacetamide (3 mL) as starting materials and in the same manner as in Example 478, the title compound (56 mg, 29%) was obtained as a pale-green powder.
¹H-NMR (DMSO-d₆, 300 MHz,) δ 1.58-1.65 (2H, m), 1.77-1.85 (2H, m), 7.04 (1H, dd, J = 8.0, 2.0 Hz), 7.12 (1H, d, J = 9.5 Hz), 7.46 (1H, t, J = 8.0 Hz), 7.52-7.60 (2H, m), 7.61-7.76 (4H, m), 7.80-7.84 (1H, m), 7.85-7.91 (1H, m), 8.10 (1H, d, J = 9.5 Hz), 8.18 (1H, s), 8.52 (1H, d, J = 2.0 Hz), 10.44 (1H, s), 11.20 (1H, s).

### Example 481

N-[6-(3-{[3-(1-cyanocyclopropyl)benzoyl]amino}phenoxy)imidazo[1,2-b]pyridazin-2-yl]-1,3-thiazole-4-carboxamide

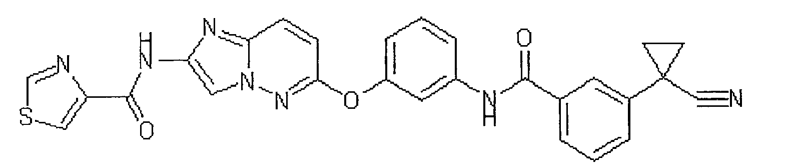

Using N-{3-[(2-aminoimidazo[1,2-b]pyridazin-6-yl)oxy]phenyl}-3-(1-cyanocyclopropyl)benzamide (100 mg, 0.224 mmol), 1,3-thiazole-4-carboxylic acid (37 mg, 0.291 mmol), oxalyl chloride (39 µL, 0.448 mmol), N,N-dimethylformamide (1 drop), tetrahydrofuran (3 mL) and N,N-dimethylacetamide (3 mL) as starting materials and in the same manner as in Example 478, the title compound (76 mg, 65%) was obtained as a pale-green powder.
¹H-NMR (DMSO-d₆, 300 MHz,) δ 1.59-1.65 (2H, m), 1.78-1.85 (2H, m), 7.02-7.08 (1H, m), 7.15 (1H, d, J = 9.6 Hz), 7.47 (1H, t, J = 8.2 Hz), 7.54-7.60 (2H, m), 7.65-7.70 (1H, m), 7.74 (1H, t, J = 2.0 Hz), 7.81-7.83 (1H, m), 7.85-7.91 (1H, m), 8.13 (1H, dd, J = 9.6, 0.6 Hz), 8.19 (1H, s), 8.60 (1H, d, J = 2.0 Hz), 9.27 (1H, d, J = 2.0 Hz), 10.45 (1H, s), 10.58 (1H, s).

### Example 482

N-[6-(3-{[3-(1-cyanocyclopropyl)benzoyl]amino}phenoxy)imidazo[1,2-b]pyridazin-2-yl]-1-methyl-1H-pyrazole-4-carboxamide

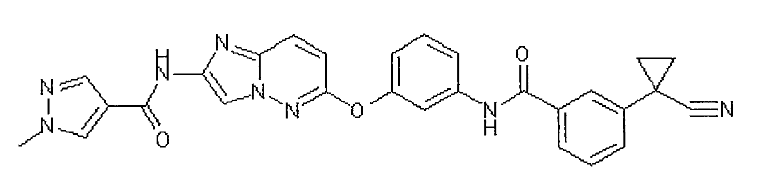

Using N-{3-[(2-aminoimidazo[1,2-b]pyridazin-6-yl)oxy]phenyl}-3-(1-cyanocyclopropyl)benzamide (100 mg, 0.224 mmol), 1-methyl-1H-pyrazole-4-carboxylic acid (36 mg, 0.291 mmol), oxalyl chloride (39 µL, 0.448 mmol), N,N-dimethylformamide (1 drop), tetrahydrofuran (3 mL) and N,N-dimethylacetamide (3 mL) as starting materials and in the same manner as in Example 478, the title compound (25 mg, 22%) was obtained as a pale-green powder.
¹H-NMR (DMSO-d₆, 300 MHz) δ 1.58-1.65 (2H, m), 1.77-1.85 (2H, m), 3.88 (3H, s), 7.04 (1H, dd, J = 7.8, 2.0 Hz), 7.10 (1H, d, J = 9.6 Hz), 7.46 (1H, t, J = 8.1 Hz), 7.51-7.61 (2H, m), 7.63-7.70 (1H, m), 7.71-7.75 (1H, m), 7.80-7.84 (1H, m), 7.85-7.91 (1H, m), 8.05-8.15 (3H, m), 8.39 (1H, s), 10.44 (1H, s), 11.00 (1H, s).

### Example 483

N-[6-(3-{[3-(1-cyanocyclopropyl)benzoyl]amino}phenoxy)imidazo[1,2-b]pyridazin-2-yl]-1-methyl-1H-imidazole-4-carboxamide

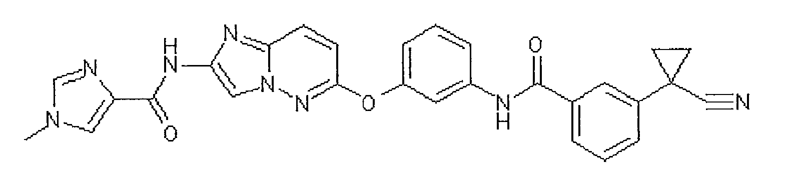

Using N-{3-[(2-aminoimidazo[1,2-b]pyridazin-6-yl)oxy]phenyl}-3-(1-cyanocyclopropyl)benzamide (170 mg, 0.381 mmol), 1-methyl-1H-imidazole-4-carboxylic acid (62 mg, 0.496 mmol), oxalyl chloride (66 µL, 0.762 mmol), N,N-dimethylformamide (1 drop), tetrahydrofuran (3 mL) and N,N-dimethylacetamide (3 mL) as starting materials and in the same manner as in Example 478, the title compound (144 mg, 73%) was obtained as a pale-green powder.
¹H-NMR (DMSO-d₆, 300 MHz) δ 1.58-1.65 (2H, m), 1.78-1.85 (2H, m), 3.73 (3H, s), 7.01-7.07 (1H, m), 7.12 (1H, d, J = 9.6 Hz), 7.46 (1H, t, J = 8.2 Hz), 7.51-7.60 (2H, m), 7.64-7.70 (1H, m), 7.73 (1H, t, J = 2.1 Hz), 7.76-7.83 (2H, m), 7.85-7.91 (2H, m), 8.07-8.14 (2H, m), 9.84 (1H, s), 10.44 (1H, s).

### Example 484

3-(1-cyano-1-methylethyl)-N-(6-{3-[(cyclopropylcarbonyl)amino]phenoxy}imidazo[1,2-b]pyridazin-2-yl)benzamide

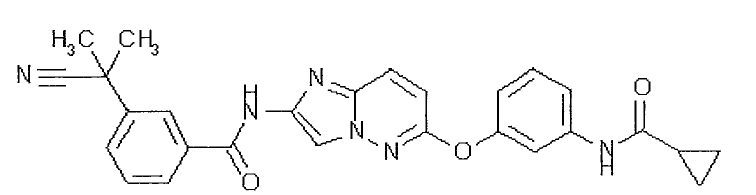

Using 3-(1-cyano-1-methylethyl)benzoic acid (37 mg, 0.19 mmol), oxalyl chloride (21 µL, 0.24 mmol), N,N-dimethylformamide (1 drop), tetrahydrofuran (2.0 mL), N-{3-[(2-aminoimidazo[1,2-b]pyridazin-6-yl)oxy]phenyl}cyclopropanecarboxamide (50 mg, 0.16 mmol) and N,N-dimethylacetamide (1.0 mL) as starting materials and in the same manner as in Example 478, the title compound (55 mg, 71%) was obtained as a white powder.
¹H-NMR (DMSO-d₆, 300 MHz) δ 0.79 (4H, d, J = 6.3 Hz), 1.64-1.85 (7H, m), 6.94 (1H, dt, J = 6.6, 2.4 Hz), 7.10 (1H, d, J = 9.6 Hz), 7.29-7.47 (2H, m), 7.50-7.66 (2H, m), 7.70-7.82 (1H, m), 8.03 (1H, ddd, J= 7.8, 1.2, 0.9 Hz), 8.10 (1H, d, J = 9.6 Hz), 8.17-8.26 (2H, m), 10.38 (1H, s), 11.52 (1H, s).

### Reference Example 1A: cloning of human ASK1 gene and preparation of recombinant Baculovirus

human ASK1 gene was cloned by PCR using human heart cDNA (Becton, Dickinson; trade name: QUICK-Clone cDNA) as a template, and a primer set:
ASK1-U: 5'-aaaagtcgacatggactacaaggacgacgatgacaaggtgaacaccattaccgaagagaagg gga - 3'(SEQ ID NO: 1) and
ASK1-L: 5'- aaagcggccgctcaagtctgtttgtttcgaaagtcaatg-3'(SEQ ID NO: 2)

### Prepared by reference to the base sequence of ASK1 gene registered in the GenBank under accession No. NM_005923.

PCR was performed according to the protocol attached to KOD plus DNA polymerase (TOYOBO CO., LTD.). The obtained PCR product was subjected to agarose gel (1%) electrophoresis, DNA fragment (2.2 kb) containing ASK1 gene was recovered from the gel, and digested with restriction enzymes Not I and Sal I. DNA after treatment with restriction enzymes was subjected to agarose gel (1%) electrophoresis, and the obtained DNA fragment was recovered, and ligated to plasmid pFASTBAC1 (Invitrogen) digested with restriction enzymes Not I and Sal I to give expression plasmid pFB-ASK1. The base sequence of the inserted fragment was confirmed to match the object sequence. Using BAC-TO-BAC Baculovirus Expression System (Invitrogen), virus stock BAC-ASK1 of recombinant Baculovirus was prepared.

### Reference Example 2A: Preparation of recombinant ASK1 enzyme

Sf-21 cells (Invitrogen) were inoculated to 150 ml Sf-900 II SFM medium (Invitrogen) containing 10% fetal bovine serum to 1x10⁶ cells/ml and cultured at 27°C for 24 hr. To the obtained culture medium was added the virus stock BAC-ASK1 of recombinant Baculovirus obtained in Reference Example 1A by 150 µl each, and the cells were further cultured for 60 hr. The culture medium was centrifuged (3000 rpm, 10 min) to separate the cells, and the cells were washed once with PBS. The cells were suspended in 10 ml of cell lysis buffer (25 mM HEPES (pH 7.5), 1% Triton X, 130 mM sodium chloride, 1 mM EDTA, 1 mM Dithiothreitol, 25 mM β-glycerophosphate, Protease inhibitor Complete (Boehringer), 1 mM sodium orthovanadate), treated 4 times in a homogenizer (POLYTRON) at 20000 rpm, 30 sec to disrupt the cells. The cell disrupt solution was centrifuged (40000 rpm, 45 min), and ASK1 was purified from the obtained supernatant using Anti-FLAG M2 Affinity Gel (Sigma Ltd.).

### Experimental Example 2: measurement of ASK1 inhibitory activity

To 37.5 µl of a reaction solution (25 mM HEPES (pH 7.5), 10 mM magnesium acetate, 1 mM DTT) containing ASK1 enzyme (30 ng) obtained in Reference Example 2A and myelin basic protein (Wako Pure Chemical Industries, Ltd., 1 µg) was added a test compound (2.5 µl) dissolved in DMSO, and the mixture was incubated at room temperature for 5 min. To the obtained mixture was added ATP solution (2.5 µM ATP, 0.1 µCi [γ-³²P]ATP, 10 µl), and the mixture was reacted at room temperature for 30 min. 50 µl of ice cooled 20% trichloroacetic acid (Wako Pure Chemical Industries, Ltd.) was added to the reaction solution to quench the reaction. The reaction solution was stood at 4°C for 30 min, and acid insoluble fraction was transferred to GF/C filter plate (Millipore) using a cell harvester (Packard). The plate was dried at 45°C for 60 min, MicroScinti0 (Packard, 40 µL) was added thereto, and radioactivity was measured with TopCount (Packard). The concentration of the test compound necessary for inhibiting ³²P uptake by the acid insoluble fraction by 50% (IC₅₀ value) was calculated by PRISM 3.0 (Graphpad software). The results are shown in Table 26.

**Table 26**

| test compound (Example No.) | IC₅₀ value (mM) |
|---|---|
| 16 | 35 |
| 17 | 130 |
| 25 | 19 |
| 42 | 13 |
| 50 | 15 |
| 51 | 14 |
| 86 | 160 |
| 103 | 32 |
| 107 | 41 |
| 140 | 160 |
| 153 | 32 |
| 174 | 34 |
| 194 | 30 |

**Formulation Example 1 (preparation of capsule)**

| | | |
|---|---|---|
| 1) | compound of Example 1 | 30 mg |
| 2) | finely divided powder cellulose | 10 mg |
| 3) | lactose | 19 mg |
| 4) | magnesium stearate | 1 mg |
| | | Total 60 mg |

| | | |
|---|---|---|
| 1), 2), 3) and 4) are mixed and filled in a gelatin capsule. | | |

**Formulation Example 2 (production of tablet)**

| | | |
|---|---|---|
| 1) | compound of Example 1 | 30 g |
| 2) | lactose | 50 g |
| 3) | cornstarch | 15 g |
| 4) | calcium carboxymethylcellulose | 44 g |
| 5) | magnesium stearate | 1 g |
| | 1000 tablets Total | 140 g |

| | | |
|---|---|---|
| The total amount of 1), 2) and 3)and 30 g of 4) are kneaded with water, vacuum dried, and sieved. The sized powder is mixed with 14 g of 4) and 1 g of 5) and punched by a tableting machine. As a result, 1000 tablets containing 30 mg of the compound of Example 1 per tablet are obtained. | | |

### Industrial Applicability

The ASK1 inhibitor of the present invention has superior activity and is useful as a pharmaceutical agent such as an agent for the prophylaxis or treatment of diabetes, inflammatory disease etc., and the like.

This application is based on a patent application No. 2006-213960 filed in Japan, the contents of which are incorporated in full herein by this reference.
## Claims

1. A compound represented by the formula (I): 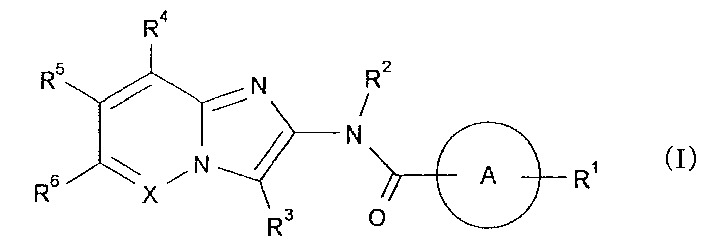 wherein
ring A is a ring which is optionally further substituted;
R¹ is a hydrogen atom or a substituent;
R² is a hydrogen atom or a substituent;
R³ is a hydrogen atom or a substituent;
R⁴ is a hydrogen atom or a substituent;
R⁵ is a hydrogen atom or a substituent;
R⁶ is a hydrogen atom or a substituent;
X is =N- or =C(Z)- (Z is a hydrogen atom or a substituent); when X is =C(Z)-, Z and R⁶ are optionally bonded to each other to form, together with the carbon atom bonded thereto, an optionally substituted ring,
provided that when X is =CH-, then R⁶ is not optionally substituted 2-piperidinyl, excluding N-imidazo[1,2-a]pyridin-2-yl-4-methyl-benzamide, N-imidazo[1,2-a]pyridin-2-yl-benzamide and N-(7-methylimidazo[1,2-a]pyridin-2-yl)-benzamide, or a salt thereof.

2. The compound of claim 1, wherein X is =C(Z)- (Z is a hydrogen atom or a substituent).

3. The compound of claim 1, wherein X is =N-, and ring A is an aromatic hydrocarbon optionally further having substituent(s) or a 5-membered heterocycle optionally having substituent(s).

4. The compound of claim 2 or 3, wherein R¹ is
(1) a hydrogen atom,
(2) C₁₋₆ alkyl optionally substituted by 1 to 3 substituents selected from
(a) an aromatic heterocyclic group,
(b) amino optionally mono- or di-substituted by substituent(s) selected from
(i) C₁₋₆ alkyl,
(ii) C₁₋₆ alkyl-carbonyl optionally substituted by 1 to 3 substituents selected from hydroxy and an aromatic heterocyclic group,
(iii) C₆₋₁₄ aryl-carbonyl,
(iv) C₁₋₆ alkyl-sulfonyl, and
(v) aromatic heterocyclyl-carbonyl,
(c) cyano,
(d) carboxyl,
(e) C₁₋₆ alkoxy-carbonyl,
(f) N-hydroxycarbamoyl,
(g) carbamoyl optionally mono- or di-substituted by C₁₋₆ alkyl optionally substituted by 1 to 3 substituents selected from (i) cyano, (ii) hydroxy, (iii) an aromatic heterocyclic group, and (iv) a non-aromatic heterocyclic group optionally having 1 to 3 C₁₋₆ alkyl,
(h) a halogen atom,
(i) di-C₁₋₆ alkylphosphoryl,
(j) hydroxy, and
(k) a non-aromatic heterocyclic group;
(3) C₃₋₁₀ cycloalkyl optionally substituted by 1 to 3 substituents selected from
(a) C₁₋₆ alkoxy-carbonyl, and
(b) carboxyl;
(4) an aromatic heterocyclic group;
(5) hydroxy optionally substituted by C₁₋₆ alkyl;
(6) amino optionally mono- or di-substituted by substituent(s) selected from
(a) C₁₋₆ alkyl, and
(b) C₁₋₆ alkyl-sulfonyl;
(7) cyano;
(8) carboxyl;
(9) C₁₋₆ alkoxy-carbonyl;
(10) C₁₋₆ alkyl-sulfonyl;
(11) sulfamoyl optionally mono- or di-substituted by substituent(s) selected from
(a) C₁₋₆ alkyl, and
(b) C₁₋₆ alkyl-carbonyl; or
(12) a halogen atom.

5. The compound of claim 2 or 3, wherein R² is a hydrogen atom.

6. The compound of claim 2 or 3, wherein R³ is a hydrogen atom, C₁₋₆ alkyl or a halogen atom.

7. The compound of claim 2 or 3, wherein R⁴ is a hydrogen atom.

8. The compound of claim 2 or 3, wherein R⁵ is a hydrogen atom.

9. The compound of claim 2 or 3, wherein R⁶ is
(1) a hydrogen atom;
(2) C₁₋₆ alkyl optionally substituted by 1 to 3 substituents selected from
(a) hydroxy,
(b) amino optionally mono- or di-substituted by C₁₋₆ alkyl, and
(c) a halogen atom;
(3) C₆₋₁₄ aryl optionally substituted by 1 to 3 substituents selected from
(a) C₁₋₆ alkyl optionally substituted by 1 to 3 halogen atoms,
(b) C₁₋₆ alkoxy optionally substituted by 1 to 3 halogen atoms,
(c) C₁₋₆ alkoxy-carbonyl,
(d) amino optionally mono- or di-substituted by substituent(s) selected from
(i) C₁₋₆ alkyl, and
(ii) C₁₋₆ alkyl-carbonyl,
(e) C₁₋₆ alkyl-sulfonyl,
(f) cyano, and
(g) C₁₋₃ alkylenedioxy;
(4) an aromatic heterocyclic group optionally substituted by 1 to 3 substituents selected from
(a) C₁₋₆ alkyl optionally substituted by 1 to 3 substituents selected from
(i) hydroxy optionally substituted by a non-aromatic heterocyclic group, and
(ii) cyano,
(b) C₆₋₁₄ aryl,
(c) an aromatic heterocyclic group,
(d) C₁₋₆ alkoxy, and
(e) a halogen atom;
(5) a non-aromatic heterocyclic group optionally substituted by 1 to 3 substituents selected from
(a) C₁₋₆ alkyl optionally substituted by 1 to 3 substituents selected from
(i) C₆₋₁₄ aryl,
(ii) an aromatic heterocyclic group, and
(iii) hydroxy optionally substituted by C₁₋₆ alkyl,
(b) C₆₋₁₄ aryl optionally substituted by 1 to 3 substituents selected from C₁₋₆ alkoxy and a halogen atom,
(c) an aromatic heterocyclic group,
(d) hydroxy,
(e) amino optionally mono- or di-substituted by C₁₋₆ alkyl-carbonyl,
(f) carboxyl,
(g) carbamoyl optionally mono- or di-substituted by C₇₋₁₃ aralkyl,
(h) C₁₋₆ alkyl-sulfonyl, and
(i) C₆₋₁₄ aryl-sulfonyl;
(6) hydroxy optionally substituted by a substituent selected from
(a) C₆₋₁₄ aryl optionally substituted by 1 to 3 substituents selected from
(i) C₁₋₆ alkyl optionally substituted by 1 to 3 substituents selected from
(A) a halogen atom,
(B) amino optionally mono- or di-substituted by C₁₋₆ alkyl, and
(C) an aromatic heterocyclic group optionally substituted by 1 to 3 C₁₋₆ alkyl optionally substituted by 1 to 3 hydroxy,
(ii) an aromatic heterocyclic group optionally substituted by 1 to 3 C₁₋₆ alkyl,
(iii) a non-aromatic heterocyclic group,
(iv) C₁₋₆ alkoxy optionally substituted by 1 to 3 substituents selected from (A) a halogen atom and (B) carboxyl,
(v) amino optionally mono- or di-substituted by substituent(s) selected from
(A) C₁₋₆ alkyl,
(B) C₁₋₆ alkyl-carbonyl,
(C) C₃₋₁₀ cycloalkyl-carbonyl,
(D) C₆₋₁₄ aryl-carbonyl optionally substituted by 1 to 3 substituents selected from (1') C₁₋₆ alkyl optionally substituted by 1 to 3 halogen atoms, (2') a halogen atom, and, (3') C₃₋₁₀ cycloalkyl optionally substituted by 1 to 3 cyano, and
(E) aromatic heterocyclyl-carbonyl optionally substituted by 1 to 3 C₁₋₆ alkyl,
(vi) carboxyl, and
(vii) non-aromatic heterocyclyl-carbonyl optionally substituted by 1 to 3 substituents selected from C₁₋₆ alkyl and oxo,
(b) an aromatic heterocyclic group optionally substituted by 1 to 3 substituents selected from
(i) C₁₋₆ alkyl,
(ii) C₁₋₆ alkyl-carbonyl, and
(iii) carboxyl, and
(c) a non-aromatic heterocyclic group optionally substituted by 1 to 3 substituents selected from C₁₋₆ alkyl and oxo;
(7) mercapto optionally substituted by
(a) an aromatic heterocyclic group optionally substituted by 1 to 3 substituents selected from
(i) C₁₋₆ alkyl optionally substituted by 1 to 3 halogen atoms,
(ii) C₆₋₁₄ aryl, and
(iii) an aromatic heterocyclic group;
(8) amino optionally mono- or di-substituted by substituent(s) selected from
(a) C₁₋₆ alkyl optionally substituted by 1 to 3 substituents selected from
(i) an aromatic heterocyclic group,
(ii) hydroxy,
(iii) C₁₋₆ alkoxy, and
(iv) amino optionally mono- or di-substituted by C₁₋₆ alkyl,
(b) C₃₋₁₀ cycloalkyl,
(c) C₇₋₁₃ aralkyl, and
(d) a non-aromatic heterocyclic group optionally substituted by 1 to 3 C₁₋₆ alkyl;
(9) cyano;
(10) C₁₋₆ alkyl-carbonyl optionally substituted by 1 to 3 hydroxy;
(11) a halogen atom; or
(12) hydroxy substituted by C₆₋₁₄ aryl fused with C₃₋₁₀ cycloalkane optionally substituted by oxo.

10. The compound of claim 2, wherein Z is a hydrogen atom, or C₁₋₆ alkyl optionally substituted by 1 to 3 hydroxy, or Z and R⁶ are bonded to each other to form, together with the carbon atom bonded thereto, an aromatic hydrocarbon or a heterocycle, each optionally substituted by 1 to 3 C₁₋₆ alkyl.

11. The following compound or a salt thereof:
4-tert-butyl-N-(1,2-dihydrofuro[2,3-e]imidazo[1,2-a]pyridin-7-yl)benzamide,
4-tert-butyl-N-[6-(1H-imidazol-1-yl)imidazo[1,2-a]pyridin-2-yl]benzamide,
6-tert-butyl-N-[6-(1H-imidazol-1-yl)imidazo[1,2-a]pyridin-2-yl]nicotinamide,
4-(2-hydroxy-1,1-dimethylethyl)-N-[6-(1H-imidazol-1-yl)imidazo[1,2-a]pyridin-2-yl]benzamide,
4-tert-butyl-N-[6-(1H-pyrazol-4-yl)imidazo[1,2-a]pyridin-2-yl]benzamide,
4-(1-cyano-1-methylethyl)-N-[6-(1H-imidazol-1-yl)imidazo[1,2-a]pyridin-2-yl]benzamide,
4-tert-butyl-N-[6-(1H-imidazol-1-yl)imidazo[1,2-b]pyridazin-2-yl]benzamide.

12. A prodrug of the compound of claim 1.

13. A pharmaceutical agent comprising the compound of claim 1, or a prodrug thereof.

14. The pharmaceutical agent of claim 13, which is an ASK1 inhibitor.

15. The pharmaceutical agent of claim 13, which is an agent for the prophylaxis or treatment of diabetes.

16. The pharmaceutical agent of claim 13, which is an agent for the prophylaxis or treatment of an inflammatory disease.

17. A method of preventing or treating diabetes and/or an inflammatory disease, comprising administering an effective amount of the compound of claim 1 or a prodrug thereof to a mammal.

18. Use of the compound of claim 1 or a prodrug thereof for the production of an agent for the prophylaxis or treatment of diabetes and/or an inflammatory disease.
